# EUROPEAN PATENT APPLICATION

(11) **EP 4 477 748 A2**
(43) Date of publication of application: **18.12.2024**
(21) Application number: 24209277.3
(22) Date of filing: 27.12.2016
(51) Int. Cl.: C12N 5/0783

(54) **METHODS OF MAKING CHIMERIC ANTIGEN RECEPTOR-EXPRESSING CELLS**

(30) Priority: 28.12.2015 US 201562271695 P; 07.12.2016 US 201662431204 P
(62) Divisional of application: 16829368.6
(71) Applicant: Novartis AG, 4056 Basel (CH); The Trustees of The University of Pennsylvania, Philadelphia, PA 19104 (US)
(72) Inventor: FACHIN, Fabio, Brookline, 02446 (US); CAO, Lan, Morris Plains, 07950 (US); GREENE, Michael R., Groton, 01450 (US); GOLOVINA, Tatiana, Havertown, 19083 (US)
(74) Representative: Mathys & Squire

(57) **Abstract**

The invention provides methods of making immune effector cells (e.g., T cells, NK cells) that can be engineered to express a chimeric antigen receptor (CAR), and compositions and reaction mixtures comprising the same.

## Description

### RELATED APPLICATIONS

This application claims priority to U.S. Serial No. 62/271695 filed December 28, 2015, and U.S. Serial No. 62/431204 filed December 7, 2016, the contents of which are incorporated herein by reference in their entireties.

### SEQUENCE LISTING

The instant application contains a Sequence Listing which has been submitted electronically in ASCII format and is hereby incorporated by reference in its entirety. Said ASCII copy, created on December 27, 2016, is named N2067-7100WO_SL.txt and is 257,808 bytes in size.

### FIELD OF THE INVENTION

The present invention relates generally to methods of making immune effector cells (e.g., T cells, NK cells) engineered to express a Chimeric Antigen Receptor (CAR), and compositions comprising the same.

### BACKGROUND OF THE INVENTION

Adoptive cell transfer (ACT) therapy with autologous T cells, especially with T cells transduced with Chimeric Antigen Receptors (CARs), has shown promise in several hematologic cancer trials.

The manufacture of autologous gene-modified T cells is currently a complex process that starts with the patient's material (e.g., obtained from leukapheresis) from which the engineered therapeutic T cells that express a CAR are derived. Patient leukapheresis material can have a high level of cell component variability. This starting material can vary greatly in cellular composition from patient to patient and within one disease state. Cell impurities can include granulocytes, monocytes, red blood cells, circulating blast cells, and platelets. Autologous cell therapy product manufacturing processes must also contend with patients' different treatment histories, state of disease, *etc.*, which will further impact the cellular content of the starting material (Burger *et al.* 2014, Kaiser *et al.* 2015, Ramos *et al.* 2009). Furthermore, such impurities from the starting material can negatively impact the manufacturing process, ultimate product quality, and therapeutic efficacy of the product.

Thus, there exists a need for methods and processes to provide a more consistent production of the CAR-expressing cell therapy product, thereby streamlining the manufacturing process, improving product quality, and maximizing the therapeutic efficacy of the product.

### SUMMARY OF THE INVENTION

The present disclosure pertains to methods of making immune effector cells (e.g., T cells, NK cells) that can be engineered to express a CAR, and compositions comprising the same.

Accordingly, in one aspect, the present disclosure features a method of making or enriching a population of immune effector cells (e.g., T cells) that can be engineered to express a chimeric antigen receptor (CAR), wherein the method includes performing elutriation. The method includes providing a frozen input sample comprising immune effector cells, thawing the frozen input sample, to produce a thawed sample, and performing elutriation on the thawed sample and collecting immune effector cells, thereby producing an output sample comprising immune effector cells that are suitable for expression of a CAR.

In one embodiment, the frozen input sample is a plasma apheresis sample.

In one embodiment, the method further comprises one, two, three or all of:
i) depleting CD19+ cells under flow conditions;
ii) performing density centrifugation using a medium comprising iodixanol, e.g., 60*%* iodixanol in water (e.g., Optiprep medium), and/or having a density greater than Ficoll (e.g., greater than 1.077 g/ml, e.g., about 1.32 g/ml));
iii) performing a wash step (e.g., on the thawed sample) with a buffer comprising dextrose and/or sodium chloride, e.g., D5 1/2 NS medium (5*%* dextrose and 0.45*%* sodium chloride), e.g., wherein the wash step is performed using a cell processing device, e.g., a cell washing device or the device used for density gradient centrifugation, e.g., a CS5 (CellSaver5+) instrument; and
iv) performing a positive selection of CD3/CD28+ cells under flow conditions.

In one embodiment, the method further comprises a step of adjusting the viscosity of the thawed sample, e.g., by adding an isotonic solution, e.g., PBS, to the thawed sample.

In one embodiment, the elutriation is performed using a flow rate of from about 30-82 mL/min or 50-80 mL/min and/or the collection volume is about 250-1250 mL or 300-1000 mL for each fraction. In one embodiment, the elutriation is performed using a flow rate of about 30, 40, 50, 60, 70, 72, or 82 mL/min, e.g., about 70 or 72 mL/min. In one embodiment, the elutriation is performed using a flow rate of about 30-40, 40-50, 50-60, 60-70, 70-72, 70-82, 72-82 mL/min. In one embodiment, the elutriation is performed using a collection volume of about 250, 400, 500, 900, or 975 mL, e.g., about 400 or 975 mL. In one embodiment, the elutriation is performed using a collection volume of about 250-400, 400-500, 500-900, 900-1000, or 1000-1259 mL. In one embodiment, the elutriation is performed at about 2400 rpm. In one embodiment, the elutriation is performed at about 2000-2800, 2200-2600, or 2300-2500 rpm.

In one embodiment, the input sample comprises at least 10*%*, 15*%*, 20*%*, 21*%*, 22*%*, 23*%*, 24*%*, 25*%*, 26*%*, 27*%*, 28*%*, 29*%*, 30*%*, 31*%*, 35*%*, or 40*%* monocytes. In one embodiment, the input sample comprises less than 60*%*, 55*%*, 50*%*, 45*%*, 40*%*, 35*%*, 30*%*, 25%, or 20% T cells. In one embodiment, the input sample comprises at least 1*%*, 2*%*, 5*%*, 10*%*, 15*%*, 20*%*, 30*%*, 35*%*, 40*%*, 45*%*, 50*%*, 55*%*, 60*%*, 65*%*, 70*%*, 75*%*, 80*%*, 85*%*, 90*%*, or 95*%* B cells.

In one embodiment, output sample comprises less than 20*%*, 15*%*, 10*%*, 9*%*, 8*%*, 7*%*, 6*%*, 5*%*, 4*%*, 2*%*, 2*%*, 1*%*, 0.5*%*,0.2*%*, or 0.1*%* monocytes. In one embodiment, the output sample comprises at least 50*%*, 55*%*, 60*%*, 65*%*, 70*%*, 75*%*, 80*%*, 85*%*, 90*%*, or 95*%* T cells. In one embodiment, the output sample comprises less than 20*%*, 15*%*, 10*%*, 9*%*, 8*%*, 7*%*, 6*%*, 5*%*, 4*%*, 2*%*, 2*%*, 1*%*, 0.5*%*,0.2*%*, or 0.1*%* B cells. In one embodiment, the output sample comprises at least 50*%*, 55*%*, 60*%*, 65*%*, 70*%*, 75*%*, 80*%*, 85*%*, 90*%*, 95*%*, 96*%*, 97*%*, 98*%*, 99*%*, 99.5*%*, 99.7*%*, or 99.9*%* CD4+CD25+ cells. In one embodiment, the output sample comprises at least 50*%*, 55*%*, 60*%*, 65*%*, 70*%*, 75*%*, 80*%*, 85*%*, 90*%*, 95*%*, 96*%*, 97*%*, 98*%*, 99*%*, 99.5*%*, 99.7*%*, or 99.9*%* CD8+CD25+ cells.

In one embodiment, the method results in a T cell yield recovery of at least 50*%*, 55*%*, 60*%*, 65*%*, 70*%*, 75*%*, 80*%*, 85*%*, 90*%*, or 95*%* T cells.

In one embodiment, the output sample is contacted with a nucleic acid encoding a CAR. In one embodiment, after contacting the output sample with a nucleic acid encoding a CAR, the output sample comprises at least 10*%*, 15*%*, 20*%*, 25*%*, 30*%*, 35*%*, 40*%*, 45*%*, or 50*%* CAR+ cells. In such embodiments, the output sample comprises at least 40*%*, 45*%*, 50*%*, 55*%*, 60*%*, 65*%*, 70*%*, 75*%*, 80*%*, 85*%*, 90*%*, or 95*%* CAR+CD4+ central memory cells. In such embodiments, the output sample comprises at least 40*%*, 45*%*, 50*%*, 55*%*, 60*%*, 65*%*, 70*%*, 75*%*, 80*%*, 85*%*, 90*%*, or 95*%* CAR+CD8+ central memory cells.

In one embodiment, after contacting the output sample with a nucleic acid encoding a CAR, the output sample produces less than 1, 0.9, 0.8, 0.7, 0.6, 0.5, 0.4, 0.3, 0.2, or 0.1 pg of IFN-gamma (IFN-γ) per CAR-expressing cell, e.g., transduced cell. IFN-gamma (IFN-γ) release assays are described herein, e.g., in the Examples. In one embodiment, after contacting the output sample with a nucleic acid encoding a CAR, the output sample comprises a cytotoxicity level (e.g., an EC50rec) of at least 5, 6, 7, 8, 9, 10, 11, 12, 13, 14, 15, 16, 17, 18, 19, 20, 25, or 30. Cyotoxicity assays are described herein, e.g., in the Examples.

In another aspect, the present disclosure features a method of making or enriching a population of immune effector cells (e.g., T cells) that can be engineered to express a CAR, wherein the method includes performing density gradient centrifugation (also referred to herein as density centrifugation). The method includes providing an input sample comprising immune effector cells, and performing a density centrifugation step using a medium comprising iodixanol, e.g., 60*%* iodixanol in water, e.g., Optiprep medium and/or having a density greater than Ficoll (e.g., greater than 1.077 g/ml, e.g., about 1.32 g/ml), thereby producing an output sample comprising immune effector cells that are suitable for expression of a CAR.

In one embodiment, the density gradient centrifugation method described herein further comprises performing one, two, three, or all of:
i) depleting CD19+ cells under flow conditions;
ii) elutriation on the input sample, wherein the input sample is optionally a thawed input sample;
iii) performing a wash step (e.g., before density centrifugation) with a buffer comprising dextrose and/or sodium chloride, e.g., D5 1/2 NS medium (5*%* dextrose and 0.45*%* sodium chloride), e.g., wherein the wash step is performed using a CS5 (CellSaver5+) instrument; and positive selection of CD3/CD28+ cells under flow conditions.

In one embodiment, the density gradient centrifugation method described herein does not comprise one or more of: using a solution comprising glycol, e.g., a Ficoll solution; or performing a wash step in a buffer comprising dextrose and/or sodium chloride, e.g., D5 1/2 NS medium, e.g., wherein the wash step is performed using a CS5 instrument; or performing a positive selection step.

In one embodiment, the density centrifugation is performed using a cell separation device, e.g., a Sepax2 device.

In one embodiment, the input sample comprises less than 60*%*, 55*%*, 50*%*, 45*%*, 40*%*, 35*%*, 30*%*, 25*%*, 20*%*, 19*%*, 18*%*, 17*%*, 16*%*, or 15*%* T cells. In one embodiment, the input sample comprises at least 10*%*, 15*%*, 20*%*, 21*%*, 22*%*, 23*%*, 24*%*, 25*%*, 26*%*, 27*%*, 28*%*, 29*%*, 30*%*, 31*%*, 35*%*, 40*%*, 45*%*, 50*%*, 55*%*, 60*%*, 65*%*, or 70*%* monocytes. In one embodiment, the input sample comprises at least 1*%*, 2*%*, 5*%*, 10*%*, 15*%*, 20*%*, 30*%*, 35*%*, 40*%*, 45*%*, 50*%*, 55*%*, 60*%*, 65*%*, 70*%*, 75*%*, or 80*%* B cells.

In one embodiment, the output sample comprises at least 25*%*, 30*%*, 35*%*, 40*%*, 45*%*, 50*%*, 55*%*, 60*%*, 65*%*, 70*%*, 75*%*, 80*%*, 85*%*, 90*%*, or 95*%* T cells. In one embodiment, the output sample comprises less than 50*%*, 45*%*, 40*%*, 35*%*, 30*%*, 25*%*, 20*%*, 15*%*, 10*%*, 9*%*, 8*%*, 7*%*, 6*%*, 5*%*, 4*%*, 2*%*, 2*%*, 1*%*, 0.5*%*, 0.2*%*, or 0.1*%* monocytes. In one embodiment, the output sample comprises less than 20*%*, 15*%*, 10*%*, 9*%*, 8*%*, 7*%*, 6*%*, 5*%*, 4*%*, 2*%*, 2*%*, 1*%*, 0.5*%*, 0.2*%*, 0.1*%*, 0.05*%*, or 0.01*%* B cells.

In one embodiment, the density gradient centrifugation method described herein results in a T cell yield recovery of at least 5*%*, 10*%*, 15*%*, 20*%*, 25*%*, 30*%*, 35*%*, 40*%*, 45*%*, 50*%*, 55*%*, 60*%*, 65*%*, 70*%*, 75*%*, 80*%*, 85*%*, 90*%*, or 95*%* T cells.

In another aspect, the present disclosure features a method of making a population of immune effector cells (e.g., T cells) that can be engineered to express a CAR, wherein the method includes a negative selection step to remove cancer-associated antigen-expressing cells, e.g., CD19-expressing (CD19+) cells. The method includes providing an input sample comprising immune effector cells, and removing CD19+ cells from the input sample under flow conditions, e.g., using a flow-through device, e.g., a cell processing system described herein, thereby producing an output sample comprising immune effector cells that are suitable for expression of a CAR. In one embodiment, the CD19+ cells comprise B cells. In one embodiment, the CD19+ cells comprise lymphoblasts.

In one embodiment, the negative selection method described herein further comprises performing one, two, three or all of:
i) elutriation on the input sample, wherein the input sample is optionally a thawed input sample;
ii) a density centrifugation step using a medium comprising iodixanol, e.g., 60*%* iodixanol in water (e.g., Optiprep medium), and/or having a density greater than Ficoll (e.g., greater than 1.077 g/ml, e.g., about 1.32 g/ml);
iii) performing a wash step (e.g., before removing CD19+ cells and/or after the input sample is thawed) with a buffer comprising dextrose and/or sodium chloride, e.g., D5 1/2 NS medium (5*%* dextrose and 0.45*%* sodium chloride), e.g., wherein the wash step is performed using a CS5 (CellSaver5+) instrument; and
iv) positive selection of CD3/CD28+ cells under flow conditions.

In one embodiment, the negative selection method described herein does not comprise performing elutriation or density centrifugation.

In one embodiment, the CD19+ cells are removed from the input sample by magnetic separation. In one embodiment, the magnetic separation comprising contacting the cells with a separation reagent. In one embodiment, the separation reagent comprises a magnetic or paramagnetic member and a CD19-binding member. In one embodiment, the magnetic separation comprises flow cytometry or FACS. In one embodiment, the CD19+ cells are removed by FACS. In one embodiment, the magnetic separation comprises use of a magnetic cell separation device, e.g., CliniMACs device. In one embodiment, the CD19+ cells are removed by a CliniMACs device. In one embodiment, the CD19+ cells are removed by a flow-through device as described herein, e.g., a cell processing system as described herein.

In one embodiment, the input sample comprises at least 1*%*, 2*%*, 5*%*, 10*%*, 15*%*, 20*%*, 30*%*, 35*%*, 40*%*, 45*%*, 50*%*, 55*%*, 60*%*, 65*%*, 70*%*, 75*%*, 80*%*, 85*%*, 90*%*, 95*%*, or 99*%* CD19+ cells. In one embodiment, the output sample comprises less than 20*%*, 15*%*, 10*%*, 9*%*, 8*%*, 7*%*, 6*%*, 5*%*, 4*%*, 2*%*, 2*%*, 1*%*, 0.5*%*,0.2*%*, 0.1*%*, 0.05*%*, or 0.01*%* CD19+ cells. In one embodiment, the output sample comprises less than 50*%*, 45*%*, 40*%*, 40*%*, 35*%*, 30*%*, 25*%*, 20*%*, 15*%*, 10*%*, 5*%*, 4*%*, 2*%*, 2*%*, or 1*%* the percentage of CD19+ cells compared to the input sample.

In one embodiment, the input sample comprises at least 10*%*, 15*%*, 20*%*, 21*%*, 22*%*, 23*%*, 24*%*, 25*%*, 26*%*, 27*%*, 28*%*, 29*%*, 30*%*, 31*%*, 35*%*, or 40*%* monocytes. In one embodiment, the input sample comprises less than 60*%*, 55*%*, 50*%*, 45*%*, 40*%*, 35*%*, 30*%*, 25*%*, or 20*%* T cells. In one embodiment, the input sample (e.g., the input sample post-wash) comprises at least 1*%*, 2*%*, 5*%*, 10*%*, 15*%*, 20*%*, 30*%*, 35*%*, 40*%*, 45*%*, 50*%,* 55*%*, 60*%*, 65*%*, 70*%*, 75*%*, or 80*%* B cells.

In one embodiment, the output sample comprises less than 20*%*, 15*%*, 10*%*, 9*%*, 8*%*, 7*%*, 6*%*, 5*%*, 4*%*, 2*%*, 2*%*, 1*%*, 0.5*%*,0.2*%*, or 0.1*%* monocytes. In one embodiment, the output sample comprises at least 50*%*, 55*%*, 60*%*, 65*%*, 70*%*, 75*%*, 80*%*, 85*%*, 90*%*, or 95*%* T cells. In one embodiment, the output sample comprises less than 20*%*, 15*%*, 10*%*, 9*%*, 8*%*, 7*%*, 6*%*, 5*%*, 4*%*, 2*%*, 2*%*, 1*%*, 0.5*%*,0 .2*%*, 0.1*%*, 0.05*%*, or 0.01*%* B cells.

In one embodiment, the negative selection method described herein results in a T cell yield recovery of at least 50*%*, 55*%*, 60*%*, 65*%*, 70*%*, 75*%*, 80*%*, 85*%*, 90*%*, or 95*%* T cells.

In another aspect, the present disclosure features a method of making a population of immune effector cells (e.g., T cells) that can be engineered to express a CAR, wherein the method comprises positive selection. The method includes providing an input sample comprising immune effector cells, and positively selecting for CD3+/CD28+ cells from the input sample under flow conditions, thereby producing an output sample comprising immune effector cells that are suitable for expression of a CAR, e.g., wherein the positive selection is performed under flow conditions.

In one embodiment, the positive selection method described herein further comprises performing one, two, three, or all of:
i) depleting CD19+ cells, e.g., under flow conditions; elutriation on the input sample, wherein the input sample is optionally a thawed input sample;
ii) performing density centrifugation using a medium comprising iodixanol, e.g., 60*%* iodixanol in water (e.g., Optiprep medium), and/or having a density greater than Ficoll (e.g., greater than 1.077 g/ml, e.g., about 1.32 g/ml); and
iii) performing a wash step (e.g., before removing CD19+ cells and/or after the input sample is thawed) with a buffer comprising dextrose and/or sodium chloride, e.g., D5 1/2 NS medium (5*%* dextrose and 0.45*%* sodium chloride), e.g., wherein the wash step is performed using a CS5 (CellSaver5+) instrument.

In one embodiment, the positive selection method described herein further comprises performing elutriation on the input sample (e.g., wherein the input sample is a thawed input sample). Optionally, the elutriation is performed together with one or more of (e.g., 1, 2, or all of) depleting CD19+ cells (e.g., as described in (i) above), performing density centrifugation (e.g., as described in (ii) above), and performing a wash step (e.g., as described in (iii) above).

In one embodiment, the positive selection method described herein further comprises performing elutriation, a wash step (optionally), and density centrifugation (e.g., using Ficoll or OptiPrep medium) prior to performing positive selection. In one embodiment, the positive selection method described herein further comprises performing a wash step (optionally) and density centrifugation (e.g., using Ficoll or OptiPrep medium) prior to performing positive selection. In one embodiment, the positive selection method described herein does not comprise performing elutriation. In one embodiment, the positive selection method described herein further comprises performing a wash with a buffer comprising dextrose and/or sodium chloride, e.g., D5 1/2 NS buffer, e.g., using a CS5+ instrument.

In one embodiment, the positive selection comprises contacting the input sample with a separation reagent, which separation reagent comprises a magnetic or paramagnetic member and a CD3 and/or CD28-binding member. In one embodiment, the positive selection for CD3+/CD28+ cells comprises incubating the input sample with a separation reagent for about 10 to 90 minutes, about 10 to 60 minutes, about 10 to 45 minutes, about 12 to 90 minutes, about 12 to 60 minutes, about 12 to 45 minutes, about 15 to 90 minutes, about 15 to 60 minutes, about 15 to 45 minutes, e.g., about 30 minutes or about 20 minutes. In one embodiment, the separation reagent comprises a bead that is coupled (e.g., covalently or non-covalently coupled) to an anti-CD3 and/or anti-CD28 antibody. In one embodiment, the positive selection uses an about 3:1 ratio of magnetic separation members (e.g., beads) to T cells.

In one embodiment, the positive selection comprises flowing a fluid that comprises the immune effector cells and magnetic separation members within an enclosed system, e.g., a chamber or a bag, where magnetic separation occurs. In one embodiment, the flowing is performed at a speed such that magnetic separation of the members (optionally bound to immune effector cells) occurs. In one embodiment, the positive selection for CD3+/CD28+ cells comprises a separation or dwell time of less than about 6, 5, 6, 3, 2, or 1 minute, or less than about 50, 40, 30, 20, 10, 5, 4, 3, 2, or 1 second.

In one embodiment, the positive selection is performed with a magnetic device, e.g., Dynamag CTS, a flow-through device comprising magnetic elements as described herein, or other arrangement of magnetic elements.

In one embodiment, the positive selection is performed using a device that includes at least one cell suspension module; at least one flow-through magnetic separation/debeading module; at least one non-magnetic output module; at least one magnetic output module; optionally, at least one magnetic component, external to the magnetic separation/debeading module, that creates magnetic forces and/or gradients; and optionally, at least one buffer module. In one embodiment, the device further comprises at least one magnetic component, external to the magnetic separation/debeading module, that creates magnetic forces and/or gradients. In one embodiment, the device further comprises at least one buffer module. In one embodiment, the magnetic separation/debeading module comprises a chamber defined by walls and having an x-direction, a y-direction, and a z-direction; an inlet and an outlet arranged on opposite ends of the chamber, e.g., in the x-direction, in the y-direction, or in the z-direction; at least two magnets adjacent or proximate to a wall of the chamber and arranged to establish a zero gradient line within the chamber between the inlet and the outlet. In one embodiment, the immune effector cells flow through the chamber, wherein each point in the chamber is within 2 cm of the magnets.

In one embodiment, the positive selection method comprises (e.g., between steps a) and b)), contacting the immune effector cells with a solution comprising dextrose and/or sodium chloride, e.g., D5 1/2 NS medium (5*%* dextrose and 0.45*%* sodium chloride), optionally, wherein the solution is at ambient temperature, e.g., at about 20-25°C. In one embodiment, the immune effector cells are present in a flexible container, e.g., a bag, e.g., during steps a) and b). In one embodiment, the method comprises (e.g., between steps a) and b), e.g., after contacting the immune effector cells with the saline solution), placing the bag on a thermal insulating material, e.g., a plurality of layers comprising paper, e.g., paper towels or wipes. In an embodiment, the method comprises (e.g., after step b)), incubating the cells at about 37°C for about 10 minutes. In an embodiment, the method comprises (e.g., after step b)), incubating the cells at about 36-38, 35-39, or 34-40°C, e.g., for about 10 minutes. In an embodiment, the incubation step lasts about 8-12, 5-15, or 5-20 minutes. In an embodiment, the incubation is performed in a Plasmatherm device.

In an embodiment of the positive selection method, the input sample comprising immune effector cells comprises at least 20*%* monocytes. In an embodiment, the input sample comprising immune effector cells comprises at least 10*%*, 15*%*, 20*%*, 25*%*, 30*%*, 35*%*, 40*%*, 50*%*, 60*%* monocytes.

In one embodiment, the positive selection method comprises one or more of (e.g., 2, 3, 4, or all of), e.g., in the order listed:
a) thawing a frozen input sample (e.g., a leukapheresis sample) comprising immune effector cells from a patient having a hematologic malignancy, optionally wherein the sample comprises >20*%* lymphoblasts;
b) washing the immune effector cells, e.g., at ambient temperature, e.g., 20-25°C, in a wash solution, e.g., X-VIVO15 medium (Lonza), called 'Modified Medium' (MM).
c) contacting the input sample with a separation reagent, which separation reagent comprises a magnetic or paramagnetic member and a CD3 and/or CD28-binding member;
d) rotating the input sample and separation reagent on a rotator, e.g., at 2-6 rpm, e.g., at 4 rpm, wherein the rotation lasts for, e.g., 10-30 minutes, e.g., 20 minutes; and
e) performing positive selection to enrich for cells that bind the separation reagent, e.g., for 30 sec to 2 minutes, e.g., for 1 minute.

In one embodiment, the positive selection method comprises one or more of (e.g., 2, 3, 4, 5, 6, or all of), e.g., in the order listed:
a) thawing a frozen input sample (e.g., a leukapheresis sample) comprising immune effector cells from a patient having a hematologic malignancy, optionally wherein the sample comprises >20*%* monocytes;
b) washing the immune effector cells, e.g., at ambient temperature, e.g., 20-25°C, in a wash solution, e.g., comprising about 5*%* dextrose and 0.45*%* sodium chloride, e.g., D5 1/2NS;
c) placing a flexible container comprising the cells on a thermal insulating material, e.g., a plurality of layers comprising paper, e.g., paper towels or wipes;
d) contacting the input sample with a separation reagent, which separation reagent comprises a magnetic or paramagnetic member and a CD3 and/or CD28-binding member;
e) incubating the input sample and separation reagent e.g., at 37°C, e.g., for 5-15 minutes, e.g., 10 minutes;
f) rotating the input sample and separation reagent on a rotator, e.g., at 2-6 rpm, e.g., at 4 rpm, wherein the rotation lasts for, e.g., 10-30 minutes, e.g., 20 minutes; and
g) performing positive selection to enrich for cells that bind the separation reagent, e.g., for 30 sec to 2 minutes, e.g., for 1 minute.

In an embodiment, the sample, e.g., the input sample, is from a patient having a hematologic malignancy, e.g., a hematologic malignancy described herein, e.g., ALL or DLBCL.

In one embodiment, the input sample comprises about 1×10⁵ nucleated cells/ml, 2×10⁵ nucleated cells/ml, 5×10⁵ nucleated cells/ml, 7×10⁵ nucleated cells/ml, 1×10⁶ nucleated cells/ml, 2×10⁶ nucleated cells/ml, 5×10⁶ nucleated cells/ml, 7×10⁶ nucleated cells/ml, 1×10⁷ nucleated cells/ml , 2×10⁷ nucleated cells/ml, 5×10⁷ nucleated cells/ml, 7×10⁷ nucleated cells/ml, 1×10 ⁷ nucleated cells/ml, 2×10⁸ nucleated cells/ml, 5×10⁸ nucleated cells/ml, and 7×10⁸ nucleated cells/ml. In one embodiment, the input sample comprises about 1-1.5 ×10⁷ T cells.

In one embodiment, the input sample comprises at least 5*%*, 10*%*, 15*%*, 20*%*, 25*%*, 30*%*, 35*%*, 40*%*, 45*%*, 50*%*, 55*%*, 60*%*, 65*%*, 70*%*, 75*%*, 80*%*, 85*%*, 90*%*, or 95*%* monocytes. In one embodiment, the input sample comprises at least 5*%*, 10*%*, 15*%*, 20*%*, 25*%*, 30*%*, 35*%*, 40*%*, 45*%*, 50*%*, 55*%*, 60*%*, 65*%*, 70*%*, 75*%*, 80*%*, 85*%*, 90*%*, or 95*%* tumor cells, e.g., lymphoblasts. In one embodiment, the input sample comprises less than 60*%*, 55*%*, 50*%*, 45*%*, 40*%*, 35*%*, 30*%*, 25*%*, or 20*%* immune effector cells, e.g., T cells. In one embodiment, the input sample comprises at least about 5*%*, 10*%*, 15*%*, 18*%*, 20*%*, 25*%*, 30*%*, 35*%*, 40*%*, 45*%*, 50*%*, 55*%*, 60*%*, 65*%*, 70*%*, 75*%*, 80*%*, 85*%*, 90*%*, or 95*%* B cells, e.g., CD45+CD19+ B cells. In one embodiment, the input sample comprises at least about 5*%*, 10*%*, 15*%*, 18*%*, 20*%*, 25*%*, 30*%*, 35*%*, 40*%*, 45*%*, 50*%*, 55*%*, 60*%*, 65*%*, 70*%*, 75*%*, 80*%*, 85*%*, 90*%*, or 95*%* B cells, e.g., CD45-CD19+ B cells.

In one embodiment, the output sample comprises less than 20*%*, 15*%*, 10*%*, 9*%*, 8*%*, 7*%*, 6*%*, 5*%*, 4*%*, 2*%*, 2*%*, 1*%*, 0.5*%*,0.2*%*, or 0.1*%* monocytes. In one embodiment, the output sample comprises less than 20*%*, 15*%*, 10*%*, 9*%*, 8*%*, 7*%*, 6*%*, 5*%*, 4*%*, 2*%*, 2*%*, 1*%*, 0.5*%*,0.2*%*, or 0.1*%* tumor cells. In one embodiment, the output sample comprises at least 50*%*, 55*%*, 60*%*, 65*%*, 70*%*, 75*%*, 80*%*, 85*%*, 90*%*, 95*%*, 96*%*, 97*%*, 98*%*, 99*%*, 99.5*%*, 99.8*%*, or 99.9*%* immune effector cells, e.g., T cells. In one embodiment, the output sample comprises at least 25*%*, 30*%*, 35*%*, 40*%*, 45*%*, 50*%*, 55*%*, 60*%*, 65*%*, 70*%*, 75*%*, 80*%*, 85*%*, 90*%*, 95*%*, 96*%*, 97*%*, 98*%*, or 99*%* T cells, e.g., CD3+CD45+ T cells. In one embodiment, the output sample comprises less than about 10*%*, 9*%*, 8*%*, 7*%*, 6*%*, 5*%*, 4*%*, 3*%*, 2*%*, or 1*%* B cells, e.g., CD45+CD19+ B cells. In one embodiment, the output sample comprises less than about 10*%*, 9*%*, 8*%*, 7*%*, 6*%*, 5*%*, 4*%*, 3*%*, 2*%*, or 1*%* B cells, e.g., CD45-CD19+ B cells. In an embodiment, the output sample comprises at least 5*%*, 6*%*, 7*%*, 8*%*, 9*%*, 10*%*, 11*%*, 12*%*, 13*%*, 14*%*, 15*%*, or 20*%* T cells.

In another aspect, the present disclosure features a method of making a population of immune effector cells (e.g., T cells) that can be engineered to express a chimeric antigen receptor (CAR), wherein the method includes:
i) providing an input sample, e.g., a frozen input sample or a fresh input sample comprising immune effector cells; optionally, wherein the input sample is a frozen input sample, thawing the frozen input sample, to produce a thawed sample;
ii) performing an enrichment step, wherein the enrichment step comprises: performing elutriation on the input sample, wherein the input sample is optionally a thawed input sample; or performing density centrifugation step using a medium comprising iodixanol, e.g., 60*%* iodixanol in water, e.g., Optiprep medium, and/or having a density greater than Ficoll (e.g., greater than 1.077 g/ml, e.g., about 1.32 g/ml); and
iii) performing a selection step, wherein the selection is a positive selection, e.g., for CD3/CD28+ cells, or a negative selection, e.g., for CD19+, CD25+, or CD14+ cells; thereby producing an output sample comprising immune effector cells that are suitable for expression of a CAR.

In another aspect, the present disclosure features a method of making a population of immune effector cells (e.g., T cells) that can be engineered to express a chimeric antigen receptor (CAR), the method comprising:
i) providing an input sample, e.g., a frozen input sample or a fresh input sample comprising immune effector cells;
ii) optionally, wherein the input sample is a frozen input sample, thawing the frozen input sample, to produce a thawed sample;
iii) performing an enrichment step, wherein the enrichment step comprises:
   1) performing elutriation on the input sample, wherein the input sample is optionally a thawed input sample; or
   2) performing density centrifugation step using a medium comprising iodixanol, e.g., 60*%* iodixanol in water, e.g., Optiprep medium, and/or having a density greater than Ficoll (e.g., greater than 1.077 g/ml, e.g., about 1.32 g/ml); and
iv) performing a selection step, wherein the selection is a positive selection, e.g., for CD3/CD28+ cells, or a negative selection, e.g., for CD19+, CD25+, or CD14+ cells;
thereby producing an output sample comprising immune effector cells that are suitable for expression of a CAR.

In another aspect, the present disclosure features a method of making a population of immune effector cells (e.g., T cells) that can be engineered to express a chimeric antigen receptor (CAR), wherein the method includes:
i) providing an input sample, e.g., a frozen input sample or a fresh input sample, comprising immune effector cells;
ii) performing an enrichment step, wherein the enrichment step comprises: performing elutriation or density centrifugation (e.g., using Ficoll or a Optiprep medium);
iii) performing a selection step, wherein the selection is a positive selection, e.g., for CD3/CD28+ cells, or a negative selection, e.g., for CD19+, CD25+, or CD14+ cells;
thereby producing an output sample comprising immune effector cells that are suitable for expression of a CAR. In one embodiment, the selection step is performed under flow conditions, e.g., by using a flow-through device.

Additional features or embodiments of any of the methods or compositions described herein include one or more of the following:
An input sample in any of the embodiments of any of the methods described herein is a biological sample from a subject that comprises immune effector cells, e.g., T cells and/or NK cells. In an embodiment, the input sample is a blood sample, e.g., a whole blood sample. In an embodiment, the input sample is an apheresis sample, e.g., a leukapheresis sample. In one embodiment, the input sample is a fresh sample, in which the sample has been obtained from the subject and is processed using any of the methods described herein within 1 day, 2 days, 5 days, or 7 days of obtaining from the subject. In one embodiment, the input sample is a frozen or cryopreserved sample, e.g., frozen at -20° C or in liquid nitrogen or frozen to -80°C at a rate of 1° per minute and stored in the vapor phase of a liquid nitrogen storage tank.

In embodiments of any of the methods described herein, the input sample comprises at least 5*%*, 10*%*, 15*%*, 20*%*, 25*%*, 30*%*, 35*%*, 40*%*, 45*%*, 50*%*, 55*%*, 60*%*, 65*%*, 70*%*, 75*%*, 80*%*, 85*%*, 90*%*, or 95*%* monocytes (and optionally up to 40*%*, 70*%*, or 95*%* monocytes). In embodiments of any of the methods described herein, the input sample comprises at least 5*%*, 10*%*, 15*%*, 20*%*, 25*%*, 30*%*, 35*%*, 40*%*, 45*%*, 50*%*, 55*%*, 60*%*, 65*%*, 70*%*, 75*%*, 80*%*, 85*%*, 90*%*, or 95*%* tumor cells, e.g., lymphoblasts (and optionally up to 50*%* or 95*%* monocytes). In embodiments of any of the methods described herein, the input sample comprises less than 60*%*, 55*%*, 50*%*, 45*%*, 40*%*, 35*%*, 30*%*, 25*%*, or 20*%* immune effector cells, e.g., T cells (and optionally greater than 20*%* T cells).

In embodiments of any of the methods described herein, the output sample comprises less than 20*%*, 15*%*, 10*%*, 9*%*, 8*%*, 7*%*, 6*%*, 5*%*, 4*%*, 2*%*, 2*%*, 1*%*, 0.5*%*,0 .2*%*, or 0.1*%* monocytes (and optionally greater than 1*%* or 0.1*%* monocytes). In embodiments of any of the methods described herein, the output sample comprises less than 20*%*, 15*%*, 10*%*, 9*%*, 8*%*, 7*%*, 6*%*, 5*%*, 4*%*, 2*%*, 2*%*, 1*%*, 0.5*%*,0.2*%*, or 0.1*%* tumor cells (and optionally greater than 1*%* or 0.1*%* tumor cells). In embodiments of any of the methods described herein, the output sample comprises at least 50*%*, 55*%*, 60*%*, 65*%*, 70*%*, 75*%*, 80*%*, 85*%*, 90*%*, 95*%*, 96*%*, 97*%*, 98*%*, 99*%*, 99.5*%*, 99.8*%*, or 99.9*%* immune effector cells, e.g., T cells (and optionally up to 60*%* or 95*%* T cells).

In embodiments of any of the methods described herein, the output sample comprises less than 50*%*, 45*%*, 40*%*, 40*%*, 35*%*, 30*%*, 25*%*, 20*%*, 15*%*, 10*%*, 5*%*, 4*%*, 2*%*, 2*%*, or 1*%* the percentage of monocytes compared to the input sample. In embodiments of any of the methods described herein, the output sample comprises less than 50*%*, 45*%*, 40*%*, 40*%*, 35*%*, 30*%*, 25*%*, 20*%*, 15*%*, 10*%*, 5*%*, 4*%*, 2*%*, 2*%*, or 1*%* the percentage of tumor cells compared to the input sample. In embodiments of any of the methods described herein, the output sample comprises at least 50*%*, 45*%*, 40*%*, 40*%*, 35*%*, 30*%*, 25*%*, 20*%*, 15*%*, 10*%*, 5*%*, 4*%*, 2*%*, 2*%*, or 1*%* the percentage of immune effector cells, e.g., T cells, compared to the input sample.

In embodiments of any of the methods described herein, the method further comprises introducing, e.g., by transduction, a nucleic acid encoding a CAR into one or more of the immune effector cells in the output sample. Other methods for introducing a nucleic acid encoding a CAR are described herein.

In embodiments of any of the methods described herein, the CAR comprises an antigen binding domain, a transmembrane domain, and an intracellular signaling domain, e.g., comprising a primary signaling domain and/or a costimulatory signaling domain.

In embodiments of any of the methods described herein, the methods further comprise a step of assaying the transduction efficiency. In embodiments of any of the methods described herein, the transduction results in a transduction efficiency of at least about 10*%*, 15*%*, 20*%*, 25*%*, 30*%*, 35*%*, 40*%*, 45*%*, or 50*%*.

In embodiments of any of the methods described herein, the methods further comprise performing a wash step on the input sample with a buffer comprising dextrose and/or sodium chloride, e.g., D5 medium, e.g., using a CS5+ instrument.

In embodiments of any of the methods described herein, the immune effector cells are human immune effector cells.

In embodiments of any of the methods described herein, the output sample comprises CD8+ T cells. In embodiments of any of the methods described herein, the output sample comprises CD4+ T cells.

In embodiments of any of the methods described herein, the input sample is from a patient that has a disease associated with a tumor antigen, e.g., a tumor antigen described herein, e.g., CD19, is selected from a proliferative disease such as a cancer or malignancy or a precancerous condition such as a myelodysplasia, a myelodysplastic syndrome or a preleukemia, or is a non-cancer related indication associated with expression of a tumor antigen described herein. In one embodiment, the disease is a cancer described herein, e.g., a cancer described herein as being associated with a target described herein. In one embodiment, the hematologic cancer is leukemia. In one embodiment, the cancer is selected from the group consisting of one or more acute leukemias including but not limited to B-cell acute lymphoid leukemia ("BALL"), T-cell acute lymphoid leukemia ("TALL"), acute lymphoid leukemia (ALL); one or more chronic leukemias including but not limited to chronic myelogenous leukemia (CML), chronic lymphocytic leukemia (CLL); additional hematologic cancers or hematologic conditions including, but not limited to B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, MALT lymphoma, mantle cell lymphoma, Marginal zone lymphoma, multiple myeloma, myelodysplasia and myelodysplastic syndrome, non-Hodgkin lymphoma, plasmablastic lymphoma, plasmacytoid dendritic cell neoplasm, Waldenstrom macroglobulinemia, and "preleukemia" which are a diverse collection of hematological conditions united by ineffective production (or dysplasia) of myeloid blood cells, and to disease associated with expression of a tumor antigen described herein include, but not limited to, atypical and/or non-classical cancers, malignancies, precancerous conditions or proliferative diseases expressing a tumor antigen as described herein; and any combination thereof. In another embodiment, the disease associated with a tumor antigen described herein is a solid tumor, e.g., a solid tumor described herein, e.g., prostatic, colorectal, pancreatic, cervical, gastric, ovarian, head, or lung cancer.

In embodiments of any of the methods described herein, the input sample is from a patient that has a cancer selected from the group consisting of one or more acute leukemias including but not limited to B-cell acute lymphoid leukemia (BALL), T-cell acute lymphoid leukemia (TALL), acute lymphoid leukemia (ALL); one or more chronic leukemias including but not limited to chronic myelogenous leukemia (CML), chronic lymphocytic leukemia (CLL); additional hematologic cancers or hematologic conditions including, but not limited to B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, MALT lymphoma, mantle cell lymphoma, Marginal zone lymphoma, multiple myeloma, myelodysplasia and myelodysplastic syndrome, non-Hodgkin lymphoma, Hodgkin lymphoma, plasmablastic lymphoma, plasmacytoid dendritic cell neoplasm, Waldenstrom macroglobulinemia, preleukemia, atypical and/or non-classical cancers, malignancies, precancerous conditions or proliferative diseases, and any combination thereof.

In embodiments of any of the methods described herein, the input sample is from a patient that has ALL.

In embodiments of any of the methods described herein, the method further comprises a step of assaying one or more cell surface markers on cells in the output sample, e.g., CD45, CD19, CD3, CD28, CD25, or CD14.

In embodiments of any of the methods described herein, the method further comprises stimulating the output sample with an agent that stimulates proliferation of the immune effector cells, e.g., stimulates a CD3/TCR complex associated signal and/or a ligand that stimulates a costimulatory molecule on the surface of the T cells, e.g., an anti-CD3 antibody and an anti-CD28 antibody.

In embodiments of any of the methods described herein, the method further comprises introducing a nucleic acid encoding a CAR, e.g., by transduction, transfection, or electroporation.

In another aspect, the present disclosure features a reaction mixture produced by a method disclosed herein, e.g., a method disclosed above.

In another aspect, the present disclosure features a reaction mixture comprising at least 80*%*, 85*%*, 90*%*, or 95*%* T cells and less than 10*%*, 9*%*, 8*%*, 7*%*, 6*%*, 5*%*, 4*%*, 3*%*, 2*%*, or 1*%* monocytes, wherein the total number of cells in the reaction mixture adds up to 100*%*. In one embodiment, the reaction mixture comprises at least 1×10⁶, 2×10⁶, 5×10⁶, 1×10⁷, 2×10⁷, 5×10⁷, 1×10⁸, 2×10⁸, or 5×10⁸ cells total. In one embodiment, the reaction mixture comprises less than 10*%*, 9*%*, 8*%*, 7*%*, 6*%*, 5*%*, 4*%*, 3*%*, 2*%*, or 1*%* B cells. In one embodiment, the reaction mixture comprises less than 10*%*, 9*%*, 8*%*, 7*%*, 6*%*, 5*%*, 4*%*, 3*%*, 2*%*, or 1*%* cancer cells, e.g. lymphoblasts.

In any of the reaction mixtures described herein, one or more of the T cells expresses a CAR, e.g., any CAR described herein.

In any of the reaction mixtures described herein, the reaction mixture further comprises a nucleic acid encoding a CAR, e.g., wherein the nucleic acid is disposed inside a T cell or outside a T cell.

Although methods and materials similar or equivalent to those described herein can be used in the practice or testing of the present invention, suitable methods and materials are described below. All publications, patent applications, patents, and other references (e.g., sequence database reference numbers) mentioned herein are incorporated by reference in their entirety. For example, all GenBank, Unigene, and Entrez sequences referred to herein, e.g., in any Table herein, are incorporated by reference. Unless otherwise specified, the sequence accession numbers specified herein, including in any Table herein, refer to the database entries current as of December 28, 2015. When one gene or protein references a plurality of sequence accession numbers, all of the sequence variants are encompassed.

In addition, the materials, methods, and examples are illustrative only and not intended to be limiting. Headings, sub-headings or numbered or lettered elements, e.g., (a), (b), (i) etc., are presented merely for ease of reading. The use of headings or numbered or lettered elements in this document does not require the steps or elements be performed in alphabetical order or that the steps or elements are necessarily discrete from one another. Other features, objects, and advantages of the invention will be apparent from the description and drawings, and from the claims.

### BRIEF DESCRIPTION OF THE FIGURES

FIG. 1A is a schematic diagram of a cell processing system with a flow-through magnetic separation/debeading module;
FIG. 1B is a schematic diagram of a cell processing system with a plurality of flow-through magnetic separation/debeading modules in parallel;
FIG. 1C is a schematic diagram of a cell processing system with a plurality of flow-through magnetic separation/debeading modules in series;
FIG. 1D is a schematic diagram of a cell processing system with a return loop;
FIG. 1E is a schematic diagram of a cell processing system with cell suspension and buffer modules separately connected to the flow-through magnetic separation/debeading module;
FIG. 1F is a schematic diagram of a cell processing system including a spinning membrane debeading module;
FIG. 1G is a schematic diagram of a cell processing system including multiple flow-through magnetic separation/debeading modules and multiple spinning membrane debeading modules;
FIG. 1H is another schematic diagram of a cell processing system with a flow-through magnetic separation/debeading module;
FIG. 2A is a transverse cross-sectional schematic diagram of a flow-through magnetic separation/debeading module in an x-oriented magnet configuration;
FIG. 2B is a semi-transparent, three-dimensional schematic diagram of the flow-through magnetic separation/debeading module of FIG. 2A;
FIG. 3 is a side longitudinal cross-sectional schematic diagram of a flow-through magnetic separation/debeading module with a membrane and sub-membrane fluid injection ports;
FIG. 4A is a transverse cross-sectional schematic diagram of a flow-through magnetic separation/debeading module in a zero-gradient configuration;
FIG. 4B is a semi-transparent, three-dimensional schematic diagram of the flow-through magnetic separation/debeading modulce of FIG. 4A;
FIG. 4C is a top longitudinal cross-sectional schematic diagram of a flow-through magnetic separation/debeading module in a zero-gradient configuration to create a zero gradient filter;
FIG. 4D is a top longitudinal cross-sectional schematic diagram of a flow-through magnetic separation/debeading module in a multiple zero-gradient configuration to create a multiple zero-gradient filter;
FIG. 5A is a longitudinal cross-sectional schematic diagram of a spinning membrane debeading module;
FIG. 5B is a transverse cross-sectional schematic diagram of a spinning membrane debeading module with a first magnet configuration;
FIG. 5C is a transverse cross-sectional schematic diagram of a spinning membrane debeading module with a second magnet configuration;
FIG. 6 is diagram of fluidic force and magnetic force on a cell;
FIGs. 7A, 7B, and 7C are diagrams of the flow-through magnetic separation/debeading module of FIG. 2B with cells present during magnetic separation;
FIGs. 8A and 8B are diagrams of the flow-through magnetic separation/debeading module of FIG. 2B with cells present during magnetic debeading;
FIG. 9 is a graph comparing the results of debeading using the flow-through magnetic separation/debeading module of FIGs. 2A and 2B and the results of debeading using a conventional stop-flow module (boxes represent quartiles and the median);
FIG. 10A is a top longitudinal x-y cross-sectional schematic diagram of a flow-through magnetic separation/debeading module with paramagnetic particle-bound cells present near the module inlet (left) and near the module outlet (right);
FIG. 10B is a side longitudinal cross-sectional schematic diagram of a flow-through magnetic separation/debeading module with paramagnetic particle-bound cells present near the module inlet (left) and near the module outlet (right);
FIG. 11 is a diagram of the flow-through magnetic separation/debeading module similar to that of FIG. 4A during magnetic separation of paramagnetic particle-bound cells and unbound cells;
FIG. 12 is a diagram of the flow-through magnetic separation/debeading module of FIG. 4C with cells present during magnetic separation of paramagnetic particle-bound cells and unbound cells;
FIG. 13 is a diagram of the flow-through magnetic separation/debeading module of FIG. 4C with cells present during magnetic separation of paramagnetic particles from debeaded, unbound cells; and
FIG. 14 is a diagram of the spinning membrane debeading module of FIG. 5 with cells present during debeading.
FIG. 15A and FIG. 15B are pictures showing the cells after Sepax SmartWash and resuspension in Modified Medium (FIG. 15A) or in aqueous D51/2NS solution (FIG. 15B).
FIG. 16A and 16B are graphs comparing cell recoveries after different wash protocols. FIG. 16A shows the comparison of cell recoveries after CS5+ wash and Sepax SmartWash procedures, where both procedures were performed in the aqueous D51/2NS solution. FIG. 16B shows the comparison of cell recoveries after CS5 wash, and after 2 hour incubation, of cells in Modified Medium compared to cells in the aqueous D51/2NS solution.

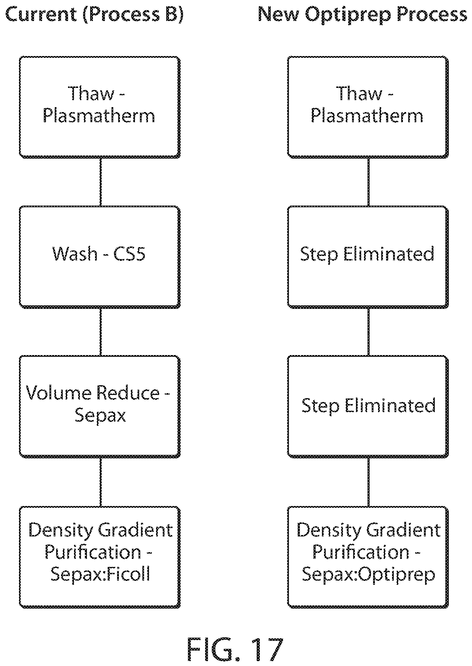

FIG. 17 is a diagram showing the comparison of steps in Process B compared to the new OptiPrep process.
FIG. 18 is a schematic showing the setup of Sepax 2 NeatCell bag and tubing kit.
FIG. 19A, 19B, 19C, 19D, and 19E are graphs showing the phenotypic comparison of products generated by Process B (density gradient centrifugation using Ficoll product) or by the new Sepax with OptiPrep method (OptiPrep product). FIG. 19A shows the T cell yield. FIG. 19B shows the B cell Yield. FIG. 19C shows the monocyte yield. FIG. 19D shows the relative composition of the final products by phenotype. FIG. 19E shows the absolute composition of the final products by phenotype.
FIG. 20 is a schematic showing the sterile bag kit for optimized positive selection (FAST).
FIG. 21 is a schematic showing the modified lid for optimized positive selection (FAST).
FIG. 22 is a graph showing the viable cell number of the clinical lots to date. Black lines indicate lots processed by Process B. Colored lines indicate lots processed by FAST method.
FIG. 23A, 23B, and 23C show the details for Experiment 1 in Example 5. FIG. 23A is a schematic overview and summary of outcomes. FIG. 23B is a series of pre and post-FAST enrichment plots for experiment arm 1. FIG. 23C is a series of pre- and post-FAST enrichment plots for experiment arm 2.
FIG. 24A, 24B, and 24C show the details of Experiment 2 in Example 5. FIG. 24A is a schematic overview and summary of outcomes. FIG. 24B is a series of pre and post-FAST enrichment plots for experiment arm 1. FIG. 24C is a series of pre- and post-FAST enrichment plots for experiment arm 2.
FIG. 25A and 25B show the details of Experiment 3 in Example 5. FIG. 25A is a schematic overview and summary of outcomes. FIG. 25B is a series of pre and post-FAST enrichment plots.
FIG. 26A and 26B are tables showing the results from performing CD19 negative selection. FIG. 26A shows the feasibility of CD19+ cell depletion in apheresis samples from a healthy donor and an ALL patient. FIG. 26B shows the distribution of different cell types, T cell, monocyte, and CD 19 B cell, before and after performing different enrichment protocols (TR149, TR150, and TR151).

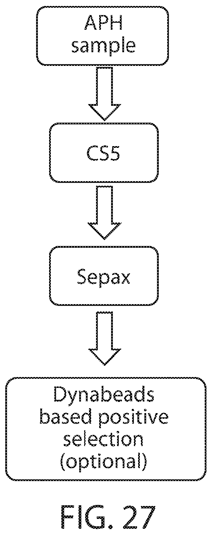

FIG. 27 is a schematic showing the steps for Process B. "APH sample" represents the input apheresis sample, which can be fresh or frozen. "CS5" represents a wash step, e.g., using a cell washing or cell processing system such as CS5+ instrument. "Sepax" represents density gradient separation using Ficoll. "Positive Selection" represents Dynabeads based "static" separation, e.g., using CD3/CD28 Dynabeads.

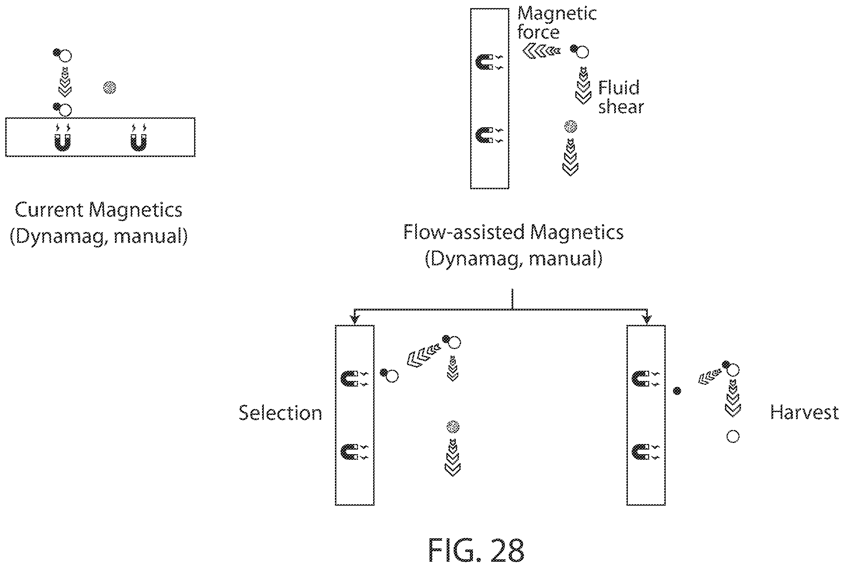

FIG. 28 is a schematic showing the current "static" magnetic separation (left) and "dynamic" magnetic separation, e.g., under flow conditions, (right). The "Selection" (middle) schematic demonstrates the separation of a magnetic separation agent associated with its target molecule, attracted to the magnetic element, thereby separating the target molecule from unwanted molecules.
FIG. 29A, 29B, and 29C show the results of an IFNyrelease assay after stimulation with CD19 (FIG. 29A), PMA (FIG. 29B), and mesothelin (FIG. 29C). The tested immune effector cell samples were prepared using the different protocols listed along the X-axis.
FIG. 30A, 30B, and 30C show the results of a cytotoxicity assay. FIG. 30A and 30B show the percentage of specific lysis for each immune effector sample prepared using the different protocols listed along the X-axis. FIG. 30C shows the cytotoxicity results obtained from FIG. 30A and 30B, expressed as reciprocal EC50 units.
FIG. 31 is a graph of the harvest yields for a non-flow-through debeading process and a flow-through debeading process as a function if input cell number; and
FIG. 32 is a binned graph of the harvest yields for a non-flow-through debeading process and a flow-through debeading process as a function of input cell number.

### DETAILED DESCRIPTION

### Definitions

Unless defined otherwise, all technical and scientific terms used herein have the same meaning as commonly understood by one of ordinary skill in the art to which the invention pertains.

The term "a" and "an" refers to one or to more than one (i.e., to at least one) of the grammatical object of the article. By way of example, "an element" means one element or more than one element.

The term "about" when referring to a measurable value such as an amount, a temporal duration, and the like, is meant to encompass variations of ±20*%* or in some instances ±10*%*, or in some instances ±5*%*, or in some instances ±1*%*, or in some instances ±0.1*%* from the specified value, as such variations are appropriate to perform the disclosed methods.

The term "Chimeric Antigen Receptor" or alternatively a "CAR" refers to a set of polypeptides, typically two in the simplest embodiments, which when in an immune effector cell, provides the cell with specificity for a target cell, typically a cancer cell, and with intracellular signal generation. In some embodiments, a CAR comprises at least an extracellular antigen binding domain, a transmembrane domain and a cytoplasmic signaling domain (also referred to herein as "an intracellular signaling domain") comprising a functional signaling domain derived from a stimulatory molecule and/or costimulatory molecule as defined below. In some embodiments, the set of polypeptides are in the same polypeptide chain (e.g., comprise a chimeric fusion protein). In some embodiments, the set of polypeptides are not contiguous with each other, e.g., are in different polypeptide chains. In some embodiments, the set of polypeptides include a dimerization switch that, upon the presence of a dimerization molecule, can couple the polypeptides to one another, e.g., can couple an antigen binding domain to an intracellular signaling domain. In one embodiment, the stimulatory molecule of the CAR is the zeta chain associated with the T cell receptor complex. In one aspect, the cytoplasmic signaling domain comprises a primary signaling domain (e.g., a primary signaling domain of CD3-zeta). In one embodiment, the cytoplasmic signaling domain further comprises one or more functional signaling domains of at least one costimulatory molecule as defined below. In one embodiment, the costimulatory molecule is a costimulatory molecule described herein, e.g., 4-1BB (i.e., CD137), CD27, ICOS, and/or CD28. In one embodiment, the CAR comprises a chimeric fusion protein comprising an extracellular antigen binding domain, a transmembrane domain and an intracellular signaling domain comprising a functional signaling domain of a stimulatory molecule. In one embodiment, the CAR comprises a chimeric fusion protein comprising an extracellular antigen binding domain, a transmembrane domain and an intracellular signaling domain comprising a functional signaling domain of a co-stimulatory molecule and a functional signaling domain of a stimulatory molecule. In one embodiment, the CAR comprises a chimeric fusion protein comprising an extracellular antigen binding domain, a transmembrane domain and an intracellular signaling domain comprising two functional signaling domains of one or more co-stimulatory molecule(s) and a functional signaling domain of a stimulatory molecule. In one embodiment, the CAR comprises a chimeric fusion protein comprising an extracellular antigen binding domain, a transmembrane domain and an intracellular signaling domain comprising at least two functional signaling domains of one or more co-stimulatory molecule(s) and a functional signaling domain of a stimulatory molecule. In one embodiment, the CAR comprises an optional leader sequence at the amino-terminus (N-terminus) of the CAR fusion protein. In one embodiment, the CAR further comprises a leader sequence at the N-terminus of the extracellular antigen binding domain, wherein the leader sequence is optionally cleaved from the antigen binding domain (e.g., a scFv) during cellular processing and localization of the CAR to the cellular membrane.

A CAR that comprises an antigen binding domain (e.g., a scFv, or TCR) that targets a specific tumor antigen X, such as those described herein, is also referred to as XCAR. For example, a CAR that comprises an antigen binding domain that targets CD19 is referred to as CD19CAR.

The term "signaling domain" refers to the functional portion of a protein which acts by transmitting information within the cell to regulate cellular activity via defined signaling pathways by generating second messengers or functioning as effectors by responding to such messengers.

The term "antibody," as used herein, refers to a protein, or polypeptide sequence derived from an immunoglobulin molecule which specifically binds with an antigen. Antibodies can be polyclonal or monoclonal, multiple or single chain, or intact immunoglobulins, and may be derived from natural sources or from recombinant sources. Antibodies can be tetramers of immunoglobulin molecules.

The term "antibody fragment" refers to at least one portion of an antibody, that retains the ability to specifically interact with (e.g., by binding, steric hindrance, stabilizing/destabilizing, spatial distribution) an epitope of an antigen. Examples of antibody fragments include, but are not limited to, Fab, Fab', F(ab')₂, Fv fragments, scFv antibody fragments, disulfide-linked Fvs (sdFv), a Fd fragment consisting of the VH and CH1 domains, linear antibodies, single domain antibodies such as sdAb (either VL or VH), camelid VHH domains, multi-specific antibodies formed from antibody fragments such as a bivalent fragment comprising two Fab fragments linked by a disulfide bridge at the hinge region, and an isolated CDR or other epitope binding fragments of an antibody. An antigen binding fragment can also be incorporated into single domain antibodies, maxibodies, minibodies, nanobodies, intrabodies, diabodies, triabodies, tetrabodies, v-NAR and bis-scFv (see, e.g., Hollinger and Hudson, Nature Biotechnology 23:1126-1136, 2005). Antigen binding fragments can also be grafted into scaffolds based on polypeptides such as a fibronectin type III (Fn3)(see U.S. Patent No.: 6,703,199, which describes fibronectin polypeptide minibodies).

The term "scFv" refers to a fusion protein comprising at least one antibody fragment comprising a variable region of a light chain and at least one antibody fragment comprising a variable region of a heavy chain, wherein the light and heavy chain variable regions are contiguously linked, e.g., via a synthetic linker, e.g., a short flexible polypeptide linker, and capable of being expressed as a single chain polypeptide, and wherein the scFv retains the specificity of the intact antibody from which it is derived. Unless specified, as used herein an scFv may have the VL and VH variable regions in either order, e.g., with respect to the N-terminal and C-terminal ends of the polypeptide, the scFv may comprise VL-linker-VH or may comprise VH-linker-VL.

The portion of a CAR comprising an antibody or antibody fragment thereof may exist in a variety of forms where the antigen binding domain is expressed as part of a contiguous polypeptide chain including, for example, a single domain antibody fragment (sdAb), a single chain antibody (scFv) and a humanized antibody (Harlow et al., 1999, In: Using Antibodies: A Laboratory Manual, Cold Spring Harbor Laboratory Press, NY; Harlow et al., 1989, In: Antibodies: A Laboratory Manual, Cold Spring Harbor, New York; Houston et al., 1988, Proc. Natl. Acad. Sci. USA 85:5879-5883; Bird et al., 1988, Science 242:423-426). In one embodiment, the antigen binding domain of a CAR comprises an antibody fragment. In a further embodiment, the CAR comprises an antibody fragment that comprises a scFv.

As used herein, the term "binding domain" or "antibody molecule" refers to a protein, e.g., an immunoglobulin chain or fragment thereof, comprising at least one immunoglobulin variable domain sequence. The term "binding domain" or "antibody molecule" encompasses antibodies and antibody fragments. In an embodiment, an antibody molecule is a multispecific antibody molecule, e.g., it comprises a plurality of immunoglobulin variable domain sequences, wherein a first immunoglobulin variable domain sequence of the plurality has binding specificity for a first epitope and a second immunoglobulin variable domain sequence of the plurality has binding specificity for a second epitope. In an embodiment, a multispecific antibody molecule is a bispecific antibody molecule. A bispecific antibody has specificity for no more than two antigens. A bispecific antibody molecule is characterized by a first immunoglobulin variable domain sequence which has binding specificity for a first epitope and a second immunoglobulin variable domain sequence that has binding specificity for a second epitope.

The portion of the CAR of the invention comprising an antibody or antibody fragment thereof may exist in a variety of forms where the antigen binding domain is expressed as part of a contiguous polypeptide chain including, for example, a single domain antibody fragment (sdAb), a single chain antibody (scFv), a humanized antibody, or bispecific antibody (Harlow et al., 1999, In: Using Antibodies: A Laboratory Manual, Cold Spring Harbor Laboratory Press, NY; Harlow et al., 1989, In: Antibodies: A Laboratory Manual, Cold Spring Harbor, New York; Houston et al., 1988, Proc. Natl. Acad. Sci. USA 85:5879-5883; Bird et al., 1988, Science 242:423-426). In one aspect, the antigen binding domain of a CAR composition of the invention comprises an antibody fragment. In a further aspect, the CAR comprises an antibody fragment that comprises a scFv.

The term "antibody heavy chain," refers to the larger of the two types of polypeptide chains present in antibody molecules in their naturally occurring conformations, and which normally determines the class to which the antibody belongs.

The term "antibody light chain," refers to the smaller of the two types of polypeptide chains present in antibody molecules in their naturally occurring conformations. Kappa (κ) and lambda (λ) light chains refer to the two major antibody light chain isotypes.

The term "complementarity determining region" or "CDR," as used herein, refers to the sequences of amino acids within antibody variable regions which confer antigen specificity and binding affinity. For example, in general, there are three CDRs in each heavy chain variable region (e.g., HCDR1, HCDR2, and HCDR3) and three CDRs in each light chain variable region (LCDR1, LCDR2, and LCDR3). The precise amino acid sequence boundaries of a given CDR can be determined using any of a number of well-known schemes, including those described by Kabat et al. (1991), "Sequences of Proteins of Immunological Interest," 5th Ed. Public Health Service, National Institutes of Health, Bethesda, MD ("Kabat" numbering scheme), Al-Lazikani et al., (1997) JMB 273,927-948 ("Chothia" numbering scheme), or a combination thereof. Under the Kabat numbering scheme, in some embodiments, the CDR amino acid residues in the heavy chain variable domain (VH) are numbered 31-35 (HCDR1), 50-65 (HCDR2), and 95-102 (HCDR3); and the CDR amino acid residues in the light chain variable domain (VL) are numbered 24-34 (LCDR1), 50-56 (LCDR2), and 89-97 (LCDR3). Under the Chothia numbering scheme, in some embodiments, the CDR amino acids in the VH are numbered 26-32 (HCDR1), 52-56 (HCDR2), and 95-102 (HCDR3); and the CDR amino acid residues in the VL are numbered 26-32 (LCDR1), 50-52 (LCDR2), and 91-96 (LCDR3). In a combined Kabat and Chothia numbering scheme, in some embodiments, the CDRs correspond to the amino acid residues that are part of a Kabat CDR, a Chothia CDR, or both. For instance, in some embodiments, the CDRs correspond to amino acid residues 26-35 (HCDR1), 50-65 (HCDR2), and 95-102 (HCDR3) in a VH, e.g., a mammalian VH, e.g., a human VH; and amino acid residues 24-34 (LCDR1), 50-56 (LCDR2), and 89-97 (LCDR3) in a VL, e.g., a mammalian VL, e.g., a human VL.

The term "recombinant antibody" refers to an antibody which is generated using recombinant DNA technology, such as, for example, an antibody expressed by a bacteriophage or yeast expression system. The term should also be construed to mean an antibody which has been generated by the synthesis of a DNA molecule encoding the antibody and which DNA molecule expresses an antibody protein, or an amino acid sequence specifying the antibody, wherein the DNA or amino acid sequence has been obtained using recombinant DNA or amino acid sequence technology which is available and well known in the art.

The term "antigen" or "Ag" refers to a molecule that provokes an immune response. This immune response may involve either antibody production, or the activation of specific immunologically-competent cells, or both. The skilled artisan will understand that any macromolecule, including virtually all proteins or peptides, can serve as an antigen. Furthermore, antigens can be derived from recombinant or genomic DNA. A skilled artisan will understand that any DNA, which comprises a nucleotide sequences or a partial nucleotide sequence encoding a protein that elicits an immune response therefore encodes an "antigen" as that term is used herein. Furthermore, one skilled in the art will understand that an antigen need not be encoded solely by a full length nucleotide sequence of a gene. It is readily apparent that the present invention includes, but is not limited to, the use of partial nucleotide sequences of more than one gene and that these nucleotide sequences are arranged in various combinations to encode polypeptides that elicit the desired immune response. Moreover, a skilled artisan will understand that an antigen need not be encoded by a "gene" at all. It is readily apparent that an antigen can be generated synthesized or can be derived from a biological sample, or might be macromolecule besides a polypeptide. Such a biological sample can include, but is not limited to a tissue sample, a tumor sample, a cell or a fluid with other biological components.

The term "autologous" refers to any material derived from the same individual to whom it is later to be re-introduced into the individual.

The term "allogeneic" refers to any material derived from a different animal of the same species as the individual to whom the material is introduced. Two or more individuals are said to be allogeneic to one another when the genes at one or more loci are not identical. In some aspects, allogeneic material from individuals of the same species may be sufficiently unlike genetically to interact antigenically

The term "xenogeneic" refers to any material derived from an animal of a different species.

The term "cancer" refers to a disease characterized by the uncontrolled growth of aberrant cells. Cancer cells can spread locally or through the bloodstream and lymphatic system to other parts of the body. Examples of various cancers are described herein and include but are not limited to, breast cancer, prostate cancer, ovarian cancer, cervical cancer, skin cancer, pancreatic cancer, colorectal cancer, renal cancer, liver cancer, brain cancer, lymphoma, leukemia, lung cancer and the like. The terms "tumor" and "cancer" are used interchangeably herein, e.g., both terms encompass solid and liquid, e.g., diffuse or circulating, tumors. As used herein, the term "cancer" or "tumor" includes premalignant, as well as malignant cancers and tumors.

"Derived from" as that term is used herein, indicates a relationship between a first and a second molecule. It generally refers to structural similarity between the first molecule and a second molecule and does not connote or include a process or source limitation on a first molecule that is derived from a second molecule. For example, in the case of an intracellular signaling domain that is derived from a CD3zeta molecule, the intracellular signaling domain retains sufficient CD3zeta structure such that is has the required function, namely, the ability to generate a signal under the appropriate conditions. It does not connote or include a limitation to a particular process of producing the intracellular signaling domain, e.g., it does not mean that, to provide the intracellular signaling domain, one must start with a CD3zeta sequence and delete unwanted sequence, or impose mutations, to arrive at the intracellular signaling domain.

The phrase "disease associated with expression of a tumor antigen as described herein" includes, but is not limited to, a disease associated with expression of a tumor antigen as described herein or condition associated with cells which express a tumor antigen as described herein including, e.g., proliferative diseases such as a cancer or malignancy or a precancerous condition such as a myelodysplasia, a myelodysplastic syndrome or a preleukemia; or a noncancer related indication associated with cells which express a tumor antigen as described herein. In one embodiment, a cancer associated with expression of a tumor antigen as described herein is a hematological cancer. In one embodiment, a cancer associated with expression of a tumor antigen as described herein is a solid cancer. Further diseases associated with expression of a tumor antigen as described herein include, but not limited to, e.g., atypical and/or non-classical cancers, malignancies, precancerous conditions or proliferative diseases associated with expression of a tumor antigen as described herein. Non-cancer related indications associated with expression of a tumor antigen as described herein include, but are not limited to, e.g., autoimmune disease, (e.g., lupus), inflammatory disorders (allergy and asthma) and transplantation. In some embodiments, the tumor antigen-expressing cells express, or at any time expressed, mRNA encoding the tumor antigen. In an embodiment, the tumor antigen-expressing cells produce the tumor antigen protein (e.g., wild-type or mutant), and the tumor antigen protein may be present at normal levels or reduced levels. In an embodiment, the tumor antigen -expressing cells produced detectable levels of a tumor antigen protein at one point, and subsequently produced substantially no detectable tumor antigen protein.

The phrase "disease associated with expression of CD19" includes, but is not limited to, a disease associated with expression of CD19 or condition associated with cells which express CD19 including, e.g., proliferative diseases such as a cancer or malignancy or a precancerous condition such as a myelodysplasia, a myelodysplastic syndrome or a preleukemia; or a noncancer related indication associated with cells which express CD19. In one aspect, a cancer associated with expression of CD19 is a hematological cancer. In one aspect, the hematological cancer is a leukemia or a lymphoma. In one aspect, a cancer associated with expression of CD19 includes cancers and malignancies including, but not limited to, e.g., one or more acute leukemias including but not limited to, e.g., acute myeloid leukemia (AML), B-cell acute Lymphoid Leukemia (BALL), T-cell acute Lymphoid Leukemia (TALL), acute lymphoid leukemia (ALL); one or more chronic leukemias including but not limited to, e.g., chronic myelogenous leukemia (CML), Chronic Lymphoid Leukemia (CLL). Additional cancers or hematologic conditions associated with expression of CD19 comprise, but are not limited to, e.g., B cell prolymphocytic leukemia, blastic plasmacytoid dendritic cell neoplasm, Burkitt's lymphoma, diffuse large B cell lymphoma, Follicular lymphoma, Hairy cell leukemia, small cell- or a large cell-follicular lymphoma, malignant lymphoproliferative conditions, MALT lymphoma, mantle cell lymphoma (MCL), Marginal zone lymphoma, multiple myeloma, myelodysplasia and myelodysplastic syndrome, non-Hodgkin lymphoma, Hodgkin lymphoma, plasmablastic lymphoma, plasmacytoid dendritic cell neoplasm, Waldenstrom macroglobulinemia, myeloproliferative neoplasm; a histiocytic disorder (e.g., a mast cell disorder or a blastic plasmacytoid dendritic cell neoplasm); a mast cell disorder, e.g., systemic mastocytosis or mast cell leukemia; B-cell prolymphocytic leukemia, plasma cell myeloma, and "preleukemia" which are a diverse collection of hematological conditions united by ineffective production (or dysplasia) of myeloid blood cells, and the like. Further diseases associated with expression of CD19expression include, but not limited to, e.g., atypical and/or non-classical cancers, malignancies, precancerous conditions or proliferative diseases associated with expression of CD19. Non-cancer related indications associated with expression of CD19 include, but are not limited to, e.g., autoimmune disease, (e.g., lupus), inflammatory disorders (allergy and asthma) and transplantation. In some embodiments, the tumor antigen-expressing cells express, or at any time expressed, mRNA encoding the tumor antigen. In an embodiment, the tumor antigen-expressing cells produce the tumor antigen protein (e.g., wild-type or mutant), and the tumor antigen protein may be present at normal levels or reduced levels. In an embodiment, the tumor antigen -expressing cells produced detectable levels of a tumor antigen protein at one point, and subsequently produced substantially no detectable tumor antigen protein. In other embodiments, the disease is a CD19-negative cancer, e.g., a CD19-negative relapsed cancer. In some embodiments, the tumor antigen (e.g., CD19)-expressing cell expresses, or at any time expressed, mRNA encoding the tumor antigen. In an embodiment, the tumor antigen (e.g., CD19)-expressing cell produces the tumor antigen protein (e.g., wild-type or mutant), and the tumor antigen protein may be present at normal levels or reduced levels. In an embodiment, the tumor antigen (e.g., CD19)-expressing cell produced detectable levels of a tumor antigen protein at one point, and subsequently produced substantially no detectable tumor antigen protein.

The phrase "disease associated with expression of a B-cell antigen" includes, but is not limited to, a disease associated with expression of one or more of CD19, CD20, CD22 or ROR1, or a condition associated with cells which express, or at any time expressed, one or more of CD19, CD20, CD22 or ROR1, including, e.g., proliferative diseases such as a cancer or malignancy or a precancerous condition such as a myelodysplasia, a myelodysplastic syndrome or a preleukemia; or a noncancer related indication associated with cells which express one or more of CD19, CD20, CD22 or ROR1. For the avoidance of doubt, a disease associated with expression of the B-cell antigen may include a condition associated with cells which do not presently express the B-cell antigen, e.g., because the antigen expression has been downregulated, e.g., due to treatment with a molecule targeting the B-cell antigen, e.g., a B-cell targeting CAR, but which at one time expressed the antigen. The phrase "disease associated with expression of a B-cell antigen" includes a disease associated with expression of CD19, as described herein. In embodiments, the CAR-expressing cells are used to treat a disease associated with a B-cell antigen. In embodiments, a CAR produced by a method herein comprises an antigen binding domain that targets a B-cell antigen.

The term "relapse" as used herein refers to reappearance of a disease (e.g., cancer) after an initial period of responsiveness, e.g., after prior treatment with a therapy, e.g., cancer therapy (e.g., complete response or partial response). The initial period of responsiveness may involve the level of cancer cells falling below a certain threshold, e.g., below 20*%*, 15*%*, 10*%*, 5*%*, 4*%*, 3*%*, 2*%*, or 1*%*. The reappearance may involve the level of cancer cells rising above a certain threshold, e.g., above 20*%*, 15*%*, 10*%*, 5*%*, 4*%*, 3*%*, 2*%*, or 1*%*. For example, e.g., in the context of B-ALL, the reappearance may involve, e.g., a reappearance of blasts in the blood, bone marrow (> 5*%*), or any extramedullary site, after a complete response. A complete response, in this context, may involve < 5*%* BM blast. More generally, in an embodiment, a response (e.g., complete response or partial response) can involve the absence of detectable MRD (minimal residual disease). In an embodiment, the initial period of responsiveness lasts at least 1, 2, 3, 4, 5, or 6 days; at least 1, 2, 3, or 4 weeks; at least 1, 2, 3, 4, 6, 8, 10, or 12 months; or at least 1, 2, 3, 4, or 5 years.

"Refractory" as used herein refers to a disease, e.g., cancer, that does not respond to a treatment. In embodiments, a refractory cancer can be resistant to a treatment before or at the beginning of the treatment. In other embodiments, the refractory cancer can become resistant during a treatment. A refractory cancer is also called a resistant cancer.

The term "conservative sequence modifications" refers to amino acid modifications that do not significantly affect or alter the binding characteristics of the antibody or antibody fragment containing the amino acid sequence. Such conservative modifications include amino acid substitutions, additions and deletions. Modifications can be introduced into an antibody or antibody fragment of the invention by standard techniques known in the art, such as site-directed mutagenesis and PCR-mediated mutagenesis. Conservative amino acid substitutions are ones in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine). Thus, one or more amino acid residues within a CAR described herein can be replaced with other amino acid residues from the same side chain family and the altered CAR can be tested using the functional assays described herein.

The term "stimulation," refers to a primary response induced by binding of a stimulatory molecule (e.g., a TCR/CD3 complex or CAR) with its cognate ligand (or tumor antigen in the case of a CAR) thereby mediating a signal transduction event, such as, but not limited to, signal transduction via the TCR/CD3 complex or signal transduction via the appropriate NK receptor or signaling domains of the CAR. Stimulation can mediate altered expression of certain molecules.

The term "stimulatory molecule," refers to a molecule expressed by an immune cell (e.g., T cell, NK cell, B cell) that provides the cytoplasmic signaling sequence(s) that regulate activation of the immune cell in a stimulatory way for at least some aspect of the immune cell signaling pathway. In one aspect, the signal is a primary signal that is initiated by, for instance, binding of a TCR/CD3 complex with an MHC molecule loaded with peptide, and which leads to mediation of a T cell response, including, but not limited to, proliferation, activation, differentiation, and the like. A primary cytoplasmic signaling sequence (also referred to as a "primary signaling domain") that acts in a stimulatory manner may contain a signaling motif which is known as immunoreceptor tyrosine-based activation motif or ITAM. Examples of an ITAM containing cytoplasmic signaling sequence that is of particular use in the invention includes, but is not limited to, those derived from CD3 zeta, common FcR gamma (FCER1G), Fc gamma RIIa, FcR beta (Fc Epsilon R1b), CD3 gamma, CD3 delta , CD3 epsilon, , CD79a, CD79b, DAP10, and DAP12. In a specific CAR of the invention, the intracellular signaling domain in any one or more CARS of the invention comprises an intracellular signaling sequence, e.g., a primary signaling sequence of CD3-zeta. In a specific CAR of the invention, the primary signaling sequence of CD3-zeta is the sequence provided as SEQ ID NO:9 (mutant CD3 zeta), or the equivalent residues from a non-human species, e.g., mouse, rodent, monkey, ape and the like. In a specific CAR of the invention, the primary signaling sequence of CD3-zeta is the sequence as provided in SEQ ID NO:10 (wild-type human CD3 zeta), or the equivalent residues from a non-human species, e.g., mouse, rodent, monkey, ape and the like.

The term "antigen presenting cell" or "APC" refers to an immune system cell such as an accessory cell (e.g., a B-cell, a dendritic cell, and the like) that displays a foreign antigen complexed with major histocompatibility complexes (MHC's) on its surface. T-cells may recognize these complexes using their T-cell receptors (TCRs). APCs process antigens and present them to T-cells.

An "intracellular signaling domain," as the term is used herein, refers to an intracellular portion of a molecule. The intracellular signaling domain can generate a signal that promotes an immune effector function of the CAR containing cell, e.g., a CART cell. Examples of immune effector function, e.g., in a CART cell, include cytolytic activity and helper activity, including the secretion of cytokines. In embodiments, the intracellular signaling domain is the portion of a protein which transduces the effector function signal and directs the cell to perform a specialized function. While the entire intracellular signaling domain can be employed, in many cases it is not necessary to use the entire chain. To the extent that a truncated portion of the intracellular signaling domain is used, such truncated portion may be used in place of the intact chain as long as it transduces the effector function signal. The term intracellular signaling domain is thus meant to include any truncated portion of the intracellular signaling domain sufficient to transduce the effector function signal.

In an embodiment, the intracellular signaling domain can comprise a primary intracellular signaling domain. Exemplary primary intracellular signaling domains include those derived from the molecules responsible for primary stimulation, or antigen dependent simulation. In an embodiment, the intracellular signaling domain can comprise a costimulatory intracellular domain. Exemplary costimulatory intracellular signaling domains include those derived from molecules responsible for costimulatory signals, or antigen independent stimulation. For example, in the case of a CART, a primary intracellular signaling domain can comprise a cytoplasmic sequence of a T cell receptor, and a costimulatory intracellular signaling domain can comprise cytoplasmic sequence from co-receptor or costimulatory molecule.

A primary intracellular signaling domain can comprise a signaling motif which is known as an immunoreceptor tyrosine-based activation motif or ITAM. Examples of ITAM containing primary cytoplasmic signaling sequences include, but are not limited to, those derived from CD3 zeta, FcR gamma, FcR beta, CD3 gamma, CD3 delta, CD3 epsilon, CD5, CD22, CD79a, CD79b, CD278 ("ICOS"), FcεRI, and CD66d, CD32, DAP10, and DAP12.

The term "zeta" or alternatively "zeta chain", "CD3-zeta" or "TCR-zeta" is defined as the protein provided as GenBank Acc. No. BAG36664.1, or the equivalent residues from a non-human species, e.g., mouse, rodent, monkey, ape and the like, and a "zeta stimulatory domain" or alternatively a "CD3-zeta stimulatory domain" or a "TCR-zeta stimulatory domain" is defined as the amino acid residues from the cytoplasmic domain of the zeta chain that are sufficient to functionally transmit an initial signal necessary for T cell activation. In one aspect the cytoplasmic domain of zeta comprises residues 52 through 164 of GenBank Acc. No. BAG36664.1 or the equivalent residues from a non-human species, e.g., mouse, rodent, monkey, ape and the like, that are functional orthologs thereof. In one aspect, the "zeta stimulatory domain" or a "CD3-zeta stimulatory domain" is the sequence provided as SEQ ID NO:9. In one aspect, the "zeta stimulatory domain" or a "CD3-zeta stimulatory domain" is the sequence provided as SEQ ID NO:10.

The term "costimulatory molecule" refers to the cognate binding partner on a T cell that specifically binds with a costimulatory ligand, thereby mediating a costimulatory response by the T cell, such as, but not limited to, proliferation. Costimulatory molecules are cell surface molecules other than antigen receptors or their ligands that are required for an efficient immune response. Costimulatory molecules include, but are not limited to MHC class I molecule, TNF receptor proteins, Immunoglobulin-like proteins, cytokine receptors, integrins, signalling lymphocytic activation molecules (SLAM proteins), activating NK cell receptors, BTLA, a Toll ligand receptor, OX40, CD2, CD7, CD27, CD28, CD30, CD40, CDS, ICAM-1, LFA-1 (CD11a/CD18), 4-1BB (CD137), B7-H3, CDS, ICAM-1, ICOS (CD278), GITR, BAFFR, LIGHT, HVEM (LIGHTR), KIRDS2, SLAMF7, NKp80 (KLRF1), NKp44, NKp30, NKp46, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a, and a ligand that specifically binds with CD83.

A costimulatory intracellular signaling domain refers to an intracellular portion of a costimulatory molecule. The intracellular signaling domain can comprise the entire intracellular portion, or the entire native intracellular signaling domain, of the molecule from which it is derived, or a functional fragment thereof.

The intracellular signaling domain can comprise the entire intracellular portion, or the entire native intracellular signaling domain, of the molecule from which it is derived, or a functional fragment thereof.

The term "4-1BB" refers to a member of the TNFR superfamily with an amino acid sequence provided as GenBank Acc. No. AAA62478.2, or the equivalent residues from a non-human species, e.g., mouse, rodent, monkey, ape and the like; and a "4-1BB costimulatory domain" is defined as amino acid residues 214-255 of GenBank Acc. No. AAA62478.2, or the equivalent residues from a non-human species, e.g., mouse, rodent, monkey, ape and the like. In one aspect, the "4-1BB costimulatory domain" is the sequence provided as SEQ ID NO:7 or the equivalent residues from a non-human species, e.g., mouse, rodent, monkey, ape and the like.

"Immune effector cell," as that term is used herein, refers to a cell that is involved in an immune response, e.g., in the promotion of an immune effector response. Examples of immune effector cells include T cells, e.g., alpha/beta T cells and gamma/delta T cells, B cells, natural killer (NK) cells, natural killer T (NKT) cells, mast cells, and myeloid-derived phagocytes.

"Immune effector function or immune effector response," as that term is used herein, refers to function or response, e.g., of an immune effector cell, that enhances or promotes an immune attack of a target cell. E.g., an immune effector function or response refers a property of a T or NK cell that promotes killing or the inhibition of growth or proliferation, of a target cell. In the case of a T cell, primary stimulation and co-stimulation are examples of immune effector function or response.

The term "effector function" refers to a specialized function of a cell. Effector function of a T cell, for example, may be cytolytic activity or helper activity including the secretion of cytokines.

The term "depletion" or "depleting", as used interchangeably herein, refers to the decrease or reduction of the level or amount of a cell, a protein, or macromolecule in a sample after a process, e.g., a selection step, e.g., a negative selection, is performed. The depletion can be a complete or partial depletion of the cell, protein, or macromolecule. In an embodiment, the depletion is at least a 1*%*, 2*%*, 5*%*, 10*%*, 15*%*, 20*%*, 25*%*, 30*%*, 35*%*, 40*%*, 45*%*, 50*%*, 55*%*, 60*%*, 65*%*, 70*%*, 75*%*, 80*%*, 85*%*, 90*%*, 95*%*, or 99*%* decrease or reduction of the level or amount of a cell, a protein, or macromolecule, as compared to the level or amount of the cell, protein or macromolecule in the sample before the process was performed.

The term "encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (e.g., rRNA, tRNA and mRNA) or a defined sequence of amino acids and the biological properties resulting therefrom. Thus, a gene, cDNA, or RNA, encodes a protein if transcription and translation of mRNA corresponding to that gene produces the protein in a cell or other biological system. Both the coding strand, the nucleotide sequence of which is identical to the mRNA sequence and is usually provided in sequence listings, and the noncoding strand, used as the template for transcription of a gene or cDNA, can be referred to as encoding the protein or other product of that gene or cDNA.

Unless otherwise specified, a "nucleotide sequence encoding an amino acid sequence" includes all nucleotide sequences that are degenerate versions of each other and that encode the same amino acid sequence. The phrase nucleotide sequence that encodes a protein or a RNA may also include introns to the extent that the nucleotide sequence encoding the protein may in some version contain an intron(s).

The term "endogenous" refers to any material from or produced inside an organism, cell, tissue or system.

The term "exogenous" refers to any material introduced from or produced outside an organism, cell, tissue or system.

The term "expression" refers to the transcription and/or translation of a particular nucleotide sequence driven by a promoter.

The term "transfer vector" refers to a composition of matter which comprises an isolated nucleic acid and which can be used to deliver the isolated nucleic acid to the interior of a cell. Numerous vectors are known in the art including, but not limited to, linear polynucleotides, polynucleotides associated with ionic or amphiphilic compounds, plasmids, and viruses. Thus, the term "transfer vector" includes an autonomously replicating plasmid or a virus. The term should also be construed to further include non-plasmid and non-viral compounds which facilitate transfer of nucleic acid into cells, such as, for example, a polylysine compound, liposome, and the like. Examples of viral transfer vectors include, but are not limited to, adenoviral vectors, adeno-associated virus vectors, retroviral vectors, lentiviral vectors, and the like.

The term "expression vector" refers to a vector comprising a recombinant polynucleotide comprising expression control sequences operatively linked to a nucleotide sequence to be expressed. An expression vector comprises sufficient cis-acting elements for expression; other elements for expression can be supplied by the host cell or in an in vitro expression system. Expression vectors include all those known in the art, including cosmids, plasmids (e.g., naked or contained in liposomes) and viruses (e.g., lentiviruses, retroviruses, adenoviruses, and adeno-associated viruses) that incorporate the recombinant polynucleotide.

The term "lentivirus" refers to a genus of the Retroviridae family. Lentiviruses are unique among the retroviruses in being able to infect non-dividing cells; they can deliver a significant amount of genetic information into the DNA of the host cell, so they are one of the most efficient methods of a gene delivery vector. HIV, SIV, and FIV are all examples of lentiviruses.

The term "lentiviral vector" refers to a vector derived from at least a portion of a lentivirus genome, including especially a self-inactivating lentiviral vector as provided in Milone et al., Mol. Ther. 17(8): 1453-1464 (2009). Other examples of lentivirus vectors that may be used in the clinic, include but are not limited to, e.g., the LENTIVECTOR^{®} gene delivery technology from Oxford BioMedica, the LENTIMAX^{™} vector system from Lentigen and the like. Nonclinical types of lentiviral vectors are also available and would be known to one skilled in the art.

The term "homologous" or "identity" refers to the subunit sequence identity between two polymeric molecules, e.g., between two nucleic acid molecules, such as, two DNA molecules or two RNA molecules, or between two polypeptide molecules. When a subunit position in both of the two molecules is occupied by the same monomeric subunit; e.g., if a position in each of two DNA molecules is occupied by adenine, then they are homologous or identical at that position. The homology between two sequences is a direct function of the number of matching or homologous positions; e.g., if half (e.g., five positions in a polymer ten subunits in length) of the positions in two sequences are homologous, the two sequences are 50% homologous; if 90% of the positions (e.g., 9 of 10), are matched or homologous, the two sequences are 90% homologous.

"Humanized" forms of non-human (e.g., murine) antibodies are chimeric immunoglobulins, immunoglobulin chains or fragments thereof (such as Fv, Fab, Fab', F(ab')2 or other antigen-binding subsequences of antibodies) which contain minimal sequence derived from non-human immunoglobulin. For the most part, humanized antibodies and antibody fragments thereof are human immunoglobulins (recipient antibody or antibody fragment) in which residues from a complementary-determining region (CDR) of the recipient are replaced by residues from a CDR of a non-human species (donor antibody) such as mouse, rat or rabbit having the desired specificity, affinity, and capacity. In some instances, Fv framework region (FR) residues of the human immunoglobulin are replaced by corresponding non-human residues. Furthermore, a humanized antibody/antibody fragment can comprise residues which are found neither in the recipient antibody nor in the imported CDR or framework sequences. These modifications can further refine and optimize antibody or antibody fragment performance. In general, the humanized antibody or antibody fragment thereof will comprise substantially all of at least one, and typically two, variable domains, in which all or substantially all of the CDR regions correspond to those of a non-human immunoglobulin and all or a significant portion of the FR regions are those of a human immunoglobulin sequence. The humanized antibody or antibody fragment can also comprise at least a portion of an immunoglobulin constant region (Fc), typically that of a human immunoglobulin. For further details, see Jones et al., Nature, 321: 522-525, 1986; Reichmann et al., Nature, 332: 323-329, 1988; Presta, Curr. Op. Struct. Biol., 2: 593-596, 1992.

"Fully human" refers to an immunoglobulin, such as an antibody or antibody fragment, where the whole molecule is of human origin or consists of an amino acid sequence identical to a human form of the antibody or immunoglobulin.

The term "isolated" means altered or removed from the natural state. For example, a nucleic acid or a peptide naturally present in a living animal is not "isolated," but the same nucleic acid or peptide partially or completely separated from the coexisting materials of its natural state is "isolated." An isolated nucleic acid or protein can exist in substantially purified form, or can exist in a non-native environment such as, for example, a host cell.

In the context of the present invention, the following abbreviations for the commonly occurring nucleic acid bases are used. "A" refers to adenosine, "C" refers to cytosine, "G" refers to guanosine, "T" refers to thymidine, and "U" refers to uridine.

The term "operably linked" or "transcriptional control" refers to functional linkage between a regulatory sequence and a heterologous nucleic acid sequence resulting in expression of the latter. For example, a first nucleic acid sequence is operably linked with a second nucleic acid sequence when the first nucleic acid sequence is placed in a functional relationship with the second nucleic acid sequence. For instance, a promoter is operably linked to a coding sequence if the promoter affects the transcription or expression of the coding sequence. Operably linked DNA sequences can be contiguous with each other and, e.g., where necessary to join two protein coding regions, are in the same reading frame.

The term "parenteral" administration of an immunogenic composition includes, e.g., subcutaneous (s.c.), intravenous (i.v.), intramuscular (i.m.), or intrasternal injection, intratumoral, or infusion techniques.

The term "nucleic acid" or "polynucleotide" refers to deoxyribonucleic acid (DNA) or ribonucleic acid (RNA), or a combination of a DNA or RNA thereof, and polymers thereof in either single- or double-stranded form. The term "nucleic acid" includes a gene, cDNA or an mRNA. In one embodiment, the nucleic acid molecule is synthetic (e.g., chemically synthesized) or recombinant. Unless specifically limited, the term encompasses nucleic acids containing analogues or derivatives of natural nucleotides that have similar binding properties as the reference nucleic acid and are metabolized in a manner similar to naturally occurring nucleotides. Unless otherwise indicated, a particular nucleic acid sequence also implicitly encompasses conservatively modified variants thereof (e.g., degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

The terms "peptide," "polypeptide," and "protein" are used interchangeably, and refer to a compound comprised of amino acid residues covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used herein, the term refers to both short chains, which also commonly are referred to in the art as peptides, oligopeptides and oligomers, for example, and to longer chains, which generally are referred to in the art as proteins, of which there are many types. "Polypeptides" include, for example, biologically active fragments, substantially homologous polypeptides, oligopeptides, homodimers, heterodimers, variants of polypeptides, modified polypeptides, derivatives, analogs, fusion proteins, among others. A polypeptide includes a natural peptide, a recombinant peptide, or a combination thereof.

The term "promoter" refers to a DNA sequence recognized by the synthetic machinery of the cell, or introduced synthetic machinery, required to initiate the specific transcription of a polynucleotide sequence.

The term "promoter/regulatory sequence" refers to a nucleic acid sequence which is required for expression of a gene product operably linked to the promoter/regulatory sequence. In some instances, this sequence may be the core promoter sequence and in other instances, this sequence may also include an enhancer sequence and other regulatory elements which are required for expression of the gene product. The promoter/regulatory sequence may, for example, be one which expresses the gene product in a tissue specific manner.

The term "constitutive" promoter refers to a nucleotide sequence which, when operably linked with a polynucleotide which encodes or specifies a gene product, causes the gene product to be produced in a cell under most or all physiological conditions of the cell.

The term "inducible" promoter refers to a nucleotide sequence which, when operably linked with a polynucleotide which encodes or specifies a gene product, causes the gene product to be produced in a cell substantially only when an inducer which corresponds to the promoter is present in the cell.

The term "tissue-specific" promoter refers to a nucleotide sequence which, when operably linked with a polynucleotide encodes or specified by a gene, causes the gene product to be produced in a cell substantially only if the cell is a cell of the tissue type corresponding to the promoter.

The terms "cancer associated antigen" or "tumor antigen" interchangeably refers to a molecule (typically protein, carbohydrate or lipid) that is preferentially expressed on the surface of a cancer cell, either entirely or as a fragment (e.g., MHC/peptide), in comparison to a normal cell, and which is useful for the preferential targeting of a pharmacological agent to the cancer cell. In some embodiments, a tumor antigen is a marker expressed by both normal cells and cancer cells, e.g., a lineage marker, e.g., CD19 on B cells. In certain aspects, the tumor antigens of the present invention are derived from, cancers including but not limited to primary or metastatic melanoma, thymoma, lymphoma, sarcoma, lung cancer, liver cancer, non-Hodgkin lymphoma, Hodgkin lymphoma, leukemias, uterine cancer, cervical cancer, bladder cancer, kidney cancer and adenocarcinomas such as breast cancer, prostate cancer, ovarian cancer, pancreatic cancer, and the like. In some embodiments, a cancer-associated antigen is a cell surface molecule that is overexpressed in a cancer cell in comparison to a normal cell, for instance, 1-fold over expression, 2-fold overexpression, 3-fold overexpression or more in comparison to a normal cell. In some embodiments, a cancer-associated antigen is a cell surface molecule that is inappropriately synthesized in the cancer cell, for instance, a molecule that contains deletions, additions or mutations in comparison to the molecule expressed on a normal cell. In some embodiments, a cancer-associated antigen will be expressed exclusively on the cell surface of a cancer cell, entirely or as a fragment (e.g., MHC/peptide), and not synthesized or expressed on the surface of a normal cell. In some embodiments, the CARs of the present invention includes CARs comprising an antigen binding domain (e.g., antibody or antibody fragment) that binds to a MHC presented peptide. Normally, peptides derived from endogenous proteins fill the pockets of Major histocompatibility complex (MHC) class I molecules, and are recognized by T cell receptors (TCRs) on CD8 + T lymphocytes. The MHC class I complexes are constitutively expressed by all nucleated cells. In cancer, virus-specific and/or tumor-specific peptide/MHC complexes represent a unique class of cell surface targets for immunotherapy. TCR-like antibodies targeting peptides derived from viral or tumor antigens in the context of human leukocyte antigen (HLA)-A1 or HLA-A2 have been described (see, e.g., Sastry et al., J Virol. 2011 85(5):1935-1942; Sergeeva et al., Blood, 2011 117(16):4262-4272; Verma et al., J Immunol 2010 184(4):2156-2165; Willemsen et al., Gene Ther 2001 8(21) :1601-1608 ; Dao et al., Sci Transl Med 2013 5(176) :176ra33 ; Tassev et al., Cancer Gene Ther 2012 19(2):84-100). For example, TCR-like antibody can be identified from screening a library, such as a human scFv phage displayed library.

The term "flexible polypeptide linker" or "linker" as used in the context of a scFv refers to a peptide linker that consists of amino acids such as glycine and/or serine residues used alone or in combination, to link variable heavy and variable light chain regions together. In one embodiment, the flexible polypeptide linker is a Gly/Ser linker and comprises the amino acid sequence (Gly-Gly-Gly-Ser)ₙ (SEQ ID NO: 15), where n is a positive integer equal to or greater than 1. For example, n=1, n=2, n=3, n=4, n=5, n=6, n=7, n=8, n=9 and n=10. In one embodiment, the flexible polypeptide linkers include, but are not limited to, (Gly₄ Ser)₄ (SEQ ID NO:27) or (Gly₄ Ser)₃ (SEQ ID NO:28). In another embodiment, the linkers include multiple repeats of (Gly₂Ser), (GlySer) or (Gly₃Ser) (SEQ ID NO:29). Also included within the scope of the invention are linkers described in WO2012/138475, incorporated herein by reference).

As used herein, a 5' cap (also termed an RNA cap, an RNA 7-methylguanosine cap or an RNA m⁷G cap) is a modified guanine nucleotide that has been added to the "front" or 5' end of a eukaryotic messenger RNA shortly after the start of transcription. The 5' cap consists of a terminal group which is linked to the first transcribed nucleotide. Its presence is critical for recognition by the ribosome and protection from RNases. Cap addition is coupled to transcription, and occurs co-transcriptionally, such that each influences the other. Shortly after the start of transcription, the 5' end of the mRNA being synthesized is bound by a cap-synthesizing complex associated with RNA polymerase. This enzymatic complex catalyzes the chemical reactions that are required for mRNA capping. Synthesis proceeds as a multi-step biochemical reaction. The capping moiety can be modified to modulate functionality of mRNA such as its stability or efficiency of translation.

As used herein, "in vitro transcribed RNA" refers to RNA, e.g., mRNA, that has been synthesized in vitro. Generally, the in vitro transcribed RNA is generated from an in vitro transcription vector. The in vitro transcription vector comprises a template that is used to generate the in vitro transcribed RNA.

As used herein, a "poly(A)" is a series of adenosines attached by polyadenylation to the mRNA. In some embodiments of a construct for transient expression, the polyA is between 50 and 5000 (SEQ ID NO: 30), e.g., greater than 64, e.g., greater than 100, e.g., greater than 300 or 400 poly(A) sequences can be modified chemically or enzymatically to modulate mRNA functionality such as localization, stability or efficiency of translation.

As used herein, "polyadenylation" refers to the covalent linkage of a polyadenylyl moiety, or its modified variant, to a messenger RNA molecule. In eukaryotic organisms, most messenger RNA (mRNA) molecules are polyadenylated at the 3' end. The 3' poly(A) tail is a long sequence of adenine nucleotides (often several hundred) added to the pre-mRNA through the action of an enzyme, polyadenylate polymerase. In higher eukaryotes, the poly(A) tail is added onto transcripts that contain a specific sequence, the polyadenylation signal. The poly(A) tail and the protein bound to it aid in protecting mRNA from degradation by exonucleases. Polyadenylation is also important for transcription termination, export of the mRNA from the nucleus, and translation. Polyadenylation occurs in the nucleus immediately after transcription of DNA into RNA, but additionally can also occur later in the cytoplasm. After transcription has been terminated, the mRNA chain is cleaved through the action of an endonuclease complex associated with RNA polymerase. The cleavage site is usually characterized by the presence of the base sequence AAUAAA near the cleavage site. After the mRNA has been cleaved, adenosine residues are added to the free 3' end at the cleavage site.

As used herein, "transient" refers to expression of a non-integrated transgene for a period of hours, days or weeks, wherein the period of time of expression is less than the period of time for expression of the gene if integrated into the genome or contained within a stable plasmid replicon in the host cell.

Apheresis is the process in which whole blood is removed from an individual, separated into select components, and the remainder returned to circulation. Generally, there are two methods for the separation of blood components, centrifugal and non-centrifugal. Leukapheresis results in the active selection and removal of the patient's white blood cells.

As used herein, the terms "treat", "treatment" and "treating" refer to the reduction or amelioration of the progression, severity and/or duration of a proliferative disorder, or the amelioration of one or more symptoms (e.g., one or more discernible symptoms) of a proliferative disorder resulting from the administration of one or more therapies (e.g., one or more therapeutic agents such as a CAR of the invention). In specific embodiments, the terms "treat", "treatment" and "treating" refer to the amelioration of at least one measurable physical parameter of a proliferative disorder, such as growth of a tumor, not necessarily discernible by the patient. In other embodiments the terms "treat", "treatment" and "treating" -refer to the inhibition of the progression of a proliferative disorder, either physically by, e.g., stabilization of a discernible symptom, physiologically by, e.g., stabilization of a physical parameter, or both. In other embodiments the terms "treat", "treatment" and "treating" refer to the reduction or stabilization of tumor size or cancerous cell count.

The term "signal transduction pathway" refers to the biochemical relationship between a variety of signal transduction molecules that play a role in the transmission of a signal from one portion of a cell to another portion of a cell. The phrase "cell surface receptor" includes molecules and complexes of molecules capable of receiving a signal and transmitting signal across the membrane of a cell.

The term "subject" is intended to include living organisms in which an immune response can be elicited (e.g., mammals, human).

The term, a "substantially purified" cell refers to a cell that is essentially free of other cell types. A substantially purified cell also refers to a cell which has been separated from other cell types with which it is normally associated in its naturally occurring state. In some instances, a population of substantially purified cells refers to a homogenous population of cells. In other instances, this term refers simply to cell that have been separated from the cells with which they are naturally associated in their natural state. In some aspects, the cells are cultured in vitro. In other aspects, the cells are not cultured in vitro.

In the context of the present invention, "tumor antigen" or "hyperproliferative disorder antigen" or "antigen associated with a hyperproliferative disorder" refers to antigens that are common to specific hyperproliferative disorders. In certain embodiments, the tumor antigen is derived from a cancer including but not limited to primary or metastatic melanoma, thymoma, lymphoma, sarcoma, lung cancer, liver cancer, non-Hodgkin lymphoma, Hodgkin lymphoma, leukemias, uterine cancer, cervical cancer, bladder cancer, kidney cancer and adenocarcinomas such as breast cancer, prostate cancer, ovarian cancer, pancreatic cancer, and the like.

The term "transfected" or "transformed" or "transduced" refers to a process by which exogenous nucleic acid is transferred or introduced into the host cell. A "transfected" or "transformed" or "transduced" cell is one which has been transfected, transformed or transduced with exogenous nucleic acid. The cell includes the primary subject cell and its progeny.

The term "specifically binds," refers to an antibody, or a ligand, which recognizes and binds with a cognate binding partner protein present in a sample, but which antibody or ligand does not substantially recognize or bind other molecules in the sample.

Ranges: throughout this disclosure, various aspects of the invention can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 2.7, 3, 4, 5, 5.3, and 6. As another example, a range such as 95-99*%* identity, includes something with 95*%*, 96*%*, 97*%*, 98*%* or 99*%* identity, and includes subranges such as 96-99*%*, 96-98*%*, 96-97*%*, 97-99*%*, 97-98*%* and 98-99*%* identity. This applies regardless of the breadth of the range.

### Description

Provided herein are methods of manufacturing immune effector cells (e.g., T cells, NK cells) that can be engineered with a CAR, e.g., a CAR described herein, and reaction mixtures and compositions comprising such cells. The methods provided herein improve the yield and quality, e.g., purity, of cells suitable for expression of a CAR. Without wishing to be bound by theory, the improved yield and quality of the cells that can be engineered to express a CAR is believed to improve the efficiency of introducing a nucleic acid encoding a CAR and improve the expansion of the resulting CAR-expressing cell. Accordingly, the methods and compositions described herein provide improved CAR-expressing cell products for use in treating a disease in a subject.

In one aspect, the present disclosure features methods that remove unwanted materials, non-target cells, or cells that can negatively impact the expression of a CAR or therapeutic efficacy of the CAR-expressing cell. For example, the methods featured herein can be used to remove or deplete one or more of any of the following: monocytes, granulocytes, red blood cells, platelets, B cells, cancer cells, e.g., lyphoblasts, cryoprotectant (from frozen samples), hemoglobin, or cellular debris. For example, the methods featured herein can be used to enrich or increase the number of one or more of any of the following: T cells (CD4+ and/or CD8+ T cells), NK cells, dendritic cells. Implementation of each method described herein alone or in any combination with each of or all of the methods described herein results in improved starting material suitable for engineering to express a CAR.

Fresh apheresis materials are commonly used in manufacturing cells suitable for expressing a CAR. Use of frozen, e.g., cryopreserved, apheresis materials provides the advantage of being easily transported, thereby removing any restriction on the proximity of location of the patient to a CAR-expressing cell product manufacturing facility, and allowing industrialization of the CAR-expressing cell manufacturing process and greater accessibility of the therapeutic product to patients in need thereof. Methods currently used for manufacturing CAR-expressing cells are optimized for processing of fresh apheresis materials, and cannot be used to obtain similar quality or yield of cells suitable for CAR expression from frozen apheresis samples. In contrast, the methods described herein can be used to process and manufacture cells suitable for CAR expression from a frozen, e.g., cryopreserved, apheresis sample. In embodiments in which the starting material is frozen, e.g., cryopreserved, the methods described herein optionally include a thawing step in which the frozen cells are allowed to thaw, e.g., without interference by an operator or a device to accelerate the thawing process, or the frozen cells are subjected to a device or process that accelerates the thawing process, e.g., by use of a thawing device, e.g., PlasmaTherm. In such embodiments, the thawed material has the same temperature as the surrounding environment, e.g., the same temperature as the ambient temperature of the room or the same temperature of the buffer into which the thawed material is added to, washed with, or incubated with. The methods described herein are particularly useful for generating or enriching a population of immune effector cells that can be engineered to express a CAR from a frozen or thawed input sample, e.g., a frozen or thawed apheresis sample.

Process B, as referred to herein, is a standard protocol for enriching immune effector cells that can be engineered to express a CAR that is currently used. Process B comprises performing density gradient purification with Ficoll, and a positive selection using CD3/CD28 Dynabeads, wherein the input sample is fresh apheresis material. The methods described herein provide greater enrichment, improved quality and yield of the desired immune effector cells suitable for expressing a CAR.

In another aspect, the disclosure features an immune effector cell (e.g., T cell, NK cell), e.g., made by any of the manufacturing methods described herein, engineered to express a CAR, wherein the engineered immune effector cell exhibits an antitumor property. In one embodiment, the CAR comprises an antigen binding domain, a transmembrane domain, and an intracellular signaling domain. An exemplary antigen is a cancer associated antigen (i.e., tumor antigen) described herein. In one aspect, a cell is transformed with the CAR and the CAR is expressed on the cell surface. In some embodiments, the cell (e.g., T cell, NK cell) is transduced with a viral vector encoding a CAR. In some embodiments, the viral vector is a retroviral vector. In some embodiments, the viral vector is a lentiviral vector. In some such embodiments, the cell may stably express the CAR. In another embodiment, the cell (e.g., T cell, NK cell) is transfected with a nucleic acid, e.g., mRNA, cDNA, DNA, encoding a CAR. In some such embodiments, the cell may transiently express the CAR.

Furthermore, the present disclosure provides CAR-expressing cell compositions and their use in medicaments or methods for treating, among other diseases, cancer or any malignancy or autoimmune diseases involving cells or tissues which express a tumor antigen as described herein.

### Elutriation

In one aspect, the methods described herein feature an elutriation method that removes unwanted cells, e.g., monocytes and blasts, thereby resulting in an improved enrichment of desired immune effector cells suitable for CAR expression. In one embodiment, the elutriation method described herein is optimized for the enrichment of desired immune effector cells suitable for CAR expression from a previously frozen sample, e.g., a thawed sample. In one embodiment, the elutriation method described herein provides a preparation of cells with improved purity as compared to a preparation of cells collected from the elutriation protocols known in the art.

In order to facilitate manufacturing logistics (remote sample collection, shipping, storage, and production unit scheduling), the cellular raw material is typically cryopreserved whole blood or apheresis materials which need to be thawed prior to the start of manufacturing. However, the density and size of cells from thawed previously frozen materials are quite different from those of fresh materials. As such, the standard elutriation protocol commonly used for isolating cells for engineering CAR expression largely fails to remove monocytes, granulocytes or any larger-sized cells from cryopreserved and thawed whole blood or apheresis materials. This situation negatively affects the outcome of subsequent CART manufacturing steps, leading to poor yields, product quality concerns, and out-of-specification process deviations. While elutriation can remove monocytes, it is not efficient in removing blast cells, since the blast cells have similar densities and sizes as T lymphocytes.

In an embodiment, the elutriation method described herein includes using an optimized viscosity of the starting sample, e.g., cell sample, e.g., thawed cell sample, by dilution with certain isotonic solutions (e.g., PBS), and using an optimized combination of flow rates and collection volume for each fraction collected by an elutriation device. An example of the modified elutriation program is described in Example 1.

Exemplary ranges of elutriation settings for separation of lymphocytes, e.g., T cells, from monocytes are provided in **Table 4**. The settings for flow rate, centrifugation, and volume for an exemplary elutriation program is also provided in Table 4 in the columns designated "Ex.".

**Table 4. Range of elutriation settings**

| **Fraction** | **Flow Rate (mL/min)** | | **Centrifugation (rpm)** | | **Volume (mL)** | |
|---|---|---|---|---|---|---|
| | **Range** | **Ex.** | **Range** | **Ex.** | **Range** | **Ex.** |
| F1 | 30.0-50.0 | 30.0 | 1800-2400 | 2400 | 100-1000 | 900 |
| F2 | 0.0-50.0 | 30.0 | 0-2400 | 2400 | 0-500 | 500 |
| F3 | 50.0-80.0 | 70.0 | 1800-2400 | 2400 | 500-1000 | 975 |
| F4 | 50.0-80.0 | 72.0 | 1800-2400 | 2400 | 500-1000 | 400 |
| F5 | 80.0-150.0 | 82.0 | 0 | 0 | 0-500 | 250 |

In one embodiment, one, two, three, four, five, six, seven, eight, nine, or ten, or more, fractions are collected from the elutriation step. In one embodiment, five fractions are collected from the elutriation step. In an embodiment where five fractions are collected, the third fraction (F3) or the fourth fraction (F4), or a combination of the third fraction and the fourth fraction, contain the desired lymphocyte population with the minimal amount of monocytes, granulocytes and other non-lymphocyte cells. In one embodiment, each fraction is collected using a different flow rate. In one embodiment, for each fraction, the flow rate is increased from the flow rate used to collect the previous fraction. In one embodiment, one or more of the fractions is collected using a different collection volume.

In one embodiment, the elutriation is performed using a flow rate of from about 20-90 mL/min, from about 30-90 mL/min, from about 40-90 mL/min, from about 50-90 mL/min, from about 60-90 mL/min, from about 70-90 mL/min, from about 40-85 mL/min, from about 50-82 mL/min, from about 60-82 mL/min, from about 70-82 mL/min, from about 50-80 mL/min, from about 60-80 mL/min, from about 70-80 mL/min. In one embodiment, the elutriation is performed using a flow rate of from about 30-82 mL/min, or from about 50-80 mL/min. In one embodiment, the elutriation is performed using a flow rate of about 30, 40, 50, 60, 70, 72, 80, or 82 mL/min. In one embodiment, the elutriation is performed using a flow rate of about 70 mL/min or 72 mL/min.

In one embodiment, the flow rate for the one or more fractions that contain the desired lymphocyte population with the minimal amount of monocytes, granulocytes, other non-lymphocyte cells, and other undesired components, is from about 20-90 mL/min, from about 30-90 mL/min, from about 40-90 mL/min, from about 50-90 mL/min, from about 60-90 mL/min, from about 70-90 mL/min, from about 40-85 mL/min, from about 50-82 mL/min, from about 60-82 mL/min, from about 70-82 mL/min, from about 50-80 mL/min, from about 60-80 mL/min, from about 70-80 mL/min. In one embodiment, the flow rate for the one or more fractions that contain the desired lymphocyte population is from about 50-82 mL/min, from about 50-80 mL/min, from about 60-82 mL/min, from about 60-80 mL/min, from about 70-82 mL/min, from about 70-80 mL/min, from about 70-75 mL/min, from about 70-72 mL/min. In one embodiment, the flow rate for the one or more fractions that contain the desired lymphocyte population is about 70 mL/min or 72 mL/min.

In one embodiment, the elutriation is performed using a collection volume of about 250-1250 mL, about 250-1000 mL, about 300-1000 mL, about 400-1000 mL, about 500-1000 mL, about 600-1000 mL, about 700-1000 mL, about 800-1000 mL, about 900-1000 mL, about 250-975 mL, about 300-975 mL, about 400-975 mL, about 500-975 mL, about 600-975 mL, about 700-975 mL, about 800-975 ml, about 300-900 mL, about 300-800 mL, about 300-700 mL, about 300-600 mL, about 300-500 mL, or about 300-400 mL. In one embodiment, the elutriation is performed using a collection volume of about 250, 400, 500, 900, or 975 mL. In one embodiment, the elutriation is performed using a collection volume of about 400 mL or about 975 mL.

In one embodiment, the collection volume for the one or more fractions that contain the desired lymphocyte population with the minimal amount of monocytes, granulocytes, other non-lymphocyte cells, and other undesired components, is from about 250-1250 mL, about 250-1000 mL, about 300-1000 mL, about 400-1000 mL, about 500-1000 mL, about 600-1000 mL, about 700-1000 mL, about 800-1000 mL, about 900-1000 mL, about 250-975 mL, about 300-975 mL, about 400-975 mL, about 500-975 mL, about 600-975 mL, about 700-975 mL, about 800-975 ml, about 300-900 mL, about 300-800 mL, about 300-700 mL, about 300-600 mL, about 300-500 mL, or about 300-400 mL. In one embodiment, the collection volume for the one or more fractions that contain the desired lymphocyte population is about 250, 400, 500, 900, or 975 mL. In one embodiment, the collection volume for the one or more fractions that contain the desired lymphocyte population is about 400 mL or about 975 mL.

In one embodiment, the elutriation method described herein is performed by an elutriation device. For example, the elutriation device is the Caridian BCT ElutraTM Cell Separation System (Terumo BCT Model 71800). The Caridian BCT ElutraTM Cell Separation System (Terumo BCT Model 71800) is a closed system that utilizes continuous counter-flow elutriation technology to perform cell separation based primarily by size and secondarily by specific gravity. The opposing forces, generated by the flow of media into the separation chamber and the sedimentation velocity created by the centrifugal force, cause the cells to arrange themselves by size and density within the separation chamber, where they are automatically siphoned into the collection bags. The customized Elutra settings are designed to allow for the distribution of lymphocytes and monocytes combined with granulocytes in different fractions. The Elutra can be operated according to the manufacturer's directions.

### Density Gradient Centrifugation

Manufacturing of adoptive cell therapeutic product requires processing the desired cells, e.g., immune effector cells, away from a complex mixture of blood cells and blood elements present in peripheral blood apheresis starting materials. Peripheral blood-derived lymphocyte samples have been successfully isolated using density gradient centrifugation through Ficoll solution. However, Ficoll is not a preferred reagent for isolating cells for therapeutic use, as Ficoll is not qualified for clinical use. In addition, Ficoll contains glycol, which has toxic potential to the cells. Furthermore, Ficoll density gradient centrifugation of thawed apheresis products after cryopreservation yields a suboptimal T cell product, e.g., as described in the Examples herein. For example, a loss of T cells in the final product, with a relative gain of non-T cells, especially undesirable B cells, blast cells and monocytes was observed in cell preparations isolated by density gradient centrifugation through Ficoll solution.

Without wishing to be bound by theory, it is believed that immune effector cells, e.g., T cells, dehydrate during cryopreservation to become denser than fresh cells. Without wishing to be bound by theory, it is also believed that immune effector cells, e.g., T cells, remain denser longer than the other blood cells, and thus are more readily lost during Ficoll density gradient separation as compared to other cells. Accordingly, without wishing to be bound by theory, a medium with a density greater than Ficoll is believed to provide improved isolation of desired immune effector cells in comparison to Ficoll or other mediums with the same density as Ficoll, e.g., 1.077 g/mL.

In one embodiment, the density gradient centrifugation method described herein includes the use of a density gradient medium comprising iodixanol. In one embodiment, the density gradient medium comprises about 60% iodixanol in water.

In one embodiment, the density gradient centrifugation method described herein includes the use of a density gradient medium having a density greater than Ficoll. In one embodiment, the density gradient centrifugation method described herein includes the use of a density gradient medium having a density greater than 1.077 g/mL, e.g., greater than 1.077 g/mL, greater than 1.1 g/mL, greater than 1.15 g/mL, greater than 1.2 g/mL, greater than 1.25 g/mL, greater than 1.3 g/mL, greater than 1.31 g/mL. In one embodiment, the density gradient medium has a density of about 1.32 g/mL.

In one embodiment, the density gradient centrifugation method described herein includes the use of a density gradient medium comprising iodixanol, e.g., about 60% iodixanol in water, and has a density greater than Ficoll, e.g., greater than 1.077 g/mL, e.g., about 1.32 g/mL. In one embodiment, the density gradient centrifugation method described herein includes the use of a density gradient medium OptiPrep^{™} (Sigma). OptiPrep^{™} is a ready-made, sterile and endotoxin-tested solution of 60% (w/v) iodixanol, with a density of 1.320 ± 0.001 g/ml. In contrast, Ficoll density gradient solution has a density of only 1.077 g/ml. Another advantage of OptiPrep^{™} over Ficoll is that OptiPrep^{™} is available in GMP grade, and therefore, qualified for therapeutic use.

Without wishing to be bound by theory, the utilization of the OptiPrep density gradient centrifugation step, e.g., with thawed apheresis material, is believed to be less likely to retain undesirable B cells and monocytes, thus is believed to further improve the collection of desired target immune effector cells, e.g., T cells, for subsequent activation and transduction steps. Accordingly, without wishing to be bound by theory, it is believed that the greater density of OptiPrep as compared to Ficoll allows both an enhanced purification and recovery of desired immune effector cells, e.g., T cells, and the concomitant removal of undesirable non-T cell types which can otherwise interfere with consistently successful outcomes of CAR-expressing immune effector cell, e.g., T cell, product manufacturing.

In one embodiment, the density gradient centrifugation is performed using a cell separation device. Examples of cell separation devices include the Sepax2 (Biosafe). In embodiments where a wash step, e.g., an improved wash step as described herein, is performed, e.g., prior to or after the density gradient centrifugation step, the wash step can be performed using the same device as used in the density gradient centrifugation step.

### Enrichment by Selection

Provided herein are methods for selection of specific cells to improve the enrichment of the desired immune effector cells suitable for CAR expression. In one embodiment, the selection comprises a positive selection, e.g., selection for the desired immune effector cells. In another embodiment, the selection comprises a negative selection, e.g., selection for unwanted cells, e.g., removal of unwanted cells. In embodiments, the positive or negative selection methods described herein are performed under flow conditions, e.g., by using a flow-through device, e.g., a flow-through device described herein.

Current selection methods, e.g., positive selection, e.g., using Dynabeads^{®} CD3/CD28 CTS^{™}, can be further optimized for enrichment. First, the amount of Dynabeads used during the selection is typically not based on the percentage of CD45+/3+ cells, e.g., CD45+/3+ cells, present in the post-density gradient centrifugation, e.g., Sepax Ficoll, sample, but rather is based on the percentage of cells, e.g., CD45+/3+ cells, present in the original patient material. Given the significant change in composition caused by the density gradient centrifugation step, e.g., Sepax Ficoll separation procedure, this calculation typically results in a decrease in T cell percentage and increase in monocyte content. Second, the two hour incubation time provides ample opportunity for both non-specific binding of Dynabeads^{®} onto non-target cells (i.e., non CD3 and /or non CD28 cells) and for bead uptake by non-target cells via endocytosis (e.g., monocytes), issues which can compromise T cell yield and purity in the positive selection product. Finally, the magnetic apparatus and operation used the selection can be sub-optimal. Currently, magnetic separation occurs within a large volume of fluid (200ml), which in turn results in a large distance between magnetically-labeled cells and the magnetic surfaces. This limits the magnetic force available for separation, hence reducing separation sensitivity and requiring longer separation times. In addition, magnetic separation is currently performed statistically, with the sample placed on top the magnetic surface for 5 minutes prior to removal of the negative fraction. Such an extended separation time is detrimental to cells, whose viability is often negatively affected during the procedure due to "pile-up" effects, and provides further opportunity for non-target cells to either bind or internalize the beads.

In contrast to the current selection methods, the selection methods described herein includes a separation that occurs "dynamically", e.g., under flow conditions, as opposed to the current "static" separation procedure. In an embodiment, separation under flow conditions comprises a magnetic separation reagent, e.g., magnetic beads that selectively bind a target antigen, an input sample, and a magnet, wherein the magnetic separation reagent and the input sample pass, e.g., flow, over a magnet. In certain embodiments, the magnetic separation reagent and the input sample pass, e.g., flow, over the magnet in continuously. Without being bound by theory, this dynamic technique enables the reduction of incubation time (i.e., contacting the sample with the separation reagent) and separation time, thus minimizing negative impacts on target cells and significantly reducing the likelihood of non-specific binding and\or bead uptake by non-target populations. In addition, the selection methods described herein do not require any modification to selection reagents (Dynabeads^{®} CD3/CD28 CTS^{™}) or to the amount of reagents used for the selection (3-to-1 bead-to-Tcell ratio).

In an embodiment, the separation or selection under flow conditions, as described herein, comprises the Flow-through Antibody-based Selection Technique (FAST) protocol. **Table 12** exhibits an overview of the parameters that differ between the current selection technique and the FAST protocol.

**Table 12: Comparison between current positive selection and FAST positive selection**

| **Unchanged parameters** | **Modified parameters** |
|---|---|
| Reagents (CD3/28 Dynabeads CTS) | Incubation time (from 2hrs to 20min) |
| Bead-to-T cell ratio (3:1) | Flow-through enrichment kit rather than static configuration (Figure 20) |
| Magnetic plate (DynaMag CTS, flatbed magnet) | Plastic lid for magnetic plate (see Figure 21) |

In one embodiment, the selection method described herein comprises a shorter incubation period than current standard protocols of the separation reagent and the input sample, followed by magnetic separation. In one embodiment, the incubation period is less than 2 hours, e.g., less than 110 minutes, less than 100 minutes, less than 90 minutes, less than 80 minutes, less than 70 minutes, less than 60 minutes, less than 50 minutes, less than 40 minutes, less than 30 minutes, less than 25 minutes, less than 20 minutes, less than 15 minutes, less than 10 minutes, or less than 5 minutes. In one embodiment, the incubation is performed under gentle rotation.

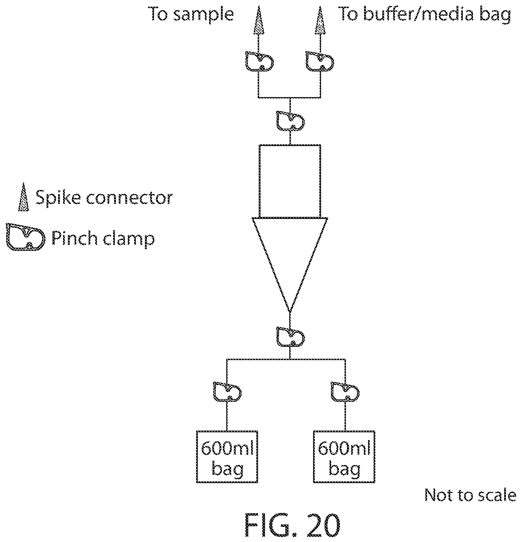

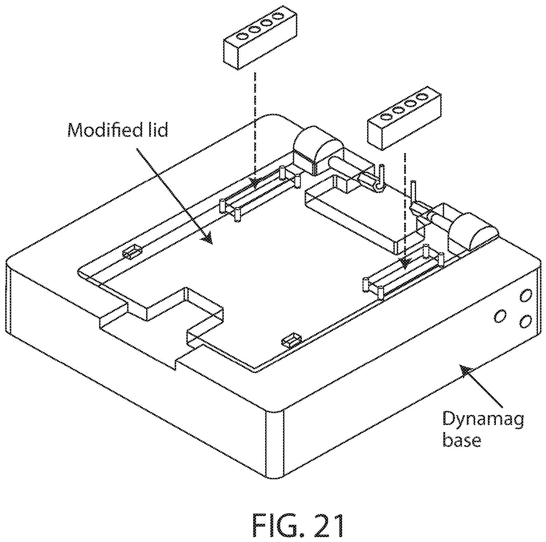

An exemplary kit for the selection method described herein is shown in **Figure 20****.** In one embodiment, the kit comprises of an assembly of three bags which can be connected to additional sample/buffer bags via spikes, or through sterile welding. In addition, a modified DynaMag lid, shown in **Figure 21****,** is employed in the separation, which limits the maximum volume during separation to a low volume, e.g., less than 100 mL, less than 90 mL less than 80 mL, less than 70 mL, less than 60 mL, less than 50 mL, less than 40 ml, e.g., about 50ml. Without wishing to be bound by theory, the low volume used during separation is believed to optimize the magnetic forces acting during the separation procedure and minimize separation times. The modified DynaMag lid also limits the maximum distance that bead:cell conjugates are displaced from the magnet, and standardizes the magnetic for experienced during position selection.

In one embodiment, one or more of the bags of the kit described herein is a triangular bag. In one embodiment, the selection bag is a triangular bag, and enables magnetic separation in "flow-through" mode, as the bag provides ports at opposite ends of the selection bag. In such embodiments, cells can be continuously flown over a magnetic element (e.g., a magnetic plate such as the DynaMag), thus enabling real-time separation of magnetically-labeled particles, while non-labeled cells will not be attracted by the magnetic field and will flow outwards. This flow-through configuration makes the system particularly amenable to automation. The modified separation bag requires the modified lid of **Figure 21** to accommodate the additional ports.

In one embodiment, the selection bag is not a triangular bag. In the embodiment where the selection bag is not a triangular bag, the incubation time is less than 2 hours, e.g., less than 110 minutes, less than 100 minutes, less than 90 minutes, less than 80 minutes, less than 70 minutes, less than 60 minutes, less than 50 minutes, less than 40 minutes, less than 30 minutes, less than 25 minutes, less than 20 minutes, less than 15 minutes, less than 10 minutes, or less than 5 minutes.

### Positive Selection

In embodiments, the positive selection methods described herein comprise selecting for, e.g., enriching, the desired immune effector cells. In one embodiment, the positive selection methods described herein comprise selecting for CD3+/CD28+ cells. In other embodiments, the positive selection methods described herein comprise selecting one or more of the following: CD3+ cells, CD28+ cells, CD4+ cells, CD8+ cells, or CD45+ cells.

Separation reagents used in the selection methods described herein comprises a magnetic or paramagnetic member, and an antigen binding member. In one embodiment, the separation reagent comprises a bead, e.g., having magnetic or paramagnetic properties that is coupled to (e.g., covalently, or non-covalently) to an antigen binding member. In one embodiment, the antigen binding member is an antibody or antibody fragment thereof. In one embodiment, the separation reagent used in positive selection for CD3+/CD28+ cells comprises a bead that is coupled to (e.g., covalently, or non-covalently) to a CD3 and/or CD28-binding member, e.g., an anti-CD3 and/or anti-CD28 antibody or antibody fragment.

### Negative Selection

Also provided herein are negative selection methods for negatively selecting for, or depleting, the input sample of unwanted cells, e.g., monocytes, granulocytes, red blood cells, platelets, and B cells, thereby enriching the resulting output sample with the desired immune effector cells, e.g., T cells. In an embodiment, the negative selection methods described herein are performed under flow conditions, e.g., using a flow through device, e.g., a flow through device described herein.

In one embodiment, the negative selection methods described herein comprise negatively selecting for one or more of monocytes, granulocytes, red blood cells, platelets, B cells, or cancer cells, e.g., lymphoblasts.

In embodiments where depletion or removal of one or more of monocytes, granulocytes, red blood cells, platelets, or B cells is desired, the negative selection method selecting for a cell expressing one or more of the following: CD19, CD25, CD14, or other surface marker or protein expressed by a monocyte, granulocyte, red blood cell, platelet, or B cell.

In embodiments where the subject has a hematological cancer, cancer cells may be present in the apheresis samples, and removal of the cancer cells may be desired. In one embodiment, the negative selection method described herein comprises negatively selecting for a CD19+ cell, e.g., a lymphoblast. In another embodiment, the negative selection method described herein comprises negatively selecting for a cancer cell expressing one or more of the following: CD19, CD33, CD123, CLL-1, BCMA, ROR1, or FLT3.

Separation reagents used in the selection methods described herein comprises a magnetic or paramagnetic member, and an antigen binding member. In one embodiment, the separation reagent comprises a bead, e.g., having magnetic or paramagnetic properties that is coupled to (e.g., covalently, or non-covalently) to an antigen binding member. In one embodiment, the antigen binding member is an antibody or antibody fragment thereof. In one embodiment, the separation reagent used in negative selection for CD19+ cells comprises a bead that is coupled to (e.g., covalently, or non-covalently) to a CD19-binding member, e.g., an anti-CD19 antibody or antibody fragment. In one embodiment, the separation reagent used in negative selection for CD14+ cells comprises a bead that is coupled to (e.g., covalently, or non-covalently) to a CD14-binding member, e.g., an anti-CD14 antibody or antibody fragment. In one embodiment, the separation reagent used in negative selection for CD25+ cells comprises a bead that is coupled to (e.g., covalently, or non-covalently) to a CD25-binding member, e.g., an anti-CD25 antibody or antibody fragment.

### Exemplary Flow-Through Device

As described herein, selection methods can be performed under flow conditions, e.g., by using a flow-through device, also referred to as a cell processing system, to further enrich a preparation of cells for desired immune effector cells, e.g., T cells, suitable for CAR expression. An exemplary flow-through device for use in such selection methods under flow conditions is described in this section.

In one embodiment, the cell processing system including at least one cell suspension module; at least one buffer module; at least one flow-through magnetic separation/debeading module; at least one non-magnetic output module; and at least one magnetic output module.

In some variations of this system, it may include at least one return loop returning upstream of at least one flow-through magnetic separation/debeading module; at least two flow-through magnetic separation/debeading modules in parallel; at least two flow-through magnetic separation/debeading modules in series, at least one additional module, or any combinations thereof. The at least one additional module may include at least one spinning membrane debeading module; at least two spinning membrane debeading modules in parallel; or at least two spinning membrane debeading modules in series. Any of the spinning membrane debeading modules may include at least one magnet adjacent or proximate to a cylindrical side-wall.

In a more specific variation, the flow-through magnetic separation/debeading module includes a chamber defined by walls and having an x-direction, a y-direction, and a z-direction; an inlet and an outlet arranged on opposite ends of the chamber in the y-direction; and at least two magnets adjacent or proximate a wall of the chamber and arranged to establish a zero gradient line within the chamber between the inlet and the outlet.

In another more specific variation, which may stand alone or be combined with the first more specific variation, the spinning membrane debeading module includes a debeading chamber define partially by a cylindrical side-wall; a porous spinning membrane having an interior and oriented co-axially with the cylindrical side-wall; a sample inlet; a waste output module connected to the interior of the spinning membrane; and a cell output module connected to the debeading chamber.

In another embodiment, the flow-through magnetic separation/debeading module includes a chamber defined by walls and having an x-direction, a y-direction, and a z-direction; an inlet and an outlet arranged on opposite ends of the chamber in the y-direction; and at least two magnets adjacent or proximate a wall of the chamber and arranged to establish a zero gradient line within the chamber between the inlet and the outlet.

In some variations of this module, it includes at least two inlets and at least two outlets; at least three magnets adjacent or proximate a wall of the chamber and arranged to establish at least two zero gradient lines within the chamber between the inlet and the outlet; at least four magnets arranged in two arrays on opposite sides of the chamber in the z-direction; at least four magnets arranged in two arrays on opposite sides of the chamber in the z-direction and cross-oriented in the x-y plane from near one inlet to near one outlet on the opposite side of the chamber in the z-direction; a sub-membrane injection ports adjacent a wall of the chamber also adjacent at least two magnets and a membrane adjacent the sub-membrane; or any combinations thereof.

In another embodiment, a spinning membrane debeading module includes a debeading chamber define partially by a cylindrical side-wall; a porous spinning membrane having an interior and oriented co-axially with the cylindrical side-wall; a sample inlet; a waste output module connected to the interior of the spinning membrane; a cell output module connected to the debeading chamber; and at least one magnet adjacent or proximate to the cylindrical side-wall.

In some variations of this module, it may include a reagent module, have a pore size greater than that of a particle to be debeaded and less than that of a cell to be debeaded, or both.

In yet another embodiment, the a method of flow-through cell processing includes flowing a cell suspension comprising paramagnetic particle-bound cells through a flow-through magnetic separation/debeading module to produce an unbound cell product. The paramagnetic particle-bound cells continue to move in the flow-through magnetic separation/debeading module through the flowing step. The flow-through magnetic separation/debeading module includes a flow chamber defined by walls through which the cell suspension flows and at least two magnets arranged adjacent or proximate at least one wall.

In some variations of this method, the cell suspension is flowed laminarly through the flow-through magnetic separation/debeading module; the cell suspension further includes unbound cells and flowing the cell suspension through the flow-through magnetic separation/debeading module separates the paramagnetic particle-bound cells and the unbound cells; the cell suspension further includes free paramagnetic particles and flowing the cell suspension through the flow-through magnetic separation/debeading module separates the free paramagnetic particles and the unbound cells, or any combinations thereof.

The methods described herein may also include flowing the separated unbound cells through the flow-through magnetic separation/debeading module a second or subsequent time using a return loop; flowing the separated paramagnetic particle-bound cells through the flow-through magnetic separation/debeading module a second or subsequent time using a return loop; debeading the paramagnetic particle-bound cells in the flow-through magnetic separation/debeading module during the second or subsequent time to produce paramagnetic particles and debeaded, unbound cells; flowing the produced paramagnetic particles and debeaded, unbound cells through the flow-through magnetic separation/debeading module a third or subsequent time to separate the paramagnetic particles and the debeaded, unbound cells, or any combinations thereof.

In another variation, combinable with all others, the magnets are oriented to establish one zero gradient line that crosses the direction of flow, such that paramagnetic-particle bound cells are pulled to the zero gradient line in one direction only, but are not affected by magnetic forces from the magnets in two other directions.

In another variation, combinable with all others, the chamber further includes a magnetic inlet through which any paramagnetic particles enter the flow chamber; a non-magnetic inlet; a magnetic outlet opposite the non-magnetic inlet; and a non-magnetic outlet opposite the magnetic inlet, wherein the zero gradient line directs all paramagnetic particles and any paramagnetic particle-bound cells to the magnetic outlet.

The cell suspension may further include unbound cells and the non-magnetic inlet may be larger than the magnetic inlet while the non-magnetic outlet is larger than magnetic outlet, so that fluid flowing from the non-magnetic inlet crosses over to the non-magnetic outlet, preventing any unbound cells from flowing into the magnetic outlet.

Alternatively, the cell suspension may further include unbound cells, and the non-magnetic inlet and magnetic inlet may be substantially the same size or the non-magnetic outlet and magnetic outlet may be substantially the same size, or both, and the respective flow rates of the fluid entering the inlets, the respective flow rates of the fluid exiting the outlets, or both may be adjusted such that fluid flowing from the non-magnetic inlet crosses over to the non-magnetic outlet, preventing any unbound cells from flowing into the magnetic outlet.

In another variation, combinable with all others, the method may include flowing the paramagnetic particle-bound cells through a spinning membrane debeading module to produce the unbound cell product. The spinning membrane debeading module may include a cylindrical debeading chamber through which the paramagnetic particle-bound cells flow, the chamber defined in part by a cylindrical side-wall and containing a co-axial spinning membrane; and at least one magnet arranged adjacent or proximate the cylindrical side-wall to establish at least one zero gradient line within the cylindrical debeading chamber.

The present disclosure relates to systems and methods for flow-through separation, debeading, paramagnetic particle separation, or any combination thereof of paramagnetic particle-bound cells or unbound cells in the presence of paramagnetic particles, in a cell suspension. The systems and methods use a flow-through magnetic separation/debeading module, a spinning membrane debeading module, or both. Although the systems and methods described herein may be used to remove paramagnetic particles from any type of cell, they are particularly well-adapted for use in removing paramagnetic particles from cells to be used in cell therapy. In addition, although some portions of the description focus on positive selection of paramagnetic particle-bound cells, as debeading is typically only performed during positive selection methods, the systems and methods may also be used for negative selection. When used for negative selection, typically any debeading modules and steps will be eliminated.

### Separation and Debeading Systems and Modules

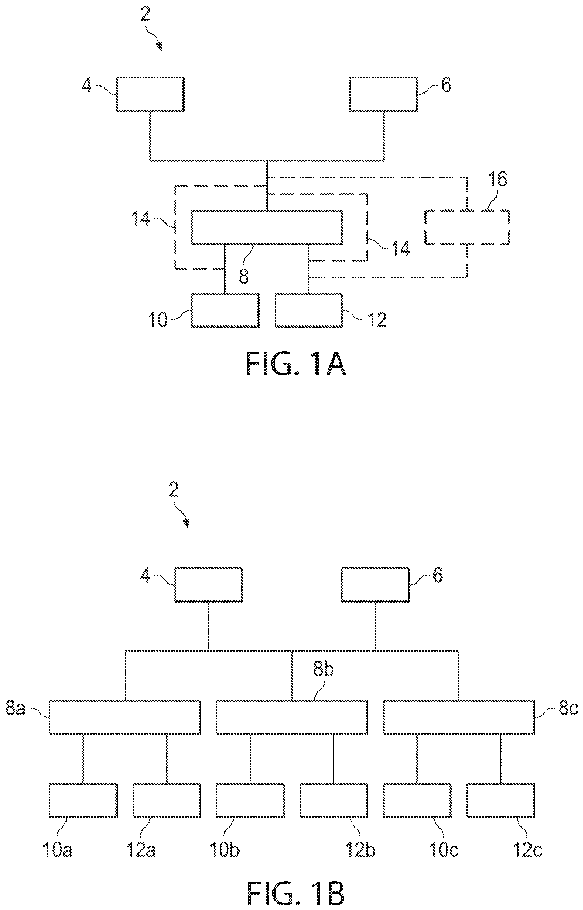

FIG. 1A is a schematic diagram of a cell processing system 2 for flow-through separation, debeading, paramagnetic particle separation, or any combination thereof of paramagnetic particle-bound cells in a cell suspension. System 2 includes cell suspension module 4, buffer module 6, flow-through magnetic separation/debeading module 8, non-magnetic output module 10, and magnetic output module 12. System 2 may optionally also include at least one additional module 16, at least one return loop 14, or both.

System 2 additionally may include fluid conduits, such as tubes or hoses, connectors, valves, switches, clamps, weld sites, housings, motors, pumps, other mechanical mechanisms, circuitry, monitoring devices, and control devices. System 2 may further include a computer programmed to control system 2 or any component thereof to perform a flow-through separation process, a flow-through debeading process, a flow-through paramagnetic particle separation process, or any combination thereof.

System 2 may have a static configuration, or it may have an adaptable configuration. One adaptable configuration may allow the exchange of modules or the insertion of additional modules. Another adaptable configuration may have an unchangeable set of modules, but may allow changes in fluid routing to at least one of the modules. Other adaptable configurations may allow both exchange and addition of modules as well as changes in fluid routing. Components of system 2 may facilitate the adaptable configuration. For instance, a programmed computer in system 2 may detect or use information regarding which modules are present or it may control fluid routing. In addition, system 2 may be have housings or fluid conduits with accompanying connectors, valves, clamps, or switches that allow removal or insertion of different modules in the same location. Modules or other components may contain identification elements, such as bar codes or radio frequency identification (RFID) chips, to allow their presence or absence to be automatically detected. Modules or other components may also contain one or more indication elements, which may ensure compliance with good manufacturing practices and other safety regulations. For instance, temperature sensitive indication elements may indicate that a module or other component has been heat sterilized or not subjected to a temperature that may compromise its integrity or effectiveness. Indication elements may also clearly identify used modules or other components. Indication elements may also be automatically detected by system 2, helping to minimize human error.

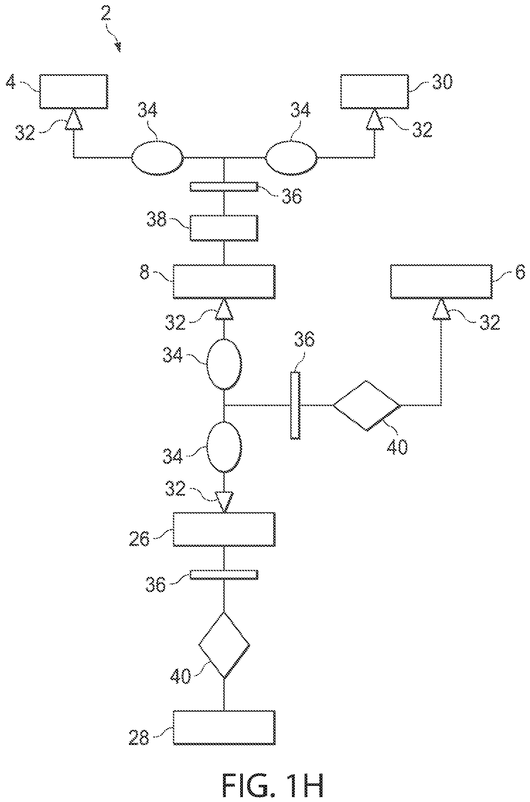

Components of system 2 not needed for a particular process may be absent, unconnected, or closed. For instance, a single output module may be present rather than a separate non-magnetic output module 10 and magnetic output module 12, as shown in FIG. 1H. In addition, also as illustrated in FIG. 1H, the components of system 2 may have fluid conduits with different routes and connections than as shown in FIG. 1A, depending on the configuration of valves, clamps, weld sites, switches, and connectors.

Cell suspension module 4 contains cells to be separated or debeaded suspended in a suspension fluid. If the cells are to be separated, then typically the cell suspension contains both paramagnetic particle-bound cells and unbound cells. The paramagnetic particle-bound cells may be the desirable cells, in which case positive selection for paramagnetic particle-bound cells will occur in system 2, or the paramagnetic particle-bound cells may be undesirable, in which case negative selection for the paramagnetic particle-bound cells will occur.

If the cells are to be debeaded, or if paramagnetic particles are to be separated from the cells, then the paramagnetic particle-bound cells are desirable cells. They may have previously been separated from undesirable cells using system 2 or another system. In instances where the presence of undesirable cells is not problematic or where there are no undesirable cells to separate, the cells to be debeaded may not have previously undergone a separation process.

The cells may be obtained directly from a biological sample, such as blood, or from a cell culture.

The suspension fluid may be any fluid able to support viability of the cells throughout the separation, debeading, or particle removal process. For instance, it may be a culture medium, a freezing agent, such as a DMSO-containing fluid, another fluid with a set or controlled pH, or another fluid with nutrients. It may also be a buffer, which may be the same as or different from the buffer in buffer module 6. The suspension fluid may have a different viscosity than the buffer. It may also have a different viscosity than the medium in which the cells enter system 2, which may be very dense, such as high density Ficoll.

The buffer in buffer module 6 may be any fluid that may be combined with the suspension fluid while allowing the suspension fluid to continue to support viability of the cells. For instance, the buffer may have a set or controlled pH. The buffer may include one or more cell-compatible salts. Although buffer is provided separately in buffer module 6, once the buffer mixes with the cell suspension, it is considered to be part of the suspension fluid.

The suspension fluid or buffer may contain antimicrobial agents, but typically will not if the cells will later be provided to a patient unless system 2 removes these agents, such as via a spinning membrane debeading module or another module, or unless they are removed later by an additional process, module, or system.

The paramagnetic particles may be formed from any paramagnetic and/or magnetizable material, such as a metal or metal alloy. Typically the paramagnetic material is not toxic to the cells or to any patient who will later receive the cells, or it is coated to avoid toxicity. The paramagnetic material may be selected to achieve a high magnetic saturation flux (mₛ). In general, which paramagnetic materials are suitable is influenced by the magnets used in system 2 and the configuration of modules using the magnets, as these elements influence driving the paramagnetic material to magnetic saturation.

The paramagnetic particles may be coated with a binding agent, such as a growth agent, a receptor or ligand, an antigen, an antibody, or any binding fragments or chimeric variants thereof, such as a chimeric antigen receptor ligand. The binding agents may be reversible in some instances, allowing detachment of the paramagnetic particles spontaneously or using a particular chemical agent. The binding agents may also include a photo-cleavable linker, in which case system 2 may include a light source, particularly a high power light source, as a module or as part of another module to allow photo-cleavage of the linker and separation of the cell and paramagnetic particle. In some instances, the coating may interact with the cells. In other instances, the coating may interact with at least one unwanted constituent of the cell suspension that is to be removed. The unwanted constituent may be active or inactive and may have previously served a useful function with respect to the cells or the cell suspension fluid. Example unwanted constituents include antibodies, growth factors, other proteins, and polymers.

Different types of paramagnetic particles, such as particles with different binding agents or formed from different magnetic materials may be present in some cell suspensions, allowing for complex separations or iterative removal of binding agents. Additional, non-paramagnetic particles, which may also be coated with any binding agent, may also be bound to cells.

Other particles that are not paramagnetic may also be present in the cell suspension and may be coated with anything used to coat the paramagnetic particles.

Modules may be formed from or lined with any biologically compatible material such as cell storage bags. Fluid conduits and any other component of system 2 that contacts the cell suspension or buffer may also be formed from or lined with any biologically compatible material.

Components of system 2 that will contact the cell suspension or buffer may be sterile prior to contact with the cell suspension.

Components of system 2 may be disposable. Components that contact the cell suspension, in particular, may be disposable to avoid contamination and sterility concerns.

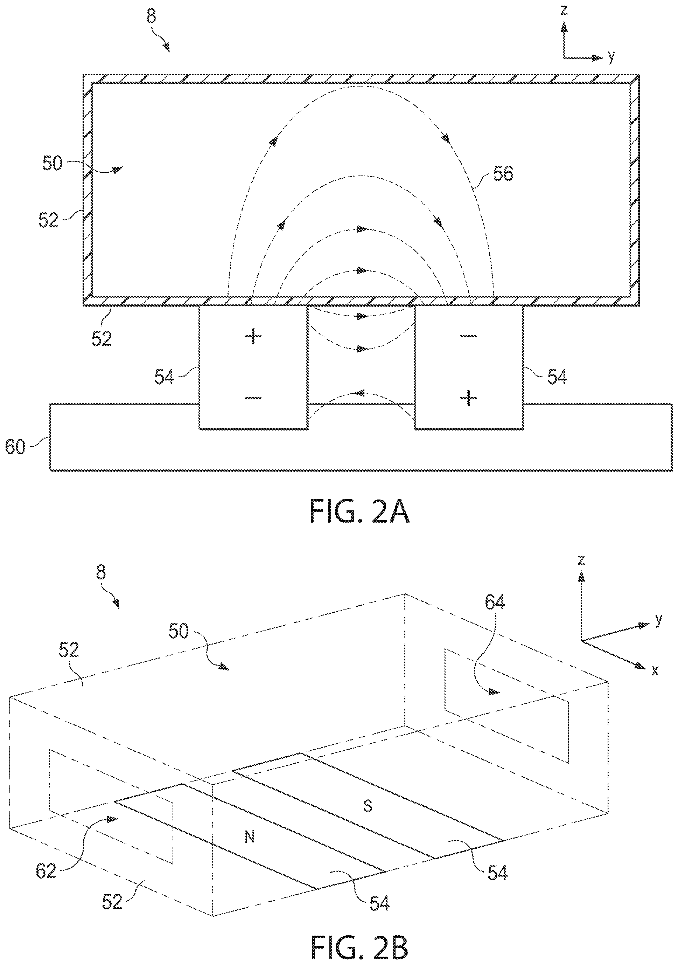

FIG. 2A is a transverse cross-sectional schematic diagram of flow-through magnetic separation/debeading module 8 in an x-oriented magnet configuration, while FIG. 2B is a semitransparent, three-dimensional schematic diagram of the same magnetic separation module 8 in the same configuration. Flow-through magnetic separation/debeading module 8 includes a flow chamber 50, defined by walls 52. External dipole magnets 54 create magnetic force lines 56. Magnets 54 are housed on movable platform 60. Flow-through magnetic separation/debeading module 8 further includes inlet 62 and outlet 64 through which a cell suspension may be flowed in the y direction through module 8.

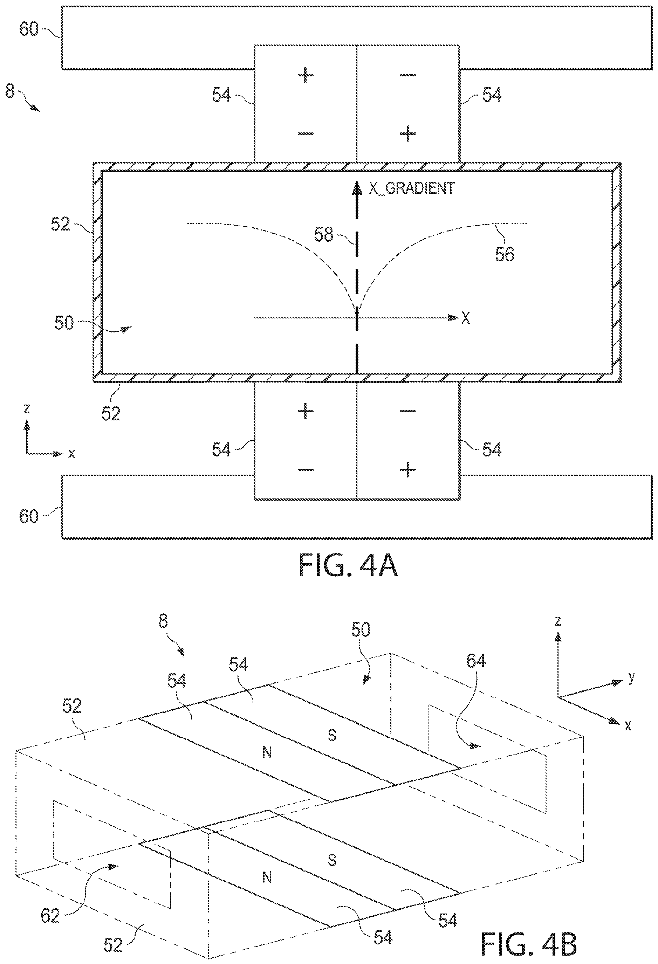

Although FIGs. 2A and 2B depict one array of magnets 54, flow-through magnetic separation/debeading module 8 may have two or more arrays, as shown in FIG. 4A, and may have more than two magnets in an array. In addition, magnets 54 may be in a permanent position, in which case separating/debeading module 8 may lack movable platform 60 or may have a movable flow chamber 50. Furthermore, although magnets 54 are shown in an x-oriented configuration, they can be at any angle in the x-y plane, including a y-orientation or an x-y cross-orientation.

Inlet 62 and outlet 64 may have any configuration sufficient to establish laminar flow of the cell suspension through chamber 50. Inlet geometry, outlet geometry, and flow rate all influence the flow of the cell suspension through chamber 50. Turbulent flow may be acceptable in some instances.

Walls 52 may be rigid structures, or they may be flexible. For instance, they may be the walls of a cell storage bag or other similar component. When walls 52 are flexible, the dimension of chamber 50 in the z direction may vary depending on the flow rate of the cell suspension through chamber 50.

The dimension of chamber 50 in the z direction may be between 5 µm and 100 µm, between 5 µm and 500 µm, or between 5 µm and 1000 µm, between 5 µm and 1 cm, or generally 100 µm, 500 µm, 1000 µm, or 1 cm or less. The dimensions in both the x, y, and z directions may be limited to achieve fluidic forces that are sufficiently high to move cells or paramagnetic particles through chamber 50.

Magnets 54 may have a high magnetic field strength. For instance, they may contain rare earth metals, such as neodymium or samarium alloyed with another metal, such as cobalt. Magnets 54 may be dipole magnets as depicted, or they may be other types of magnets, such as quadrapole magnets. Magnets 54 may have an adjustable magnetic field strength. For example, they may be electromagnets. Magnets 54 may be arranged to maximize magnetic attraction for magnets on the same side of chamber 50, to maximize magnetic repulsion for magnets on opposite sides of chamber 50, or both. Although FIG. 2A depicts a particular magnet configuration, configurations in which magnet polarity is opposite or concordant may be used depending upon the effect to be achieved.

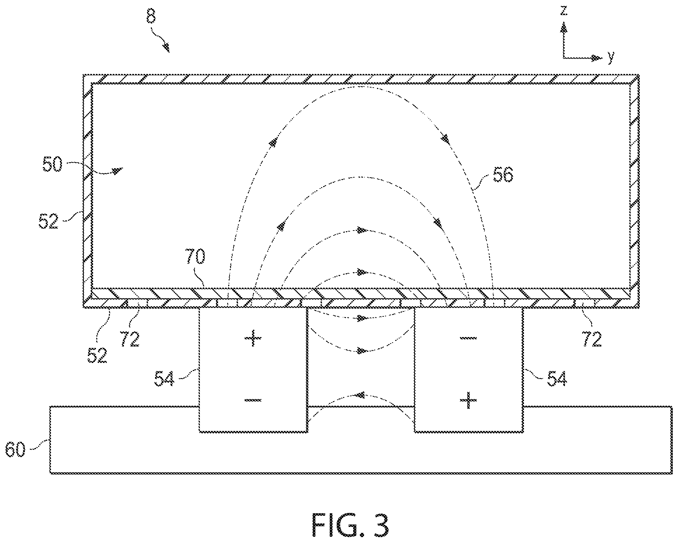

FIG. 3 is a side longitudinal cross-sectional schematic diagram of a flow-through magnetic separation/debeading module 8 with membrane 70 located above sub-membrane fluid injection ports 72 and magnets 54. Fluid from buffer module 6 or another fluid module may be introduced through fluid injection ports 72 to help debead cells located near membrane 70.

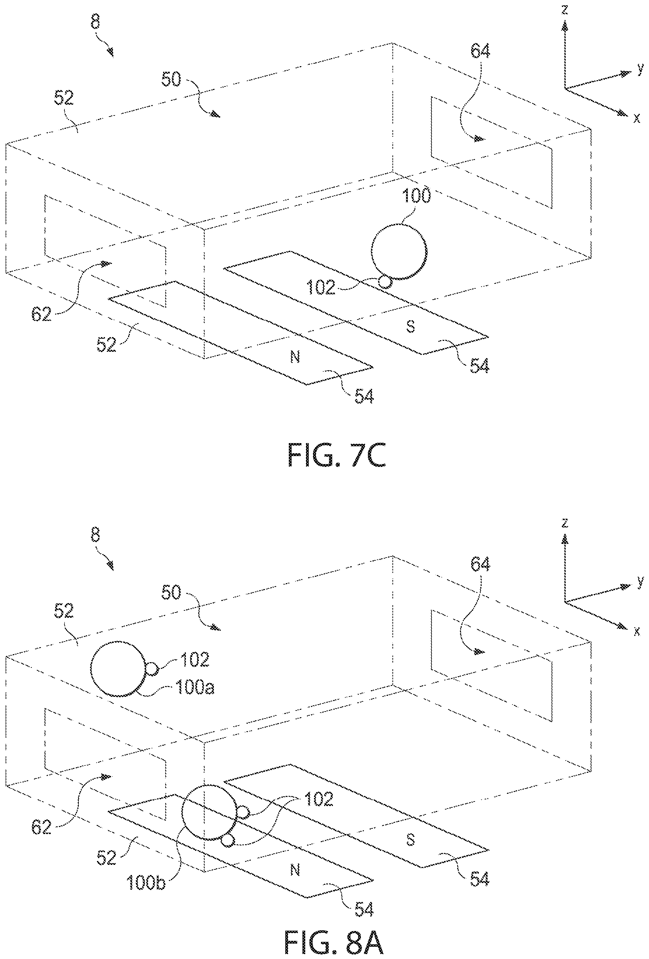

Movable platform 60 may be movable in the z direction allowing the movement of magnets 54 in the z direction from a position adjacent to chamber 50 as shown in FIGs. 2A and 2B, or proximate chamber 50 (not shown), to a position distant from chamber 50 (as shown in FIG. 7C). For example, the position distant from chamber 50 may be at least 1 cm from the nearest wall 52. The position distant is sufficient to prevent any substantial influence of magnets 54, via their magnetic fields, on any paramagnetic particle in chamber 50. The position distance may be substantially less if a magnetically insulating material is inserted between magnets 54 and chamber 50. If the magnets 54 have an adjustable magnetic field strength, rather than being moved, they may simply be adjusted to a lower magnetic field strength or zero magnetic field strength to avoid any substantial influence on any paramagnetic particles in chamber 50.

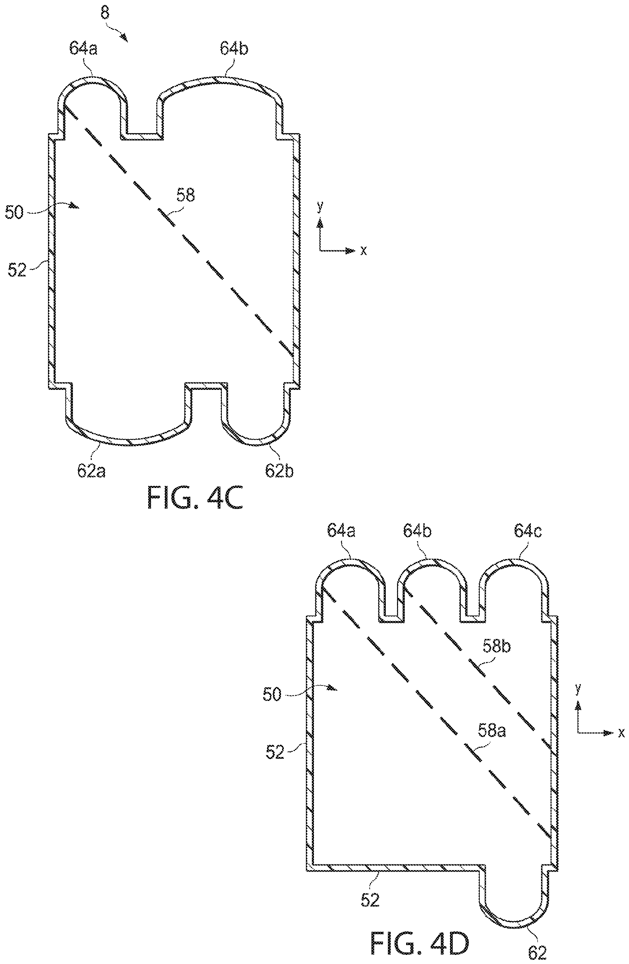

Particularly when using a zero-gradient configuration, module 8 may have a top array of magnets 54 and a bottom array of magnets 54, as depicted in FIGs. 4A and 4B. Movable platform 60 may be rotatable in the x-y plane, or magnets 54 may be permanently oriented such that magnets 54 are in an x-y cross-oriented configuration, such as that depicted in the top longitudinal cross-sectional schematic diagram of a flow-through magnetic separation/debeading module 8 of FIG. 4C. For use in a zero-gradient configuration, flow-through magnetic separation/debeading module 8 may have two inlets 62, a non-magnetic inlet 62a and a magnetic inlet 62b as well as two outlets 64, a magnetic outlet 64a and a non-magnetic outlet 64b. In this instance, the zero gradient line 58 in an x-y cross-oriented direction forms a zero gradient filter when module 8 is in use. Inclusion of additional magnets 54 may provide two zero gradient lines, 58a and 58b in the same module 8, as illustrated in FIG. 4D, allowing the separation of different paramagnetic particles into different outlets 64a and 64 b, or providing a back-up filter.

Zero gradient line 58 may be a zero gradient band, having a dimension in the x direction, if magnets 54 are spaced sufficiently apart from one another rather than being adjacent as depicted in FIGs. 2A and 2B.

Magnets for use with a flow-through magnetic separation/debeading module may be located external to the chamber that the cell suspension flows through, or internal to the chamber. If the magnets are internal, they may be coated with a biocompatible material. Particularly if the magnets are internal, they may be disposable.

FIG. 1B is a schematic diagram of a cell processing system 2, which includes a plurality of flow-through magnetic separation/debeading modules 8a, 8b and 8c in parallel. Although only three flow-through magnetic separation/debeading modules 8 are illustrated, the plurality may be any number greater than two. When flow-through magnetic separation/debeading modules 8 are in parallel, the modules will typically be of the same type and in the same configuration so that the same function is performed by each. Parallel flow-through magnetic separation/debeading modules 8 may be particularly useful for rapid cell suspension processing or management of fluid volume when combined with additional modules. In addition, placing flow-through magnetic separation/debeading modules 8 in parallel provides flexibility in controlling fluid flow, as the modules need to all be used at the same time.

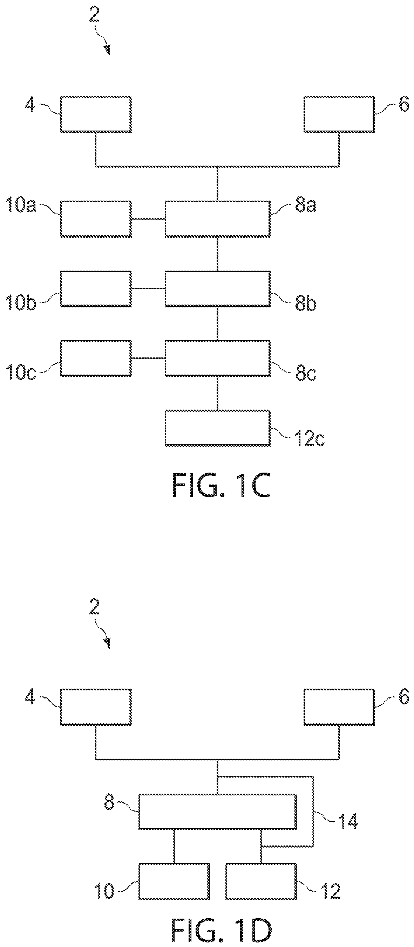

FIG. 1C is a schematic diagram of a cell processing system 2, which includes a plurality of flow-through magnetic separation/debeading modules 8a, 8b and 8c in series. Although only three flow-through magnetic separation/debeading modules 8 are illustrated, the plurality may be any number greater than two. When flow-through magnetic separation/debeading modules 8 are in series, they may be of the same type and configuration so that the same function is performed by each, but, typically, they will be of different types and configurations so that different functions are performed by each. For instance module 8a may separate paramagnetic particle-bound cells and unbound cells, module 8b may debead paramagnetic particle-bound cells, and module 8c may debead paramagnetic particle-bound cells under greater magnetic field gradients.

FIG. 1D is a schematic diagram of a cell processing system 2 in which return loop 14 directs paramagnetic particle-bound cells back through flow-through magnetic separation/debeading module 8. Such a system may be used to achieve better separation of paramagnetic particle-bound cells and unbound cells, or better debeading or separation of unbound cells and paramagnetic particles as compared to a similar system with no return loop 14. Fluid may be directed to return loop 14 or to magnetic output module 12 by a valve or switch.

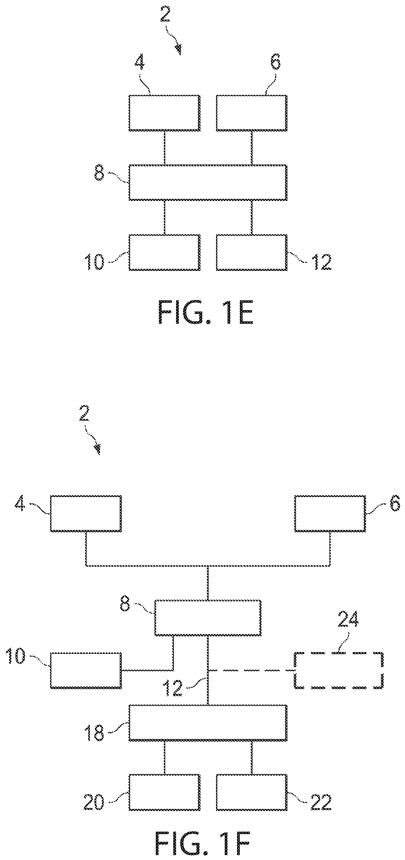

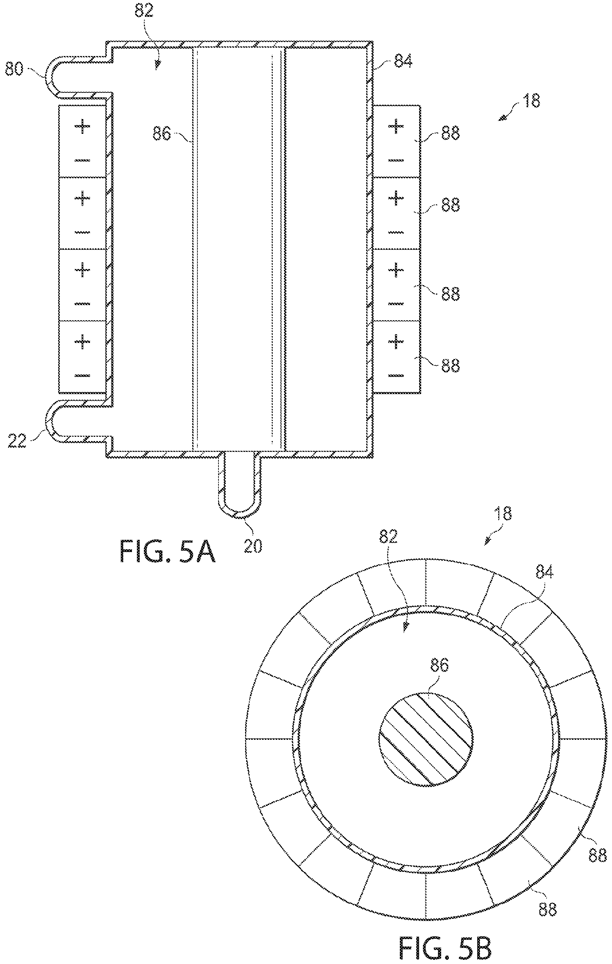

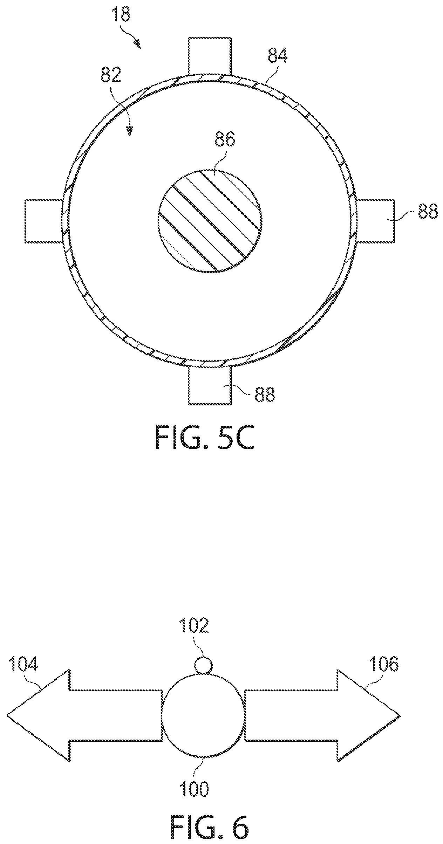

FIG. 1E is a schematic diagram of a cell processing system 2 in which cell suspension module 4 and buffer module 6 are separately connected to flow-through magnetic separation/debeading module 8. Such a system may be particularly useful when flow-through magnetic separation/debeading module 8 has two inlets 62 and two outlets 64 and magnets 54 in an x-y cross-oriented configuration as shown in and described with respect to FIG. 5.

FIG. 1F is a schematic diagram of a cell processing system 2 with a spinning membrane debeading module 18 located downstream of flow-through magnetic separation/debeading module 8. Spinning membrane debeading module 18 is connected to waste output module 20 and cell output module 22. In this system 2, flow-through magnetic separation/debeading module 8 separates paramagnetic particle-bound cells and unbound cells, while a spinning membrane debeading module 18 conducts all debeading, or flow-through magnetic separation/debeading module 8 may conduct debeading as well. Reagent module 24 may optionally be present if a reagent, such as a chemical agent, is added to the suspension fluid in spinning membrane debeading module 18. Although one spinning membrane debeading module 18 is illustrated in FIG. 1F, system 2 may include a plurality of modules 18 in series or in parallel. When modules 18 are in series, a chemical agent may only be added to the last module 18 to minimize cell exposure to the chemical agent.

The reagent in reagent module 24 may be any chemical agent that weakens the bond between a particle and a cell. The particle may be the paramagnetic particle, or it may be a non-paramagnetic particle.

FIG. 5A is a longitudinal cross-sectional schematic diagram of a spinning membrane debeading module 18. This module 18 includes sample inlet 80, which allows a cell suspension fluid to flow into cylindrical debeading chamber 82, which is defined in party by cylindrical side-wall 84 and contains co-axially oriented cylindrical spinning membrane 86. Wall 84 is lined on the exterior with magnets 88. Debeading chamber 18 allows fluid that has passed through spinning membrane 86 to exit via waste output module 20, while the remaining fluid and cells exit through cell output module 22. Spinning membrane 86 has an average pore size smaller than the average diameter of the cells, but larger than any non-paramagnetic particle to be removed by debeading. The average pore size may also be larger than any paramagnetic particle to be removed, allowing removal of these paramagnetic particles by the spinning membrane either as a primary particle removal method, or as a back-up to magnetic removal.

Magnets 88 may substantially surround wall 84 as shown in FIG. 5B, or they may be spaced at intervals along wall 84, as shown in FIG. 5C. Magnets 88 may be mounted on a movable platform to allow them to be moved from a position adjacent to wall 88, as shown in FIGS. 5A-5C, or proximate walls 88 (not shown) to a position distant from wall 84. For example, the position distant may be at least 1 cm from wall 84. This movement to a position distant prevents magnets 88, via their magnetic field, from having a substantial influence on any paramagnetic particles in chamber 82. If a magnetically insulating material is inserted between magnets 88 and chamber 82, the position distant may be less than if the magnetically insulating material were not present. If the magnets 88 have an adjustable magnetic field strength, rather than being moved, they may simply be adjusted to a lower magnetic field strength or zero magnetic field strength to avoid an substantial influence on any paramagnetic particles in chamber 82.

Although multiple magnets 88 are shown in FIG. 5, it is possible to have only a single magnet 88.

Magnets 88 may have a high magnetic field strength. For instance, they may contain rare earth metals, such as neodymium or samarium alloyed with another metal, such as cobalt. Magnets 88 may be dipole magnets, quadrapole magnets, or any other type of magnets. Magnets 88 may have an adjustable magnetic field strength, for example, they may be electromagnets. Magnets 88 may be arranged to maximize magnetic attraction, for instance in a wrapped configuration.

Magnets for use with a spinning membrane module may be located external to the chamber that the cell suspension flows through, or internal to the chamber. If the magnets are internal, they may be coated with a biocompatible material. Particularly if the magnets are internal, they may be disposable.

Example spinning membranes suitable for use in modules disclosed herein include the 4-µm track-etched polycarbonate spinning membrane used in the LOVO^{®} cell processing system (Fresenius Kabi, Fenwal, Lake Zurich, IL), and the spinning membrane used in the ISOLEX^{®} magnetic cell separation systems (Baxter, Deerfield, IL).

Elements from FIGs. 1A-1F, including flow-through magnetic separation/debeading modules 8 as described in FIGS. 2-4 or magnetic spinning membrane debeading modules 18 as described in FIG. 5 may be combined with one another in cell processing system 2 depending on the specific cell processing to be performed. The elements may be combined as depicted, or in other reasonable variations. For instance, a reagent module 24 may be included in a system otherwise a depicted in FIG. 1A so that a chemical agent may be added to suspension fluid in flow-through magnetic separation/debeading module 8 when it is used for debeading. Modules, including additional buffer modules or output modules, may be arranged and used to ensure proper fluid volumes and flow rates, particularly in modules 8 and 18.

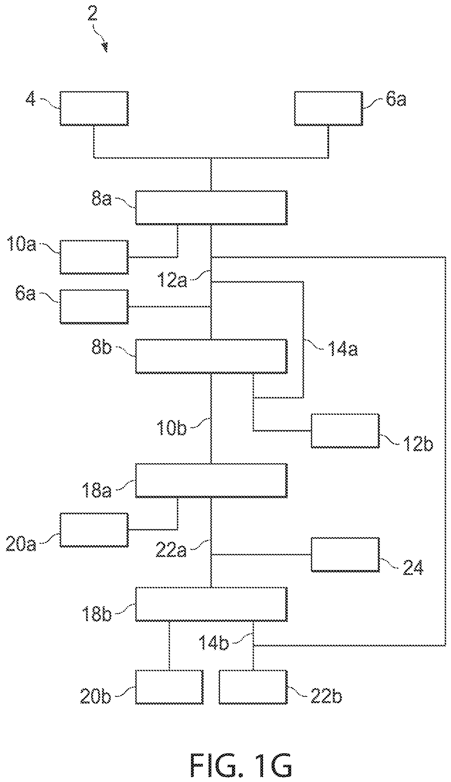

One example cell processing system 2 combining multiple modules and loops is illustrated in FIG. 1G. This system includes cell suspension module 4 and buffer module 6a connected to first flow-through magnetic separation/debeading module 8a, which has non-magnetic output module 10a and magnetic output module 12a. Magnetic output module 12a is connected to second, in-series flow-through magnetic separation/debeading module 8b, which is also connected to buffer module 6b and has non-magnetic output module 10b and magnetic output module 12, and return loop 14a. Return loop 14a leads back to module 8b. Magnetic output module 12b leads to first spinning membrane debeading module 18a, which has waste output module 20a and cell output module 22a. Cell output module 22a leads to second, in-series spinning membrane debeading module 18b, which is also connected to reagent module 24 as well as waste output module 20b, return loop 14b, and cell output module 22b. Return loop 14b leads back to second, in-series, flow-through magnetic separation/debeading module 8b.

Another example cell processing system 2, having various additional modules with specific fluid conduits, is shown in FIG. 1H. Cell suspension module 4 and satellite module 30, which may be empty or may contain buffer, are connected to flow-through magnetic separation/debeading module 8. Flow-through magnetic separation/debeading module 8 is separately connected to buffer module 6 and reservoir module 26. Reservoir module 26 is further connected to recovery module 28. Many connections are made using spike tubing 32. The system further contains, along various fluid conduits, roller clamps 34, weld sites 36, a slide clamp 38, and pinch clamps 40.

Cell processing system 2 may contain a variety of additional modules 16, such as magnetic columns, other physical separation modules, cell washing modules, cell concentration modules, and media exchange modules.

### Cell Separation and Debeading Methods

System 2 may be used to separate paramagnetic particle-bound cells and unbound cells, to debead magnetic-particle bound cells, to separate paramagnetic particles and unbound cells, or any combination thereof, in a flow-through process. In a flow-through process, all cells continue to move while in flow-through magnetic separation/debeading module 8. None, no more than 0.01%, or no more than 0.05 %, or no more than 1% of paramagnetic particle-bound cells passing through module 8 stop along walls 52.

### Flow-Through Magnetic Separation/Debeading Processes

In a flow-through process using the system of FIG. 1A, flow-through magnetic separation/debeading module 8 may optionally be primed by flowing buffer from buffer module 6 through it to either non-magnetic output module 10 or magnetic output module 12, or another output or additional module 16. A cell suspension containing paramagnetic particle-bound cells is flowed through module 8.

If system 2 is configured for separation, the cell suspension is directed to non-magnetic output module 10. Module 8 may periodically be configured to not attract paramagnetic particles while buffer from buffer 6 flows through it to magnetic output module 12. In order to ensure better separation of cells, and high purity of the magnetic or non-magnetic cell products, the cell suspension may be directed through a loop 14 for a second or subsequent passage through module 8. Paramagnetic particle-bound cells may be directed to an additional component 16, such as a flow-through magnetic separation/debeading module 8 configured for debeading or to a spinning membrane debeading module 18.

If system 2 is configured for debeading, after flowing through module 8, the cell suspension is directed to non-magnetic output module 10. Alternatively, the cell suspension may be directed through loop 14 for a second or subsequent passage through module 8 prior to direction to non-magnetic output module 10.

Processes using flow-through magnetic separation/debeading module 8 subject each paramagnetic particle-bound cell 100 with at least one bound paramagnetic particle 102 to a fluidic (shear or drag) force 104. Each cell 100 is also subjected to magnetic force 106, as shown in FIG. 6. Fluidic force 104 and magnetic force 106 may be combined in such a way that paramagnetic particle 102 remains bound to paramagnetic particle-bound cell 100, allowing cell 100 to be separated form unbound cells. Fluidic force 104 and magnetic force 106 may also be combined in such a way as to cause paramagnetic particle 102 to detach from paramagnetic particle-bound cell, allowing debeading of cell 100. Secondary forces, such a diffusion and gravity, also act upon paramagnetic particle 102, but their effects on debeading are typically much less than fluidic force and magnetic force and are often ignored when calculating the proper flow rate through a module.

System 2 may generally be configured so that, as quickly as possible, desirable cells are no longer subject to passage through modules, reducing trauma to the cells. For instance, only the magnetic output module 12 may contain a return loop to flow-through magnetic separation/debeading module 8 in a debeading configuration, allowing more easily debeaded cells to pass through module 8 fewer times than those with more recalcitrantly bound paramagnetic particles.

Flow-through module 8 may be laminar or turbulent. Magnetic force 106 is influenced by the saturation flux (mₛ) of paramagnetic particle 102. Magnetic force 106 is also influenced by the strength of the magnetic field 56 to which paramagnetic particle 102 is subjected, which is influenced by the strength of magnets 54 as well as depth of chamber 50 in the z direction and cell 100's location within that chamber. Magnetic force 106 is further influenced by the magnetic field gradient to which paramagnetic particle 102 is subjected as it moves through chamber 50, which is influenced by the strength and placement of magnets 54 as well as paramagnetic particle 102's location with respect to magnets 54.

In addition, fluidic forces are affected by the fluid flow velocity, which is typically highest in the center of the chamber and zero at the walls, meaning that cells attached to the walls may not experience sufficient fluidic force to detach them from their paramagnetic particle and that cells along the wall may be largely stationary and may shield downstream cells from fluidic forces. This results in loss of desirable cells and is avoided by continuous flow of the cell suspension through chamber 50. This loss may be avoided by a flow-through approach, in which cells are not rendered stationary by magnetic force. It may also be avoided by modules in which velocity is not zero or near zero at the walls, such as plug flow modules where fluid flow velocity is uniform across the chamber.

### Flow-through Separation Process

A flow-through separation process may be conducted using flow-through magnetic separation/debeading module 8 in a configuration as shown in FIGs. 2A and 2B. Flow rate is such that fluidic force 104 and magnetic force 106 allow paramagnetic particle 102 to remain bound to cell 100. Flow rate is also such that cell 100 is not lysed by fluidic forces.

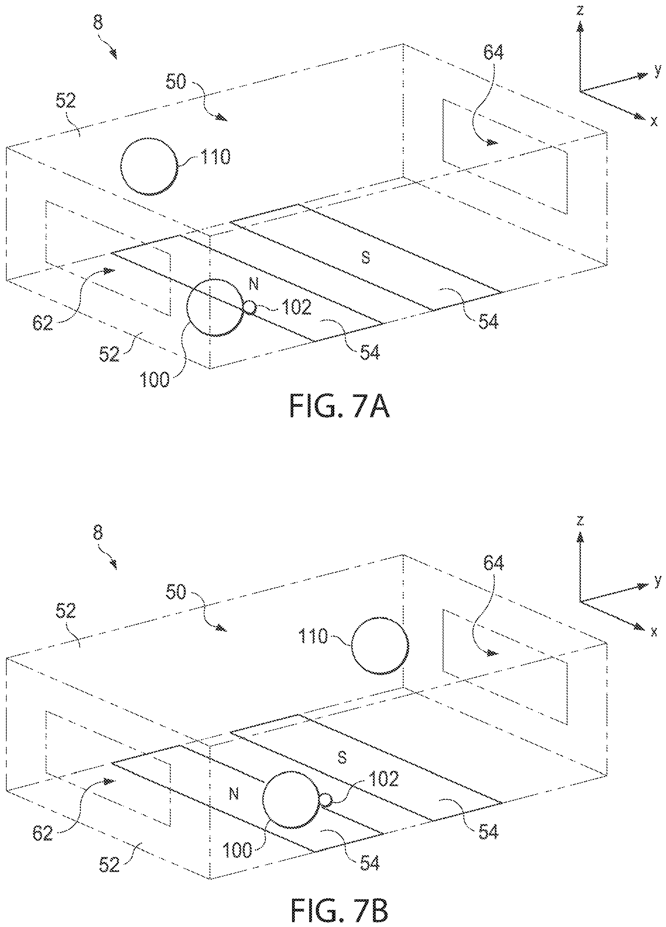

FIG. 7A shows module 8 of FIG. 2B when paramagnetic particle-bound cell 100 and unbound cell 110 have recently entered chamber 50. In FIG. 7B, cell 100 has stopped, while unbound cell 110 has continued on at it original velocity. In FIG. 7C, the magnets have been moved away and cell 100 continues to move, but unbound cell 110 has exited chamber 50.

Suspension fluid exiting chamber 50 while magnets 54 are adjacent to or proximate chamber 50 enters non-magnetic output module 10. Periodically, cell suspension flow from cell suspension module 4 is stopped and buffer is flowed into chamber 50 from buffer module 6 while magnets 54 are moved away from chamber 50 to allow paramagnetic particle-bound cells 100 to be flushed into magnetic output module 12 by the buffer.

This flow-through separation process, particularly when repeated to allow multiple passages of cells through module 8, may remove at least 80%, at least 90%, at least 95%, or at least 99% of paramagnetic particle-bound cells 100 from the cell suspension prior to its entry into non-magnetic output module 10. Efficiency may be lower for a single pass process, in which cells pass through module 8 only once. For instance, in a single pass process, module 8 may remove at least 25% or at least 50% of paramagnetic particle-bound cells 100 from the cell suspension prior to its entry into non-magnetic output module 10. Single or multiple passes of either the non-magnetic output or the magnetic output through flow-through magnetic separation/debeading module 8 may result in paramagnetic particle-bound cell product with no more than 1% unbound cells 110 and containing at least 99% of the paramagnetic particle-bound cells 100 found in the cell suspension prior to the flow-through separation process, an unbound cell product with no more than 1% paramagnetic particle-bound cells 100 and containing at least 99% of the unbound cells 110 found in the cell suspension prior to the flow-through process, or both.

### Flow-through Debeading Process

A flow-through debeading process may also be conducted using flow-through magnetic separation/debeading module 8 in a configuration as shown in FIGs. 2A and 2B. Flow rate is such that fluidic force 104 and magnetic force 106 detach, on average over the cells in the cell suspension, at least one paramagnetic particle 102 from cell 100 while cell 100 passes through chamber 50. Flow rate is also such that the cells are not lysed by fluidic forces.

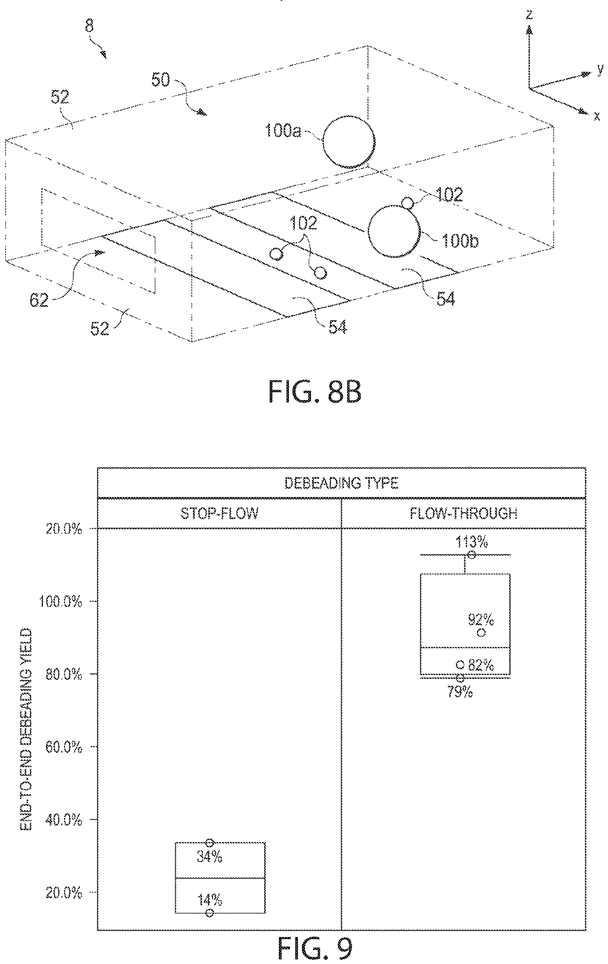

FIG. 8A shows module 8 of FIG. 2B when paramagnetic particle-bound cells 100a and 100b have recently entered chamber 50. Cell 100a has one paramagnetic particle 102, while cell 100b has two paramagnetic particles 102. In FIG. 8B, one paramagnetic particle 102 each has detached from both cell 100a and cell 100b and has come to rest on wall 52, while cells 100a and 100b continue to pass through chamber 50. Cell 100a no longer has any paramagnetic particles 102, while cell 100b retains one paramagnetic particle 102. Cell 100b may be passed through module 8 a second time to remove this second paramagnetic particle 102. Alternatively, module 8 may be configured such that cell 100b remains in chamber 50, similar to the paramagnetic particle-bound cell in FIG. 7, while cell 100a passes out of chamber 50.

This flow-through debeading process may remove at least 99% of paramagnetic particles from cells.

When a system 2 containing a recirculation loop 14 from magnetic output module 12 was used to debead CLT0119 T cells, cells in the magnetic output were resuspended in buffer from buffer module 6 and passed through module 8 a second time and then again a third time. A comparison of the results of this process to the results of a conventional stop-flow process are provided in FIG. 9. FIG. 9 presents end-to-end yields, which are the ratios of the number of cells in the final product to the number of cells that entered the debeading system.

The flow-through magnetic separation/debeading module 8 shown in FIG. 3 may be used to debead cells in a manner similar to module 8 as shown in FIGs. 2A and 2B. Fluid introduced through ports 72 supplies a force to cells 100 sufficient to dislodge them from membrane 70. Because velocity of the suspension fluid approached zero near membrane 70, these cells might otherwise not be separated or debeaded or may be lost.

This flow-through debeading process may also remove at least 99% of paramagnetic particles from the cells.

### Flow-through Zero Gradient Filter Process

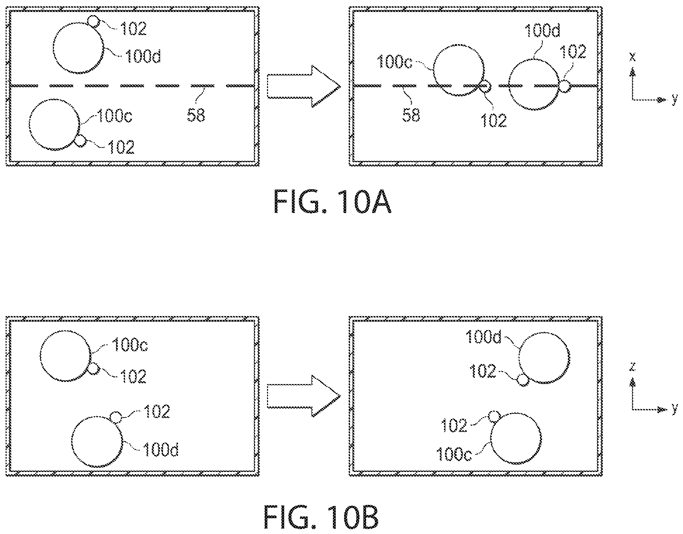

A flow-through separation process may be conducted using flow-through magnetic separation/debeading module 8 in a configuration as shown in FIGs. 4A and 4B. Flow rate is such that fluidic force 104 and magnetic force 106 allow paramagnetic particle 102 to remain bound to cell 100. Flow rate is also such that cell 100 does not stop in chamber 50 and also is not lysed by fluidic forces. Various configurations are shown in FIGs. 10-13 and may be modified, for example to adjust the number and proportional size of inlets 62 and outlets 64, for use with different zero gradient filter processes. For instance, the same effects achieve by having inlets 62 and outlets 64 of different sizes can also be the achieved by providing different flow rates, typically controlled by pumps, through same-size inlets and outlets.

FIG. 10 presents a basic description of how paramagnetic particle-bound cells 100 flow-through module 8 in a zero gradient configuration. As illustrated in FIG. 10A, cells 100 after entering module 8 (left figure) are pulled in the x-direction to zero gradient line 58 by the time it they are nearly exiting module 8 (right figure). As illustrated in FIGs. 10A and 10B, cells 100 are not subject to magnetic force effects in the y-direction or the z-direction when entering module 8 (left figure) or even when close to exiting (right figure) .

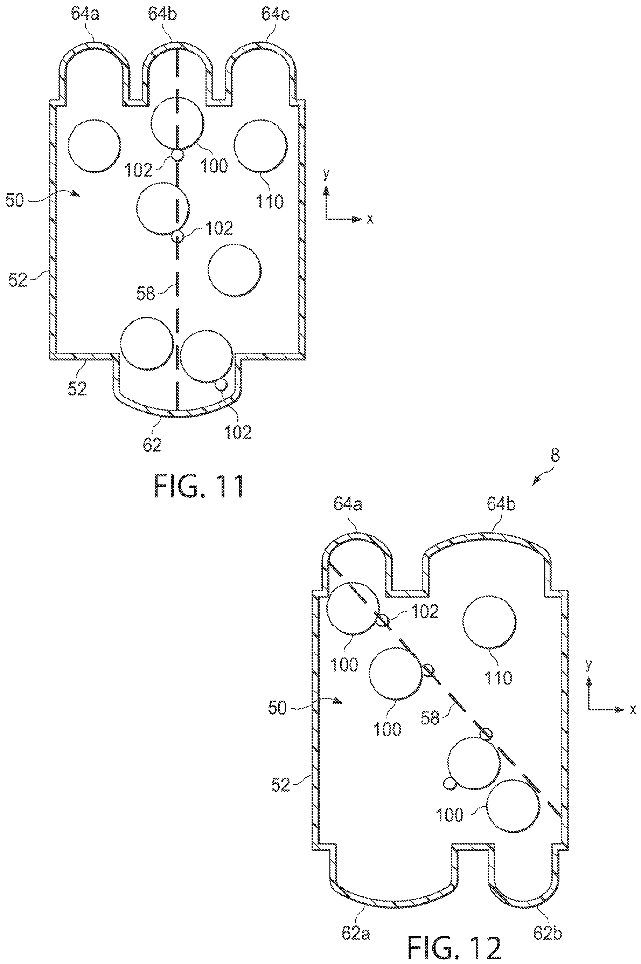

FIG. 11 shows a zero-gradient module 8 with magnets 54 oriented as shown in FIG. 4A. In this module, fluid enters via inlet 62. Cells 100 with magnetic particles 102 follow zero gradient line 58 and are directed to magnetic outlet 64b. Unbound cells 110 are unaffected by zero gradient line 58 and flow to non-magnetic outlets 64a and 64c. Some unbound cells 110 will also enter magnetic outlet 64b in this configuration.

FIG. 12 shows cells moving through module 8 of FIG. 4C. Cells 100 with paramagnetic particles 102 follow zero gradient line 58 and are directed to magnetic outlet 64a. Unbound cells 110 are unaffected by zero gradient line 58 and flow to non-magnetic outlet 64b. Thus, zero gradient line 58 acts as a magnetic filter while allowing all cells to continue to move through chamber 50 without being pushed towards any wall 52. As illustrated non-magnetic inlet 62a is larger than magnetic inlet 62b and non-magnetic outlet 64b is larger than magnetic outlet 64a. If fluid flow in module 8 is laminar, fluid from non-magnetic inlet 62a crosses over to non-magnetic outlet 64b, preventing any unbound cells 110 from entering magnetic outlet 64a. This method may also be used with turbulent flow, but with less efficiency due to loss of unbound cells to the magnetic outlet 64a.

This flow-through separation process may remove at least 80%, at least 90%, at least 95%, or at least 99% of paramagnetic particle-bound cells 100 from the cell suspension prior to its entry into non-magnetic output module 10. Multiple passes of either the non-magnetic output or the magnetic output through flow-through magnetic separation/debeading module 8 may result in a paramagnetic particle-bound cell product with no more than 1% unbound cells 11 and containing at least 99% of the paramagnetic particle-bound cells 100 found in the cell suspension prior to the flow-through separation process, an unbound cell product with no more than 1% paramagnetic particle-bound cells 100 and containing at least 99% of the unbound cells 110 found in the cell suspension prior to the flow-through process, or both.

### Flow-through Zero Gradient Paramagnetic Particle Separation Process

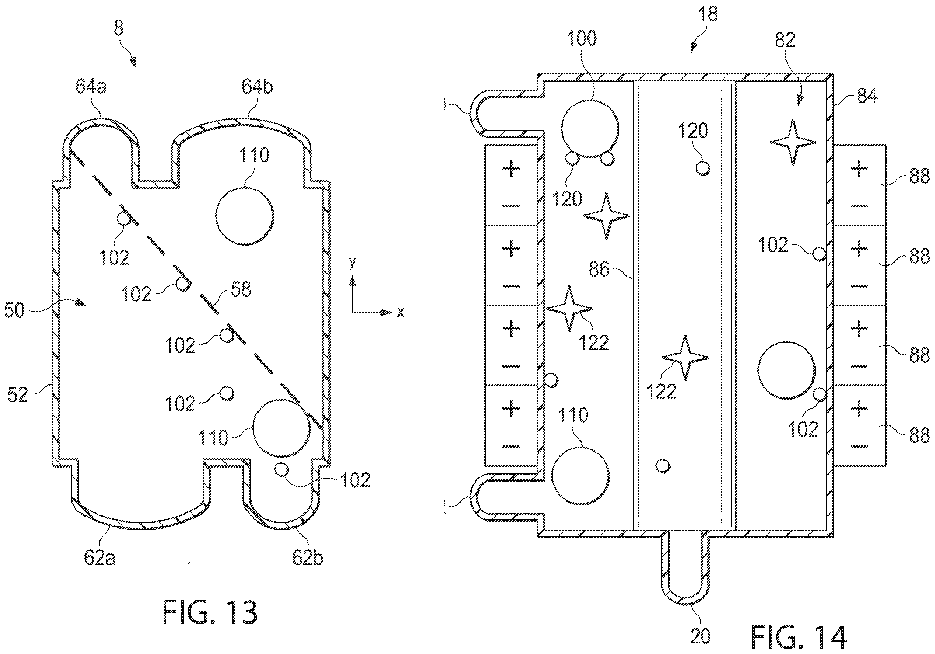

A flow-through paramagnetic particle separation process may also be conducted using flow-through magnetic separation/debeading module 8 in a configuration as shown in FIG. 4C. FIG. 13 shows debeaded, unbound cells 110 and paramagnetic particles 102 moving through module 8. Cells 110 were previously debeaded, for instance by spinning membrane debeading module 18, a non-spinning membrane debeading module, or the same or a separate module 8 in a debeading configuration. Alternatively, cells 110 may have already been in the presence of, but not bound to paramagnetic particles 102. Paramagnetic particles 102 follow zero gradient line 58 and are directed to magnetic outlet 64a. Debeaded, unbound cells 110 are unaffected by zero gradient line 58 and flow to non-magnetic outlet 64b. Thus, zero gradient line 58 acts as a magnetic filter while allowing cells 110 to continue to move through chamber 50 without being pushed towards any wall 52.

This flow-through paramagnetic particle separation process may remove at least 80%, at least 90%, at least 95%, or at least 99% of beads from the cell suspension.

This process, in conjunction with removing the paramagnetic particles, may remove an unwanted constituent of the cell suspension as well.

Although the cell separation process and the particle separation process are described separately above, they may both occur simultaneously in the same module or system. For example, a separation module will typically remove both paramagnetic particle-bound cells and free paramagnetic particles from the cell suspension.

### Spinning membrane Debeading Processes

In a flow-through process using system 2 of FIG. 1F, separated paramagnetic particle-bound cells 100 are directed into spinning membrane module 18 as shown in FIG. 14 via sample inlet 80. Within debeading chamber 82, spinning membrane 86 generates recirculating Taylor-Couette flows in the cell suspension which cause fluidic forces in addition to the fluidic forces generated by flow through chamber 82 from inlet 80 to outlets 20 and 22. As a result, although paramagnetic particles 102 still experience a fluidic force and a magnetic force, the relationship of these forces and how they cause debeading is difficult to model. However, fluidic forces are affected by at least the size and spin rate of spinning membrane 80, the cell suspension flow rate through chamber 82, and the viscosity of the suspension fluid. Magnetic force is affected by the nature of paramagnetic particles 102, the nature of magnets 88, and the design of module 18, particularly distance between the cells 100 and magnets 88. Fluidic forces are typically not so high as to lyse the cells. The Taylor-Couette flows are sufficient to keep the cells away from and out of contact with wall 84 and spinning membrane 86.

Paramagnetic particles 102 that are removed from cells 100 migrate to wall 84, and particularly to zero gradient lines or bands along wall 84. Any non-paramagnetic particles 120 are also removed from cells 100 by fluidic forces alone. Non-paramagnetic particles 120 pass through pores in spinning membrane 86 and then exit chamber 82 via waste outlet module 20. Any chemical agent 122 added from optional reagent chamber 24 also passes through the pores in spinning membrane 86 and exits chamber 82 via waste outlet module 20. This limits exposure of cells 100 to chemical agent 120. Debeaded, unbound cells 110 exit chamber 82 via cell outlet module 22.

Paramagnetic particles 102 may be removed from wall 84 periodically, for instance by stopping cell suspension flow through chamber 82, moving magnets 88 to a position distant from wall 84, then flowing buffer through chamber 82.

Some paramagnetic particles 102 may also pass through spinning membrane 80 and be removed. If magnets 88 are absent or sufficiently distant from chamber 82, all paramagnetic particle removal may be accomplished by spinning membrane 80.

This flow-through debeading process may also remove at least 80%, at least 90%, at least 95%, or 99% of paramagnetic particles from the cells, at least 80%, at least 90%, at least 95%, or at least 99% of all non-paramagnetic particles from the cells, or both.

The spinning membrane may also be used to separate paramagnetic particles 102 from unbound cells 102.

Spinning membrane 86 may have a pore size small enough to exclude all cells in the cell suspension.

Spinning membrane module 18 may also be used to remove unwanted constituents from the cell suspension. These constituents may simply be filtered by spinning membrane 88, or they may interact with the coating of paramagnetic particles 102, non-paramagnetic particles 120, or both, and be removed with the particles. Debeading and unwanted constituent removal may occur separately or simultaneously.

### Other Debeading and Paramagnetic Particle Separation Processes

System 2, due to it modular design, is also compatible with other debeading and paramagnetic particle separation processes. One need only insert an appropriate additional module 16. For instance, columns, including magnetic columns and physical separation methods are often used to debead cells and may be included as an additional module 16.

### Other Incorporated Processes

System 2, due to its modular design, is compatible with other incorporated processes. These processes may occur in at least one additional module 16. For instance, a module may be used to wash cells. A module may also be used to concentrate cells. A module may be used to exchange the media in which cells are located. One module may be used for more than one of these steps.

### Multiple-Module Flow-Through Process

System 2 as shown in FIG. 1G may be used in a multiple-module flow-through process. Optionally, buffer from buffer module 6a may be flowed through flow-through magnetic separation/debeading module 8a and optionally also one or more of modules 8b, 18a, and 18b. A cell suspension containing desirable, paramagnetic particle and non-paramagnetic particle-bound cells and undesirable unbound cells is flowed through module 8a, which is configured as shown in FIG. 4 for separation, but alternatively may be configured as shown in FIGs. 2A and 2B for separation. Unbound cells are directed to non-magnetic output module 10a as waste. Paramagnetic particle-bound cells are directed via magnetic output module 12a to flow-through magnetic separation/debeading module 8b. Module 8b is configured as shown in FIGs. 2A and 2B for debeading. Paramagnetic particle-bound cells are flowed through return loop 14a at least once prior to entering magnetic output module 12b as waste. Debeaded, unbound cells are sent via non-magnetic output module 10b to spinning membrane debeading module 18a. Any remaining paramagnetic particles and non-paramagnetic particles are removed, with non-paramagnetic particles flowing into waste output module 20a, while unbound paramagnetic particles remain in module 18 and unbound cells and cells with paramagnetic particles, non-paramagnetic particles, or both flow into cell output module 22a. Cell output module 22a leads to a second spinning membrane module 18b. A chemical agent able to facilitate removal of either the paramagnetic particle or the non-paramagnetic particle, or both, from the cells is added from reagent module 24. Non-paramagnetic particles and the chemical agent flow into waste module 20b. Paramagnetic particles remain in module 18. Unbound cells and cells with either paramagnetic particles, non-paramagnetic particles, or both flow into return loop 14b at least once prior to being sent to cell output module 22b as the final cell product of the flow-through process.

### Clinical Applications

All of the processes herein may be conducted according to clinical good manufacturing practice (cGMP) standards.

The processes may be used for cell purification, enrichment, harvesting, washing, concentration or for cell media exchange, particularly during the collection of raw, starting materials (particularly cells) at the start of the manufacturing process, as well as during the manufacturing process for the selection or expansion of cells for cell therapy.

The cells may include any plurality of cells. The cells may be of the same cell type, or mixed cell types. In addition, the cells may be from one donor, such as an autologous donor or a single allogenic donor for cell therapy. The cells may be obtained from patients by, for example, leukapheresis or apheresis. The cells may include T cells, for example may include a population that has greater than 50% T cells, greater than 60% T cells, greater than 70% T cells, greater than 80% T cells, or 90% T cells.

Selection processes may be particularly useful in selecting cells prior to culture and expansion. For instance, paramagnetic particles coated with anti-CD3 and/or anti CD28 may be used to select T cells for expansion or for introduction of a nucleic acid encoding a chimeric antigen receptor (CAR) or other protein. Such a process is used to produce CTL019 T cells for treatment of acute lymphoblastic leukemia (ALL).

The debeading processes and modules disclosed herein may be particularly useful in the manufacture of cells for cell therapy, for example in purifying cells prior to, or after, culture and expansion. For instance, paramagnetic particles coated with anti-CD3 and/or anti CD28 antibodies may be used to selectively expand T cells, for example T cells that are, or will be, modified by introduction of a nucleic acid encoding a chimeric antigen receptor (CAR) or other protein, such that the CAR is expressed by the T cells. During the manufacture of such T cells, the debeading processes or modules may be used to separate T cells from the paramagnetic particles. Such a debeading process or module is used to produce, for example, CTL019 T cells for treatment of acute lymphoblastic leukemia (ALL).

In one such process, illustrated here by way of example, cells, for example, T cells, are collected from a donor (for example, a patient to be treated with an autologous chimeric antigen receptor T cell product) via apheresis (e.g., leukapheresis). Collected cells may then be optionally purified, for example, by an elutriation step. Paramagnetic particles, for example, anti-CD3/anti-CD28-coated paramagnetic particles, may then be added to the cell population, to expand the T cells. The process may also include a transduction step, wherein nucleic acid encoding one or more desired proteins, for example, a CAR, for example a CAR targeting CD19, is introduced into the cell. The nucleic acid may be introduced in a lentiviral vector. The cells, e.g., the lentivirally transduced cells, may then be expanded for a period of days, for example, 1, 2, 3, 4, 5, 6, 7, 8, 9, 10, or more days, for example in the presence of a suitable medium. After expansion, the debeading processes/modules disclosed herein may be used to separate the desired T cells from the paramagnetic particles. The process may include one or more debeading steps according to the processes of the present disclosure. The debeaded cells may then be formulated for administration to the patient. Examples of CAR T cells and their manufacture are further described, for example, in WO2012/079000, which is incorporated herein by reference in its entirety. The systems and methods of the present disclosure may be used for any cell separation/purification/debeading processes described in or associated with WO2012/079000.

The systems and methods herein may similarly benefit other cell therapy products by wasting fewer desirable cells, causing less cell trauma, and more reliably removing magnetic and any non-paramagnetic particles from cells with less or no exposure to chemical agents, as compared to conventional systems and methods.

Although only exemplary embodiments of the disclosure are specifically described above, it will be appreciated that modifications and variations of these examples are possible without departing from the spirit and intended scope of the disclosure. For example, the magnetic modules and systems containing them may be arranged and used in a variety of configurations in addition to those described. In addition, the systems and methods may include additional components and steps not specifically described herein. For instance, methods may include priming, where a fluid is first introduced into a component to remove bubbles and reduce resistance to cell suspension or buffer movement. Furthermore, embodiments may include only a portion of the systems described herein for use with the methods described herein. For example, embodiments may relate to disposable modules, hoses, etc. usable within non-disposable equipment to form a complete system able to separate or debead cells to produce a cell product.

Embodiments of various aspects of a flow-through device or cell processing system described herein can be defined in any of the following numbered paragraphs:
1. A cell processing system comprising:
   at least one cell suspension module;
   at least one buffer module;
   at least one flow-through magnetic separation/debeading module;
   at least one non-magnetic output module; and
   at least one magnetic output module.
2. The cell processing system of Paragraph 1, further comprising at least one return loop returning upstream of at least one flow-through magnetic separation/debeading module.
3. The cell processing system of Paragraph 1, comprising at least two flow-through magnetic separation/debeading modules in parallel.
4. The cell processing system of Paragraph 1, comprising at least two flow-through magnetic separation/debeading modules in series.
5. The cell processing system of Paragraph 1, further comprising at least one additional module.
6. The cell processing system of Paragraph 5, wherein the at least one additional module comprises at least one spinning membrane debeading module.
7. The cell processing system of Paragraph 6, comprising at least two spinning membrane debeading modules in parallel.
8. The cell processing system of Paragraph 6, comprising at least two spinning membrane debeading modules in series.
9. The cell processing system of Paragraph 5, wherein the at least one additional module comprises at least one physical separation module.
10. The cell processing system of Paragraph 9, wherein the at least one additional module comprises at least one magnetic column module.
11. The cell processing system of Paragraph 5, wherein the at least one additional module comprises at least one media exchange module.
12. The cell processing system of Paragraph 5, wherein the at least one additional module comprises at least one cell concentration module.
13. The cell processing system of Paragraph 5, wherein the at least one additional module comprises at least one cell washing module.
14. The cell processing system of Paragraph 1, wherein the flow-through magnetic separation/debeading module comprises:
   a chamber defined by walls and having an x-direction, a y-direction, and a z-direction;
   an inlet and an outlet arranged on opposite ends of the chamber in the y-direction; and at least two magnets adjacent or proximate a wall of the chamber and arranged to establish a zero gradient line within the chamber between the inlet and the outlet.
15. The cell processing system of Paragraph 6, wherein the spinning membrane debeading module comprises:
   a debeading chamber define partially by a cylindrical side-wall;
   a porous spinning membrane having an interior and oriented co-axially with the cylindrical side-wall;
   a sample inlet;
   a waste output module connected to the interior of the spinning membrane; and
   a cell output module connected to the debeading chamber.
16. The cell processing system of Paragraph 15, wherein the spinning membrane debeading module further comprises at least one magnet adjacent or proximate to the cylindrical side-wall.
17. A flow-through magnetic separation/debeading module comprising:
   a chamber defined by walls and having an x-direction, a y-direction, and a z-direction;
   an inlet and an outlet arranged on opposite ends of the chamber in the y-direction; and
   at least two magnets adjacent or proximate a wall of the chamber and arranged to establish a zero gradient line within the chamber between the inlet and the outlet.
18. The module of Paragraph 17, comprising at least two inlets and at least two outlets.
19. The module of Paragraph 17, further comprising at least three magnets adjacent or proximate a wall of the chamber and arranged to establish at least two zero gradient lines within the chamber between the inlet and the outlet.
20. The module of Paragraph 17, further comprising at least four magnets arranged in two arrays on opposite sides of the chamber in the z-direction.
21. The module of Paragraph 18, further comprising at least four magnets arranged in two arrays on opposite side of the chamber in the z-direction and cross-oriented in the x-y plane from near one inlet to near one outlet on the opposite side of the chamber in the z-direction.
22. The module of Paragraph 17, further comprising:
   sub-membrane injection ports adjacent a wall of the chamber also adjacent at least two magnets; and
   a membrane adjacent the sub-membrane.
23. A spinning membrane debeading module comprising:
   a debeading chamber define partially by a cylindrical side-wall;
   a porous spinning membrane having an interior and oriented co-axially with the cylindrical side-wall;
   a sample inlet;
   a waste output module connected to the interior of the spinning membrane;
   a cell output module connected to the debeading chamber; and
   at least one magnet adjacent or proximate to the cylindrical side-wall.
24. The spinning membrane debeading module of Paragraph 23, further comprising a reagent module.
25. The spinning membrane debeading module of Paragraph 23, wherein the porous spinning membrane has a pore size greater than that of a particle to be debeaded and less than that of a cell to be debeaded.
26. A method of flow-through cell processing comprising flowing a cell suspension comprising paramagnetic particle-bound cells through a flow-through magnetic separation/debeading module to produce an unbound cell product,
   wherein the paramagnetic particle-bound cells continue to move in the flow-through magnetic separation/debeading module through the flowing step, and
   wherein the flow-through magnetic separation/debeading module comprises:
      a flow chamber defined by walls through which the cell suspension flows; and
      at least two magnets arranged adjacent or proximate at least one wall.
27. The method of Paragraph 26, wherein the cell suspension is flowed laminarly through the flow-through magnetic separation/debeading module.
28. The method of Paragraph 26, wherein the cell suspension further comprises unbound cells and flowing the cell suspension through the flow-through magnetic separation/debeading module separates the paramagnetic particle-bound cells and the unbound cells.
29. The method of Paragraph 28, wherein the cell suspension further comprises free paramagnetic particles and flowing the cell suspension through the flow-through magnetic separation/debeading module separates the free paramagnetic particles and the unbound cells.
30. The method of Paragraph 28, further comprising flowing the separated unbound cells through the flow-through magnetic separation/debeading module a second or subsequent time using a return loop.
31. The method of Paragraph 28, further comprising flowing the separated paramagnetic particle-bound cells through the flow-through magnetic separation/debeading module a second or subsequent time using a return loop.
32. The method of Paragraph 31, further comprising debeading the paramagnetic particle-bound cells in the flow-through magnetic separation/debeading module during the second or subsequent time to produce paramagnetic particles and debeaded, unbound cells.
33. The method of Paragraph 32, further comprising flowing the produced paramagnetic particles and debeaded, unbound cells through the flow-through magnetic separation/debeading module a third or subsequent time to separate the paramagnetic particles and the debeaded, unbound cells.
34. The method of Paragraph 26, wherein the magnets are oriented to establish one zero gradient line that crosses the direction of flow, such that paramagnetic-particle bound cells are pulled to the zero gradient line in one direction only, but are not affected by magnetic forces of the two magnets in two other directions.
35. The method of Paragraph 26, wherein the chamber further comprises:
   a magnetic inlet through which any paramagnetic particles enter the flow chamber;
   a non-magnetic inlet;
   a magnetic outlet opposite the non-magnetic inlet; and
   a non-magnetic outlet opposite the magnetic inlet, wherein the zero gradient line directs all paramagnetic particles and any bound cells to the magnetic outlet.
36. The method of Paragraph 35, wherein the cell suspension further comprises unbound cells and wherein non-magnetic inlet is larger than the magnetic inlet and the non-magnetic outlet is larger than magnetic outlet, wherein fluid flowing from the non-magnetic inlet crosses over to the non-magnetic outlet, preventing any unbound cells from flowing into the magnetic outlet.
37. The method of Paragraph 35, wherein the cell suspension further comprises unbound cells, wherein the non-magnetic inlet and magnetic inlet are substantially the same size or the non-magnetic out and magnetic outlet are substantially the same size, or both, and wherein respective flow rates of the fluid enter the inlets, the respective flow rates of the fluid exiting the outlets, or both are adjusted such that fluid flowing from the non-magnetic inlet crosses over to the non-magnetic outlet, preventing any unbound cells from flowing into the magnetic outlet.
38. The method of Paragraph 26, further comprising flowing the paramagnetic particle-bound cells through a spinning membrane debeading module to produce the unbound cell product, wherein the spinning membrane debeading module comprises:
   a cylindrical debeading chamber through which the paramagnetic particle-bound cells flow, the chamber defined in part by a cylindrical side-wall and containing a co-axial spinning membrane; and
   at least one magnet arranged adjacent or proximate the cylindrical side-wall to establish at least one zero gradient line within the cylindrical debeading chamber.
39. The method of Paragraph 26, further comprising flowing the paramagnetic particle-bound cells through a magnetic column module to produce the unbound cell product.
40. The method of Paragraph 26, further comprising flowing the paramagnetic particle-bound cells or the unbound cell product through a cell washing module.
41. The method of Paragraph 26, further comprising flowing the paramagnetic particle-bound cells or the unbound cell product through a media exchange module.
42. The method of Paragraph 26, further comprising flowing the paramagnetic particle-bound cells or the unbound cell product through a cell concentration module.
43. A method of manufacturing a cell therapy composition, said method comprising:
   contacting a cell population with paramagnetic particles coated with one or more agents which assist in expanding one or more cell types within the cell population;
   introducing nucleic acid into cells within the cell population;
   expanding cells within the cell population;
   debeading the cell population according to the method of any of Paragraphs 26 to 42, or using the system of any of Paragraphs 1 to 16 or the module of any of Paragraphs 17-25; and
   formulating the cell population for cell therapy.
44. The method of Paragraph 43, wherein the one or more agents which assist in expanding one or more cell types comprises anti-CD3 antibody or antigen binding fragment thereof, anti-CD28 antibody or antigen binding fragment thereof, and combinations thereof.
45. The method of any of Paragraphs 43 to 44, wherein the nucleic acid is introduced by lentivirus or mRNA transduction.
46. The method of any of Paragraphs 43 to 45, wherein the cell therapy is a chimeric antigen receptor T cell therapy.
47. The method of paragraph 46, wherein the cell therapy is an anti-CD19 chimeric antigen receptor T cell therapy.

### Improved Wash Step

The cellular composition of apheresis, e.g., leukapheresis, products vary greatly from patient to patient. Leukapheresis products with high percentages of granulocytes (e.g. neutrophils) have been correlated with instances of elevated cell clumping during CAR T cell manufacturing using Process B. Without wishing to be bound by theory, such irreversible clumping is believed to reduce available cell numbers and negatively impacts cell yields by interfering with the enrichment process (e.g. positive selection) which results in an overall reduction in cell numbers and purity. In addition, without wishing to be bound by theory, reduction in cell purity and yield directly impacts subsequent process performance (e.g., transduction efficiency and expansion), and final product cell numbers and quality. The net outcome of this reduces the ability to manufacture product able to meet dose specifications at the end of the processing cycle. Thus, without wishing to be bound by theory, it is believed that prevention of clumping can reduce the cell loss and improve the T cell purity which can generate better quality and quantity of starting material for the subsequent processing steps and result in an overall improved therapeutic product.

In the current manufacturing processes in the art, e.g., Process B, patient cellular leukapheresis material is thawed on the Plasmatherm (Genesis), washed using the CellSaver 5+ instrument (Haemonetics), and is then resuspended in either a cell expansion medium based on X-VIVO15 medium (Lonza), called 'Modified Medium', or into a buffered isotonic saline solution such as phosphate-buffered saline (PBS) for the subsequent Ficoll selection of lymphocytes. Modified Medium is prepared according to the protocol provided in Example 2. However, as described in Example 2, transfer of thawed cells into either Modified Medium or into PBS solution can cause the cells to clump.

Accordingly, also provided herein are improved methods for washing cells to prevent clumping, is compatible with subsequent manufacturing steps, e.g., positive selection by stimulation, e.g., with anti- CD3/CD28 CTS Dynabeads (Thermo Fisher). In addition, the improved wash step described herein is performed, e.g., on thawed cells, to remove subcellular debris, free hemoglobin and cryoprotectants, to achieve volume reduction, and to enable subsequent density gradient separation. In an embodiment, the wash step is performed with an alternative cell resuspension buffer to Modified Medium or PBS solution. In an embodiment, the wash step is performed with a buffer comprising dextrose and/or sodium chloride. In an embodiment, the buffer comprises about 5% and about 0.45% sodium chloride, e.g., D5 1/2 NS medium. In an embodiment, the buffer stabilizes the cell suspension and prevents clumping, e.g., for at least 30 minutes, 45 minutes, 1 hour, 1.5 hours, 2 hours, 2.5 hours, 3 hours, 3.5 hours, 4 hours, 4.5 hours, 5 hours, 5.5 hours, or 6 hours.

In one embodiment, the improved wash step described herein is performed using a device, e.g., a cell separation device, e.g., the same device used for density gradient centrifugation. For example, the improved wash step is performed using the Sepax 2 RM device (Biosafe).

In an embodiment, the wash step disclosed herein can be used for a fresh apheresis sample or a previously frozen, e.g., thawed, apheresis sample. In embodiments, the wash step disclosed herein can be used before or after any of the elutriation, density gradient centrifugation, or selection methods described herein. In another embodiment, the wash step disclosed herein is performed after a density gradient centrifugation step, e.g., a density gradient centrifugation using OptiPrep medium.

### Improved Manufacturing Process

Provided herein are methods for improving the quality and yield of immune effector cells suitable for expressing CAR to a greater degree than methods currently used in the art. In one embodiment, the elutriation, density gradient centrifugation, positive and negative selection under flow conditions, and improved wash step described in the preceding sections can be used in any combination with each other or with additional methods currently used in the art or described herein to isolate or enrich for the desired immune effector cells that are suitable for expressing a CAR.

Generally, a method for generating or enriching for a population of immune effector cells that can be engineered to express a CAR includes: providing an input sample, performing an enrichment step, and performing a selection step, thereby producing an output sample comprising the immune effector cells that are suitable for expression of a CAR. Methods for producing a population of immune effector cells that express a CAR comprise the methods for generating or enriching the population of immune effector cells that can be engineered to express a CAR, and further comprise a stimulation step, e.g., wherein the cells are stimulated to proliferate or persist, and further comprises the introduction of a nucleic acid encoding a CAR. Additional disclosure regarding the stimulation and introduction/expression of a CAR are further described in the following sections.

In one embodiment, the input sample is a fresh sample, e.g., a fresh apheresis, leukapheresis, or whole blood sample, obtained from a subject. In another embodiment, the input sample is a frozen sample. In embodiments where the input sample is a frozen sample, e.g., a frozen or cryopreserved apheresis, leukapheresis, or whole blood sample, the method comprises thawing the frozen sample or providing a thawed sample. Frozen, e.g., cryopreserved, samples can be thawed by passive or active means. Thawing by passive means includes allowing the sample to thaw, e.g., reach the temperature of the surrounding environment, e.g., reach room temperature or reach the temperature of the buffer or solution in which the sample is transferred to or mixed with. Thawing by active means includes using a device that thaws the sample, e.g., brings the sample to the temperature of the surrounding environment faster than if thawing by passive means.

In one embodiment, the enrichment step comprises performing elutriation or density gradient centrifugation. The elutriation can be performed using elutriation conditions known in the art, or the improved settings described herein for elutriation of a frozen or previously frozen sample. The density gradient centrifugation can be performed using Ficoll or a media comprising iodixanol, e.g., about 60% iodixanol in water, e.g., OptiPrep ^{™}.

In any of the methods described herein, the selection step comprises performing a positive selection step and/or a negative selection step. The positive selection step can comprise selecting for CD3+/CD28+ cells, e.g., using a separation agent, e.g., a bead coupled to an anti-CD3 and/or anti-CD28 antibody, either under static or flow conditions, e.g., using a. The negative selection step can comprise negatively selecting for CD19+ B cells or CD19+ lymphoblasts, e.g., using a separation agent, e.g., a bead coupled to an anti-CD19 antibody.

In any of the methods described herein, a wash step can be performed after sample collection, after thawing of the sample, before the enrichment step, after the enrichment step, before the selection step, or after the selection step, or any combination thereof.

Exemplary methods for generating or enriching for a population of immune effector cells that can be engineered to express a CAR that include one or more of the elutriation, density gradient centrifugation, positive or negative selection, e.g., under flow conditions, or improved wash step are further described herein.

In one embodiment, a method for generating or enriching for a population of immune effector cells that can be engineered to express a CAR includes providing a frozen input sample comprising immune effector cells; thawing the frozen input sample, to produce a thawed sample; performing an enrichment step, wherein the enrichment step comprises performing elutriation on the input sample, wherein the input sample is optionally a thawed input sample; and performing a selection step, wherein the selection is a positive selection, e.g., for CD3/CD28+ cells, or a negative selection, e.g., for CD19+, CD25+, or CD14+ cells.

In another embodiment, a method for generating or enriching for a population of immune effector cells that can be engineered to express a CAR includes providing a fresh or frozen input sample comprising immune effector cells; and optionally, wherein the input sample is a frozen input sample, thawing the frozen input sample, to produce a thawed sample; performing an enrichment step, wherein the enrichment step comprises performing density centrifugation step using a medium comprising iodixanol, e.g., 60% iodixanol in water, e.g., OptiPrep medium, and/or having a density greater than Ficoll (e.g., greater than 1.077 g/ml, e.g., about 1.32 g/ml); and performing a selection step, wherein the selection is a positive selection, e.g., for CD3/CD28+ cells, or a negative selection, e.g., for CD19+, CD25+, or CD14+ cells.

In another embodiment, a method for generating or enriching for a population of immune effector cells that can be engineered to express a CAR includes providing a fresh or frozen input sample comprising immune effector cells; performing an enrichment step, wherein the enrichment step comprises performing elutriation or density centrifugation (e.g., using Ficoll or a Optiprep medium); and performing a positive selection step under flow conditions, e.g., for CD3/CD28+ cells.

In another embodiment, a method for generating or enriching for a population of immune effector cells that can be engineered to express a CAR includes providing a fresh or frozen input sample comprising immune effector cells; performing an enrichment step, wherein the enrichment step comprises performing elutriation or density centrifugation (e.g., using Ficoll or a Optiprep medium); and performing a negative selection step under flow conditions, e.g., for CD19+, CD25+, or CD14+ cells;

In any of the methods described herein, a wash step can be performed after sample collection, after thawing of the sample, before the enrichment step, after the enrichment step, before the selection step, or after the selection step, or any combination thereof.

Control limits can be defined that identifies the range or threshold of a property of the input sample or after one or more steps in the methods described herein, and dictates or determines the next step, in order to optimize enrichment of the desired immune effector cells, and ensure manufacturing success and product quality. In embodiments, the control limits may be different depending on the type of cancer of the subject from which the input sample is obtained from. By way of example, the control limits for the presence of monocytes in the input sample obtained from a subject having ALL or DLBCL, are as follows: if the monocytes are >20% of the input sample, e.g., leukapheresis whole blood cell, the optimal method comprises elutriation and/or CD3/CD28 positive selection under flow conditions; or if the monocytes are <20% of the input sample, the input sample is washed and the optimal method is determined based on blast content. In another example, the control limits for the presence of blast cells in the input sample obtained from a subject having ALL or DLBCL are as follows: if blast cells are ≥20% of incoming leukapheresis WBC, elutriation (to remove monocytes, granulocytes and cell debris) and/or modified CD19 negative selection (to remove blasts), or other technologies to deplete blasts should be performed; or if blast cells are <20% of incoming leukapheresis, then leukapheresis material will be washed and process will be determined based on the monocyte content.

After enrichment of the immune effector cells suitable for expressing a CAR, in one embodiment, the immune effector cells are stimulated, e.g., to proliferate, using any of the methods known in the art or described herein, e.g., as described in the section titled "Activation and Expansion of Immune Effector Cells".

After enrichment of the immune effector cells suitable for expressing a CAR, and optionally, after stimulation and/or expansion as described herein, a nucleic acid encoding a CAR, e.g., a CAR described herein, can be introduced to the immune effector cells. Methods for introducing a nucleic acid, e.g., encoding a CAR, are well known in the art and described herein, e.g., as described in the sections titled "Nucleic Acid Constructs Encoding a CAR", "RNA Transfection", and "Non-viral Delivery Methods".

### Sources of Immune Effector Cells

This section provides additional methods or steps for obtaining an input sample comprising desired immune effector cells, isolating and processing desired immune effector cells, e.g., T cells, and removing unwanted materials, e.g., unwanted cells. The additional methods or steps described in this section can be used in combination with any of the elutriation, density gradient centrifugation, selection under flow conditions, or improved wash step described in the preceding sections.

A source of cells, e.g., T cells or natural killer (NK) cells, can be obtained from a subject. Examples of subjects include humans, monkeys, chimpanzees, dogs, cats, mice, rats, and transgenic species thereof. T cells can be obtained from a number of sources, including peripheral blood mononuclear cells, bone marrow, lymph node tissue, cord blood, thymus tissue, tissue from a site of infection, ascites, pleural effusion, spleen tissue, and tumors.

In certain aspects of the present disclosure, immune effector cells, e.g., T cells, can be obtained from a unit of blood collected from a subject using any number of techniques known to the skilled artisan, and any of the methods disclosed herein, in any combination of steps thereof. In one aspect, cells from the circulating blood of an individual are obtained by apheresis. The apheresis product typically contains lymphocytes, including T cells, monocytes, granulocytes, B cells, other nucleated white blood cells, red blood cells, and platelets. In one aspect, the cells collected by apheresis may be washed to remove the plasma fraction and, optionally, to place the cells in an appropriate buffer or media for subsequent processing steps. In one embodiment, the cells are washed with phosphate buffered saline (PBS). In an alternative embodiment, the wash solution lacks calcium and may lack magnesium or may lack many if not all divalent cations. In another embodiment, the cells are washed using the improved wash step described herein.

Initial activation steps in the absence of calcium can lead to magnified activation. As those of ordinary skill in the art would readily appreciate a washing step may be accomplished by methods known to those in the art, such as by using a semi-automated "flow-through" centrifuge (for example, the Cobe 2991 cell processor, the Baxter CytoMate, or the Haemonetics Cell Saver 5) according to the manufacturer's instructions. After washing, the cells may be resuspended in a variety of biocompatible buffers, such as, for example, Ca-free, Mg-free PBS, PlasmaLyte A, or other saline solution with or without buffer. Alternatively, the undesirable components of the apheresis sample may be removed and the cells directly resuspended in culture media.

In one aspect, desired immune effector cells, e.g., T cells, are isolated from peripheral blood lymphocytes by lysing the red blood cells and depleting the monocytes, for example, by centrifugation through a PERCOLL^{™} gradient or by counterflow centrifugal elutriation.

The methods described herein can include, e.g., selection of a specific subpopulation of immune effector cells, e.g., T cells, that are a T regulatory cell-depleted population, CD25+ depleted cells, using, e.g., a negative selection technique, e.g., described herein. In some embodiments, the population of T regulatory-depleted cells contains less than 30%, 25%, 20%, 15%, 10%, 5%, 4%, 3%, 2%, 1% of CD25+ cells.

In one embodiment, T regulatory cells, e.g., CD25+ T cells, are removed from the population using an anti-CD25 antibody, or fragment thereof, or a CD25-binding ligand, e.g. IL-2. In one embodiment, the anti-CD25 antibody, or fragment thereof, or CD25-binding ligand is conjugated to a substrate, e.g., a bead, or is otherwise coated on a substrate, e.g., a bead. In one embodiment, the anti-CD25 antibody, or fragment thereof, is conjugated to a substrate as described herein.

In one embodiment, the T regulatory cells, e.g., CD25+ T cells, are removed from the population using CD25 depleting reagent from Miltenyi^{™}. In one embodiment, the ratio of cells to CD25 depletion reagent is 1e7 cells to 20 uL, or 1e7 cells to 15 uL, or 1e7 cells to 10 uL, or 1e7 cells to 5 uL, or 1e7 cells to 2.5 uL, or 1e7 cells to 1.25 uL. In one embodiment, e.g., for T regulatory cells, e.g., CD25+ depletion, greater than 500 million cells/ml is used. In a further aspect, a concentration of cells of 600, 700, 800, or 900 million cells/ml is used.

In one embodiment, the population of immune effector cells to be depleted includes about 6 × 10⁹ CD25+ T cells. In other aspects, the population of immune effector cells to be depleted include about 1 × 10⁹ to 1× 10¹⁰ CD25+ T cell, and any integer value in between. In one embodiment, the resulting population T regulatory-depleted cells has 2 × 10⁹ T regulatory cells, e.g., CD25+ cells, or less (e.g., 1 × 10⁹, 5 × 10⁸, 1 × 10⁸, 5 × 10⁷, 1 × 10⁷, or less CD25+ cells).

In one embodiment, the T regulatory cells, e.g., CD25+ cells, are removed from the population using the CliniMAC system with a depletion tubing set, such as, e.g., tubing 162-01. In one embodiment, the CliniMAC system is run on a depletion setting such as, e.g., DEPLETION2.1.

Without wishing to be bound by a particular theory, decreasing the level of negative regulators of immune cells (e.g., decreasing the number of unwanted immune cells, e.g., T_{REG} cells), in a subject prior to apheresis or during manufacturing of a CAR-expressing cell product significantly reduces the risk of subject relapse. For example, methods of depleting T_{REG} cells are known in the art. Methods of decreasing T_{REG} cells include, but are not limited to, cyclophosphamide, anti-GITR antibody (an anti-GITR antibody described herein), CD25-depletion, mTOR inhibitor, and combinations thereof.

In some embodiments, the manufacturing methods comprise reducing the number of (e.g., depleting) T_{REG} cells prior to manufacturing of the CAR-expressing cell. For example, manufacturing methods comprise contacting the sample, e.g., the apheresis sample, with an anti-GITR antibody and/or an anti-CD25 antibody (or fragment thereof, or a CD25-binding ligand), e.g., to deplete T_{REG} cells prior to manufacturing of the CAR-expressing cell (e.g., T cell, NK cell) product.

Without wishing to be bound by a particular theory, decreasing the level of negative regulators of immune cells (e.g., decreasing the number of unwanted immune cells, e.g., T_{REG} cells), in a subject prior to apheresis or during manufacturing of a CAR-expressing cell product can reduce the risk of a subject's relapse. In an embodiment, a subject is pre-treated with one or more therapies that reduce T_{REG} cells prior to collection of cells for CAR-expressing cell product manufacturing, thereby reducing the risk of subject relapse to CAR-expressing cell treatment. In an embodiment, methods of decreasing T_{REG} cells include, but are not limited to, administration to the subject of one or more of cyclophosphamide, anti-GITR antibody, CD25-depletion, or a combination thereof. In an embodiment, methods of decreasing T_{REG} cells include, but are not limited to, administration to the subject of one or more of cyclophosphamide, anti-GITR antibody, CD25-depletion, mTOR inhibitor, or a combination thereof. Administration of one or more of cyclophosphamide, anti-GITR antibody, CD25-depletion, or a combination thereof, can occur before, during or after an infusion of the CAR-expressing cell product. Administration of one or more of cyclophosphamide, anti-GITR antibody, CD25-depletion, mTOR inhibitor, or a combination thereof, can occur before, during or after an infusion of the CAR-expressing cell product.

In some embodiments, the manufacturing methods comprise reducing the number of (e.g., depleting) T_{REG} cells prior to manufacturing of the CAR-expressing cell. For example, manufacturing methods comprise contacting the sample, e.g., the apheresis sample, with an anti-GITR antibody and/or an anti-CD25 antibody (or fragment thereof, or a CD25-binding ligand), e.g., to deplete T_{REG} cells prior to manufacturing of the CAR-expressing cell (e.g., T cell, NK cell) product.

In an embodiment, a subject is pre-treated with cyclophosphamide prior to collection of cells for CAR-expressing cell product manufacturing, thereby reducing the risk of subject relapse to CAR-expressing cell treatment (e.g., CTL019 treatment). In an embodiment, a subject is pre-treated with an anti-GITR antibody prior to collection of cells for CAR-expressing cell (e.g., T cell or NK cell) product manufacturing, thereby reducing the risk of subject relapse to CAR-expressing cell treatment.

In an embodiment, the CAR-expressing cell (e.g., T cell, NK cell) manufacturing process is modified to deplete TREG cells prior to manufacturing of the CAR-expressing cell (e.g., T cell, NK cell) product (e.g., a CTL019 product). In an embodiment, CD25-depletion is used to deplete TREG cells prior to manufacturing of the CAR-expressing cell (e.g., T cell, NK cell) product (e.g., a CTL019 product).

In one embodiment, the population of cells to be removed are neither the regulatory T cells or tumor cells, but cells that otherwise negatively affect the expansion and/or function of CART cells, e.g. cells expressing CD14, CD11b, CD33, CD15, or other markers expressed by potentially immune suppressive cells. In one embodiment, such cells are envisioned to be removed concurrently with regulatory T cells and/or tumor cells, or following said depletion, or in another order.

The methods described herein can include more than one selection step, e.g., more than one depletion step. Enrichment of a T cell population by negative selection can be accomplished, e.g., with a combination of antibodies directed to surface markers unique to the negatively selected cells. One method is cell sorting and/or selection via negative magnetic immunoadherence or flow cytometry that uses a cocktail of monoclonal antibodies directed to cell surface markers present on the cells negatively selected. For example, to enrich for CD4+ cells by negative selection, a monoclonal antibody cocktail can include antibodies to CD14, CD20, CD11b, CD16, HLA-DR, and CD8.

The methods described herein can further include removing cells from the population which express a tumor antigen, e.g., a tumor antigen that does not comprise CD25, e.g., CD19, CD30, CD38, CD123, CD20, CD14 or CD11b, to thereby provide a population of T regulatory-depleted, e.g., CD25+ depleted, and tumor antigen depleted cells that are suitable for expression of a CAR, e.g., a CAR described herein. In one embodiment, tumor antigen expressing cells are removed simultaneously with the T regulatory, e.g., CD25+ cells. For example, an anti-CD25 antibody, or fragment thereof, and an anti-tumor antigen antibody, or fragment thereof, can be attached to the same substrate, e.g., bead, which can be used to remove the cells or an anti-CD25 antibody, or fragment thereof, or the anti-tumor antigen antibody, or fragment thereof, can be attached to separate beads, a mixture of which can be used to remove the cells. In other embodiments, the removal of T regulatory cells, e.g., CD25+ cells, and the removal of the tumor antigen expressing cells is sequential, and can occur, e.g., in either order.

Also provided are methods that include removing cells from the population which express a check point inhibitor, e.g., a check point inhibitor described herein, e.g., one or more of PD1+ cells, LAG3+ cells, and TIM3+ cells, to thereby provide a population of T regulatory-depleted, e.g., CD25+ depleted cells, and check point inhibitor depleted cells, e.g., PD1+, LAG3+ and/or TIM3+ depleted cells. Exemplary check point inhibitors include PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGF (e.g., TGF beta), e.g., as described herein. In one embodiment, check point inhibitor expressing cells are removed simultaneously with the T regulatory, e.g., CD25+ cells. For example, an anti-CD25 antibody, or fragment thereof, and an anti-check point inhibitor antibody, or fragment thereof, can be attached to the same bead which can be used to remove the cells, or an anti-CD25 antibody, or fragment thereof, and the anti-check point inhibitor antibody, or fragment there, can be attached to separate beads, a mixture of which can be used to remove the cells. In other embodiments, the removal of T regulatory cells, e.g., CD25+ cells, and the removal of the check point inhibitor expressing cells is sequential, and can occur, e.g., in either order.

Methods described herein can include a positive selection step. For example, T cells can isolated by incubation with anti-CD3/anti-CD28 (e.g., 3x28)-conjugated beads, such as DYNABEADS^{®} M-450 CD3/CD28 T, for a time period sufficient for positive selection of the desired T cells. In one embodiment, the time period is about 30 minutes. In a further embodiment, the time period ranges from 30 minutes to 36 hours or longer and all integer values there between. In a further embodiment, the time period is at least 1, 2, 3, 4, 5, or 6 hours. In yet another embodiment, the time period is 10 to 24 hours, e.g., 24 hours. Longer incubation times may be used to isolate T cells in any situation where there are few T cells as compared to other cell types, such in isolating tumor infiltrating lymphocytes (TIL) from tumor tissue or from immunocompromised individuals. Further, use of longer incubation times can increase the efficiency of capture of CD8+ T cells. Thus, by simply shortening or lengthening the time T cells are allowed to bind to the CD3/CD28 beads and/or by increasing or decreasing the ratio of beads to T cells (as described further herein), subpopulations of T cells can be preferentially selected for or against at culture initiation or at other time points during the process. Additionally, by increasing or decreasing the ratio of anti-CD3 and/or anti-CD28 antibodies on the beads or other surface, subpopulations of T cells can be preferentially selected for or against at culture initiation or at other desired time points.

In one embodiment, a T cell population can be selected that expresses one or more of IFN-γ, TNFα, IL-17A, IL-2, IL-3, IL-4, GM-CSF, IL-10, IL-13, granzyme B, and perforin, or other appropriate molecules, e.g., other cytokines. Methods for screening for cell expression can be determined, e.g., by the methods described in PCT Publication No.: WO 2013/126712.

For isolation of a desired population of cells by positive or negative selection, the concentration of cells and surface (e.g., particles such as beads) can be varied. In certain aspects, it may be desirable to significantly decrease the volume in which beads and cells are mixed together (e.g., increase the concentration of cells), to ensure maximum contact of cells and beads. For example, in one aspect, a concentration of 10 billion cells/ml, 9 billion/ml, 8 billion/ml, 7 billion/ml, 6 billion/ml, or 5 billion/ml is used. In one aspect, a concentration of 1 billion cells/ml is used. In yet one aspect, a concentration of cells from 75, 80, 85, 90, 95, or 100 million cells/ml is used. In further aspects, concentrations of 125 or 150 million cells/ml can be used.

Using high concentrations can result in increased cell yield, cell activation, and cell expansion. Further, use of high cell concentrations allows more efficient capture of cells that may weakly express target antigens of interest, such as CD28-negative T cells, or from samples where there are many tumor cells present (e.g., leukemic blood, tumor tissue, etc.). Such populations of cells may have therapeutic value and would be desirable to obtain. For example, using high concentration of cells allows more efficient selection of CD8+ T cells that normally have weaker CD28 expression.

In a related aspect, it may be desirable to use lower concentrations of cells. By significantly diluting the mixture of T cells and surface (e.g., particles such as beads), interactions between the particles and cells is minimized. This selects for cells that express high amounts of desired antigens to be bound to the particles. For example, CD4+ T cells express higher levels of CD28 and are more efficiently captured than CD8+ T cells in dilute concentrations. In one aspect, the concentration of cells used is 5 × 10⁶/ml. In other aspects, the concentration used can be from about 1 × 10⁵/ml to 1 × 10⁶/ml, and any integer value in between.

In other aspects, the cells may be incubated on a rotator for varying lengths of time at varying speeds at either 2-10°C or at room temperature.

In one embodiment, a plurality of the immune effector cells of the population do not express diaglycerol kinase (DGK), e.g., is DGK-deficient. In one embodiment, a plurality of the immune effector cells of the population do not express Ikaros, e.g., is Ikaros-deficient. In one embodiment, a plurality of the immune effector cells of the population do not express DGK and Ikaros, e.g., is both DGK and Ikaros-deficient.

T cells for stimulation can also be frozen after a washing step. Wishing not to be bound by theory, the freeze and subsequent thaw step provides a more uniform product by removing granulocytes and to some extent monocytes in the cell population. After the washing step that removes plasma and platelets, the cells may be suspended in a freezing solution. While many freezing solutions and parameters are known in the art and will be useful in this context, one method involves using PBS containing 20% DMSO and 8% human serum albumin, or culture media containing 10% Dextran 40 and 5% Dextrose, 20% Human Serum Albumin and 7.5% DMSO, or 31.25% Plasmalyte-A, 31.25% Dextrose 5%, 0.45% NaCl, 10% Dextran 40 and 5% Dextrose, 20% Human Serum Albumin, and 7.5% DMSO or other suitable cell freezing media containing for example, Hespan and PlasmaLyte A, the cells then are frozen to -80°C at a rate of 1° per minute and stored in the vapor phase of a liquid nitrogen storage tank. Other methods of controlled freezing may be used as well as uncontrolled freezing immediately at -20° C or in liquid nitrogen.

In certain aspects, cryopreserved cells are thawed and washed as described herein and allowed to rest for one hour at room temperature prior to activation using the methods of the present invention.

Also contemplated in the context of the invention is the collection of blood samples or apheresis product from a subject at a time period prior to when the expanded cells as described herein might be needed. As such, the source of the cells to be expanded can be collected at any time point necessary, and desired cells, such as T cells, isolated and frozen for later use in immune effector cell therapy for any number of diseases or conditions that would benefit from immune effector cell therapy, such as those described herein. In one aspect a blood sample or an apheresis is taken from a generally healthy subject. In certain aspects, a blood sample or an apheresis is taken from a generally healthy subject who is at risk of developing a disease, but who has not yet developed a disease, and the cells of interest are isolated and frozen for later use. In certain aspects, the T cells may be expanded, frozen, and used at a later time. In certain aspects, samples are collected from a patient shortly after diagnosis of a particular disease as described herein but prior to any treatments. In a further aspect, the cells are isolated from a blood sample or an apheresis from a subject prior to any number of relevant treatment modalities, including but not limited to treatment with agents such as natalizumab, efalizumab, antiviral agents, chemotherapy, radiation, immunosuppressive agents, such as cyclosporin, azathioprine, methotrexate, mycophenolate, and FK506, antibodies, or other immunoablative agents such as CAMPATH, anti-CD3 antibodies, cytoxan, fludarabine, cyclosporin, FK506, rapamycin, mycophenolic acid, steroids, FR901228, and irradiation.

In a further aspect of the present invention, T cells are obtained from a patient directly following treatment that leaves the subject with functional T cells. In this regard, it has been observed that following certain cancer treatments, in particular treatments with drugs that damage the immune system, shortly after treatment during the period when patients would normally be recovering from the treatment, the quality of T cells obtained may be optimal or improved for their ability to expand ex vivo. Likewise, following ex vivo manipulation using the methods described herein, these cells may be in a preferred state for enhanced engraftment and in vivo expansion. Thus, it is contemplated within the context of the present invention to collect blood cells, including T cells, dendritic cells, or other cells of the hematopoietic lineage, during this recovery phase. Further, in certain aspects, mobilization (for example, mobilization with GM-CSF) and conditioning regimens can be used to create a condition in a subject wherein repopulation, recirculation, regeneration, and/or expansion of particular cell types is favored, especially during a defined window of time following therapy. Illustrative cell types include T cells, B cells, dendritic cells, and other cells of the immune system.

In one embodiment, the immune effector cells expressing a CAR molecule, e.g., a CAR molecule described herein, are obtained from a subject that has received a low, immune enhancing dose of an mTOR inhibitor. In an embodiment, the population of immune effector cells, e.g., T cells, to be engineered to express a CAR, are harvested after a sufficient time, or after sufficient dosing of the low, immune enhancing, dose of an mTOR inhibitor, such that the level of PD1 negative immune effector cells, e.g., T cells, or the ratio of PD1 negative immune effector cells, e.g., T cells/ PD1 positive immune effector cells, e.g., T cells, in the subject or harvested from the subject has been, at least transiently, increased.

In other embodiments, population of immune effector cells, e.g., T cells, which have, or will be engineered to express a CAR, can be treated ex vivo by contact with an amount of an mTOR inhibitor that increases the number of PD1 negative immune effector cells, e.g., T cells or increases the ratio of PD1 negative immune effector cells, e.g., T cells/ PD1 positive immune effector cells, e.g., T cells.

It is recognized that the methods of the application can utilize culture media conditions comprising 5% or less, for example 2%, human AB serum, and employ known culture media conditions and compositions, for example those described in Smith et al., "Ex vivo expansion of human T cells for adoptive immunotherapy using the novel Xeno-free CTS Immune Cell Serum Replacement" Clinical & Translational Immunology (2015) 4, e31; doi: 10. 103 8/cti. 2014.3 1.

In one embodiment, the methods of the application can utilize culture media conditions comprising serum-free medium. In one embodiment, the serum free medium is OpTmizer CTS (LifeTech), Immunocult XF (Stemcell technologies), CellGro (CellGenix), TexMacs (Miltenyi), Stemline (Sigma), Xvivo15 (Lonza), PrimeXV (Irvine Scientific), or StemXVivo (RandD systems). The serum-free medium can be supplemented with a serum substitute such as ICSR (immune cell serum replacement) from LifeTech. The level of serum substitute (e.g., ICSR) can be, e.g., up to 5%, e.g., about 1%, 2%, 3%, 4%, or 5%.

In one embodiment, a T cell population is diaglycerol kinase (DGK)-deficient. DGK-deficient cells include cells that do not express DGK RNA or protein, or have reduced or inhibited DGK activity. DGK-deficient cells can be generated by genetic approaches, e.g., administering RNA-interfering agents, e.g., siRNA, shRNA, miRNA, to reduce or prevent DGK expression. Alternatively, DGK-deficient cells can be generated by treatment with DGK inhibitors described herein.

In one embodiment, a T cell population is Ikaros-deficient. Ikaros-deficient cells include cells that do not express Ikaros RNA or protein, or have reduced or inhibited Ikaros activity, Ikaros-deficient cells can be generated by genetic approaches, e.g., administering RNA-interfering agents, e.g., siRNA, shRNA, miRNA, to reduce or prevent Ikaros expression. Alternatively, Ikaros-deficient cells can be generated by treatment with Ikaros inhibitors, e.g., lenalidomide.

In embodiments, a T cell population is DGK-deficient and Ikaros-deficient, e.g., does not express DGK and Ikaros, or has reduced or inhibited DGK and Ikaros activity. Such DGK and Ikaros-deficient cells can be generated by any of the methods described herein.

In an embodiment, the NK cells are obtained from the subject. In another embodiment, the NK cells are an NK cell line, e.g., NK-92 cell line (Conkwest).

### Allogeneic CAR-expressing Cells

In embodiments described herein, the immune effector cell can be an allogeneic immune effector cell, e.g., T cell or NK cell. For example, the cell can be an allogeneic T cell, e.g., an allogeneic T cell lacking expression of a functional T cell receptor (TCR) and/or human leukocyte antigen (HLA), e.g., HLA class I and/or HLA class II.

A T cell lacking a functional TCR can be, e.g., engineered such that it does not express any functional TCR on its surface, engineered such that it does not express one or more subunits that comprise a functional TCR (e.g., engineered such that it does not express (or exhibits reduced expression) of TCR alpha, TCR beta, TCR gamma, TCR delta, TCR epsilon, and/or TCR zeta) or engineered such that it produces very little functional TCR on its surface. Alternatively, the T cell can express a substantially impaired TCR, e.g., by expression of mutated or truncated forms of one or more of the subunits of the TCR. The term "substantially impaired TCR" means that this TCR will not elicit an adverse immune reaction in a host.

A T cell described herein can be, e.g., engineered such that it does not express a functional HLA on its surface. For example, a T cell described herein, can be engineered such that cell surface expression HLA, e.g., HLA class 1 and/or HLA class II, is downregulated. In some embodiments, downregulation of HLA may be accomplished by reducing or eliminating expression of beta-2 microglobulin (B2M).

In some embodiments, the T cell can lack a functional TCR and a functional HLA, e.g., HLA class I and/or HLA class II.

Modified T cells that lack expression of a functional TCR and/or HLA can be obtained by any suitable means, including a knock out or knock down of one or more subunit of TCR or HLA. For example, the T cell can include a knock down of TCR and/or HLA using siRNA, shRNA, clustered regularly interspaced short palindromic repeats (CRISPR) transcription-activator like effector nuclease (TALEN), or zinc finger endonuclease (ZFN).

In some embodiments, the allogeneic cell can be a cell which does not express or expresses at low levels an inhibitory molecule, e.g. by any method described herein. For example, the cell can be a cell that does not express or expresses at low levels an inhibitory molecule, e.g., that can decrease the ability of a CAR-expressing cell to mount an immune effector response. Examples of inhibitory molecules include PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGF (e.g., TGF beta). Inhibition of an inhibitory molecule, e.g., by inhibition at the DNA, RNA or protein level, can optimize a CAR-expressing cell performance. In embodiments, an inhibitory nucleic acid, e.g., an inhibitory nucleic acid, e.g., a dsRNA, e.g., an siRNA or shRNA, a clustered regularly interspaced short palindromic repeats (CRISPR), a transcription-activator like effector nuclease (TALEN), or a zinc finger endonuclease (ZFN), e.g., as described herein, can be used.

### siRNA and shRNA to inhibit TCR or HLA

In some embodiments, TCR expression and/or HLA expression can be inhibited using siRNA or shRNA that targets a nucleic acid encoding a TCR and/or HLA , and/or an inhibitory molecule described herein (e.g., PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGF beta), in a cell, e.g., T cell.

Expression systems for siRNA and shRNAs, and exemplary shRNAs, are described, e.g., in paragraphs 649 and 650 of International Application WO2015/142675, filed March 13, 2015, which is incorporated by reference in its entirety.

### CRISPR to inhibit TCR or HLA

"CRISPR" or "CRISPR to TCR and/or HLA" or "CRISPR to inhibit TCR and/or HLA" as used herein refers to a set of clustered regularly interspaced short palindromic repeats, or a system comprising such a set of repeats. "Cas", as used herein, refers to a CRISPR-associated protein. A "CRISPR/Cas" system refers to a system derived from CRISPR and Cas which can be used to silence or mutate a TCR and/or HLA gene, and/or an inhibitory molecule described herein (e.g., PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGF beta), in a cell, e.g., T cell.

The CRISPR/Cas system, and uses thereof, are described, e.g., in paragraphs 651-658 of International Application WO2015/142675, filed March 13, 2015, which is incorporated by reference in its entirety.

### TALEN to inhibit TCR and/or HLA

"TALEN" or "TALEN to HLA and/or TCR" or "TALEN to inhibit HLA and/or TCR" refers to a transcription activator-like effector nuclease, an artificial nuclease which can be used to edit the HLA and/or TCR gene, and/or an inhibitory molecule described herein (e.g., PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGF beta), in a cell, e.g., T cell.

TALENs , and uses thereof, are described, e.g., in paragraphs 659-665 of International Application WO2015/142675, filed March 13, 2015, which is incorporated by reference in its entirety.

### Zinc finger nuclease to inhibit HLA and/or TCR

"ZFN" or "Zinc Finger Nuclease" or "ZFN to HLA and/or TCR" or "ZFN to inhibit HLA and/or TCR" refer to a zinc finger nuclease, an artificial nuclease which can be used to edit the HLA and/or TCR gene, and/or an inhibitory molecule described herein (e.g., PD1, PD-L1, PD-L2, CTLA4, TIM3, CEACAM (e.g., CEACAM-1, CEACAM-3 and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGF beta), in a cell, e.g., T cell.

ZFNs, and uses thereof, are described, e.g., in paragraphs 666-671 of International Application WO2015/142675, filed March 13, 2015, which is incorporated by reference in its entirety.

### Telomerase expression

Telomeres play a crucial role in somatic cell persistence, and their length is maintained by telomerase (TERT). Telomere length in CLL cells may be very short (Roth et al., "Significantly shorter telomeres in T-cells of patients with ZAP-70+/CD38 chronic lymphocytic leukaemia" British Journal of Haematology, 143, 383-386., August 28 2008), and may be even shorter in manufactured CAR-expressing cells, e.g., CART19 cells, limiting their potential to expand after adoptive transfer to a patient. Telomerase expression can rescue CAR-expressing cells from replicative exhaustion.

While not wishing to be bound by any particular theory, in some embodiments, a therapeutic T cell has short term persistence in a patient, due to shortened telomeres in the T cell; accordingly, transfection with a telomerase gene can lengthen the telomeres of the T cell and improve persistence of the T cell in the patient. See Carl June, "Adoptive T cell therapy for cancer in the clinic", Journal of Clinical Investigation, 117:1466-1476 (2007). Thus, in an embodiment, an immune effector cell, e.g., a T cell, ectopically expresses a telomerase subunit, e.g., the catalytic subunit of telomerase, e.g., TERT, e.g., hTERT. In some aspects, this disclosure provides a method of producing a CAR-expressing cell, comprising contacting a cell with a nucleic acid encoding a telomerase subunit, e.g., the catalytic subunit of telomerase, e.g., TERT, e.g., hTERT. The cell may be contacted with the nucleic acid before, simultaneous with, or after being contacted with a construct encoding a CAR.

Telomerase expression may be stable (e.g., the nucleic acid may integrate into the cell's genome) or transient (e.g., the nucleic acid does not integrate, and expression declines after a period of time, e.g., several days). Stable expression may be accomplished by transfecting or transducing the cell with DNA encoding the telomerase subunit and a selectable marker, and selecting for stable integrants. Alternatively or in combination, stable expression may be accomplished by site-specific recombination, e.g., using the Cre/Lox or FLP/FRT system.

Transient expression may involve transfection or transduction with a nucleic acid, e.g., DNA or RNA such as mRNA. In some embodiments, transient mRNA transfection avoids the genetic instability sometimes associated with stable transfection with TERT. Transient expression of exogenous telomerase activity is described, e.g., in International Application WO2014/130909, which is incorporated by reference herein in its entirety. In embodiments, mRNA-based transfection of a telomerase subunit is performed according to the messenger RNA Therapeutics^{™} platform commercialized by Moderna Therapeutics. For instance, the method may be a method described in US Pat. No. 8710200, 8822663, 8680069, 8754062, 8664194, or 8680069.

In an embodiment, hTERT has the amino acid sequence of GenBank Protein ID AAC51724.1 (Meyerson et al., "hEST2, the Putative Human Telomerase Catalytic Subunit Gene, Is Up-Regulated in Tumor Cells and during Immortalization" Cell Volume 90, Issue 4, 22 August 1997, Pages 785-795):








































(SEQ ID NO: 108)

In an embodiment, the hTERT has a sequence at least 80%, 85%, 90%, 95%, 96%, 97%, 98%, or 99% identical to the sequence of SEQ ID NO: 108. In an embodiment, the hTERT has a sequence of SEQ ID NO: 108. In an embodiment, the hTERT comprises a deletion (e.g., of no more than 5, 10, 15, 20, or 30 amino acids) at the N-terminus, the C-terminus, or both. In an embodiment, the hTERT comprises a transgenic amino acid sequence (e.g., of no more than 5, 10, 15, 20, or 30 amino acids) at the N-terminus, the C-terminus, or both.

In an embodiment, the hTERT is encoded by the nucleic acid sequence of GenBank Accession No. AF018167 (Meyerson et al., "hEST2, the Putative Human Telomerase Catalytic Subunit Gene, Is Up-Regulated in Tumor Cells and during Immortalization" Cell Volume 90, Issue 4, 22 August 1997, Pages 785-795):

1 caggcagcgt ggtcctgctg cgcacgtggg aagccctggc cccggccacc cccgcgatgc
61 cgcgcgctcc ccgctgccga gccgtgcgct ccctgctgcg cagccactac cgcgaggtgc
121 tgccgctggc cacgttcgtg cggcgcctgg ggccccaggg ctggcggctg gtgcagcgcg
181 gggacccggc ggctttccgc gcgctggtgg cccagtgcct ggtgtgcgtg ccctgggacg
241 cacggccgcc ccccgccgcc ccctccttcc gccaggtgtc ctgcctgaag gagctggtgg
301 cccgagtgct gcagaggctg tgcgagcgcg gcgcgaagaa cgtgctggcc ttcggcttcg
361 cgctgctgga cggggcccgc gggggccccc ccgaggcctt caccaccagc gtgcgcagct
421 acctgcccaa cacggtgacc gacgcactgc gggggagcgg ggcgtggggg ctgctgttgc
481 gccgcgtggg cgacgacgtg ctggttcacc tgctggcacg ctgcgcgctc tttgtgctgg
541 tggctcccag ctgcgcctac caggtgtgcg ggccgccgct gtaccagctc ggcgctgcca
601 ctcaggcccg gcccccgcca cacgctagtg gaccccgaag gcgtctggga tgcgaacggg
661 cctggaacca tagcgtcagg gaggccgggg tccccctggg cctgccagcc ccgggtgcga
721 ggaggcgcgg gggcagtgcc agccgaagtc tgccgttgcc caagaggccc aggcgtggcg
781 ctgcccctga gccggagcgg acgcccgttg ggcaggggtc ctgggcccac ccgggcagga
841 cgcgtggacc gagtgaccgt ggtttctgtg tggtgtcacc tgccagaccc gccgaagaag
901 ccacctcttt ggagggtgcg ctctctggca cgcgccactc ccacccatcc gtgggccgcc
961 agcaccacgc gggcccccca tccacatcgc ggccaccacg tccctgggac acgccttgtc
1021 ccccggtgta cgccgagacc aagcacttcc tctactcctc aggcgacaag gagcagctgc
1081 ggccctcctt cctactcagc tctctgaggc ccagcctgac tggcgctcgg aggctcgtgg
1141 agaccatctt tctgggttcc aggccctgga tgccagggac tccccgcagg ttgccccgcc
1201 tgccccagcg ctactggcaa atgcggcccc tgtttctgga gctgcttggg aaccacgcgc
1261 agtgccccta cggggtgctc ctcaagacgc actgcccgct gcgagctgcg gtcaccccag
1321 cagccggtgt ctgtgcccgg gagaagcccc agggctctgt ggcggccccc gaggaggagg
1381 acacagaccc ccgtcgcctg gtgcagctgc tccgccagca cagcagcccc tggcaggtgt
1441 acggcttcgt gcgggcctgc ctgcgccggc tggtgccccc aggcctctgg ggctccaggc
1501 acaacgaacg ccgcttcctc aggaacacca agaagttcat ctccctgggg aagcatgcca
1561 agctctcgct gcaggagctg acgtggaaga tgagcgtgcg gggctgcgct tggctgcgca
1621 ggagcccagg ggttggctgt gttccggccg cagagcaccg tctgcgtgag gagatcctgg
1681 ccaagttcct gcactggctg atgagtgtgt acgtcgtcga gctgctcagg tctttctttt
1741 atgtcacgga gaccacgttt caaaagaaca ggctcttttt ctaccggaag agtgtctgga
1801 gcaagttgca aagcattgga atcagacagc acttgaagag ggtgcagctg cgggagctgt

1861 cggaagcaga ggtcaggcag catcgggaag ccaggcccgc cctgctgacg tccagactcc
1921 gcttcatccc caagcctgac gggctgcggc cgattgtgaa catggactac gtcgtgggag
1981 ccagaacgtt ccgcagagaa aagagggccg agcgtctcac ctcgagggtg aaggcactgt
2041 tcagcgtgct caactacgag cgggcgcggc gccccggcct cctgggcgcc tctgtgctgg
2101 gcctggacga tatccacagg gcctggcgca ccttcgtgct gcgtgtgcgg gcccaggacc
2161 cgccgcctga gctgtacttt gtcaaggtgg atgtgacggg cgcgtacgac accatccccc
2221 aggacaggct cacggaggtc atcgccagca tcatcaaacc ccagaacacg tactgcgtgc
2281 gtcggtatgc cgtggtccag aaggccgccc atgggcacgt ccgcaaggcc ttcaagagcc
2341 acgtctctac cttgacagac ctccagccgt acatgcgaca gttcgtggct cacctgcagg
2401 agaccagccc gctgagggat gccgtcgtca tcgagcagag ctcctccctg aatgaggcca
2461 gcagtggcct cttcgacgtc ttcctacgct tcatgtgcca ccacgccgtg cgcatcaggg
2521 gcaagtccta cgtccagtgc caggggatcc cgcagggctc catcctctcc acgctgctct
2581 gcagcctgtg ctacggcgac atggagaaca agctgtttgc ggggattcgg cgggacgggc
2641 tgctcctgcg tttggtggat gatttcttgt tggtgacacc tcacctcacc cacgcgaaaa
2701 ccttcctcag gaccctggtc cgaggtgtcc ctgagtatgg ctgcgtggtg aacttgcgga
2761 agacagtggt gaacttccct gtagaagacg aggccctggg tggcacggct tttgttcaga
2821 tgccggccca cggcctattc ccctggtgcg gcctgctgct ggatacccgg accctggagg
2881 tgcagagcga ctactccagc tatgcccgga cctccatcag agccagtctc accttcaacc
2941 gcggcttcaa ggctgggagg aacatgcgtc gcaaactctt tggggtcttg cggctgaagt
3001 gtcacagcct gtttctggat ttgcaggtga acagcctcca gacggtgtgc accaacatct
3061 acaagatcct cctgctgcag gcgtacaggt ttcacgcatg tgtgctgcag ctcccatttc
3121 atcagcaagt ttggaagaac cccacatttt tcctgcgcgt catctctgac acggcctccc
3181 tctgctactc catcctgaaa gccaagaacg cagggatgtc gctgggggcc aagggcgccg
3241 ccggccctct gccctccgag gccgtgcagt ggctgtgcca ccaagcattc ctgctcaagc
3301 tgactcgaca ccgtgtcacc tacgtgccac tcctggggtc actcaggaca gcccagacgc
3361 agctgagtcg gaagctcccg gggacgacgc tgactgccct ggaggccgca gccaacccgg
3421 cactgccctc agacttcaag accatcctgg actgatggcc acccgcccac agccaggccg
3481 agagcagaca ccagcagccc tgtcacgccg ggctctacgt cccagggagg gaggggcggc
3541 ccacacccag gcccgcaccg ctgggagtct gaggcctgag tgagtgtttg gccgaggcct
3601 gcatgtccgg ctgaaggctg agtgtccggc tgaggcctga gcgagtgtcc agccaagggc
3661 tgagtgtcca gcacacctgc cgtcttcact tccccacagg ctggcgctcg gctccacccc
3721 agggccagct tttcctcacc aggagcccgg cttccactcc ccacatagga atagtccatc
3781 cccagattcg ccattgttca cccctcgccc tgccctcctt tgccttccac ccccaccatc
3841 caggtggaga ccctgagaag gaccctggga gctctgggaa tttggagtga ccaaaggtgt
3901 gccctgtaca caggcgagga ccctgcacct ggatgggggt ccctgtgggt caaattgggg
3961 ggaggtgctg tgggagtaaa atactgaata tatgagtttt tcagttttga aaaaaaaaaa
4021 aaaaaaa
(SEQ ID NO: 23)

In an embodiment, the hTERT is encoded by a nucleic acid having a sequence at least 80%, 85%, 90%, 95%, 96%, 97%, 98%, or 99% identical to the sequence of SEQ ID NO: 23. In an embodiment, the hTERT is encoded by a nucleic acid of SEQ ID NO: 23.

### Chimeric Antigen Receptor (CAR)

The present invention provides immune effector cells (e.g., T cells, NK cells) that are engineered to contain one or more CARs that direct the immune effector cells to cancer. This is achieved through an antigen binding domain on the CAR that is specific for a cancer associated antigen. There are two classes of cancer associated antigens (tumor antigens) that can be targeted by the CARs described herein: (1) cancer associated antigens that are expressed on the surface of cancer cells; and (2) cancer associated antigens that itself is intracellar, however, a fragment of such antigen (peptide) is presented on the surface of the cancer cells by MHC (major histocompatibility complex).

Accordingly, an immune effector cell, e.g., obtained by a method described herein, can be engineered to contain a CAR that target one of the following cancer associated antigens (tumor antigens): CD19, CD123, CD22, CD30, CD171, CS-1, CLL-1, CD33, EGFRvIII, GD2, GD3, BCMA, Tn Ag, PSMA, ROR1, FLT3, FAP, TAG72, CD38, CD44v6, CEA, EPCAM, B7H3, KIT, IL-13Ra2, Mesothelin, IL-11Ra, PSCA, VEGFR2, LewisY, CD24, PDGFR-beta, PRSS21, SSEA-4, CD20, Folate receptor alpha, ERBB2 (Her2/neu), MUC1, EGFR, NCAM, Prostase, PAP, ELF2M, Ephrin B2, IGF-I receptor, CAIX, LMP2, gp100, bcr-abl, tyrosinase, EphA2, Fucosyl GM1, sLe, GM3, TGS5, HMWMAA, o-acetyl-GD2, Folate receptor beta, TEM1/CD248, TEM7R, CLDN6, TSHR, GPRC5D, CXORF61, CD97, CD179a, ALK, Plysialic acid, PLAC1, GloboH, NY-BR-1, UPK2, HAVCR1, ADRB3, PANX3, GPR20, LY6K, OR51E2, TARP, WT1, NY-ESO-1, LAGE-1a, legumain, HPV E6,E7, MAGE-A1, MAGE A1, ETV6-AML, sperm protein 17, XAGE1, Tie 2, MAD-CT-1, MAD-CT-2, Fos-related antigen 1, p53, p53 mutant, prostein, survivin and telomerase, PCTA-1/Galectin 8, MelanA/MART1, Ras mutant, hTERT, sarcoma translocation breakpoints, ML-IAP, ERG (TMPRSS2 ETS fusion gene), NA17, PAX3, Androgen receptor, Cyclin B1, MYCN, RhoC, TRP-2, CYP1B1, BORIS, SART3, PAX5, OY-TES1, LCK, AKAP-4, SSX2, RAGE-1, human telomerase reverse transcriptase, RU1, RU2, intestinal carboxyl esterase, and mut hsp70-2.

### Bispecific CARs

In an embodiment a multispecific antibody molecule is a bispecific antibody molecule. A bispecific antibody has specificity for no more than two antigens. A bispecific antibody molecule is characterized by a first immunoglobulin variable domain sequence which has binding specificity for a first epitope and a second immunoglobulin variable domain sequence that has binding specificity for a second epitope. In an embodiment the first and second epitopes are on the same antigen, e.g., the same protein (or subunit of a multimeric protein). In an embodiment the first and second epitopes overlap. In an embodiment the first and second epitopes do not overlap. In an embodiment the first and second epitopes are on different antigens, e.g., different proteins (or different subunits of a multimeric protein). In an embodiment a bispecific antibody molecule comprises a heavy chain variable domain sequence and a light chain variable domain sequence which have binding specificity for a first epitope and a heavy chain variable domain sequence and a light chain variable domain sequence which have binding specificity for a second epitope. In an embodiment a bispecific antibody molecule comprises a half antibody having binding specificity for a first epitope and a half antibody having binding specificity for a second epitope. In an embodiment a bispecific antibody molecule comprises a half antibody, or fragment thereof, having binding specificity for a first epitope and a half antibody, or fragment thereof, having binding specificity for a second epitope. In an embodiment a bispecific antibody molecule comprises a scFv, or fragment thereof, have binding specificity for a first epitope and a scFv, or fragment thereof, have binding specificity for a second epitope.

In certain embodiments, the antibody molecule is a multi-specific (e.g., a bispecific or a trispecific) antibody molecule. Protocols for generating bispecific or heterodimeric antibody molecules, and various configurations for bispecific antibody molecules, are described in, e.g., paragraphs 455-458 of WO2015/142675, filed March 13, 2015, which is incorporated by reference in its entirety.

In one aspect, the bispecific antibody molecule is characterized by a first immunoglobulin variable domain sequence, e.g., a scFv, which has binding specificity for CD19, e.g., comprises a scFv as described herein, or comprises the light chain CDRs and/or heavy chain CDRs from a scFv described herein, and a second immunoglobulin variable domain sequence that has binding specificity for a second epitope on a different antigen.

### Chimeric TCR

In one aspect, the antibodies and antibody fragments of the present invention (e.g., CD19 antibodies and fragments) can be grafted to one or more constant domain of a T cell receptor ("TCR") chain, for example, a TCR alpha or TCR beta chain, to create a chimeric TCR. Without being bound by theory, it is believed that chimeric TCRs will signal through the TCR complex upon antigen binding. For example, an scFv as disclosed herein, can be grafted to the constant domain, e.g., at least a portion of the extracellular constant domain, the transmembrane domain and the cytoplasmic domain, of a TCR chain, for example, the TCR alpha chain and/or the TCR beta chain. As another example, an antibody fragment, for example a VL domain as described herein, can be grafted to the constant domain of a TCR alpha chain, and an antibody fragment, for example a VH domain as described herein, can be grafted to the constant domain of a TCR beta chain (or alternatively, a VL domain may be grafted to the constant domain of the TCR beta chain and a VH domain may be grafted to a TCR alpha chain). As another example, the CDRs of an antibody or antibody fragment may be grafted into a TCR alpha and/or beta chain to create a chimeric TCR. For example, the LCDRs disclosed herein may be grafted into the variable domain of a TCR alpha chain and the HCDRs disclosed herein may be grafted to the variable domain of a TCR beta chain, or vice versa. Such chimeric TCRs may be produced, e.g., by methods known in the art (For example, Willemsen RA et al, Gene Therapy 2000; 7: 1369-1377; Zhang T et al, Cancer Gene Ther 2004; 11: 487-496; Aggen et al, Gene Ther. 2012 Apr;19(4):365-74).

### Non-Antibody Scaffolds

In embodiments, the antigen binding domain comprises a non-antibody scaffold, e.g., a fibronectin, ankyrin, domain antibody, lipocalin, small modular immuno-pharmaceutical, maxybody, Protein A, or affilin. The non-antibody scaffold has the ability to bind to target antigen on a cell. In embodiments, the antigen binding domain is a polypeptide or fragment thereof of a naturally occurring protein expressed on a cell. In some embodiments, the antigen binding domain comprises a non-antibody scaffold. A wide variety of non-antibody scaffolds can be employed so long as the resulting polypeptide includes at least one binding region which specifically binds to the target antigen on a target cell.

Non-antibody scaffolds include: fibronectin (Novartis, MA), ankyrin (Molecular Partners AG, Zurich, Switzerland), domain antibodies (Domantis, Ltd., Cambridge, MA, and Ablynx nv, Zwijnaarde, Belgium), lipocalin (Pieris Proteolab AG, Freising, Germany), small modular immuno-pharmaceuticals (Trubion Pharmaceuticals Inc., Seattle, WA), maxybodies (Avidia, Inc., Mountain View, CA), Protein A (Affibody AG, Sweden), and affilin (gammacrystallin or ubiquitin) (Scil Proteins GmbH, Halle, Germany).

In an embodiment the antigen binding domain comprises the extracellular domain, or a counter-ligand binding fragment thereof, of molecule that binds a counterligand on the surface of a target cell.

The immune effector cells can comprise a recombinant DNA construct comprising sequences encoding a CAR, wherein the CAR comprises an antigen binding domain (e.g., antibody or antibody fragment, TCR or TCR fragment) that binds specifically to a tumor antigen, e.g., an tumor antigen described herein, and an intracellular signaling domain. The intracellular signaling domain can comprise a costimulatory signaling domain and/or a primary signaling domain, e.g., a zeta chain. As described elsewhere, the methods described herein can include transducing a cell, e.g., from the population of T regulatory-depleted cells, with a nucleic acid encoding a CAR, e.g., a CAR described herein.

In specific aspects, a CAR comprises a scFv domain, wherein the scFv may be preceded by an optional leader sequence such as provided in SEQ ID NO: 1, and followed by an optional hinge sequence such as provided in SEQ ID NO:2 or SEQ ID NO:36 or SEQ ID NO:38, a transmembrane region such as provided in SEQ ID NO:6, an intracellular signalling domain that includes SEQ ID NO:7 or SEQ ID NO:16 and a CD3 zeta sequence that includes SEQ ID NO:9 or SEQ ID NO:10, e.g., wherein the domains are contiguous with and in the same reading frame to form a single fusion protein.

In one aspect, an exemplary CAR constructs comprise an optional leader sequence (e.g., a leader sequence described herein), an extracellular antigen binding domain (e.g., an antigen binding domain described herein), a hinge (e.g., a hinge region described herein), a transmembrane domain (e.g., a transmembrane domain described herein), and an intracellular stimulatory domain (e.g., an intracellular stimulatory domain described herein). In one aspect, an exemplary CAR construct comprises an optional leader sequence (e.g., a leader sequence described herein), an extracellular antigen binding domain (e.g., an antigen binding domain described herein), a hinge (e.g., a hinge region described herein), a transmembrane domain (e.g., a transmembrane domain described herein), an intracellular costimulatory signaling domain (e.g., a costimulatory signaling domain described herein) and/or an intracellular primary signaling domain (e.g., a primary signaling domain described herein).

An exemplary leader sequence is provided as SEQ ID NO: 1. An exemplary hinge/spacer sequence is provided as SEQ ID NO: 2 or SEQ ID NO:36 or SEQ ID NO:38. An exemplary transmembrane domain sequence is provided as SEQ ID NO:6. An exemplary sequence of the intracellular signaling domain of the 4-1BB protein is provided as SEQ ID NO: 7. An exemplary sequence of the intracellular signaling domain of CD27 is provided as SEQ ID NO:16. An exemplary CD3zeta domain sequence is provided as SEQ ID NO: 9 or SEQ ID NO: 10.

In one aspect, the immune effector cell comprises a recombinant nucleic acid construct comprising a nucleic acid molecule encoding a CAR, wherein the nucleic acid molecule comprises a nucleic acid sequence encoding an antigen binding domain, wherein the sequence is contiguous with and in the same reading frame as the nucleic acid sequence encoding an intracellular signaling domain. An exemplary intracellular signaling domain that can be used in the CAR includes, but is not limited to, one or more intracellular signaling domains of, e.g., CD3-zeta, CD28, CD27, 4-1BB, and the like. In some instances, the CAR can comprise any combination of CD3-zeta, CD28, 4-1BB, and the like.

The nucleic acid sequences coding for the desired molecules can be obtained using recombinant methods known in the art, such as, for example by screening libraries from cells expressing the nucleic acid molecule, by deriving the nucleic acid molecule from a vector known to include the same, or by isolating directly from cells and tissues containing the same, using standard techniques. Alternatively, the nucleic acid of interest can be produced synthetically, rather than cloned.

Nucleic acids encoding a CAR can be introduced into the immune effector cells using, e.g., a retroviral or lentiviral vector construct.

Nucleic acids encoding a CAR can also be introduced into the immune effector cell using, e.g., an RNA construct that can be directly transfected into a cell. A method for generating mRNA for use in transfection involves *in vitro* transcription (IVT) of a template with specially designed primers, followed by polyA addition, to produce a construct containing 3' and 5' untranslated sequence ("UTR") (e.g., a 3' and/or 5' UTR described herein), a 5' cap (e.g., a 5' cap described herein) and/or Internal Ribosome Entry Site (IRES) (e.g., an IRES described herein), the nucleic acid to be expressed, and a polyA tail, typically 50-2000 bases in length (e.g., described in the Examples, e.g., SEQ ID NO:35). RNA so produced can efficiently transfect different kinds of cells. In one embodiment, the template includes sequences for the CAR. In an embodiment, an RNA CAR vector is transduced into a cell, e.g., a T cell by electroporation.

### Antigen binding domain

In one aspect, a plurality of the immune effector cells, e.g., the population of T regulatory-depleted cells, include a nucleic acid encoding a CAR that comprises a targetspecific binding element otherwise referred to as an antigen binding domain. The choice of binding element depends upon the type and number of ligands that define the surface of a target cell. For example, the antigen binding domain may be chosen to recognize a ligand that acts as a cell surface marker on target cells associated with a particular disease state. Thus, examples of cell surface markers that may act as ligands for the antigen binding domain in a CAR described herein include those associated with viral, bacterial and parasitic infections, autoimmune disease and cancer cells.

In one aspect, the portion of the CAR comprising the antigen binding domain comprises an antigen binding domain that targets a tumor antigen, e.g., a tumor antigen described herein.

The antigen binding domain can be any domain that binds to the antigen including but not limited to a monoclonal antibody, a polyclonal antibody, a recombinant antibody, a human antibody, a humanized antibody, and a functional fragment thereof, including but not limited to a single-domain antibody such as a heavy chain variable domain (VH), a light chain variable domain (VL) and a variable domain (VHH) of camelid derived nanobody, and to an alternative scaffold known in the art to function as antigen binding domain, such as a recombinant fibronectin domain, a T cell receptor (TCR), or a fragment there of, e.g., single chain TCR, and the like. In some instances, it is beneficial for the antigen binding domain to be derived from the same species in which the CAR will ultimately be used in. For example, for use in humans, it may be beneficial for the antigen binding domain of the CAR to comprise human or humanized residues for the antigen binding domain of an antibody or antibody fragment.

In an embodiment, the antigen binding domain comprises an anti-CD19 antibody, or fragment thereof, e.g., an scFv. For example, the antigen binding domain comprises a variable heavy chain and a variable light chain listed in Table 1. The linker sequence joining the variable heavy and variable light chains can be, e.g., any of the linker sequences described herein, or alternatively, can be GSTSGSGKPGSGEGSTKG (SEQ ID NO: 104).

**Table 1: Anti-CD19 antibody binding domains**

| | | |
|---|---|---|
| CD19 | huscFv1 (SEQ ID NO: 39) | EIVMTQSPATLSLSPGERATLSCRASQDISKYLNWYQQKPGQAPRLLIYHTSRL HSGIPARFSGSGSGTDYTLTISSLQPEDFAVYFCQQGNTLPYTFGQGTKLEIKG GGGSGGGGSGGGGSQVQLQESGPGLVKPSETLSLTCTVSGVSLPDYGVSWIRQP PGKGLEWIGVIWGSETTYYSSSLKSRVTISKDNSKNQVSLKLSSVTAADTAVYY CAKHYYYGGSYAMDYWGQGTLVTVSS |
| CD19 | huscFv2 | Eivmtqspatlslspgeratlscrasqdiskylnwyqqkpgqaprlliyhtsrlhsgip arfsgsgsgtdytltisslqpedfavyfcqqgntlpytfgqgtkleikggggsggggsg |
| | (SEQ ID NO: 40) | gggsqvqlqesgpglvkpsetlsltctvsgvslpdygvswirqppgkglewigviwgse ttyyqsslksrvtiskdnsknqvslklssvtaadtavyycakhyyyggsyamdywgqgt lvtvss |
| CD19 | huscFv3 (SEQ ID NO: 41) | Qvqlqesgpglvkpsetlsltctvsgvslpdygvswirqppgkglewigviwgsettyy ssslksrvtiskdnsknqvslklssvtaadtavyycakhyyyggsyamdywgqgtlvtv ssggggsggggsggggseivmtqspatls_spgeratlscrasqdiskylnwyqqkpgq aprlliyhtsrlhsgiparfsgsgsgtdytltisslqpedfavyfcqqgntlpytfgqg tkleik |
| CD19 | huscFv4 (SEQ ID NO: 42) | Qvqlqesgpglvkpsetlsltctvsgvslpdygvswirqppgkglewigviwgsettyy qsslksrvtiskdnsknqvslklssvtaadtavyycakhyyyggsyamdywgqgtlvtv ssggggsggggsggggseivmtqspatls_spgeratlscrasqdiskylnwyqqkpgq aprlliyhtsrlhsgiparfsgsgsgtdytltisslqpedfavyfcqqgntlpytfgqg tkleik |
| CD19 | huscFv5 (SEQ ID NO: 43) | Eivmtqspatlslspgeratlscrasqdiskylnwyqqkpgqaprlliyhtsrlhsgip arfsgsgsgtdytltisslqpedfavyfcqqgntlpytfgqgtkleikggggsggggsg gggsggggsqvqlqesgpglvkpsetlsltctvsgvslpdygvswirqppgkglewigv iwgsettyyssslksrvtiskdnsknqvslklssvtaadtavyycakhyyyggsyamdy wgqgtlvtvss |
| CD19 | huscFv6 (SEQ ID NO: 44) | Eivmtqspatlslspgeratlscrasqdiskylnwyqqkpgqaprlliyhtsrlhsgip arfsgsgsgtdytltisslqpedfavyfcqqgntlpytfgqgtkleikggggsggggsg gggsggggsqvqlqesgpglvkpsetlsltctvsgvslpdygvswirqppgkglewigv iwgsettyyqsslksrvtiskdnsknqvslklssvtaadtavyycakhyyyggsyamdy wgqgtlvtvss |
| CD19 | huscFv7 (SEQ ID NO: 45) | Qvqlqesgpglvkpsetlsltctvsgvslpdygvswirqppgkglewigviwgsettyy ssslksrvtiskdnsknqvslklssvtaadtavyycakhyyyggsyamdywgqgtlvtv ssggggsggggsggggsggggseivmtqspatlslspgeratlscrasqdiskylnwyq qkpgqaprllliyhtsrlhsgiparfsgsgsgtdytltisslqpedfavyfcqqgntlpy tfgqgtkleik |
| CD19 | huscFv8 (SEQ ID NO: 46) | Qvqlqesgpglvkpsetlsltctvsgvslpdygvswirqppgkglewigviwgsettyy qsslksrvtiskdnsknqvslklssvtaadtavyycakhyyyggsyamdywgqgtlvtv ssggggsggggsggggsggggseivmtqspatlslspgeratlscrasqdiskylnwyq qkpgqaprlliyhtsrlhsgiparfsgsgsgtdytltisslqpedfavyfcqqgntlpy tfgqgtkleik |
| CD19 | huscFv9 (SEQ ID NO: 47) | Eivmtqspatlslspgeratlscrasqdiskylnwyqqkpgqaprlliyhtsrlhsgip arfsgsgsgtdytltisslqpedfavyfcqqgntlpytfgqgtkleikggggsggggsg gggsggggsqvqlqesgpglvkpsetlsltctvsgvslpdygvswirqppgkglewigv iwgsettyynsslksrvtiskdnsknqvslklssvtaadtavyycakhyyyggsyamdy wgqgtlvtvss |
| CD19 | Hu scFv10 (SEQ ID NO: 48) | Qvqlqesgpglvkpsetlsltctvsgvslpdygvswirqppgkglewigviwgsettyy nsslksrvtiskdnsknqvslklssvtaadtavyycakhyyyggsyamdywgqgtlvtv ssggggsggggsggggsggggseivmtqspatlslspgeratlscrasqdiskylnwyq qkpgqaprllliyhtsrlhsgiparfsgsgsgtdytltisslqpedfavyfcqqgntlpy tfgqgtkleik |
| CD19 | Hu scFv11 (SEQ ID NO: 49) | Eivmtqspatlslspgeratlscrasqdiskylnwyqqkpgqaprlliyhtsrlhsgip arfsgsgsgtdytltisslqpedfavyfcqqgntlpytfgqgtkleikggggsggggsg gggsqvqlqesgpglvkpsetlsltctvsgvslpdygvswirqppgkglewigviwgse ttyynsslksrvtiskdnsknqvslklssvtaadtavyycakhyyyggsyamdywgqgt lvtvss |
| CD19 | Hu scFv12 (SEQ ID NO: 50) | Qvqlqesgpglvkpsetlsltctvsgvslpdygvswirqppgkglewigviwgsettyy nsslksrvtiskdnsknqvslklssvtaadtavyycakhyyyggsyamdywgqgtlvtv ssggggsggggsggggseivmtqspatlslspgeratlscrasqdiskylnwyqqkpgq aprlliyhtsrlhsgiparfsgsgsgtdytltisslqpedfavyfcqqgntlpytfgqg tkleik |
| CD19 | muCTL0 19 (SEQ ID NO: 51) | Diqmtqttsslsaslgdrvtiscrasqdiskylnwyqqkpdgtvklliyhtsrlhsgvp srfsgsgsgtdysltisnleqediatyfcqqgntlpytfgggtkleitggggsggggsg gggsevklqesgpglvapsqslsvtctvsgvslpdygvswirqpprkglewlgviwgse ttyynsalksrltiikdnsksqvflkmnslqtddtaiyycakhyyyggsyamdywgqgt |
| | | svtvss |

**Table 2: Additional anti-CD19 antibody binding domains**

| Antibody | VH Sequence | VL Sequence |
|---|---|---|
| SSJ25-C1 | QVQLLESGAELVRPGSSVKISCKASGYAFSS YWMNWVKQRPGQGLEWIGQIYPGDGDTNYNG KFKGQATLTADKSSSTAYMQLSGLTSEDSAV YSCARKTISSVVDFYFDYWGQGTTVT (SEQ ID NO: 3) | ELVLTQSPKFMSTSVGDRVSVTCKASQNVGTNVA WYQQKPGQSPKPL[?]IYSATYRNSGVPDRFTGSGSG TDFTLTITNVQSKDLADYFYFCQYNRYPYTSGGG TKLEIKRRS (SEQ ID NO: 4) |

**Table 2A: Additional murine anti-CD19 antibody binding domains**

| | | |
|---|---|---|
| **mCAR1 scFv** | SEQ ID NO: 124 | QVQLLESGAELVRPGSSVKISCKASGYAFSSYWMNWVKQRPGQGLEWIGQIYPGDG DTNYNGKFKGQATLTADKSSSTAYMQLSGLTSEDSAVYSCARKTISSVVDFYFDYW GQGTTVTGGGSGGGSGGGSGGGSELVLTQSPKFMSTSVGDRVSVTCKASQNVGTNV AWYQQKPGQSPKPLIYSATYRNSGVPDRFTGSGSGTDFTLTITNVQSKDLADYFCQ YNRYPYTSFFFTKLEIKRRS |
| **mCAR2 scFv** | SEQ ID NO: 125 | DIQMTQTTSSLSASLGDRVTISCRASQDISKYLNWYQQKPDGTVKLLIYHTSRLHS GVPSRFSGSGSGTDYSLTISNLEQEDIATYFCQQGNTLPYTFGGGTKLEITGSTSG SGKPGSGEGSTKGEVKLQESGPGLVAPSQSLSVTCTVSGVSLPDYGVSWIRQPPRK GLEWLGVIWGSETTYYNSALKSRLTIIKDNSKSQVFLKMNSLQTDDTAIYYCAKHY YYGGSYAMDYWGQGTSVTVSSE |
| **mCAR3 scFv** | SEQ ID NO: 126 | DIQMTQTTSSLSASLGDRVTISCRASQDISKYLNWYQQKPDGTVKLLIYHTSRLHS GVPSRFSGSGSGTDYSLTISNLEQEDIATYFCQQGNTLPYTFGGGTKLEITGSTSG SGKPGSGEGSTKGEVKLQESGPGLVAPSQSLSVTCTVSGVSLPDYGVSWIRQPPRK GLEWLGVIWGSETTYYNSALKSRLTIIKDNSKSQVFLKMNSLQTDDTAIYYCAKHY YYGGSYAMDYWGQGTSVTVSS |

Any CD19 CAR, e.g., the CD19 antigen binding domain of any known CD19 CAR, can be used in accordance with the present disclosure. For example, LG-740; CD19 CAR described in the US Pat. No. 8,399,645; US Pat. No. 7,446,190; Xu et al., Leuk Lymphoma. 2013 54(2):255-260(2012); Cruz et al., Blood 122(17):2965-2973 (2013); Brentjens et al., Blood, 118(18):4817-4828 (2011); Kochenderfer et al., Blood 116(20):4099-102 (2010); Kochenderfer et al., Blood 122 (25):4129-39(2013); and 16th Annu Meet Am Soc Gen Cell Ther (ASGCT) (May 15-18, Salt Lake City) 2013, Abst 10.

Exemplary target antigens that can be targeted using the CAR-expressing cells, include, but are not limited to, CD19, CD123, EGFRvIII, mesothelin, among others, as described in, for example, WO 2014/130635, WO 2014/130657, and WO 2015/090230, each of which is herein incorporated by reference in its entirety.

In one embodiment, the CAR T cell that specifically binds to CD19 has the USAN designation TISAGENLECLEUCEL-T. CTL019 is made by a gene modification of T cells is mediated by stable insertion via transduction with a self-inactivating, replication deficient Lentiviral (LV) vector containing the CTL019 transgene under the control of the EF-1 alpha promoter. CTL019 can be a mixture of transgene positive and negative T cells that are delivered to the subject on the basis of percent transgene positive T cells.

In other embodiments, the CAR-expressing cells can specifically bind to human CD19, *e.g.,* can include a CAR molecule, or an antigen binding domain (e.g., a humanized antigen binding domain) according to Table 3 of WO2014/153270, incorporated herein by reference.

In other embodiments, the CAR-expressing cells can specifically bind to CD123, *e.g.,* can include a CAR molecule (*e.g.,* any of the CAR1-CAR8), or an antigen binding domain according to Tables 1-2 of WO 2014/130635, incorporated herein by reference.

In an embodiment, the CAR molecule comprises a CD123 CAR described herein, e.g., a CD123 CAR described in US2014/0322212A1 or US2016/0068601A1, both incorporated herein by reference. In embodiments, the CD123 CAR comprises an amino acid, or has a nucleotide sequence shown in US2014/0322212A1 or US2016/0068601A1, both incorporated herein by reference.

In other embodiments, the CAR-expressing cells can specifically bind to EGFRvIII, *e.g.,* can include a CAR molecule, or an antigen binding domain according to Table 2 or SEQ ID NO:11 of WO 2014/130657, incorporated herein by reference.

In an embodiment, the CAR molecule comprises an EGFRvIII CAR molecule described herein, e.g., an EGFRvIII CAR described US2014/0322275A1, incorporated herein by reference. In embodiments, the EGFRvIII CAR comprises an amino acid, or has a nucleotide sequence shown in US2014/0322275A1, incorporated herein by reference.

In other embodiments, the CAR-expressing cells can specifically bind to mesothelin, *e.g.,* can include a CAR molecule, or an antigen binding domain according to Tables 2-3 of WO 2015/090230, incorporated herein by reference.

In an embodiment, the CAR molecule comprises a mesothelin CAR described herein, e.g., a mesothelin CAR described in WO 2015/090230, incorporated herein by reference. In embodiments, the mesothelin CAR comprises an amino acid, or has a nucleotide sequence shown in WO 2015/090230, incorporated herein by reference.

In one embodiment, CAR molecule comprises a BCMA CAR molecule described herein, e.g., a BCMA CAR described in US-2016-0046724-A1. In embodiments, the BCMA CAR comprises an amino acid, or has a nucleotide sequence shown in US-2016-0046724-A1, incorporated herein by reference.

In an embodiment, the CAR molecule comprises a CLL1 CAR described herein, e.g., a CLL1 CAR described in US2016/0051651A1, incorporated herein by reference. In embodiments, the CLL1 CAR comprises an amino acid, or has a nucleotide sequence shown in US2016/0051651A1, incorporated herein by reference.

In an embodiment, the CAR molecule comprises a CD33 CAR described herein, e.ga CD33 CAR described in US2016/0096892A1, incorporated herein by reference. In embodiments, the CD33 CAR comprises an amino acid, or has a nucleotide sequence shown in US2016/0096892A1, incorporated herein by reference.

In accordance with any method or composition described herein, in embodiments, a CAR molecule comprises a CD123 CAR described herein, e.g., a CD123 CAR described in US2014/0322212A1 or US2016/0068601A1, both incorporated herein by reference. In embodiments, the CD123 CAR comprises an amino acid, or has a nucleotide sequence shown in US2014/0322212A1 or US2016/0068601A1, both incorporated herein by reference. In other embodiments, a CAR molecule comprises a CD19 CAR molecule described herein, e.g., a CD19 CAR molecule described in US-2015-0283178-A1, e.g., CTL019. In embodiments, the CD19 CAR comprises an amino acid, or has a nucleotide sequence shown in US-2015-0283178-A1, incorporated herein by reference. In one embodiment, CAR molecule comprises a BCMA CAR molecule described herein, e.g., a BCMA CAR described in US-2016-0046724-A1. In embodiments, the BCMA CAR comprises an amino acid, or has a nucleotide sequence shown in US-2016-0046724-A1, incorporated herein by reference. In an embodiment, the CAR molecule comprises a CLL1 CAR described herein, e.g., a CLL1 CAR described in US2016/0051651A1, incorporated herein by reference. In embodiments, the CLL1 CAR comprises an amino acid, or has a nucleotide sequence shown in US2016/0051651A1, incorporated herein by reference. In an embodiment, the CAR molecule comprises a CD33 CAR described herein, e.g., a CD33 CAR described in US2016/0096892A1, incorporated herein by reference. In embodiments, the CD33 CAR comprises an amino acid, or has a nucleotide sequence shown in US2016/0096892A1, incorporated herein by reference. In an embodiment, the CAR molecule comprises an EGFRvIII CAR molecule described herein, e.g., an EGFRvIII CAR described US2014/0322275A1, incorporated herein by reference. In embodiments, the EGFRvIII CAR comprises an amino acid, or has a nucleotide sequence shown in US2014/0322275A1, incorporated herein by reference. In an embodiment, the CAR molecule comprises a mesothelin CAR described herein, e.g., a mesothelin CAR described in WO 2015/090230, incorporated herein by reference. In embodiments, the mesothelin CAR comprises an amino acid, or has a nucleotide sequence shown in WO 2015/090230, incorporated herein by reference.

Exemplary CD19 CARs include CD19 CARs described herein, e.g., in one or more tables described herein, or an anti-CD19 CAR described in Xu et al. Blood 123.24(2014):3750-9; Kochenderfer et al. Blood 122.25(2013):4129-39, Cruz et al. Blood 122.17(2013):2965-73, NCT00586391, NCT01087294, NCT02456350, NCT00840853, NCT02659943, NCT02650999, NCT02640209, NCT01747486, NCT02546739, NCT02656147, NCT02772198, NCT00709033, NCT02081937, NCT00924326, NCT02735083, NCT02794246, NCT02746952, NCT01593696, NCT02134262, NCT01853631, NCT02443831, NCT02277522, NCT02348216, NCT02614066, NCT02030834, NCT02624258, NCT02625480, NCT02030847, NCT02644655, NCT02349698, NCT02813837, NCT02050347, NCT01683279, NCT02529813, NCT02537977, NCT02799550, NCT02672501, NCT02819583, NCT02028455, NCT01840566, NCT01318317, NCT01864889, NCT02706405, NCT01475058, NCT01430390, NCT02146924, NCT02051257, NCT02431988, NCT01815749, NCT02153580, NCT01865617, NCT02208362, NCT02685670, NCT02535364, NCT02631044, NCT02728882, NCT02735291, NCT01860937, NCT02822326, NCT02737085, NCT02465983, NCT02132624, NCT02782351, NCT01493453, NCT02652910, NCT02247609, NCT01029366, NCT01626495, NCT02721407, NCT01044069, NCT00422383, NCT01680991, NCT02794961, or NCT02456207, each of which is incorporated herein by reference in its entirety.

In one embodiment, the antigen binding domain comprises one, two three (e.g., all three) heavy chain CDRs, HC CDR1, HC CDR2 and HC CDR3, from an antibody described herein (e.g., an antibody described in WO2015/142675, US-2015-0283178-A1, US-2016-0046724-A1, US2014/0322212A1, US2016/0068601A1, US2016/0051651A1, US2016/0096892A1, US2014/0322275A1, or WO2015/090230, incorporated herein by reference), and/or one, two, three (e.g., all three) light chain CDRs, LC CDR1, LC CDR2 and LC CDR3, from an antibody described herein (e.g., an antibody described in WO2015/142675, US-2015-0283178-A1, US-2016-0046724-A1, US2014/0322212A1, US2016/0068601A1, US2016/0051651A1, US2016/0096892A1, US2014/0322275A1, or WO2015/090230, incorporated herein by reference). In one embodiment, the antigen binding domain comprises a heavy chain variable region and/or a variable light chain region of an antibody listed above.

In embodiments, the antigen binding domain is an antigen binding domain described in WO2015/142675, US-2015-0283178-A1, US-2016-0046724-A1, US2014/0322212A1, US2016/0068601A1, US2016/0051651A1, US2016/0096892A1, US2014/0322275A1, or WO2015/090230, incorporated herein by reference.

In embodiments, the antigen binding domain targets BCMA and is described in US-2016-0046724-A1.

In embodiments, the antigen binding domain targets CD19 and is described in US-2015-0283178-A1.

In embodiments, the antigen binding domain targets CD123 and is described in US2014/0322212A1, US2016/0068601A1.

In embodiments, the antigen binding domain targets CLL1 and is described in US2016/0051651A1.

In embodiments, the antigen binding domain targets CD33 and is described in US2016/0096892A1.

Exemplary target antigens that can be targeted using the CAR-expressing cells, include, but are not limited to, CD19, CD123, EGFRvIII, CD33, mesothelin, BCMA, and GFR ALPHA-4, among others, as described in, for example, WO2014/153270, WO 2014/130635, WO2016/028896, WO 2014/130657, WO2016/014576, WO 2015/090230, WO2016/014565, WO2016/014535, and WO2016/025880, each of which is herein incorporated by reference in its entirety.

In other embodiments, the CAR-expressing cells can specifically bind to humanized CD19, e.g., can include a CAR molecule, or an antigen binding domain (e.g., a humanized antigen binding domain) according to Table 3 of WO2014/153270, incorporated herein by reference. The amino acid and nucleotide sequences encoding the CD19 CAR molecules and antigen binding domains (e.g., including one, two, three VH CDRs; and one, two, three VL CDRs according to Kabat or Chothia), are specified in WO2014/153270.

In other embodiments, the CAR-expressing cells can specifically bind to CD123, e.g., can include a CAR molecule (e.g., any of the CAR1 to CAR8), or an antigen binding domain according to Tables 1-2 of WO 2014/130635, incorporated herein by reference. The amino acid and nucleotide sequences encoding the CD123 CAR molecules and antigen binding domains (e.g., including one, two, three VH CDRs; and one, two, three VL CDRs according to Kabat or Chothia), are specified in WO 2014/130635.

In other embodiments, the CAR-expressing cells can specifically bind to CD123, e.g., can include a CAR molecule (e.g., any of the CAR123-1 to CAR123-4 and hzCAR123-1 to hzCAR123-32), or an antigen binding domain according to Tables 2, 6, and 9 of WO2016/028896, incorporated herein by reference. The amino acid and nucleotide sequences encoding the CD123 CAR molecules and antigen binding domains (e.g., including one, two, three VH CDRs; and one, two, three VL CDRs according to Kabat or Chothia), are specified in WO2016/028896.

In other embodiments, the CAR-expressing cells can specifically bind to EGFRvIII, e.g., can include a CAR molecule, or an antigen binding domain according to Table 2 or SEQ ID NO:11 of WO 2014/130657, incorporated herein by reference. The amino acid and nucleotide sequences encoding the EGFRvIII CAR molecules and antigen binding domains (e.g., including one, two, three VH CDRs; and one, two, three VL CDRs according to Kabat or Chothia), are specified in WO 2014/130657.

In other embodiments, the CAR-expressing cells can specifically bind to CD33, e.g., can include a CAR molecule (e.g., any of CAR33-1 to CAR-33-9), or an antigen binding domain according to Table 2 or 9 of WO2016/014576, incorporated herein by reference. The amino acid and nucleotide sequences encoding the CD33 CAR molecules and antigen binding domains (e.g., including one, two, three VH CDRs; and one, two, three VL CDRs according to Kabat or Chothia), are specified in WO2016/014576.

In other embodiments, the CAR-expressing cells can specifically bind to mesothelin, e.g., can include a CAR molecule, or an antigen binding domain according to Tables 2-3 of WO 2015/090230, incorporated herein by reference. The amino acid and nucleotide sequences encoding the mesothelin CAR molecules and antigen binding domains (e.g., including one, two, three VH CDRs; and one, two, three VL CDRs according to Kabat or Chothia), are specified in WO 2015/090230.

In other embodiments, the CAR-expressing cells can specifically bind to BCMA, e.g., can include a CAR molecule, or an antigen binding domain according to Table 1 or 16, SEQ ID NO: 271 or SEQ ID NO: 273 of WO2016/014565, incorporated herein by reference. The amino acid and nucleotide sequences encoding the BCMA CAR molecules and antigen binding domains (*e.g*., including one, two, three VH CDRs; and one, two, three VL CDRs according to Kabat or Chothia), are specified in WO2016/014565.

In other embodiments, the CAR-expressing cells can specifically bind to CLL-1, e.g., can include a CAR molecule, or an antigen binding domain according to Table 2 of WO2016/014535, incorporated herein by reference. The amino acid and nucleotide sequences encoding the CLL-1 CAR molecules and antigen binding domains (e.g., including one, two, three VH CDRs; and one, two, three VL CDRs according to Kabat or Chothia), are specified in WO2016/014535.

In other embodiments, the CAR-expressing cells can specifically bind to GFR ALPHA-4, e.g., can include a CAR molecule, or an antigen binding domain according to Table 2 of WO2016/025880, incorporated herein by reference. The amino acid and nucleotide sequences encoding the GFR ALPHA-4 CAR molecules and antigen binding domains (e.g., including one, two, three VH CDRs; and one, two, three VL CDRs according to Kabat or Chothia), are specified in WO2016/025880.

In one embodiment, the antigen binding domain of any of the CAR molecules described herein (e.g., any of CD19, CD123, EGFRvIII, CD33, mesothelin, BCMA, and GFR ALPHA-4) comprises one, two three (e.g., all three) heavy chain CDRs, HC CDR1, HC CDR2 and HC CDR3, from an antibody listed above, and/or one, two, three (e.g., all three) light chain CDRs, LC CDR1, LC CDR2 and LC CDR3, from an antigen binding domain listed above. In one embodiment, the antigen binding domain comprises a heavy chain variable region and/or a variable light chain region of an antibody listed or described above.

In one embodiment, the antigen binding domain comprises one, two three (e.g., all three) heavy chain CDRs, HC CDR1, HC CDR2 and HC CDR3, from an antibody listed above, and/or one, two, three (e.g., all three) light chain CDRs, LC CDR1, LC CDR2 and LC CDR3, from an antibody listed above. In one embodiment, the antigen binding domain comprises a heavy chain variable region and/or a variable light chain region of an antibody listed or described above.

In some embodiments, the tumor antigen is a tumor antigen described in International Application WO2015/142675, filed March 13, 2015, which is herein incorporated by reference in its entirety. In some embodiments, the tumor antigen is chosen from one or more of: CD19; CD123; CD22; CD30; CD171; CS-1 (also referred to as CD2 subset 1, CRACC, SLAMF7, CD319, and 19A24); C-type lectin-like molecule-1 (CLL-1 or CLECL1); CD33; epidermal growth factor receptor variant III (EGFRvIII); ganglioside G2 (GD2); ganglioside GD3 (aNeu5Ac(2-8)aNeu5Ac(2-3)bDGalp(1-4)bDGlcp(1-1)Cer); TNF receptor family member B cell maturation (BCMA); Tn antigen ((Tn Ag) or (GalNAcα-Ser/Thr)); prostate-specific membrane antigen (PSMA); Receptor tyrosine kinase-like orphan receptor 1 (ROR1); Fms-Like Tyrosine Kinase 3 (FLT3); Tumor-associated glycoprotein 72 (TAG72); CD38; CD44v6; Carcinoembryonic antigen (CEA); Epithelial cell adhesion molecule (EPCAM); B7H3 (CD276); KIT (CD117); Interleukin-13 receptor subunit alpha-2 (IL-13Ra2 or CD213A2); Mesothelin; Interleukin 11 receptor alpha (IL-11Ra); prostate stem cell antigen (PSCA); Protease Serine 21 (Testisin or PRSS21); vascular endothelial growth factor receptor 2 (VEGFR2); Lewis(Y) antigen; CD24; Platelet-derived growth factor receptor beta (PDGFR-beta); Stage-specific embryonic antigen-4 (SSEA-4); CD20; Folate receptor alpha; Receptor tyrosine-protein kinase ERBB2 (Her2/neu); Mucin 1, cell surface associated (MUC1); epidermal growth factor receptor (EGFR); neural cell adhesion molecule (NCAM); Prostase; prostatic acid phosphatase (PAP); elongation factor 2 mutated (ELF2M); Ephrin B2; fibroblast activation protein alpha (FAP); insulin-like growth factor 1 receptor (IGF-I receptor), carbonic anhydrase IX (CAIX); Proteasome (Prosome, Macropain) Subunit, Beta Type, 9 (LMP2); glycoprotein 100 (gp100); oncogene fusion protein consisting of breakpoint cluster region (BCR) and Abelson murine leukemia viral oncogene homolog 1 (Abl) (bcr-abl); tyrosinase; ephrin type-A receptor 2 (EphA2); Fucosyl GM1; sialyl Lewis adhesion molecule (sLe); ganglioside GM3 (aNeu5Ac(2-3)bDGalp(1-4)bDGlcp(1-1)Cer); transglutaminase 5 (TGS5); high molecular weight-melanoma-associated antigen (HMWMAA); o-acetyl-GD2 ganglioside (OAcGD2); Folate receptor beta; tumor endothelial marker 1 (TEM1/CD248); tumor endothelial marker 7-related (TEM7R); claudin 6 (CLDN6); thyroid stimulating hormone receptor (TSHR); G protein-coupled receptor class C group 5, member D (GPRC5D); chromosome X open reading frame 61 (CXORF61); CD97; CD179a; anaplastic lymphoma kinase (ALK); Polysialic acid; placenta-specific 1 (PLAC1); hexasaccharide portion of globoH glycoceramide (GloboH); mammary gland differentiation antigen (NY-BR-1); uroplakin 2 (UPK2); Hepatitis A virus cellular receptor 1 (HAVCR1); adrenoceptor beta 3 (ADRB3); pannexin 3 (PANX3); G protein-coupled receptor 20 (GPR20); lymphocyte antigen 6 complex, locus K 9 (LY6K); Olfactory receptor 51E2 (OR51E2); TCR Gamma Alternate Reading Frame Protein (TARP); Wilms tumor protein (WT1); Cancer/testis antigen 1 (NY-ESO-1); Cancer/testis antigen 2 (LAGE-1a); Melanoma-associated antigen 1 (MAGE-A1); ETS translocation-variant gene 6, located on chromosome 12p (ETV6-AML); sperm protein 17 (SPA17); X Antigen Family, Member 1A (XAGE1); angiopoietin-binding cell surface receptor 2 (Tie 2); melanoma cancer testis antigen-1 (MAD-CT-1); melanoma cancer testis antigen-2 (MAD-CT-2); Fos-related antigen 1; tumor protein p53 (p53); p53 mutant; prostein; surviving; telomerase; prostate carcinoma tumor antigen-1 (PCTA-1 or Galectin 8), melanoma antigen recognized by T cells 1 (MelanA or MART1); Rat sarcoma (Ras) mutant; human Telomerase reverse transcriptase (hTERT); sarcoma translocation breakpoints; melanoma inhibitor of apoptosis (ML-IAP); ERG (transmembrane protease, serine 2 (TMPRSS2) ETS fusion gene); N-Acetyl glucosaminyl-transferase V (NA17); paired box protein Pax-3 (PAX3); Androgen receptor; Cyclin B1; v-myc avian myelocytomatosis viral oncogene neuroblastoma derived homolog (MYCN); Ras Homolog Family Member C (RhoC); Tyrosinase-related protein 2 (TRP-2); Cytochrome P450 1B1 (CYP1B1); CCCTC-Binding Factor (Zinc Finger Protein)-Like (BORIS or Brother of the Regulator of Imprinted Sites), Squamous Cell Carcinoma Antigen Recognized By T Cells 3 (SART3); Paired box protein Pax-5 (PAX5); proacrosin binding protein sp32 (OY-TES1); lymphocyte-specific protein tyrosine kinase (LCK); A kinase anchor protein 4 (AKAP-4); synovial sarcoma, X breakpoint 2 (SSX2); Receptor for Advanced Glycation Endproducts (RAGE-1); renal ubiquitous 1 (RU1); renal ubiquitous 2 (RU2); legumain; human papilloma virus E6 (HPV E6); human papilloma virus E7 (HPV E7); intestinal carboxyl esterase; heat shock protein 70-2 mutated (mut hsp70-2); CD79a; CD79b; CD72; Leukocyte-associated immunoglobulin-like receptor 1 (LAIR1); Fc fragment of IgA receptor (FCAR or CD89); Leukocyte immunoglobulin-like receptor subfamily A member 2 (LILRA2); CD300 molecule-like family member f (CD300LF); C-type lectin domain family 12 member A (CLEC12A); bone marrow stromal cell antigen 2 (BST2); EGF-like modulecontaining mucin-like hormone receptor-like 2 (EMR2); lymphocyte antigen 75 (LY75); Glypican-3 (GPC3); Fc receptor-like 5 (FCRL5); and immunoglobulin lambda-like polypeptide 1 (IGLL1).

In one embodiment, the antigen binding domain comprises one, two three (e.g., all three) heavy chain CDRs, HC CDR1, HC CDR2 and HC CDR3, from an antibody listed above, and/or one, two, three (e.g., all three) light chain CDRs, LC CDR1, LC CDR2 and LC CDR3, from an antibody listed above. In one embodiment, the antigen binding domain comprises a heavy chain variable region and/or a variable light chain region of an antibody listed or described above.

In one aspect, the anti-tumor antigen binding domain is a fragment, e.g., a single chain variable fragment (scFv). In one aspect, the anti-a cancer associate antigen as described herein binding domain is a Fv, a Fab, a (Fab')2, or a bi-functional (e.g. bi-specific) hybrid antibody (e.g., Lanzavecchia et al., Eur. J. Immunol. 17, 105 (1987)). In one aspect, the antibodies and fragments thereof of the invention binds a cancer associate antigen as described herein protein with wild-type or enhanced affinity.

In some instances, scFvs can be prepared according to a method known in the art (see, for example, Bird et al., (1988) Science 242:423-426 and Huston et al., (1988) Proc. Natl. Acad. Sci. USA 85:5879-5883). ScFv molecules can be produced by linking VH and VL regions together using flexible polypeptide linkers. The scFv molecules comprise a linker (e.g., a Ser-Gly linker) with an optimized length and/or amino acid composition. The linker length can greatly affect how the variable regions of a scFv fold and interact. In fact, if a short polypeptide linker is employed (e.g., between 5-10 amino acids) intrachain folding is prevented. Interchain folding is also required to bring the two variable regions together to form a functional epitope binding site. For examples of linker orientation and size see, e.g., Hollinger et al. 1993 Proc Natl Acad. Sci. U.S.A. 90:6444-6448, U.S. Patent Application Publication Nos. 2005/0100543, 2005/0175606, 2007/0014794, and PCT publication Nos. WO2006/020258 and WO2007/024715, which are incorporated herein by reference.

An scFv can comprise a linker of at least 1, 2, 3, 4, 5, 6, 7, 8, 9, 10, 11, 12, 13, 14, 15, 16, 17, 18, 19, 20, 25, 30, 35, 40, 45, 50, or more amino acid residues between its VL and VH regions. The linker sequence may comprise any naturally occurring amino acid. In some embodiments, the linker sequence comprises amino acids glycine and serine. In another embodiment, the linker sequence comprises sets of glycine and serine repeats such as (Gly₄Ser)n, where n is a positive integer equal to or greater than 1 (SEQ ID NO:25). In one embodiment, the linker can be (Gly₄Ser)₄ (SEQ ID NO:27) or (Gly₄Ser)₃(SEQ ID NO:28). Variation in the linker length may retain or enhance activity, giving rise to superior efficacy in activity studies.

In another aspect, the antigen binding domain is a T cell receptor ("TCR"), or a fragment thereof, for example, a single chain TCR (scTCR). Methods to make such TCRs are known in the art. See, e.g., Willemsen RA et al, Gene Therapy 7: 1369-1377 (2000); Zhang T et al, Cancer Gene Ther 11: 487-496 (2004); Aggen et al, Gene Ther. 19(4):365-74 (2012) (references are incorporated herein by its entirety). For example, scTCR can be engineered that contains the Vα and Vβ genes from a T cell clone linked by a linker (e.g., a flexible peptide). This approach is very useful to cancer associated target that itself is intracellar, however, a fragment of such antigen (peptide) is presented on the surface of the cancer cells by MHC.

### Transmembrane domain

With respect to the transmembrane domain, in various embodiments, a CAR can be designed to comprise a transmembrane domain that is attached to the extracellular domain of the CAR. A transmembrane domain can include one or more additional amino acids adjacent to the transmembrane region, e.g., one or more amino acid associated with the extracellular region of the protein from which the transmembrane was derived (e.g., 1, 2, 3, 4, 5, 6, 7, 8, 9, 10 up to 15 amino acids of the extracellular region) and/or one or more additional amino acids associated with the intracellular region of the protein from which the transmembrane protein is derived (e.g., 1, 2, 3, 4, 5, 6, 7, 8, 9, 10 up to 15 amino acids of the intracellular region). In one aspect, the transmembrane domain is one that is associated with one of the other domains of the CAR. In some instances, the transmembrane domain can be selected or modified by amino acid substitution to avoid binding of such domains to the transmembrane domains of the same or different surface membrane proteins, e.g., to minimize interactions with other members of the receptor complex. In one aspect, the transmembrane domain is capable of homodimerization with another CAR on the cell surface of a CAR-expressing cell. In a different aspect, the amino acid sequence of the transmembrane domain may be modified or substituted so as to minimize interactions with the binding domains of the native binding partner present in the same CART.

The transmembrane domain may be derived either from a natural or from a recombinant source. Where the source is natural, the domain may be derived from any membrane-bound or transmembrane protein. In one aspect the transmembrane domain is capable of signaling to the intracellular domain(s) whenever the CAR has bound to a target. A transmembrane domain of particular use in this invention may include at least the transmembrane region(s) of e.g., the alpha, beta or zeta chain of the T-cell receptor, CD28, CD27, CD3 epsilon, CD45, CD4, CD5, CD8, CD9, CD16, CD22, CD33, CD37, CD64, CD80, CD86, CD134, CD137, CD154. In some embodiments, a transmembrane domain may include at least the transmembrane region(s) of, e.g., KIR2DS2, OX40, CD2, CD27, LFA-1 (CD11a, CD18), ICOS (CD278), 4-1BB (CD137), GITR, CD40, BAFFR, HVEM (LIGHTR), SLAMF7, NKp80 (KLRF1), NKp44, NKp30, NKp46, CD160, CD19, IL2R beta, IL2R gamma, IL7R α, ITGA1, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), SLAMF6 (NTB-A, Ly108), SLAM (SLAMF1, CD150, IPO-3), BLAME (SLAMF8), SELPLG (CD162), LTBR, PAG/Cbp, NKG2D, NKG2C, or CD19.

In some instances, the transmembrane domain can be attached to the extracellular region of the CAR, e.g., the antigen binding domain of the CAR, via a hinge, e.g., a hinge from a human protein. For example, in one embodiment, the hinge can be a human Ig (immunoglobulin) hinge, e.g., an IgG4 hinge, or a CD8a hinge. In one embodiment, the hinge or spacer comprises (e.g., consists of) the amino acid sequence of SEQ ID NO:2. In one aspect, the transmembrane domain comprises (e.g., consists of) a transmembrane domain of SEQ ID NO: 6.

In one aspect, the hinge or spacer comprises an IgG4 hinge. For example, in one embodiment, the hinge or spacer comprises a hinge of the amino acid sequence ESKYGPPCPPCPAPEFLGGPSVFLFPPKPKDTLMISRTPEVTCVVVDVSQEDPEVQFNW YVDGVEVHNAKTKPREEQFNSTYRVVSVLTVLHQDWLNGKEYKCKVSNKGLPSSIEK TISKAKGQPREPQVYTLPPSQEEMTKNQVSLTCLVKGFYPSDIAVEWESNGQPENNYK TTPPVLDSDGSFFLYSRLTVDKSRWQEGNVFSCSVMHEALHNHYTQKSLSLSLGKM (SEQ ID NO:36). In some embodiments, the hinge or spacer comprises a hinge encoded by a nucleotide sequence of

GAGAGCAAGTACGGCCCTCCCTGCCCCCCTTGCCCTGCCCCCGAGTTCCTGGGCGG
ACCCAGCGTGTTCCTGTTCCCCCCCAAGCCCAAGGACACCCTGATGATCAGCCGGA
CCCCCGAGGTGACCTGTGTGGTGGTGGACGTGTCCCAGGAGGACCCCGAGGTCCA
GTTCAACTGGTACGTGGACGGCGTGGAGGTGCACAACGCCAAGACCAAGCCCCGG
GAGGAGCAGTTCAATAGCACCTACCGGGTGGTGTCCGTGCTGACCGTGCTGCACCA
GGACTGGCTGAACGGCAAGGAATACAAGTGTAAGGTGTCCAACAAGGGCCTGCCC
AGCAGCATCGAGAAAACCATCAGCAAGGCCAAGGGCCAGCCTCGGGAGCCCCAGG
TGTACACCCTGCCCCCTAGCCAAGAGGAGATGACCAAGAACCAGGTGTCCCTGAC
CTGCCTGGTGAAGGGCTTCTACCCCAGCGACATCGCCGTGGAGTGGGAGAGCAAC
GGCCAGCCCGAGAACAACTACAAGACCACCCCCCCTGTGCTGGACAGCGACGGCA
GCTTCTTCCTGTACAGCCGGCTGACCGTGGACAAGAGCCGGTGGCAGGAGGGCAA
CGTCTTTAGCTGCTCCGTGATGCACGAGGCCCTGCACAACCACTACACCCAGAAGA
GCCTGAGCCTGTCCCTGGGCAAGATG (SEQ ID NO:37).

In one aspect, the hinge or spacer comprises an IgD hinge. For example, in one embodiment, the hinge or spacer comprises a hinge of the amino acid sequence RWPESPKAQASSVPTAQPQAEGSLAKATTAPATTRNTGRGGEEKKKEKEKEEQEERET KTPECPSHTQPLGVYLLTPAVQDLWLRDKATFTCFVVGSDLKDAHLTWEVAGKVPTG GVEEGLLERHSNGSQSQHSRLTLPRSLWNAGTSVTCTLNHPSLPPQRLMALREPAAQA PVKLSLNLLASSDPPEAASWLLCEVSGFSPPNILLMWLEDQREVNTSGFAPARPPPQPG STTFWAWSVLRVPAPPSPQPATYTCVVSHEDSRTLLNASRSLEVSYVTDH (SEQ ID NO:38). In some embodiments, the hinge or spacer comprises a hinge encoded by a nucleotide sequence of

AGGTGGCCCGAAAGTCCCAAGGCCCAGGCATCTAGTGTTCCTACTGCACAGCCCCA
GGCAGAAGGCAGCCTAGCCAAAGCTACTACTGCACCTGCCACTACGCGCAATACT
GGCCGTGGCGGGGAGGAGAAGAAAAAGGAGAAAGAGAAAGAAGAACAGGAAGA
GAGGGAGACCAAGACCCCTGAATGTCCATCCCATACCCAGCCGCTGGGCGTCTATC

TCTTGACTCCCGCAGTACAGGACTTGTGGCTTAGAGATAAGGCCACCTTTACATGT
TTCGTCGTGGGCTCTGACCTGAAGGATGCCCATTTGACTTGGGAGGTTGCCGGAAA
GGTACCCACAGGGGGGGTTGAGGAAGGGTTGCTGGAGCGCCATTCCAATGGCTCT
CAGAGCCAGCACTCAAGACTCACCCTTCCGAGATCCCTGTGGAACGCCGGGACCTC
TGTCACATGTACTCTAAATCATCCTAGCCTGCCCCCACAGCGTCTGATGGCCCTTAG
AGAGCCAGCCGCCCAGGCACCAGTTAAGCTTAGCCTGAATCTGCTCGCCAGTAGTG
ATCCCCCAGAGGCCGCCAGCTGGCTCTTATGCGAAGTGTCCGGCTTTAGCCCGCCC
AACATCTTGCTCATGTGGCTGGAGGACCAGCGAGAAGTGAACACCAGCGGCTTCG
CTCCAGCCCGGCCCCCACCCCAGCCGGGTTCTACCACATTCTGGGCCTGGAGTGTC
TTAAGGGTCCCAGCACCACCTAGCCCCCAGCCAGCCACATACACCTGTGTTGTGTC
CCATGAAGATAGCAGGACCCTGCTAAATGCTTCTAGGAGTCTGGAGGTTTCCTACG
TGACTGACCATT (SEQ ID NO:103).

In one aspect, the transmembrane domain may be recombinant, in which case it will comprise predominantly hydrophobic residues such as leucine and valine. In one aspect a triplet of phenylalanine, tryptophan and valine can be found at each end of a recombinant transmembrane domain.

Optionally, a short oligo- or polypeptide linker, between 2 and 10 amino acids in length may form the linkage between the transmembrane domain and the cytoplasmic region of the CAR. A glycine-serine doublet provides a particularly suitable linker. For example, in one aspect, the linker comprises the amino acid sequence of GGGGSGGGGS (SEQ ID NO: 5). In some embodiments, the linker is encoded by a nucleotide sequence of
GGTGGCGGAGGTTCTGGAGGTGGAGGTTCC (SEQ ID NO: 8).

In one aspect, the hinge or spacer comprises a KIR2DS2 hinge.

### Cytoplasmic domain

The cytoplasmic domain or region of the CAR includes an intracellular signaling domain. An intracellular signaling domain is generally responsible for activation of at least one of the normal effector functions of the immune cell in which the CAR has been introduced.

Examples of intracellular signaling domains for use in a CAR described herein include the cytoplasmic sequences of the T cell receptor (TCR) and co-receptors that act in concert to initiate signal transduction following antigen receptor engagement, as well as any derivative or variant of these sequences and any recombinant sequence that has the same functional capability.

It is known that signals generated through the TCR alone are insufficient for full activation of the T cell and that a secondary and/or costimulatory signal is also required. Thus, T cell activation can be said to be mediated by two distinct classes of cytoplasmic signaling sequences: those that initiate antigen-dependent primary activation through the TCR (primary intracellular signaling domains) and those that act in an antigen-independent manner to provide a secondary or costimulatory signal (secondary cytoplasmic domain, e.g., a costimulatory domain).

A primary signaling domain regulates primary activation of the TCR complex either in a stimulatory way, or in an inhibitory way. Primary intracellular signaling domains that act in a stimulatory manner may contain signaling motifs which are known as immunoreceptor tyrosine-based activation motifs or ITAMs.

Examples of ITAM containing primary intracellular signaling domains that are of particular use in the invention include those of TCR zeta, FcR gamma, FcR beta, CD3 gamma, CD3 delta , CD3 epsilon, CD5, CD22, CD79a, CD79b, CD278 (also known as "ICOS"), FcεRI, DAP10, DAP12, and CD66d. In one embodiment, a CAR of the invention comprises an intracellular signaling domain, e.g., a primary signaling domain of CD3-zeta, e.g., a CD3-zeta sequence described herein.

In one embodiment, a primary signaling domain comprises a modified ITAM domain, e.g., a mutated ITAM domain which has altered (e.g., increased or decreased) activity as compared to the native ITAM domain. In one embodiment, a primary signaling domain comprises a modified ITAM-containing primary intracellular signaling domain, e.g., an optimized and/or truncated ITAM-containing primary intracellular signaling domain. In an embodiment, a primary signaling domain comprises one, two, three, four or more ITAM motifs.

### Costimulatory Signaling Domain

The intracellular signalling domain of the CAR can comprise the CD3-zeta signaling domain by itself or it can be combined with any other desired intracellular signaling domain(s) useful in the context of a CAR of the invention. For example, the intracellular signaling domain of the CAR can comprise a CD3 zeta chain portion and a costimulatory signaling domain. The costimulatory signaling domain refers to a portion of the CAR comprising the intracellular domain of a costimulatory molecule. In one embodiment, the intracellular domain is designed to comprise the signaling domain of CD3-zeta and the signaling domain of CD28. In one aspect, the intracellular domain is designed to comprise the signaling domain of CD3-zeta and the signaling domain of ICOS.

A costimulatory molecule can be a cell surface molecule other than an antigen receptor or its ligands that is required for an efficient response of lymphocytes to an antigen. Examples of such molecules include CD27, CD28, 4-1BB (CD137), OX40, CD30, CD40, PD-1, ICOS, lymphocyte function-associated antigen-1 (LFA-1), CD2, CD7, LIGHT, NKG2C, B7-H3, and a ligand that specifically binds with CD83, and the like. For example, CD27 costimulation has been demonstrated to enhance expansion, effector function, and survival of human CART cells in vitro and augments human T cell persistence and antitumor activity in vivo (Song et al. Blood. 2012; 119(3):696-706). Further examples of such costimulatory molecules include CDS, ICAM-1, GITR, BAFFR, HVEM (LIGHTR), SLAMF7, NKp80 (KLRF1), NKp30, NKp44, NKp46, CD160, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, NKG2D, NKG2C and PAG/Cbp.

The intracellular signaling sequences within the cytoplasmic portion of the CAR may be linked to each other in a random or specified order. Optionally, a short oligo- or polypeptide linker, for example, between 2 and 10 amino acids (e.g., 2, 3, 4, 5, 6, 7, 8, 9, or 10 amino acids) in length may form the linkage between intracellular signaling sequences. In one embodiment, a glycine-serine doublet can be used as a suitable linker. In one embodiment, a single amino acid, e.g., an alanine, a glycine, can be used as a suitable linker.

In one aspect, the intracellular signaling domain is designed to comprise two or more, e.g., 2, 3, 4, 5, or more, costimulatory signaling domains. In an embodiment, the two or more, e.g., 2, 3, 4, 5, or more, costimulatory signaling domains, are separated by a linker molecule, e.g., a linker molecule described herein. In one embodiment, the intracellular signaling domain comprises two costimulatory signaling domains. In some embodiments, the linker molecule is a glycine residue. In some embodiments, the linker is an alanine residue.

In one aspect, the intracellular signaling domain is designed to comprise the signaling domain of CD3-zeta and the signaling domain of CD28. In one aspect, the intracellular signaling domain is designed to comprise the signaling domain of CD3-zeta and the signaling domain of 4-1BB. In one aspect, the signaling domain of 4-1BB is a signaling domain of SEQ ID NO: 7. In one aspect, the signaling domain of CD3-zeta is a signaling domain of SEQ ID NO: 9.

In one aspect, the intracellular signaling domain is designed to comprise the signaling domain of CD3-zeta and the signaling domain of CD27. In one aspect, the signaling domain of CD27 comprises an amino acid sequence of QRRKYRSNKGESPVEPAEPCRYSCPREEEGSTIPIQEDYRKPEPACSP (SEQ ID NO:16). In one aspect, the signalling domain of CD27 is encoded by a nucleic acid sequence of AGGAGTAAGAGGAGCAGGCTCCTGCACAGTGACTACATGAACATGACTCCCCGCC GCCCCGGGCCCACCCGCAAGCATTACCAGCCCTATGCCCCACCACGCGACTTCGCA GCCTATCGCTCC (SEQ ID NO:14).

In one aspect, the CAR-expressing cell described herein can further comprise a second CAR, e.g., a second CAR that includes a different antigen binding domain, e.g., to the same target or a different target (e.g., a target other than a cancer associated antigen described herein or a different cancer associated antigen described herein, e.g., CD19, CD33, CLL-1, CD34, FLT3, or folate receptor beta). In one embodiment, the second CAR includes an antigen binding domain to a target expressed the same cancer cell type as the cancer associated antigen. In one embodiment, the CAR-expressing cell comprises a first CAR that targets a first antigen and includes an intracellular signaling domain having a costimulatory signaling domain but not a primary signaling domain, and a second CAR that targets a second, different, antigen and includes an intracellular signaling domain having a primary signaling domain but not a costimulatory signaling domain. While not wishing to be bound by theory, placement of a costimulatory signaling domain, e.g., 4-1BB, CD28, ICOS, CD27 or OX-40, onto the first CAR, and the primary signaling domain, e.g., CD3 zeta, on the second CAR can limit the CAR activity to cells where both targets are expressed. In one embodiment, the CAR expressing cell comprises a first cancer associated antigen CAR that includes an antigen binding domain that binds a target antigen described herein, a transmembrane domain and a costimulatory domain and a second CAR that targets a different target antigen (e.g., an antigen expressed on that same cancer cell type as the first target antigen) and includes an antigen binding domain, a transmembrane domain and a primary signaling domain. In another embodiment, the CAR expressing cell comprises a first CAR that includes an antigen binding domain that binds a target antigen described herein, a transmembrane domain and a primary signaling domain and a second CAR that targets an antigen other than the first target antigen (e.g., an antigen expressed on the same cancer cell type as the first target antigen) and includes an antigen binding domain to the antigen, a transmembrane domain and a costimulatory signaling domain.

In another aspect, the disclosure features a population of CAR-expressing cells, e.g., CART cells. In some embodiments, the population of CAR-expressing cells comprises a mixture of cells expressing different CARs.

For example, in one embodiment, the population of CART cells can include a first cell expressing a CAR having an antigen binding domain to a cancer associated antigen described herein, and a second cell expressing a CAR having a different antigen binding domain, e.g., an antigen binding domain to a different a cancer associated antigen described herein, e.g., an antigen binding domain to a cancer associated antigen described herein that differs from the cancer associate antigen bound by the antigen binding domain of the CAR expressed by the first cell.

As another example, the population of CAR-expressing cells can include a first cell expressing a CAR that includes an antigen binding domain to a cancer associated antigen described herein, and a second cell expressing a CAR that includes an antigen binding domain to a target other than a cancer associate antigen as described herein. In one embodiment, the population of CAR-expressing cells includes, e.g., a first cell expressing a CAR that includes a primary intracellular signaling domain, and a second cell expressing a CAR that includes a secondary signaling domain.

In another aspect, the disclosure features a population of cells wherein at least one cell in the population expresses a CAR having an antigen binding domain to a cancer associated antigen described herein, and a second cell expressing another agent, e.g., an agent which enhances the activity of a CAR-expressing cell. For example, in one embodiment, the agent can be an agent which inhibits an inhibitory molecule. Inhibitory molecules, e.g., PD-1, can, in some embodiments, decrease the ability of a CAR-expressing cell to mount an immune effector response. Examples of inhibitory molecules include PD-1, PD-L1, PD-L2, CTLA4, TIM3, CEACAM (CEACAM-1, CEACAM-3, and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGF (e.g., TGF beta). In one embodiment, the agent which inhibits an inhibitory molecule comprises a first polypeptide, e.g., an inhibitory molecule, associated with a second polypeptide that provides a positive signal to the cell, e.g., an intracellular signaling domain described herein. In one embodiment, the agent comprises a first polypeptide, e.g., of an inhibitory molecule such as PD-1, PD-L1, CTLA4, TIM3, CEACAM (CEACAM-1, CEACAM-3, and/or CEACAM-5), LAG3, VISTA, BTLA, TIGIT, LAIR1, CD160, 2B4 and TGF beta, or a fragment of any of these, and a second polypeptide which is an intracellular signaling domain described herein (e.g., comprising a costimulatory domain (e.g., 41BB, CD27, OX40 or CD28, e.g., as described herein) and/or a primary signaling domain (e.g., a CD3 zeta signaling domain described herein). In one embodiment, the agent comprises a first polypeptide of PD-1 or a fragment thereof, and a second polypeptide of an intracellular signaling domain described herein (e.g., a CD28 signaling domain described herein and/or a CD3 zeta signaling domain described herein).

The sequences of anti-CD19 binding domains are provided herein in Table 1. Full CAR constructs can be generated using any of the antigen binding domains described in Table 1 with one or more additional CAR component provided below.
- leader (amino acid sequence) (SEQ ID NO: 1)
   MALPVTALLLPLALLLHAARP
- **leader (nucleic acid sequence) (SEQ ID NO: 12)** 
ATGGCCCTGCCTGTGACAGCCCTGCTGCTGCCTCTGGCTCTGCTGCTGCATG
CCGCTAGACCC
- **leader (nucleic acid sequence 2) (SEQ ID NO: 127)** 
ATGGCCCTCCCTGTCACCGCCCTGCTGCTTCCGCTGGCTCTTCTGCTCCACGCCGCT
CGGCCC
- **leader (nucleic acid sequence 3) (SEQ ID NO: 128)** 
ATGGCCTTACCAGTGACCGCCTTGCTCCTGCCGCTGGCCTTGCTGCTCCACGCCGCC
AGGCCG
- CD8 hinge (amino acid sequence) (SEQ ID NO: 2)
   TTTPAPRPPTPAPTIASQPLSLRPEACRPAAGGAVHTRGLDFACD
- **CD8 hinge (nucleic acid sequence) (SEQ ID NO: 13)** 
ACCACGACGCCAGCGCCGCGACCACCAACACCGGCGCCCACCATCGCGTC
GCAGCCCCTGTCCCTGCGCCCAGAGGCGTGCCGGCCAGCGGCGGGGGCGCAGTG
CACACGAGGGGGCTGGACTTCGCCTGTGAT
- **CD8 hinge (nucleic acid sequence 2) (SEQ ID NO: 129)** 
ACCACTACCCCAGCACCGAGGCCACCCACCCCGGCTCCTACCATCGCCTCC
CAGCCTCTGTCCCTGCGTCCGGAGGCATGTAGACCCGCAGCTGGTGGGGCCGTGCA
TACCCGGGGTCTTGACTTCGCCTGCGAT
- CD8 transmembrane (amino acid sequence) (SEQ ID NO: 6)
   IYIWAPLAGTCGVLLLSLVITLYC
- **transmembrane (nucleic acid sequence) (SEQ ID NO: 17)** 
ATCTACATCTGGGCGCCCTTGGCCGGGACTTGTGGGGTCCTTCTCCTGTCAC
TGGTTATCACCCTTTACTGC
- **transmembrane (nucleic acid sequence 2) (SEQ ID NO: 130)** 
ATCTACATTTGGGCCCCTCTGGCTGGTACTTGCGGGGTCCTGCTGCTTTCAC
TCGTGATCACTCTTTACTGT
- 4-1BB Intracellular domain (amino acid sequence) (SEQ ID NO: 7)
   KRGRKKLLYIFKQPFMRPVQTTQEEDGCSCRFPEEEEGGCEL
- **4-1BB Intracellular domain (nucleic acid sequence) (SEQ ID NO: 18)** 
AAACGGGGCAGAAAGAAACTCCTGTATATATTCAAACAACCATTTATGAG
ACCAGTACAAACTACTCAAGAGGAAGATGGCTGTAGCTGCCGATTTCCAGAAGAA
GAAGAAGGAGGATGTGAACTG
- **4-1BB Intracellular domain (nucleic acid sequence 2) (SEQ ID NO: 131)** 
AAGCGCGGTCGGAAGAAGCTGCTGTACATCTTTAAGCAACCCTTCATGAGG
CCTGTGCAGACTACTCAAGAGGAGGACGGCTGTTCATGCCGGTTCCCAGAGGAGG
AGGAAGGCGGCTGCGAACTG
- CD3 zeta domain (amino acid sequence) (SEQ ID NO: 9) 
RVKFSRSADAPAYKQGQNQLYNELNLGRREEYDVLDKRRGRDPEMGGKPRR
KNPQEGLYNELQKDKMAEAYSEIGMKGERRRGKGHDGLYQGLSTATKDTYDALHM
QALPPR
- **CD3 zeta (nucleic acid sequence) (SEQ ID NO: 20)** 
AGAGTGAAGTTCAGCAGGAGCGCAGACGCCCCCGCGTACAAGCAGGGCCA
GAACCAGCTCTATAACGAGCTCAATCTAGGACGAAGAGAGGAGTACGATGTTTTG
GACAAGAGACGTGGCCGGGACCCTGAGATGGGGGGAAAGCCGAGAAGGAAGAAC
CCTCAGGAAGGCCTGTACAATGAACTGCAGAAAGATAAGATGGCGGAGGCCTACA
GTGAGATTGGGATGAAAGGCGAGCGCCGGAGGGGCAAGGGGCACGATGGCCTTTA
CCAGGGTCTCAGTACAGCCACCAAGGACACCTACGACGCCCTTCACATGCAGGCCC
TGCCCCCTCGC
- **CD3 zeta (nucleic acid sequence 2) (SEQ ID NO: 132)** 
CGCGTGAAATTCAGCCGCAGCGCAGATGCTCCAGCCTACAAGCAGGGGCA
GAACCAGCTCTACAACGAACTCAATCTTGGTCGGAGAGAGGAGTACGACGTGCTG
GACAAGCGGAGAGGACGGGACCCAGAAATGGGCGGGAAGCCGCGCAGAAAGAAT
CCCCAAGAGGGCCTGTACAACGAGCTCCAAAAGGATAAGATGGCAGAAGCCTATA
GCGAGATTGGTATGAAAGGGGAACGCAGAAGAGGCAAAGGCCACGACGGACTGT
ACCAGGGACTCAGCACCGCCACCAAGGACACCTATGACGCTCTTCACATGCAGGC
CCTGCCGCCTCGG
- **CD3 zeta domain (amino acid sequence; NCBI Reference Sequence NM_000734.3) (SEQ ID NO:10)** 
RVKFSRSADAPAYQQGQNQLYNELNLGRREEYDVLDKRRGRDPEMGGKPRR
KNPQEGLYNELQKDKMAEAYSEIGMKGERRRGKGHDGLYQGLSTATKDTYDALHM
QALPPR
- **CD3 zeta (nucleic acid sequence; NCBI Reference Sequence NM_000734.3); (SEQ ID NO:21)** 
agagtgaagttcagcaggagcgcagacgcccccgcgtaccagcagggccagaaccagctctataacgagctcaatctag
gacgaagagaggagtacgatgttttggacaagagacgtggccgggaccctgagatgggggaaagccgagaaggaagaaccctca
ggaaggcctgtacaatgaactgcagaaagataagatggcggaggcctacagtgagattgggatgaaaggcgagcgccggaggggca
aggggcacgatggcctttaccagggtctcagtacagccaccaaggacacctacgacgcccttcacatgcaggccctgccccctcgc
   **IgG4 Hinge (amino acid sequence) (SEQ ID NO:36)** 
ESKYGPPCPPCPAPEFLGGPSVFLFPPKPKDTLMISRTPEVTCVVVDVSQEDPEV
QFNWYVDGVEVHNAKTKPREEQFNSTYRVVSVLTVLHQDWLNGKEYKCKVSNKGLP
SSIEKTISKAKGQPREPQVYTLPPSQEEMTKNQVSLTCLVKGFYPSDIAVEWESNGQPE
NNYKTTPPVLDSDGSFFLYSRLTVDKSRWQEGNVFSCSVMHEALHNHYTQKSLSLSLG
KM
   **IgG4 Hinge (nucleotide sequence) (SEQ ID NO:37)** 
GAGAGCAAGTACGGCCCTCCCTGCCCCCCTTGCCCTGCCCCCGAGTTCCTG
GGCGGACCCAGCGTGTTCCTGTTCCCCCCCAAGCCCAAGGACACCCTGATGATCAG
CCGGACCCCCGAGGTGACCTGTGTGGTGGTGGACGTGTCCCAGGAGGACCCCGAG
GTCCAGTTCAACTGGTACGTGGACGGCGTGGAGGTGCACAACGCCAAGACCAAGC
CCCGGGAGGAGCAGTTCAATAGCACCTACCGGGTGGTGTCCGTGCTGACCGTGCTG
CACCAGGACTGGCTGAACGGCAAGGAATACAAGTGTAAGGTGTCCAACAAGGGCC
TGCCCAGCAGCATCGAGAAAACCATCAGCAAGGCCAAGGGCCAGCCTCGGGAGCC
CCAGGTGTACACCCTGCCCCCTAGCCAAGAGGAGATGACCAAGAACCAGGTGTCC
CTGACCTGCCTGGTGAAGGGCTTCTACCCCAGCGACATCGCCGTGGAGTGGGAGA
GCAACGGCCAGCCCGAGAACAACTACAAGACCACCCCCCCTGTGCTGGACAGCGA
CGGCAGCTTCTTCCTGTACAGCCGGCTGACCGTGGACAAGAGCCGGTGGCAGGAG
GGCAACGTCTTTAGCTGCTCCGTGATGCACGAGGCCCTGCACAACCACTACACCCA
GAAGAGCCTGAGCCTGTCCCTGGGCAAGATG
   EF1 alpha promoter 
CGTGAGGCTCCGGTGCCCGTCAGTGGGCAGAGCGCACATCGCCCACAGTCC
CCGAGAAGTTGGGGGGAGGGGTCGGCAATTGAACCGGTGCCTAGAGAAGGTGGCG
CGGGGTAAACTGGGAAAGTGATGTCGTGTACTGGCTCCGCCTTTTTCCCGAGGGTG
GGGGAGAACCGTATATAAGTGCAGTAGTCGCCGTGAACGTTCTTTTTCGCAACGGG
TTTGCCGCCAGAACACAGGTAAGTGCCGTGTGTGGTTCCCGCGGGCCTGGCCTCTT
TACGGGTTATGGCCCTTGCGTGCCTTGAATTACTTCCACCTGGCTGCAGTACGTGAT
TCTTGATCCCGAGCTTCGGGTTGGAAGTGGGTGGGAGAGTTCGAGGCCTTGCGCTT
AAGGAGCCCCTTCGCCTCGTGCTTGAGTTGAGGCCTGGCCTGGGCGCTGGGGCCGC
CGCGTGCGAATCTGGTGGCACCTTCGCGCCTGTCTCGCTGCTTTCGATAAGTCTCTA
GCCATTTAAAATTTTTGATGACCTGCTGCGACGCTTTTTTTCTGGCAAGATAGTCTT
GTAAATGCGGGCCAAGATCTGCACACTGGTATTTCGGTTTTTGGGGCCGCGGGCGG
CGACGGGGCCCGTGCGTCCCAGCGCACATGTTCGGCGAGGCGGGGCCTGCGAGCG
CGGCCACCGAGAATCGGACGGGGGTAGTCTCAAGCTGGCCGGCCTGCTCTGGTGC
CTGGCCTCGCGCCGCCGTGTATCGCCCCGCCCTGGGCGGCAAGGCTGGCCCGGTCG
GCACCAGTTGCGTGAGCGGAAAGATGGCCGCTTCCCGGCCCTGCTGCAGGGAGCT
CAAAATGGAGGACGCGGCGCTCGGGAGAGCGGGCGGGTGAGTCACCCACACAAA
GGAAAAGGGCCTTTCCGTCCTCAGCCGTCGCTTCATGTGACTCCACGGAGTACCGG
GCGCCGTCCAGGCACCTCGATTAGTTCTCGAGCTTTTGGAGTACGTCGTCTTTAGGT
TGGGGGGAGGGGTTTTATGCGATGGAGTTTCCCCACACTGAGTGGGTGGAGACTG
AAGTTAGGCCAGCTTGGCACTTGATGTAATTCTCCTTGGAATTTGCCCTTTTTGAGT
TTGGATCTTGGTTCATTCTCAAGCCTCAGACAGTGGTTCAAAGTTTTTTTCTTCCAT
TTCAGGTGTCGTGA (SEQ ID NO: 11).
   Gly/Ser (SEQ ID NO:25)
      GGGGS
   Gly/Ser (SEQ ID NO:26): This sequence may encompass 1-6 "Gly Gly Gly Gly Ser" repeating units
      GGGGSGGGGS GGGGSGGGGS GGGGSGGGGS
   Gly/Ser (SEQ ID NO:27)
      GGGGSGGGGS GGGGSGGGGS
   Gly/Ser (SEQ ID NO:28)
      GGGGSGGGGS GGGGS
   Gly/Ser (SEQ ID NO:29)
      GGGS
      PolyA (SEQ ID NO:30): A5000
      Poly A (SEQ ID NO:31): A100
      PolyT (SEQ ID NO:32): T5000
      Poly A (SEQ ID NO:33): A5000
      Poly A (SEQ ID NO:34): A400
      Poly A (SEQ ID NO:35)" A2000
- Gly/Ser (SEQ ID NO:15): This sequence may encompass 1-10 "Gly Gly Gly Ser" repeating units
   GGGSGGGSGG GSGGGSGGGS GGGSGGGSGG GSGGGSGGGS

Exemplary CD19 CAR constructs that can be used in the methods described herein are shown in **Table 3:**

**Table 3: CD19 CAR Constructs**

| **Name** | **SEQ ID** | **Sequence** |
|---|---|---|
| **CAR 1** | | |
| **CAR1 scFv domain** | 39 | EIVMTQSPATLSLSPGERATLSCRASQDISKYLNWYQQKPGQAPRLLIYHT SRLHSGIPARFSGSGSGTDYTLTISSLQPEDFAVYFCQQGNTLPYTFGQGT KLEIKGGGGSGGGGSGGGGSQVQLQESGPGLVKPSETLSLTCTVSGVSLPD YGVSWIRQPPGKGLEWIGVIWGSETTYYSSSLKSRVTISKDNSKNQVSLKL SSVTAADTAVYYCAKHYYYGGSYAMDYWGQGTLVTVSS |
| **103101 CAR1 Soluble scFv - nt** | 52 | atggccctccctgtcaccgccctgctgcttccgctggctcttctgctccacgccgc tcggcccgaaattgtgatgacccagtcacccgccactcttagcctttcacccggtg agcgcgcaaccctgtcttgcagagcctcccaagacatctcaaaataccttaattgg tatcaacagaagcccggacaggctcctcgccttctgatctaccacaccagccggct |
| | | ccattctggaatccctgccaggttcagcggtagcggatctgggaccgactacaccc tcactatcagctcactgcagccagaggacttcgctgtctatttctgtcagcaaggg aacaccctgccctacacctttggacagggcaccaagctcgagattaaaggtggagg tggcagcggaggaggtgggtccggcggtggaggaagccaggtccaactccaagaaa gcggaccgggtcttgtgaagccatcagaaactctttcactgacttgtactgtgagc ggagtgtctctccccgattacggggtgtcttggatcagacagccaccggggaaggg tctggaatggattggagtgatttggggctctgagactacttactactcttcatccc tcaagtcacgcgtcaccatctcaaaggacaactctaagaatcaggtgtcactgaaa ctgtcatctgtgaccgcagccgacaccgccgtgtactattgcgctaagcattacta ttatggcgggagctacgcaatggattactggggacagggtactctggtcaccgtgt ccagccaccaccatcatcaccatcaccat |
| **103101 CAR1 Soluble scFv** - **aa** | 64 | MALPVTALLLPLALLLHAARPeivmtqspatlslspgeratlscrasqdiskylnw yqqkpgqaprlliyhtsrlhsgiparfsgsgsgtdytltisslqpedfavyfcqqg ntlpytfgqgtkleikggggsggggsggggsqvqlqesgpglvkpsetlsltctvs gvslpdygvswirqppgkglewigviwgsettyyssslksrvtiskdnsknqvslk lssvtaadtavyycakhyyyggsyamdywgqgtlvtvsshhhhhhhh |
| **104875 CAR 1-Full** - **nt** | 90 | atggccctccctgtcaccgccctgctgcttccgctggctcttctgctccacgccgc tcggcccgaaattgtgatgacccagtcacccgccactcttagcctttcacccggtg agcgcgcaaccctgtcttgcagagcctcccaagacatctcaaataccttaattgg tatcaacagaagcccggacaggctcctcgccttctgatctaccacaccagccggct ccattctggaatccctgccaggttcagcggtagcggatctgggaccgactacaccc tcactatcagctcactgcagccagaggacttcgctgtctatttctgtcagcaaggg aacaccctgccctacacctttggacagggcaccaagctcgagattaaaggtggagg tggcagcggaggaggtgggtccggcggtggaggaagccaggtccaactccaagaaa gcggaccgggtcttgtgaagccatcagaaactctttcactgacttgtactgtgagc ggagtgtctctccccgattacggggtgtcttggatcagacagccaccggggaaggg tctggaatggattggagtgatttggggctctgagactacttactactcttcatccc tcaagtcacgcgtcaccatctcaaaggacaactctaagaatcaggtgtcactgaaa ctgtcatctgtgaccgcagccgacaccgccgtgtactattgcgctaagcattacta ttatggcgggagctacgcaatggattactggggacagggtactctggtcaccgtgt ccagcaccactaccccagcaccgaggccacccaccccggctcctaccatcgcctcc cagcctctgtccctgcgtccggaggcatgtagacccgcagctggtggggccgtgca tacccggggtcttgacttcgcctgcgatatctacatttgggcccctctggctggta cttgcggggtcctgctgctttcactcgtgatcactctttactgtaagcgcggtcgg aagaagctgctgtacatctttaagcaacccttcatgaggcctgtgcagactactca agaggaggacggctgttcatgccggttcccagaggaggaggaaggcggctgcgaac tgcgcgtgaaattcagccgcagcgcagatgctccagcctacaagcaggggcagaac cagctctacaacgaactcaatcttggtcggagagaggagtacgacgtgctggacaa |
| | | gcggagaggacgggacccagaaatgggcgggaagccgcgcagaaagaatccccaag agggcctgtacaacgagctccaaaaggataagatggcagaagcctatagcgagatt ggtatgaaaggggaacgcagaagaggcaaaggccacgacggactgtaccagggact cagcaccgccaccaaggacacctatgacgctcttcacatgcaggccctgccgcctc gg |
| **104875 CAR 1-Full** - **aa** | 77 | MALPVTALLLPLALLLHAARPeivmtqspatlslspgeratlscrasqdiskylnw yqqkpgqaprlliyhtsrlhsgiparfsgsgsgtdytltisslqpedfavyfcqqg ntlpytfgqgtkleikggggsggggsggggsqvqlqesgpglvkpsetlsltctvs gvslpdygvswirqppgkglewigviwgsettyyssslksrvtiskdnsknqvslk lssvtaadtavyycakhyyyggsyamdywgqgtlvtvssttpaprpptpaptias qplslrpeacrpaaggavhtrgldfacdiyiwaplagtcgvlllslvitlyckrgr kkllyifkqpfmrpvqttqeedgcscrfpeeeeggcelrvkfsrsadapaykqgqn qlynelnlgrreeydvldkrrgrdpemggkprrknpqeglynelqkdkmaeaysei gmkgerrrgkghdglyqglstatkdtydalhmqalppr |

| **CAR 2** | | |
|---|---|---|
| **CAR2 scFv domain** | 40 | eivmtqspatlslspgeratlscrasqdiskylnwyqqkpgqaprlliyhtsrlhs giparfsgsgsgtdytltisslqpedfavyfcqqgntlpytfgqgtkleikggggs ggggsggggsqvqlqesgpglvkpsetlsltctvsgvslpdygvswirqppgkgle wigviwgsettyyqsslksrvtiskdnsknqvslklssvtaadtavyycakhyyyg gsyamdywgqgtlvtvss |
| **103102 CAR2** - **Soluble scFv** - **nt** | 53 | atggccctccctgtcaccgccctgctgcttccgctggctcttctgctccacgccgc tcggcccgaaattgtgatgacccagtcacccgccactcttagcctttcacccggtg agcgcgcaaccctgtcttgcagagcctcccaagacatctcaaaataccttaattgg tatcaacagaagcccggacaggctcctcgccttctgatctaccacaccagccggct ccattctggaatccctgccaggttcagcggtagcggatctgggaccgactacaccc tcactatcagctcactgcagccagaggacttcgctgtctatttctgtcagcaaggg aacaccctgccctacacctttggacagggcaccaagctcgagattaaaggtggagg tggcagcggaggaggtgggtccggcggtggaggaagccaggtccaactccaagaaa gcggaccgggtcttgtgaagccatcagaaactctttcactgacttgtactgtgagc ggagtgtctctccccgattacggggtgtcttggatcagacagccaccggggaaggg tctggaatggattggagtgatttggggctctgagactacttactaccaatcatccc tcaagtcacgcgtcaccatctcaaaggacaactctaagaatcaggtgtcactgaaa ctgtcatctgtgaccgcagccgacaccgccgtgtactattgcgctaagcattacta ttatggcgggagctacgcaatggattactggggacagggtactctggtcaccgtgt ccagccaccaccatcatcaccatcaccat |
| **103102 CAR2** - **Soluble** | 65 | MALPVTALLLPLALLLHAARPeivmtqspatlslspgeratlscrasqdiskylnw yqqkpgqaprlliyhtsrlhsgiparfsgsgsgtdytltisslqpedfavyfcqqg ntlpytfgqgtkleikggggsggggsggggsqvqlqesgpglvkpsetlsltctvs |
| **scFv** - **aa** | | gvslpdygvswirqppgkglewigviwgsettyyqsslksrvtiskdnsknqvslk lssvtaadtavyycakhyyyggsyamdywgqgtlvtvsshhhhhhhh |
| **104876 CAR 2** - **Full** - **nt** | 91 | atggccctccctgtcaccgccctgctgcttccgctggctcttctgctccacgccgc tcggcccgaaattgtgatgacccagtcacccgccactcttagcctttcacccggtg agcgcgcaaccctgtcttgcagagcctcccaagacatctcaaatacctta attgg tatcaacagaagcccggacaggctcctcgccttctgatctaccacaccagccggct ccattctggaatccctgccaggttcagcggtagcggatctgggaccgactacaccc tcactatcagctcactgcagccagaggacttcgctgtctatttctgtcagcaaggg aacaccctgccctacacctttggacagggcaccaagctcgagattaaaggtggagg tggcagcggaggaggtgggtccggcggtggaggaagccaggtccaactccaagaaa gcggaccgggtcttgtgaagccatcagaaactctttcactgacttgtactgtgagc ggagtgtctctccccgattacggggtgtcttggatcagacagccaccggggaaggg tctggaatggattggagtgatttggggctctgagactacttactaccaatcatccc tcaagtcacgcgtcaccatctcaaaggacaactctaagaatcaggtgtcactgaaa ctgtcatctgtgaccgcagccgacaccgccgtgtactattgcgctaagcattacta ttatggcgggagctacgcaatggattactggggacagggtactctggtcaccgtgt ccagcaccactaccccagcaccgaggccacccaccccggctcctaccatcgcctcc cagcctctgtccctgcgtccggaggcatgtagacccgcagctggtggggccgtgca tacccggggtcttgacttcgcctgcgatatctacatttgggcccctctggctggta cttgcggggtcctgctgctttcactcgtgatcactctttactgtaagcgcggtcgg aagaagctgctgtacatctttaagcaacccttcatgaggcctgtgcagactactca agaggaggacggctgttcatgccggttcccagaggaggaggaaggcggctgcgaac tgcgcgtgaaattcagccgcagcgcagatgctccagcctacaagcaggggcagaac cagctctacaacgaactcaatcttggtcggagagaggagtacgacgtgctggacaa gcggagaggacgggacccagaaatgggcgggaagccgcgcagaaagaatccccaag agggcctgtacaacgagctccaaaaggataagatggcagaagcctatagcgagatt ggtatgaaaggggaacgcagaagaggcaaaggccacgacggactgtaccagggact cagcaccgccaccaaggacacctatgacgctcttcacatgcaggccctgccgcctc gg |
| **104876 CAR 2** - **Full** - **aa** | 78 | MALPVTALLLPLALLLHAARPeivmtqspatlslspgeratlscrasqdiskylnw yqqkpgqaprlliyhtsrlhsgiparfsgsgsgtdytltisslqpedfavyfcqqg ntlpytfgqgtkleikggggsggggsggggsqvqlqesgpglvkpsetlsltctvs gvslpdygvswirqppgkglewigviwgsettyyqsslksrvtiskdnsknqvslk lssvtaadtavyycakhyyyggsyamdywgqgtlvtvsstttpaprpptpaptias qplslrpeacrpaaggavhtrgldfacdiyiwaplagtcgvlllslvitlyckrgr kkllyifkqpfmrpvqttqeedgcscrfpeeeeggcelrvkfsrsadapaykqgqn qlynelnlgrreeydvldkrrgrdpemggkprrknpqeglynelqkdkmaeaysei gmkgerrrgkghdglyqglstatkdtydalhmqalppr |

| **CAR 3** | | |
|---|---|---|
| **CAR3 scFv domain** | 41 | qvqlqesgpglvkpsetlsltctvsgvslpdygvswirqppgkglewigviwgset tyysssslksrvtiskdnsknqvslklssvtaadtavyycakhyyyggsyamdywgq gtlvtvssggggsggggsggggseivmtqspatlslspgeratlscrasqdiskyl nwyqqkpgqaprlliyhtsrlhsgiparfsgsgsgtdytltisslqpedfavyfcq qgntlpytfgqgtkleik |
| **103104 CAR 3** - **Soluble scFv** - **nt** | 54 | atggctctgcccgtgaccgcactcctcctgccactggctctgctgcttcacgccgc tcgcccacaagtccagcttcaagaatcagggcctggtctggtgaagccatctgaga ctctgtccctcacttgcaccgtgagcggagtgtccctcccagactacggagtgagc tggattagacagcctcccggaaagggactggagtggatcggagtgatttggggtag cgaaaccacttactattcatcttccctgaagtcacgggtcaccatttcaaaggata actcaaagaatcaagtgagcctcaagctctcatcagtcaccgccgctgacaccgcc gtgtattactgtgccaagcattactactatggaggtcctacgccatggactactg ggccagggaactctggtcactgtgtcatctggtggaggaggtagcggaggaggcg ggagcggtggaggtggctccgaaatcgtgatgacccagagccctgcaaccctgtcc ctttctcccggggaacggctaccctttcttgtcgggcatcacaagatatctcaaa atacctcaattggtatcaacagaagccgggacaggcccctaggcttcttatctacc acacctctcgcctgcatagcgggattcccgcacgctttagcgggtctggaagcggg accgactacactctgaccatctcatctctccagcccgaggacttcgccgtctactt ctgccagcagggtaacaccctgccgtacaccttcggccagggcaccaagcttgaga tcaaacatcaccaccatcatcaccatcac |
| **103104 CAR 3** - **Soluble scFv** - **aa** | 66 | MALPVTALLLPLALLLHAARPqvqlqesgpglvkpsetlsltctvsgvslpdygvs wirqppgkglewigviwgsettyyssslksrvtiskdnsknqvslklssvtaadta vyycakhyyyggsyamdywgqgtlvtvssggggsggggsggggseivmtqspatls lspgeratlscrasqdiskylnwyqqkpgqaprlliyhtsrlhsgiparfsgsgsg tdytltisslqpedfavyfcqqgntlpytfgqgtkleikhhhhhhhh |
| **104877 CAR 3 - Full** - **nt** | 92 | atggctctgcccgtgaccgcactcctcctgccactggctctgctgcttcacgccgc tcgcccacaagtccagcttcaagaatcagggcctggtctggtgaagccatctgaga ctctgtccctcacttgcaccgtgagcggagtgtccctcccagactacggagtgagc tggattagacagcctcccggaaagggactggagtggatcggagtgatttgggtag cgaaaccacttactattcatcttccctgaagtcacgggtcaccatttcaaaggata actcaaagaatcaagtgagcctcaagctctcatcagtcaccgccgctgacaccgcc gtgtattactgtgccaagcattactactatggagggtcctacgccatggactactg gggccagggaactctggtcactgtgtcatctggtggaggaggtagcggaggaggcg ggagcggtggaggtggctccgaaatcgtgatgacccagagccctgcaaccctgtcc ctttctcccggggaacggctaccctttcttgtcggcatcacaagatatctcaaa atacctcaattggtatcaacagaagccgggacaggcccctaggcttcttatctacc acacctctcgcctgcatagcgggattcccgcacgctttagcggtctggaagcggg |
| | | accgactacactctgaccatctcatctctccagcccgaggacttcgccgtctactt ctgccagcagggtaacaccctgccgtacaccttcggccagggcaccaagcttgaga tcaaaaccactactcccgctccaaggccacccacccctgccccgaccatcgcctct cagccgctttccctgcgtccggaggcatgtagacccgcagctggtggggccgtgca tacccggggtcttgacttcgcctgcgatatctacatttgggcccctctggctggta cttgcggggtcctgctgctttcactcgtgatcactctttactgtaagcgcggtcgg aagaagctgctgtacatctttaagcaacccttcatgaggcctgtgcagactactca agaggaggacggctgttcatgccggttcccagaggaggaggaaggcggctgcgaac tgcgcgtgaaattcagccgcagcgcagatgctccagcctacaagcaggggcagaac cagctctacaacgaactcaatcttggtcggagagaggagtacgacgtgctggacaa gcggagaggacgggacccagaaatgggcgggaagccgcgcagaaagaatccccaag agggcctgtacaacgagctccaaaaggataagatggcagaagcctatagcgagatt ggtatgaaaggggaacgcagaagaggcaaaggccacgacggactgtaccagggact cagcaccgccaccaaggacacctatgacgctcttcacatgcaggccctgccgcctc gg |
| **104877 CAR 3 - Full** - **aa** | 79 | MALPVTALLLPLALLLHAARPqvqlqesgpglvkpsetlsltctvsgvslpdygvs wirqppgkglewigviwgsettyyssslksrvtiskdnsknqvslklssvtaadta vyycakhyyyggsyamdywgqgtlvtvssggggsggggsggggseivmtqspatls lspgeratlscrasqdiskylnwyqqkpgqaprlliyhtsrlhsgiparfsgsgsg tdytltisslqpedfavyfcqqgntlpytfgqgtkleikttpaprpptpaptias qplslrpeacrpaaggavhtrgldfacdiyiwaplagtcgvlllslvitlyckrgr kkllyifkqpfmrpvqttqeedgcscrfpeeeeggcelrvkfsrsadapaykqgqn qlynelnlgrreeydvldkrrgrdpemggkprrknpqeglynelqkdkmaeaysei gmkgerrrgkghdglyqglstatkdtydalhmqalppr |

| **CAR 4** | | |
|---|---|---|
| **CAR4 scFv domain** | 42 | qvqlqesgpglvkpsetlsltctvsgvslpdygvswirqppgkglewigviwgset tyyqsslksrvtiskdnsknqvslklssvtaadtavyycakhyyyggsyamdywgq gtlvtvssggggsggggsggggseivmtqspatlslspgeratlscrasqdiskyl nwyqqkpgqaprlliyhtsrlhsgiparfsgsgsgtdytltisslqpedfavyfcq qgntlpytfgqgtkleik |
| **103106 CAR4-Soluble scFv** - **nt** | 55 | atggctctgcccgtgaccgcactcctcctgccactggctctgctgcttcacgccgc tcgcccacaagtccagcttcaagaatcagggcctggtctggtgaagccatctgaga ctctgtccctcacttgcaccgtgagcggagtgtccctcccagactacggagtgagc tggattagacagcctcccggaaagggactggagtggatcggagtgatttgggtag cgaaaccacttactatcaatcttccctgaagtcacgggtcaccatttcaaaggata actcaaagaatcaagtgagcctcaagctctcatcagtcaccgccgctgacaccgcc gtgtattactgtgccaagcattactactatggaggtcctacgccatggactactg gggccagggaactctggtcactgtgtcatctggtggaggaggtagcggaggaggcg |
| | | ggagcggtggaggtggctccgaaatcgtgatgacccagagccctgcaaccctgtcc ctttctcccggggaacgggctaccctttcttgtcgggcatcacaagatatctcaaa atacctcaattggtatcaacagaagccgggacaggcccctaggcttcttatctacc acacctctcgcctgcatagcgggattcccgcacgctttagcgggtctggaagcggg accgactacactctgaccatctcatctctccagcccgaggacttcgccgtctactt ctgccagcagggtaacaccctgccgtacaccttcggccagggcaccaagcttgaga tcaaacatcaccaccatcatcaccatcac |
| **103106 CAR4-Soluble scFv -aa** | 67 | MALPVTALLLPLALLLHAARPqvqlqesgpglvkpsetlsltctvsgvslpdygvs wirqppgkglewigviwgsettyyqsslksrvtiskdnsknqvslklssvtaadta vyycakhyyyggsyamdywgqgtlvtvssggggsggggsggggseivmtqspatls lspgeratlscrasqdiskylnwyqqkpgqaprlliyhtsrlhsgiparfsgsgsg tdytltisslqpedfavyfcqqgntlpytfgqgtkleikhhhhhhhh |
| **104878 CAR 4 - Full** - **nt** | 93 | atggctctgcccgtgaccgcactcctcctgccactggctctgctgcttcacgccgc tcgcccacaagtccagcttcaagaatcagggcctggtctggtgaagccatctgaga ctctgtccctcacttgcaccgtgagcggagtgtccctcccagactacggagtgagc tggattagacagcctcccggaaagggactggagtggatcggagtgatttgggtag cgaaaccacttactatcaatcttccctgaagtcacgggtcaccatttcaaaggata actcaaagaatcaagtgagcctcaagctctcatcagtcaccgccgctgacaccgcc gtgtattactgtgccaagcattactactatggagggtcctacgccatggactactg gggccagggaactctggtcactgtgtcatctggtggaggaggtagcggaggaggcg ggagcggtggaggtggctccgaaatcgtgatgacccagagccctgcaaccctgtcc ctttctcccggggaacgggctaccctttcttgtcgggcatcacaagatatctcaaa atacctcaattggtatcaacagaagccgggacaggcccctaggcttcttatctacc acacctctcgcctgcatagcgggattcccgcacgctttagcgggtctggaagcggg accgactacactctgaccatctcatctctccagcccgaggacttcgccgtctactt ctgccagcagggtaacaccctgccgtacaccttcggccagggcaccaagcttgaga tcaaaaccactactcccgctccaaggccacccacccctgccccgaccatcgcctct cagccgctttccctgcgtccggaggcatgtagacccgcagctggtggggccgtgca tacccggggtcttgacttcgcctgcgatatctacatttgggcccctctggctggta cttgcggggtcctgctgctttcactcgtgatcactctttactgtaagcgcggtcgg aagaagctgctgtacatctttaagcaacccttcatgaggcctgtgcagactactca agaggaggacggctgttcatgccggttcccagaggaggaggaaggcggctgcgaac tgcgcgtgaaattcagccgcagcgcagatgctccagcctacaagcaggggcagaac cagctctacaacgaactcaatcttggtcggagagaggagtacgacgtgctggacaa gcggagaggacgggacccagaaatgggcgggaagccgcgcagaaagaatccccaag agggcctgtacaacgagctccaaaaggataagatggcagaagcctatagcgagatt ggtatgaaaggggaacgcagaagaggcaaaggccacgacggactgtaccaggact cagcaccgccaccaaggacacctatgacgctcttcacatgcaggccctgccgcctc |
| | | gg |
| 104878 CAR 4 - Full - aa | 80 | MALPVTALLLPLALLLHAARPqvqlqesgpglvkpsetlsltctvsgvslpdygvs wirqppgkglewigviwgsettyyqsslksrvtiskdnsknqvslklssvtaadta vyycakhyyyggsyamdywgqgtlvtvssggggsggggsggggseivmtqspatls lspgeratlscrasqdiskylnwyqqkpgqaprlliyhtsrlhsgiparfsgsgsg tdytltisslqpedfavyfcqqgntlpytfgqgtkleikttpaprpptpaptias qplslrpeacrpaaggavhtrgldfacdiyiwaplagtcgvlllslvitlyckrgr kkllyifkqpfmrpvqttqeedgcscrfpeeeeggcelrvkfsrsadapaykqgqn qlynelnlgrreeydvldkrrgrdpemggkprrknpqeglynelqkdkmaeaysei gmkgerrrgkghdglyqglstatkdtydalhmqalppr |

| **CAR 5** | | |
|---|---|---|
| **CAR 5 scFv domain** | 43 | eivmtqspatlslspgeratlscrasqdiskylnwyqqkpgqaprlliyhtsrlhs gipar fsgsgsgtdytltisslqpedfavyfcqqgntlpytfgqgtkleikggggs ggggsggggsggggsqvqlqesgpglvkpsetlsltctvsgvslpdygvswirqpp gkglewigviwgsettyysssslksrvtiskdnsknqvslklssvtaadtavyycak hyyyggsyamdywgqgtlvtvss |
| **99789 CAR5** - **Soluble scFv** - **nt** | 56 | atggccctcccagtgaccgctctgctgctgcctctcgcacttcttctccatgccgc tcggcctgagatcgtcatgacccaaagccccgctaccctgtccctgtcacccggcg agagggcaaccctttcatgcagggccagccaggacatttctaagtacctcaactgg tatcagcagaagccagggcaggctcctcgcctgctgatctaccacaccagccgcct ccacagcggtatccccgccagatttccgggagcgggtctggaaccgactacaccc tcaccatctcttctctgcagcccgaggatttcgccgtctatttctgccagcagggg aatactctgccgtacaccttcggtcaaggtaccaagctggaaatcaaggaggcgg aggatcaggcggtggcggaagcggaggaggtggctccggaggaggaggttcccaag tgcagcttcaagaatcaggacccggacttgtgaagccatcagaaaccctctccctg acttgtaccgtgtccggtgtgagcctccccgactacggagtctcttggattcgcca gcctccggggaagggtcttgaatggattggggtgatttggggatcagagactactt actactcttcatcacttaagtcacgggtcaccatcagcaaagataatagcaagaac caagtgtcacttaagctgtcatctgtgaccgccgctgacaccgccgtgtactattg tgccaaacattactattacggagggtcttatgctatggactactggggacagggga ccctggtgactgtctctagccatcaccatcaccaccatcatcac |
| **99789 CAR5** - **Soluble scFv -aa** | 68 | MALPVTALLLPLALLLHAARPeivmtqspatlslspgeratlscrasqdiskylnw yqqkpgqaprlliyhtsrlhsgiparfsgsgsgtdytltisslqpedfavyfcqqg ntlpytfgqgtkleikggggsggggsggggsggggsqvqlqesgpglvkpsetlsl tctvsgvslpdygvswirqppgkglewigviwgsettyysssslksrvtiskdnskn qvslklssvtaadtavyycakhyyyggsyamdywgqgtlvtvsshhhhhhhh |
| **104879 CAR 5 -** | 94 | atggccctccctgtcaccgccctgctgcttccgctggctcttctgctccacgccgc tcggcccgaaattgtgatgacccagtcacccgccactcttagcctttcacccggtg |
| **Full** - **nt** | | agcgcgcaaccctgtcttgcagagcctcccaagacatctcaaaataccttaattgg tatcaacagaagcccggacaggctcctcgccttctgatctaccacaccagccggct ccattctggaatccctgccaggttcagcggtagcggatctgggaccgactacaccc tcactatcagctcactgcagccagaggacttcgctgtctatttctgtcagcaaggg aacaccctgccctacacctttggacagggcaccaagctcgagattaaaggtggagg tggcagcggaggaggtgggtccggcggtggaggaagcggcggaggcgggagccagg tccaactccaagaaagcggaccgggtcttgtgaagccatcagaaactctttcactg acttgtactgtgagcggagtgtctctccccgattacggggtgtcttggatcagaca gccaccggggaagggtctggaatggattggagtgatttggggctctgagactactt actactcttcatccctcaagtcacgcgtcaccatctcaaaggacaactctaagaat caggtgtcactgaaactgtcatctgtgaccgcagccgacaccgccgtgtactattg cgctaagcattactattatggcgggagctacgcaatggattactggggacagggta ctctggtcaccgtgtccagcaccactaccccagcaccgaggccacccaccccggct cctaccatcgcctcccagcctctgtccctgcgtccggaggcatgtagacccgcagc tggtggggccgtgcatacccggggtcttgacttcgcctgcgatatctacatttggg cccctctggctggtacttgcggggtcctgctgctttcactcgtgatcactctttac tgtaagcgcggtcggaagaagctgctgtacatctttaagcaacccttcatgaggcc tgtgcagactactcaagaggaggacggctgttcatgccggttcccagaggaggagg aaggcggctgcgaactgcgcgtgaaattcagccgcagcgcagatgctccagcctac aagcaggggcagaaccagctctacaacgaactcaatcttggtcggagagaggagta cgacgtgctggacaagcggagaggacgggacccagaaatgggcgggaagccgcgca gaaagaatccccaagagggcctgtacaacgagctccaaaaggataagatggcagaa gcctatagcgagattggtatgaaaggggaacgcagaagaggcaaaggccacgacgg actgtaccagggactcagcaccgccaccaaggacacctatgacgctcttcacatgc aggccctgccgcctcgg |
| **104879 CAR 5 - Full** - **aa** | 81 | MALPVTALLLPLALLLHAARPeivmtqspatlslspgeratlscrasqdiskylnw yqqkpgqaprlliyhtsrlhsgiparfsgsgsgtdytltisslqpedfavyfcqqg ntlpytfgqgtkleikggggsggggsggggsggggsqvqlqesgpglvkpsetlsl tctvsgvslpdygvswirqppgkglewigviwgsettyyssslksrvtiskdnskn qvslklssvtaadtavyycakhyyyggsyamdywgqgtlvtvsstttpaprpptpa ptiasqplslrpeacrpaaggavhtrgldfacdiyiwaplagtcgvlllslvitly ckrgrkkllyifkqpfmrpvqttqeedgcscrfpeeeeggcelrvkfsrsadapay kqgqnqlynelnlgrreeydvldkrrgrdpemggkprrknpqeglynelqkdkmae ayseigmkgerrrgkghdglyqglstatkdtydalhmqalppr |

| **CAR 6** | | |
|---|---|---|
| **CAR6 scFv domain** | 44 | eivmtqspatlslspgeratlscrasqdiskylnwyqqkpgqaprlliyhtsrlhs giparfsgsgsgtdytltisslqpedfavyfcqqgntlpytfgqgtkleikggggs ggggsggggsggggsqvqlqesgpglvkpsetlsltctvsgvslpdygvswirqpp |
| | | gkglewigviwgsettyyqsslksrvtiskdnsknqvslklssvtaadtavyycak hyyyggsyamdywgqgtlvtvss |
| **99790 CAR6** - **Soluble scFv** - **nt** | 57 | atggccctcccagtgaccgctctgctgctgcctctcgcacttcttctccatgccgc tcggcctgagatcgtcatgacccaaagccccgctaccctgtccctgtcacccggcg agagggcaaccctttcatgcagggccagccaggacatttctaagtacctcaactgg tatcagcagaagccagggcaggctcctcgcctgctgatctaccacaccagccgcct ccacagcggtatccccgccagatttccgggagcgggtctggaaccgactacaccc tcaccatctcttctctgcagcccgaggatttcgccgtctatttctgccagcagggg aatactctgccgtacaccttcggtcaaggtaccaagctggaaatcaaggaggcgg aggatcaggcggtggcggaagcggaggaggtggctccggaggaggaggttcccaag tgcagcttcaagaatcaggacccggacttgtgaagccatcagaaaccctctccctg acttgtaccgtgtccggtgtgagcctccccgactacggagtctcttggattcgcca gcctccggggaagggtcttgaatggattggggtgatttggggatcagagactactt actaccagtcatcacttaagtcacgggtcaccatcagcaaagataatagcaagaac caagtgtcacttaagctgtcatctgtgaccgccgctgacaccgccgtgtactattg tgccaaacattactattacggagggtcttatgctatggactactggggacagggga ccctggtgactgtctctagccatcaccatcaccaccatcatcac |
| **99790 CAR6** - **Soluble scFv** - **aa** | 69 | MALPVTALLLPLALLLHAARPeivmtqspatlslspgeratlscrasqdiskylnw yqqkpgqaprlliyhtsrlhsgiparfsgsgsgtdytltisslqpedfavyfcqqg ntlpytfgqgtkleikggggsggggsggggsggggsqvqlqesgpglvkpsetlsl tctvsgvslpdygvswirqppgkglewigviwgsettyyqsslksrvtiskdnskn qvslklssvtaadtavyycakhyyyggsyamdywgqgtlvtvsshhhhhhhh |
| **104880 CAR6-Full** - **nt** | 95 | atggccctccctgtcaccgccctgctgcttccgctggctcttctgctccacgccgc tcggcccgaaattgtgatgacccagtcacccgccactcttagcctttcacccggtg agcgcgcaaccctgtcttgcagagcctcccaagacatctcaaataccttaattgg tatcaacagaagcccggacaggctcctcgccttctgatctaccacaccagccggct ccattctggaatccctgccaggttcagcggtagcggatctgggaccgactacaccc tcactatcagctcactgcagccagaggacttcgctgtctatttctgtcagcaaggg aacaccctgccctacacctttggacagggcaccaagctcgagattaaaggtggagg tggcagcggaggaggtgggtccggcggtggaggaagcggaggcggagggagccagg tccaactccaagaaagcggaccgggtcttgtgaagccatcagaaactctttcactg acttgtactgtgagcggagtgtctctccccgattacggggtgtcttggatcagaca gccaccggggaagggtctggaatggattggagtgatttggggctctgagactactt actaccaatcatccctcaagtcacgcgtcaccatctcaaaggacaactctaagaat caggtgtcactgaaactgtcatctgtgaccgcagccgacaccgccgtgtactattg cgctaagcattactattatggcgggagctacgcaatggattactggggacagggta ctctggtcaccgtgtccagcaccactaccccagcaccgaggccacccaccccggct cctaccatcgcctcccagcctctgtccctgcgtccggaggcatgtagacccgcagc |
| | | tggtggggccgtgcatacccggggtcttgacttcgcctgcgatatctacatttggg cccctctggctggtacttgcggggtcctgctgctttcactcgtgatcactctttac tgtaagcgcggtcggaagaagctgctgtacatctttaagcaacccttcatgaggcc tgtgcagactactcaagaggaggacggctgttcatgccggttcccagaggaggagg aaggcggctgcgaactgcgcgtgaaattcagccgcagcgcagatgctccagcctac aagcaggggcagaaccagctctacaacgaactcaatcttggtcggagagaggagta cgacgtgctggacaagcggagaggacgggacccagaaatgggcggaagccgcgca gaaagaatccccaagagggcctgtacaacgagctccaaaaggataagatggcagaa gcctatagcgagattggtatgaaaggggaacgcagaagaggcaaaggccacgacgg actgtaccagggactcagcaccgccaccaaggacacctatgacgctcttcacatgc aggccctgccgcctcgg |
| **104880 CAR6-Full** - **aa** | 82 | MALPVTALLLPLALLLHAARPeivmtqspatlslspgeratlscrasqdiskylnw yqqkpgqaprlliyhtsrlhsgiparfsgsgsgtdytltisslqpedfavyfcqqg ntlpytfgqgtkleikggggsggggsggggsggggsqvqlqesgpglvkpsetlsl tctvsgvslpdygvswirqppgkglewigviwgsettyyqsslksrvtiskdnskn qvslklssvtaadtavyycakhyyyggsyamdywgqgtlvtvsstttpaprpptpa ptiasqplslrpeacrpaaggavhtrgldfacdiyiwaplagtcgvlllslvitly ckrgrkkllyifkqpfmrpvqttqeedgcscrfpeeeeggcelrvkfsrsadapay kqgqnqlynelnlgrreeydvldkrrgrdpemggkprrknpqeglynelqkdkmae ayseigmkgerrrgkghdglyqglstatkdtydalhmqalppr |

| **CAR 7** | | |
|---|---|---|
| **CAR7 scFv domain** | 45 | qvqlqesgpglvkpsetlsltctvsgvslpdygvswirqppgkglewigviwgset tyysssslksrvtiskdnsknqvslklssvtaadtavyycakhyyyggsyamdywgq gtlvtvssggggsggggsggggsggggseivmtqspatlslspgeratlscrasqd iskylnwyqqkpgqaprlliyhtsrlhsgiparfsgsgsgtdytltisslqpedfa vyfcqqgntlpytfgqgtkleik |
| **100796 CAR7** - **Soluble scFv** - **nt** | 58 | atggcactgcctgtcactgccctcctgctgcctctggccctccttctgcatgccgc caggccccaagtccagctgcaagagtcaggacccggactggtgaagccgtctgaga ctctctcactgacttgtaccgtcagcggcgtgtccctccccgactacggagtgtca tggatccgccaacctcccgggaaagggcttgaatggattggtgtcatctggggttc tgaaaccacctactactcatcttccctgaagtccagggtgaccatcagcaaggata attccaagaaccaggtcagccttaagctgtcatctgtgaccgctgctgacaccgcc gtgtattactgcgccaagcactactattacggaggaagctacgctatggactattg gggacagggcactctcgtgactgtgagcagcggcggtggagggtctggaggtggag gatccggtggtggtgggtcaggcggaggagggagcgagattgtgatgactcagtca ccagccaccctttctctttcacccggcgagagagcaaccctgagctgtagagccag ccaggacatttctaagtacctcaactggtatcagcaaaaaccggggcaggcccctc gcctcctgatctaccatacctcacgccttcactctggtatccccgctcggtttagc |
| | | ggatcaggatctggtaccgactacactctgaccatttccagcctgcagccagaaga tttcgcagtgtatttctgccagcagggcaatacccttccttacaccttcggtcagg gaaccaagctcgaaatcaagcaccatcaccatcatcaccaccat |
| **100796 CAR7** - **Soluble scFv** - **aa** | 70 | MALPVTALLLPLALLLHAARPqvqlqesgpglvkpsetlsltctvsgvslpdygvs wirqppgkglewigviwgsettyyssslksrvtiskdnsknqvslklssvtaadta vyycakhyyyggsyamdywgqgtlvtvssggggsggggsggggsggggseivmtqs patlslspgeratlscrasqdiskylnwyqqkpgqaprlliyhtsrlhsgiparfs gsgsgtdytltisslqpedfavyfcqqgntlpytfgqgtkleikhhhhhhhh |
| **104881 CAR 7 Full** - **nt** | 96 | atggctctgcccgtgaccgcactcctcctgccactggctctgctgcttcacgccgc tcgcccacaagtccagcttcaagaatcagggcctggtctggtgaagccatctgaga ctctgtccctcacttgcaccgtgagcggagtgtccctcccagactacggagtgagc tggattagacagcctcccggaaagggactggagtggatcggagtgatttgggtag cgaaaccacttactattcatcttccctgaagtcacgggtcaccatttcaaaggata actcaaagaatcaagtgagcctcaagctctcatcagtcaccgccgctgacaccgcc gtgtattactgtgccaagcattactactatggaggtcctacgccatggactactg gggccagggaactctggtcactgtgtcatctggtggaggaggtagcggaggaggcg gagcggtggaggtggctccggaggtggcggaagcgaaatcgtgatgacccagagc cctgcaaccctgtccctttctcccggggaacgggctaccctttcttgtcgggcatc acaagatatctcaaaatacctcaattggtatcaacagaagccgggacaggcccta ggcttcttatctaccacacctctcgcctgcatagcgggattcccgcacgctttagc gggtctggaagcgggaccgactacactctgaccatctcatctctccagcccgagga cttcgccgtctacttctgccagcagggtaacaccctgccgtacaccttcggccagg gcaccaagcttgagatcaaaaccactactcccgctccaaggccacccacccctgcc ccgaccatcgcctctcagccgctttccctgcgtccggaggcatgtagacccgcagc tggtggggccgtgcatacccgggtcttgacttcgcctgcgatatctacatttggg cccctctggctggtacttgcggggtcctgctgctttcactcgtgatcactctttac tgtaagcgcggtcggaagaagctgctgtacatctttaagcaacccttcatgaggcc tgtgcagactactcaagaggaggacggctgttcatgccggttcccagaggaggagg aaggcggctgcgaactgcgcgtgaaattcagccgcagcgcagatgctccagcctac aagcaggggcagaaccagctctacaacgaactcaatcttggtcggagagaggagta cgacgtgctggacaagcggagaggacgggacccagaaatgggcggaagccgcgca gaaagaatccccaagagggcctgtacaacgagctccaaaaggataagatggcagaa gcctatagcgagattggtatgaaaggggaacgcagaagaggcaaaggccacgacgg actgtaccagggactcagcaccgccaccaaggacacctatgacgctcttcacatgc aggccctgccgcctcgg |
| **104881 CAR 7 Full** - **aa** | 83 | MALPVTALLLPLALLLHAARPqvqlqesgpglvkpsetlsltctvsgvslpdygvs wirqppgkglewigviwgsettyyssslksrvtiskdnsknqvslklssvtaadta vyycakhyyyggsyamdywgqgtlvtvssggggsggggsggggsggggseivmtqs |
| | | patlslspgeratlscrasqdiskylnwyqqkpgqaprlliyhtsrlhsgiparfs gsgsgtdytltisslqpedfavyfcqqgntlpytfgqgtkleikttttpaprpptpa ptiasqplslrpeacrpaaggavhtrgldfacdiyiwaplagtcgvlllslvitly ckrgrkkllyifkqpfmrpvqttqeedgcscrfpeeeeggcelrvkfsrsadapay kqgqnqlynelnlgrreeydvldkrrgrdpemggkprrknpqeglynelqkdkmae ayseigmkgerrrgkghdglyqglstatkdtydalhmqalppr |

| **CAR 8** | | |
|---|---|---|
| **CAR8 scFv domain** | 46 | qvqlqesgpglvkpsetlsltctvsgvslpdygvswirqppgkglewigviwgset tyyqsslksrvtiskdnsknqvslklssvtaadtavyycakhyyyggsyamdywgq gtlvtvssggggsggggsggggsggggseivmtqspatlslspgeratlscrasqd iskylnwyqqkpgqaprlliyhtsrlhsgiparfsgsgsgtdytltisslqpedfa vyfcqqgntlpytfgqgtkleik |
| **100798 CARS** - **Soluble scFv** - **nt** | 59 | atggcactgcctgtcactgccctcctgctgcctctggccctccttctgcatgccgc caggccccaagtccagctgcaagagtcaggacccggactggtgaagccgtctgaga ctctctcactgacttgtaccgtcagcggcgtgtccctccccgactacggagtgtca tggatccgccaacctcccgggaaagggcttgaatggattggtgtcatctggggttc tgaaaccacctactaccagtcttccctgaagtccagggtgaccatcagcaaggata attccaagaaccaggtcagccttaagctgtcatctgtgaccgctgctgacaccgcc gtgtattactgcgccaagcactactattacggaggaagctacgctatggactattg gggacagggcactctcgtgactgtgagcagcggcggtggagggtctggaggtggag gatccggtggtggtgggtcaggcggaggagggagcgagattgtgatgactcagtca ccagccaccctttctctttcacccggcgagagagcaaccctgagctgtagagccag ccaggacatttctaagtacctcaactggtatcagcaaaaaccggggcaggcccctc gcctcctgatctaccatacctcacgccttcactctggtatccccgctcggtttagc ggatcaggatctggtaccgactacactctgaccatttccagcctgcagccagaaga tttcgcagtgtatttctgccagcagggcaatacccttccttacaccttcggtcagg gaaccaagctcgaaatcaagcaccatcaccatcatcatcaccac |
| **100798 CARS** - **Soluble scFv** - **aa** | 71 | MALPVTALLLPLALLLHAARPqvqlqesgpglvkpsetlsltctvsgvslpdygvs wirqppgkglewigviwgsettyyqsslksrvtiskdnsknqvslklssvtaadta vyycakhyyyggsyamdywgqgtlvtvssggggsggggsggggsggggseivmtqs patlslspgeratlscrasqdiskylnwyqqkpgqaprlliyhtsrlhsgiparfs gsgsgtdytltisslqpedfavyfcqqgntlpytfgqgtkleikhhhhhhhh |
| **104882 CAR 8 - Full** - **nt** | 97 | atggctctgcccgtgaccgcactcctcctgccactggctctgctgcttcacgccgc tcgcccacaagtccagcttcaagaatcagggcctggtctggtgaagccatctgaga ctctgtccctcacttgcaccgtgagcggagtgtccctcccagactacggagtgagc tggattagacagcctcccggaaagggactggagtggatcggagtgatttgggtag cgaaaccacttactatcaatcttccctgaagtcacgggtcaccatttcaaaggata actcaaagaatcaagtgagcctcaagctctcatcagtcaccgccgctgacaccgcc |
| | | gtgtattactgtgccaagcattactactatggagggtcctacgccatggactactg gggccagggaactctggtcactgtgtcatctggtggaggaggtagcggaggaggcg ggagcggtggaggtggctccggaggcggtgggtcagaaatcgtgatgacccagagc cctgcaaccctgtccctttctcccggggaacggctaccctttcttgtcgggcatc acaagatatctcaaaatacctcaattggtatcaacagaagccgggacaggcccta ggcttcttatctaccacacctctcgcctgcatagcgggattcccgcacgctttagc gggtctggaagcgggaccgactacactctgaccatctcatctctccagcccgagga cttcgccgtctacttctgccagcagggtaacaccctgccgtacaccttcggccagg gcaccaagcttgagatcaaaaccactactcccgctccaaggccacccacccctgcc ccgaccatcgcctctcagccgctttccctgcgtccggaggcatgtagacccgcagc tggtggggccgtgcatacccggggtcttgacttcgcctgcgatatctacatttggg cccctctggctggtacttgcggggtcctgctgctttcactcgtgatcactctttac tgtaagcgcggtcggaagaagctgctgtacatctttaagcaacccttcatgaggcc tgtgcagactactcaagaggaggacggctgttcatgccggttcccagaggaggagg aaggcggctgcgaactgcgcgtgaaattcagccgcagcgcagatgctccagcctac aagcaggggcagaaccagctctacaacgaactcaatcttggtcggagagaggagta cgacgtgctggacaagcggagaggacgggacccagaaatgggcgggaagccgcgca gaaagaatccccaagagggcctgtacaacgagctccaaaaggataagatggcagaa gcctatagcgagattggtatgaaaggggaacgcagaagaggcaaaggccacgacgg actgtaccagggactcagcaccgccaccaaggacacctatgacgctcttcacatgc aggccctgccgcctcgg |
| **104882 CAR 8 - Full - aa** | 84 | MALPVTALLLPLALLLHAARPqvqlqesgpglvkpsetlsltctvsgvslpdygvs wirqppgkglewigviwgsettyyqsslksrvtiskdnsknqvslklssvtaadta vyycakhyyyggsyamdywgqgtlvtvssggggsggggsggggsggggseivmtqs patlslspgeratlscrasqdiskylnwyqqkpgqaprlliyhtsrlhsgiparfs gsgsgtdytltisslqpedfavyfcqqgntlpytfgqgtkleikttttpaprpptpa ptiasqplslrpeacrpaaggavhtrgldfacdiyiwaplagtcgvlllslvitly ckrgrkkllyifkqpfmrpvqttqeedgcscrfpeeeeggcelrvkfsrsadapay kqgqnqlynelnlgrreeydvldkrrgrdpemggkprrknpqeglynelqkdkmae ayseigmkgerrrgkghdglyqglstatkdtydalhmqalppr |

| **CAR 9** | | |
|---|---|---|
| **CAR9 scFv domain** | 47 | eivmtqspatlslspgeratlscrasqdiskylnwyqqkpgqaprlliyhtsrlhs gipar fsgsgsgtdytltisslqpedfavyfcqqgntlpytfgqgtkleikggggs ggggsggggsggggsqvqlqesgpglvkpsetlsltctvsgvslpdygvswirqpp gkglewigviwgsettyynsslksrvtiskdnsknqvslklssvtaadtavyycak hyyyggsyamdywgqgtlvtvss |
| **99789 CAR9** - | 60 | atggccctcccagtgaccgctctgctgctgcctctcgcacttcttctccatgccgc tcggcctgagatcgtcatgacccaaagccccgctaccctgtccctgtcacccggcg |
| **Soluble scFv - nt** | | agagggcaaccctttcatgcagggccagccaggacatttctaagtacctcaactgg tatcagcagaagccagggcaggctcctcgcctgctgatctaccacaccagccgcct ccacagcggtatccccgccagatttccgggagcgggtctggaaccgactacaccc tcaccatctcttctctgcagcccgaggatttcgccgtctatttctgccagcagggg aatactctgccgtacaccttcggtcaaggtaccaagctggaaatcaaggaggcgg aggatcaggcggtggcggaagcggaggaggtggctccggaggaggaggttcccaag tgcagcttcaagaatcaggacccggacttgtgaagccatcagaaaccctctccctg acttgtaccgtgtccggtgtgagcctccccgactacggagtctcttggattcgcca gcctccggggaagggtcttgaatggattggggtgatttggggatcagagactactt actacaattcatcacttaagtcacgggtcaccatcagcaaagataatagcaagaac caagtgtcacttaagctgtcatctgtgaccgccgctgacaccgccgtgtactattg tgccaaacattactattacggagggtcttatgctatggactactggggacagggga ccctggtgactgtctctagccatcaccatcaccaccatcatcac |
| **99789 CAR9 - Soluble scFv - aa** | 72 | MALPVTALLLPLALLLHAARPeivmtqspatlslspgeratlscrasqdiskylnw yqqkpgqaprlliyhtsrlhsgiparfsgsgsgtdytltisslqpedfavyfcqqg ntlpytfgqgtkleikggggsggggsggggsggggsqvqlqesgpglvkpsetlsl tctvsgvslpdygvswirqppgkglewigviwgsettyynsslksrvtiskdnskn qvslklssvtaadtavyycakhyyyggsyamdywgqgtlvtvsshhhhhhhh |
| **105974 CAR 9 - Full - nt** | 98 | atggccctccctgtcaccgccctgctgcttccgctggctcttctgctccacgccgc tcggcccgaaattgtgatgacccagtcacccgccactcttagcctttcacccggtg agcgcgcaaccctgtcttgcagagcctcccaagacatctcaaataccttaattgg tatcaacagaagcccggacaggctcctcgccttctgatctaccacaccagccggct ccattctggaatccctgccaggttcagcggtagcggatctgggaccgactacaccc tcactatcagctcactgcagccagaggacttcgctgtctatttctgtcagcaaggg aacaccctgccctacacctttggacagggcaccaagctcgagattaaaggtggagg tggcagcggaggaggtgggtccggcggtggaggaagcggaggcggtgggagccagg tccaactccaagaaagcggaccgggtcttgtgaagccatcagaaactctttcactg acttgtactgtgagcggagtgtctctccccgattacggggtgtcttggatcagaca gccaccggggaagggtctggaatggattggagtgatttggggctctgagactactt actacaactcatccctcaagtcacgcgtcaccatctcaaaggacaactctaagaat caggtgtcactgaaactgtcatctgtgaccgcagccgacaccgccgtgtactattg cgctaagcattactattatggcgggagctacgcaatggattactggggacagggta ctctggtcaccgtgtccagcaccactaccccagcaccgaggccacccaccccggct cctaccatcgcctcccagcctctgtccctgcgtccggaggcatgtagacccgcagc tggtggggccgtgcatacccggggtcttgacttcgcctgcgatatctacatttggg cccctctggctggtacttgcggggtcctgctgctttcactcgtgatcactctttac tgtaagcgcggtcggaagaagctgctgtacatctttaagcaacccttcatgaggcc tgtgcagactactcaagaggaggacggctgttcatgccggttcccagaggaggagg |
| | | aaggcggctgcgaactgcgcgtgaaattcagccgcagcgcagatgctccagcctac aagcaggggcagaaccagctctacaacgaactcaatcttggtcggagagaggagta cgacgtgctggacaagcggagaggacgggacccagaaatgggcgggaagccgcgca gaaagaatccccaagagggcctgtacaacgagctccaaaaggataagatggcagaa gcctatagcgagattggtatgaaaggggaacgcagaagaggcaaaggccacgacgg actgtaccagggactcagcaccgccaccaaggacacctatgacgctcttcacatgc aggccctgccgcctcgg |
| **105974 CAR 9 - Full - aa** | 85 | MALPVTALLLPLALLLHAARPeivmtqspatlslspgeratlscrasqdiskylnw yqqkpgqaprlliyhtsrlhsgiparfsgsgsgtdytltisslqpedfavyfcqqg ntlpytfgqgtkleikggggsggggsggggsggggsqvqlqesgpglvkpsetlsl tctvsgvslpdygvswirqppgkglewigviwgsettyynsslksrvtiskdnskn qvslklssvtaadtavyycakhyyyggsyamdywgqgtlvtvsstttpaprpptpa ptiasqplslrpeacrpaaggavhtrgldfacdiyiwaplagtcgvlllslvitly ckrgrkkllyifkqpfmrpvqttqeedgcscrfpeeeeggcelrvkfsrsadapay kqgqnqlynelnlgrreeydvldkrrgrdpemggkprrknpqeglynelqkdkmae ayseigmkgerrrgkghdglyqglstatkdtydalhmqalppr |

| **CAR10** | | |
|---|---|---|
| **CAR10 scFv domain** | 48 | qvqlqesgpglvkpsetlsltctvsgvslpdygvswirqppgkglewigviwgset tyynsslksrvtiskdnsknqvslklssvtaadtavyycakhyyyggsyamdywgq gtlvtvssggggsggggsggggsggggseivmtqspatlslspgeratlscrasqd iskylnwyqqkpgqaprlliyhtsrlhsgiparfsgsgsgtdytltisslqpedfa vyfcqqgntlpytfgqgtkleik |
| **100796 CAR10 - Soluble scFv - nt** | 61 | atggcactgcctgtcactgccctcctgctgcctctggccctccttctgcatgccgc caggccccaagtccagctgcaagagtcaggacccggactggtgaagccgtctgaga ctctctcactgacttgtaccgtcagcggcgtgtccctccccgactacggagtgtca tggatccgccaacctcccgggaaagggcttgaatggattggtgtcatctggggttc tgaaaccacctactacaactcttccctgaagtccagggtgaccatcagcaaggata attccaagaaccaggtcagccttaagctgtcatctgtgaccgctgctgacaccgcc gtgtattactgcgccaagcactactattacggaggaagctacgctatggactattg gggacagggcactctcgtgactgtgagcagcggcggtggagggtctggaggtggag gatccggtggtggtgggtcaggcggaggagggagcgagattgtgatgactcagtca ccagccaccctttctctttcacccggcgagagagcaaccctgagctgtagagccag ccaggacatttctaagtacctcaactggtatcagcaaaaaccggggcaggcccctc gcctcctgatctaccatacctcacgccttcactctggtatccccgctcggtttagc ggatcaggatctggtaccgactacactctgaccatttccagcctgcagccagaaga tttcgcagtgtatttctgccagcagggcaatacccttccttacaccttcggtcagg gaaccaagctcgaaatcaagcaccatcaccatcatcaccaccat |
| **100796** | 73 | MALPVTALLLPLALLLHAARPqvqlqesgpglvkpsetlsltctvsgvslpdygvs |
| **CAR10** - **Soluble scFv - aa** | | wirqppgkglewigviwgsettyynsslksrvtiskdnsknqvslklssvtaadta vyycakhyyyggsyamdywgqgtlvtvssggggsggggsggggsggggseivmtqs patlslspgeratlscrasqdiskylnwyqqkpgqaprlliyhtsrlhsgiparfs gsgsgtdytltisslqpedfavyfcqqgntlpytfgqgtkleikhhhhhhhh |
| **105975 CAR 10 Full - nt** | 99 | atggccctccctgtcaccgccctgctgcttccgctggctcttctgctccacgccgc tcggcccgaaattgtgatgacccagtcacccgccactcttagcctttcacccggtg agcgcgcaaccctgtcttgcagagcctcccaagacatctcaaataccttaattgg tatcaacagaagcccggacaggctcctcgccttctgatctaccacaccagccggct ccattctggaatccctgccaggttcagcggtagcggatctgggaccgactacaccc tcactatcagctcactgcagccagaggacttcgctgtctatttctgtcagcaaggg aacaccctgccctacacctttggacagggcaccaagctcgagattaaaggtggagg tggcagcggaggaggtgggtccggcggtggaggaagcggaggcggtgggagccagg tccaactccaagaaagcggaccgggtcttgtgaagccatcagaaactctttcactg acttgtactgtgagcggagtgtctctccccgattacggggtgtcttggatcagaca gccaccggggaagggtctggaatggattggagtgatttggggctctgagactactt actacaactcatccctcaagtcacgcgtcaccatctcaaaggacaactctaagaat caggtgtcactgaaactgtcatctgtgaccgcagccgacaccgccgtgtactattg cgctaagcattactattatggcgggagctacgcaatggattactggggacagggta ctctggtcaccgtgtccagcaccactaccccagcaccgaggccacccaccccggct cctaccatcgcctcccagcctctgtccctgcgtccggaggcatgtagacccgcagc tggtggggccgtgcatacccggggtcttgacttcgcctgcgatatctacatttggg cccctctggctggtacttgcggggtcctgctgctttcactcgtgatcactctttac tgtaagcgcggtcggaagaagctgctgtacatctttaagcaacccttcatgaggcc tgtgcagactactcaagaggaggacggctgttcatgccggttcccagaggaggagg aaggcggctgcgaactgcgcgtgaaattcagccgcagcgcagatgctccagcctac aagcaggggcagaaccagctctacaacgaactcaatcttggtcggagagaggagta cgacgtgctggacaagcggagaggacgggacccagaaatgggcggaagccgcgca gaaagaatccccaagagggcctgtacaacgagctccaaaaggataagatggcagaa gcctatagcgagattggtatgaaaggggaacgcagaagaggcaaaggccacgacgg actgtaccagggactcagcaccgccaccaaggacacctatgacgctcttcacatgc aggccctgccgcctcgg |
| **105975 CAR 10 Full - aa** | 86 | MALPVTALLLPLALLLHAARPEIVMTQSPATLSLSPGERATLSCRASQDISKYLNW YQQKPGQAPRLLIYHTSRLHSGIPARFSGSGSGTDYTLTISSLQPEDFAVYFCQQG NTLPYTFGQGTKLEIKGGGGSGGGGSGGGGSGGGGSQVQLQESGPGLVKPSETLSL TCTVSGVSLPDYGVSWIRQPPGKGLEWIGVIWGSETTYYNSSLKSRVTISKDNSKN QVSLKLSSVTAADTAVYYCAKHYYYGGSYAMDYWGQGTLVTVSSTTTPAPRPPTPA PTIASQPLSLRPEACRPAAGGAVHTRGLDFACDIYIWAPLAGTCGVLLLSLVITLY |
| | | CKRGRKKLLYIFKQPFMRPVQTTQEEDGCSCRFPEEEEGGCELRVKFSRSADAPAY KQGQNQLYNELNLGRREEYDVLDKRRGRDPEMGGKPRRKNPQEGLYNELQKDKMAE AYSEIGMKGERRRGKGHDGLYQGLSTATKDTYDALHMQALPPR |

| **CAR11** | | |
|---|---|---|
| **CAR11 scFv domain** | 49 | eivmtqspatlslspgeratlscrasqdiskylnwyqqkpgqaprlliyhtsrlhs gipar fsgsgsgtdytltisslqpedfavyfcqqgntlpytfgqgtkleikggggs ggggsggggsqvqlqesgpglvkpsetlsltctvsgvslpdygvswirqppgkgle wigviwgsettyynsslksrvtiskdnsknqvslklssvtaadtavyycakhyyyg gsyamdywgqgtlvtvss |
| **103101 CAR11** - **Soluble scFv - nt** | 62 | Atggccctccctgtcaccgccctgctgcttccgctggctcttctgctccacgccgc tcggcccgaaattgtgatgacccagtcacccgccactcttagcctttcacccggtg agcgcgcaaccctgtcttgcagagcctcccaagacatctcaaaataccttaattgg tatcaacagaagcccggacaggctcctcgccttctgatctaccacaccagccggct ccattctggaatccctgccaggttcagcggtagcggatctgggaccgactacaccc tcactatcagctcactgcagccagaggacttcgctgtctatttctgtcagcaaggg aacaccctgccctacacctttggacagggcaccaagctcgagattaaaggtggagg tggcagcggaggaggtgggtccggcggtggaggaagccaggtccaactccaagaaa gcggaccgggtcttgtgaagccatcagaaactctttcactgacttgtactgtgagc ggagtgtctctccccgattacggggtgtcttggatcagacagccaccggggaaggg tctggaatggattggagtgatttggggctctgagactacttactacaattcatccc tcaagtcacgcgtcaccatctcaaaggacaactctaagaatcaggtgtcactgaaa ctgtcatctgtgaccgcagccgacaccgccgtgtactattgcgctaagcattacta ttatggcgggagctacgcaatggattactggggacagggtactctggtcaccgtgt ccagccaccaccatcatcaccatcaccat |
| **103101 CAR11 - Soluble scFv - aa** | 74 | MALPVTALLLPLALLLHAARPeivmtqspatlslspgeratlscrasqdiskylnw yqqkpgqaprlliyhtsrlhsgiparfsgsgsgtdytltisslqpedfavyfcqqg ntlpytfgqgtkleikggggsggggsggggsqvqlqesgpglvkpsetlsltctvs gvslpdygvswirqppgkglewigviwgsettyynsslksrvtiskdnsknqvslk lssvtaadtavyycakhyyyggsyamdywgqgtlvtvsshhhhhhhh |
| **105976 CAR 11 Full - nt** | 100 | atggctctgcccgtgaccgcactcctcctgccactggctctgctgcttcacgccgc tcgcccacaagtccagcttcaagaatcagggcctggtctggtgaagccatctgaga ctctgtccctcacttgcaccgtgagcggagtgtccctcccagactacggagtgagc tggattagacagcctcccggaaagggactggagtggatcggagtgatttgggtag cgaaaccacttactataactcttccctgaagtcacgggtcaccatttcaaaggata actcaaagaatcaagtgagcctcaagctctcatcagtcaccgccgctgacaccgcc gtgtattactgtgccaagcattactactatggagggtcctacgccatggactactg gggccagggaactctggtcactgtgtcatctggtggaggaggtagcggaggaggcg |
| | | ggagcggtggaggtggctccggaggtggcggaagcgaaatcgtgatgacccagagc cctgcaaccctgtccctttctcccggggaacggctaccctttcttgtcggcatc acaagatatctcaaaatacctcaattggtatcaacagaagccgggacaggcccta ggcttcttatctaccacacctctcgcctgcatagcgggattcccgcacgctttagc gggtctggaagcgggaccgactacactctgaccatctcatctctccagcccgagga cttcgccgtctacttctgccagcagggtaacaccctgccgtacaccttcggccagg gcaccaagcttgagatcaaaaccactactcccgctccaaggccacccacccctgcc ccgaccatcgcctctcagccgctttccctgcgtccggaggcatgtagacccgcagc tggtggggccgtgcatacccggggtcttgacttcgcctgcgatatctacatttggg cccctctggctggtacttgcggggtcctgctgctttcactcgtgatcactctttac tgtaagcgcggtcggaagaagctgctgtacatctttaagcaacccttcatgaggcc tgtgcagactactcaagaggaggacggctgttcatgccggttcccagaggaggagg aaggcggctgcgaactgcgcgtgaaattcagccgcagcgcagatgctccagcctac aagcaggggcagaaccagctctacaacgaactcaatcttggtcggagagaggagta cgacgtgctggacaagcggagaggacgggacccagaaatgggcgggaagccgcgca gaaagaatccccaagagggcctgtacaacgagctccaaaaggataagatggcagaa gcctatagcgagattggtatgaaaggggaacgcagaagaggcaaaggccacgacgg actgtaccagggactcagcaccgccaccaaggacacctatgacgctcttcacatgc aggccctgccgcctcgg |
| **105976 CAR 11 Full - aa** | 87 | MALPVTALLLPLALLLHAARPQVQLQESGPGLVKPSETLSLTCTVSGVSLPDYGVS WIRQPPGKGLEWIGVIWGSETTYYNSSLKSRVTISKDNSKNQVSLKLSSVTAADTA VYYCAKHYYYGGSYAMDYWGQGTLVTVSSGGGGSGGGGSGGGGSGGGGSEIVMTQS PATLSLSPGERATLSCRASQDISKYLNWYQQKPGQAPRLLIYHTSRLHSGIPARFS GSGSGTDYTLTISSLQPEDFAVYFCQQGNTLPYTFGQGTKLEIKTTTPAPRPPTPA PTIASQPLSLRPEACRPAAGGAVHTRGLDFACDIYIWAPLAGTCGVLLLSLVITLY CKRGRKKLLYIFKQPFMRPVQTTQEEDGCSCRFPEEEEGGCELRVKFSRSADAPAY KQGQNQLYNELNLGRREEYDVLDKRRGRDPEMGGKPRRKNPQEGLYNELQKDKMAE AYSEIGMKGERRRGKGHDGLYQGLSTATKDTYDALHMQALPPR |

| **CAR12** | | |
|---|---|---|
| **CAR12 scFv domain** | 50 | qvqlqesgpglvkpsetlsltctvsgvslpdygvswirqppgkglewigviwgset tyynsslksrvtiskdnsknqvslklssvtaadtavyycakhyyyggsyamdywgq gtlvtvssggggsggggsggggseivmtqspatlslspgeratlscrasqdiskyl nwyqqkpgqaprllliyhtsrlhsgiparfsgsgsgtdytltisslqpedfavyfcq qgntlpytfgqgtkleik |
| **103104 CAR12 -** | 63 | atggctctgcccgtgaccgcactcctcctgccactggctctgctgcttcacgccgc tcgcccacaagtccagcttcaagaatcagggcctggtctggtgaagccatctgaga |
| **Soluble scFv - nt** | | ctctgtccctcacttgcaccgtgagcggagtgtccctcccagactacggagtgagc tggattagacagcctcccggaaagggactggagtggatcggagtgatttgggtag cgaaaccacttactataactcttccctgaagtcacgggtcaccatttcaaaggata actcaaagaatcaagtgagcctcaagctctcatcagtcaccgccgctgacaccgcc gtgtattactgtgccaagcattactactatggagggtcctacgccatggactactg gggccagggaactctggtcactgtgtcatctggtggaggaggtagcggaggaggcg ggagcggtggaggtggctccgaaatcgtgatgacccagagccctgcaaccctgtcc ctttctcccggggaacggctaccctttcttgtcggcatcacaagatatctcaaa atacctcaattggtatcaacagaagccgggacaggcccctaggcttcttatctacc acacctctcgcctgcatagcgggattcccgcacgctttagcggtctggaagcggg accgactacactctgaccatctcatctctccagcccgaggacttcgccgtctactt ctgccagcagggtaacaccctgccgtacaccttcggccagggcaccaagcttgaga tcaaacatcaccaccatcatcaccatcac |
| **103104 CAR12 - Soluble scFv -aa** | 75 | MALPVTALLLPLALLLHAARPqvqlqesgpglvkpsetlsltctvsgvslpdygvs wirqppgkglewigviwgsettyynsslksrvtiskdnsknqvslklssvtaadta vyycakhyyyggsyamdywgqgtlvtvssggggsggggsggggseivmtqspatls lspgeratlscrasqdiskylnwyqqkpgqaprlliyhtsrlhsgiparfsgsgsg tdytltisslqpedfavyfcqqgntlpytfgqgtkleikhhhhhhhh |
| **105977 CAR 12 - Full - nt** | 101 | atggccctccctgtcaccgccctgctgcttccgctggctcttctgctccacgccgc tcggcccgaaattgtgatgacccagtcacccgccactcttagcctttcacccggtg agcgcgcaaccctgtcttgcagagcctcccaagacatctcaaataccttaattgg tatcaacagaagcccggacaggctcctcgccttctgatctaccacaccagccggct ccattctggaatccctgccaggttcagcggtagcggatctgggaccgactacaccc tcactatcagctcactgcagccagaggacttcgctgtctatttctgtcagcaaggg aacaccctgccctacacctttggacagggcaccaagctcgagattaaaggtggagg tggcagcggaggaggtgggtccggcggtggaggaagccaggtccaactccaagaaa gcggaccgggtcttgtgaagccatcagaaactctttcactgacttgtactgtgagc ggagtgtctctccccgattacggggtgtcttggatcagacagccaccggggaaggg tctggaatggattggagtgatttggggctctgagactacttactacaactcatccc tcaagtcacgcgtcaccatctcaaaggacaactctaagaatcaggtgtcactgaaa ctgtcatctgtgaccgcagccgacaccgccgtgtactattgcgctaagcattacta ttatggcgggagctacgcaatggattactggggacagggtactctggtcaccgtgt ccagcaccactaccccagcaccgaggccacccaccccggctcctaccatcgcctcc cagcctctgtccctgcgtccggaggcatgtagacccgcagctggtggggccgtgca tacccggggtcttgacttcgcctgcgatatctacatttgggcccctctggctggta cttgcggggtcctgctgctttcactcgtgatcactctttactgtaagcgcggtcgg aagaagctgctgtacatctttaagcaacccttcatgaggcctgtgcagactactca agaggaggacggctgttcatgccggttcccagaggaggaggaaggcggctgcgaac |
| | | tgcgcgtgaaattcagccgcagcgcagatgctccagcctacaagcaggggcagaac cagctctacaacgaactcaatcttggtcggagagaggagtacgacgtgctggacaa gcggagaggacgggacccagaaatgggcgggaagccgcgcagaaagaatccccaag agggcctgtacaacgagctccaaaaggataagatggcagaagcctatagcgagatt ggtatgaaaggggaacgcagaagaggcaaaggccacgacggactgtaccaggact cagcaccgccaccaaggacacctatgacgctcttcacatgcaggccctgccgcctc gg |
| **105977 CAR 12 - Full - aa** | 88 | MALPVTALLLPLALLLHAARPEIVMTQSPATLSLSPGERATLSCRASQDISKYLNW YQQKPGQAPRLLIYHTSRLHSGIPARFSGSGSGTDYTLTISSLQPEDFAVYFCQQG NTLPYTFGQGTKLEIKGGGGSGGGGSGGGGSQVQLQESGPGLVKPSETLSLTCTVS GVSLPDYGVSWIRQPPGKGLEWIGVIWGSETTYYNSSLKSRVTISKDNSKNQVSLK LSSVTAADTAVYYCAKHYYYGGSYAMDYWGQGTLVTVSSTTTPAPRPPTPAPTIAS QPLSLRPEACRPAAGGAVHTRGLDFACDIYIWAPLAGTCGVLLLSLVITLYCKRGR KKLLYIFKQPFMRPVQTTQEEDGCSCRFPEEEEGGCELRVKFSRSADAPAYKQGQN QLYNELNLGRREEYDVLDKRRGRDPEMGGKPRRKNPQEGLYNELQKDKMAEAYSEI GMKGERRRGKGHDGLYQGLSTATKDTYDALHMQALPPR |

| **CTL019** | | |
|---|---|---|
| **CTL019 - Soluble scFv-Histag - nt** | 22 | atggccctgcccgtcaccgctctgctgctgccccttgctctgcttcttcatgcagc aaggccggacatccagatgacccaaaccacctcatccctctctgcctctcttggag acagggtgaccatttcttgtcgcgccagccaggacatcagcaagtatctgaactgg tatcagcagaagccggacggaaccgtgaagctcctgatctaccatacctctcgcct gcatagcggcgtgccctcacgcttctctggaagcggatcaggaaccgattattctc tcactatttcaaatcttgagcaggaagatattgccacctatttctgccagcagggt aataccctgccctacaccttcggaggagggaccaagctcgaaatcaccggtggagg aggcagcggcggtggagggtctggtggaggtggttctgaggtgaagctgcaagaat caggccctggacttgtggccccttcacagtccctgagcgtgacttgcaccgtgtcc ggagtctccctgcccgactacggagtgtcatggatcagacaacctccacggaaagg actggaatggctcggtgtcatctggggtagcgaaactacttactacaattcagccc tcaaaagcaggctgactattatcaaggacaacagcaagtcccaagtctttcttaag atgaactcactccagactgacgacaccgcaatctactattgtgctaagcactacta ctacggaggatcctacgctatggattactggggacaaggtacttccgtcactgtct cttcacaccatcatcaccatcaccatcac |
| **CTL019 - Soluble scFv-Histag - aa** | 76 | MALPVTALLLPLALLLHAARPdiqmtqttsslsaslgdrvtiscrasqdiskylnw yqqkpdgtvklliyhtsrlhsgvpsrfsgsgsgtdysltisnleqediatyfcqqg ntlpytfgggtkleitggggsggggsggggsevklqesgpglvapsqslsvtctvs gvslpdygvswirqpprkglewlgviwgsettyynsalksrltiikdnsksqvflk |
| | | mnslqtddtaiyycakhyyyggsyamdywgqgtsvtvss**hhhhhhhh** |
| **CTL019 Full - nt** | 102 | atggccttaccagtgaccgccttgctcctgccgctggccttgctgctccacgccgc caggccggacatccagatgacacagactacatcctccctgtctgcctctctgggag acagagtcaccatcagttgcagggcaagtcaggacattagtaaatatttaaattgg tatcagcagaaaccagatggaactgttaaactcctgatctaccatacatcaagatt acactcaggagtcccatcaaggttcagtggcagtgggtctggaacagattattctc tcaccattagcaacctggagcaagaagatattgccacttacttttgccaacagggt aatacgcttccgtacacgttcggaggggggaccaagctggagatcacaggtggcgg tggctcgggcggtggtgggtcgggtggcggcggatctgaggtgaaactgcaggagt caggacctggcctggtggcgccctcacagagcctgtccgtcacatgcactgtctca ggggtctcattacccgactatggtgtaagctggattcgccagcctccacgaaaggg tctggagtggctgggagtaatatggggtagtgaaaccacatactataattcagctc tcaaatccagactgaccatcatcaaggacaactccaagagccaagttttcttaaaa atgaacagtctgcaaactgatgacacagccatttactactgtgccaaacattatta ctacggtggtagctatgctatggactactggggccaaggaacctcagtcaccgtct cctcaaccacgacgccagcgccgcgaccaccaacaccggcgcccaccatcgcgtcg cagcccctgtccctgcgcccagaggcgtgccggccagcggcgggggcgcagtgca cacgagggggctggacttcgcctgtgatatctacatctgggcgcccttggccggga cttgtggggtccttctcctgtcactggttatcaccctttactgcaaacggggcaga aagaaactcctgtatatattcaaacaaccatttatgagaccagtacaaactactca agaggaagatggctgtagctgccgatttccagaagaagaagaaggaggatgtgaac tgagagtgaagttcagcaggagcgcagacgcccccgcgtacaagcagggccagaac cagctctataacgagctcaatctaggacgaagagaggagtacgatgttttggacaa gagacgtggccgggaccctgagatggggggaaagccgagaaggaagaaccctcagg aaggcctgtacaatgaactgcagaaagataagatggcggaggcctacagtgagatt gggatgaaaggcgagcgccggaggggcaaggggcacgatggcctttaccagggtct cagtacagccaccaaggacacctacgacgcccttcacatgcaggccctgccccctc gc |
| **CTL019 Full - aa** | 89 | MALPVTALLLPLALLLHAARPdiqmtqttsslsaslgdrvtiscrasqdiskylnw yqqkpdgtvklliyhtsrlhsgvpsrfsgsgsgtdysltisnleqediatyfcqqg ntlpytfgggtkleitggggsggggsggggsevklqesgpglvapsqslsvtctvs gvslpdygvswirqpprkglewlgviwgsettyynsalksrltiikdnsksqvflk mnslqtddtaiyycakhyyyggsyamdywgqgtsvtvsstttpaprpptpaptias qplslrpeacrpaaggavhtrgldfacdiyiwaplagtcgvlllslvitlyckrgr kkllyifkqpfmrpvqttqeedgcscrfpeeeeggcelrvkfsrsadapaykqgqn qlynelnlgrreeydvldkrrgrdpemggkprrknpqeglynelqkdkmaeaysei gmkgerrrgkghdglyqglstatkdtydalhmqalppr |
| **CTL019** | 51 | diqmtqttsslsaslgdrvtiscrasqdiskylnwyqqkpdgtvklliyhtsrlhs |
| **scFv domain** | | gvpsrfsgsgsgtdysltisnleqediatyfcqqgntlpytfgggtkleitggggs ggggsggggsevklqesgpglvapsqslsvtctvsgvslpdygvswirqpprkgle wlgviwgsettyynsalksrltiikdnsksqvflkmnslqtddtaiyycakhyyyg gsyamdywgqgtsvtvss |

In some embodiments, the CDRs are defined according to the Kabat numbering scheme, the Chothia numbering scheme, or a combination thereof.

The sequences of humanized CDR sequences of the scFv domains are shown in **Table 3A** for the heavy chain variable domains and in **Table 3B** for the light chain variable domains. "ID" stands for the respective SEQ ID NO for each CDR.

**Table 3A. Heavy Chain Variable Domain CDRs (Kabat)**

| Candidate | FW | HCDR1 | ID | HCDR2 | ID | HCDR3 | ID |
|---|---|---|---|---|---|---|---|
| murine_CART19 | | DYGVS | 133 | VIWGSETTYYNSALKS | 134 | HYYYGGSYAMDY | 138 |
| humanized_CART19 a | VH4 | DYGVS | 133 | VIWGSETTYY*S*S*S*LKS | 135 | HYYYGGSYAMDY | 138 |
| humanized_CART19 b | VH4 | DYGVS | 133 | VIWGSETTYY*Q*S*S*LKS | 136 | HYYYGGSYAMDY | 138 |
| humanized_CART19 c | VH4 | DYGVS | 133 | VIWGSETTYYN*S*ALKS | 137 | HYYYGGSYAMDY | 138 |

**Table 3B Light Chain Variable Domain CDRs**

| Candidate | FW | LCDR1 | ID | LCDR2 | ID | LCDR3 | ID |
|---|---|---|---|---|---|---|---|
| murine_CART19 | | RASQDISKYLN | 139 | HTSRLHS | 140 | QQGNTLPYT | 141 |
| humanized_CART19 a | VK3 | RASQDISKYLN | 139 | HTSRLHS | 140 | QQGNTLPYT | 141 |
| humanized_CART19 b | VK3 | RASQDISKYLN | 139 | HTSRLHS | 140 | QQGNTLPYT | 141 |
| humanized_CART19 c | VK3 | RASQDISKYLN | 139 | HTSRLHS | 140 | QQGNTLPYT | 141 |

### Co-expression of CAR with Other Molecules or Agents

### Co-expression of a Second CAR

In one aspect, the CAR-expressing cell described herein can further comprise a second CAR, e.g., a second CAR that includes a different antigen binding domain, e.g., to the same target (e.g., CD19) or a different target (e.g., a target other than CD19, e.g., a target described herein). In one embodiment, the CAR-expressing cell comprises a first CAR that targets a first antigen and includes an intracellular signaling domain having a costimulatory signaling domain but not a primary signaling domain, and a second CAR that targets a second, different, antigen and includes an intracellular signaling domain having a primary signaling domain but not a costimulatory signaling domain. Placement of a costimulatory signaling domain, e.g., 4-1BB, CD28, CD27, OX-40 or ICOS, onto the first CAR, and the primary signaling domain, e.g., CD3 zeta, on the second CAR can limit the CAR activity to cells where both targets are expressed. In one embodiment, the CAR expressing cell comprises a first CAR that includes an antigen binding domain, a transmembrane domain and a costimulatory domain and a second CAR that targets another antigen and includes an antigen binding domain, a transmembrane domain and a primary signaling domain. In another embodiment, the CAR expressing cell comprises a first CAR that includes an antigen binding domain, a transmembrane domain and a primary signaling domain and a second CAR that targets another antigen and includes an antigen binding domain to the antigen, a transmembrane domain and a costimulatory signaling domain.

In one embodiment, the CAR-expressing cell comprises an XCAR described herein and an inhibitory CAR. In one embodiment, the inhibitory CAR comprises an antigen binding domain that binds an antigen found on normal cells but not cancer cells, e.g., normal cells that also express X. In one embodiment, the inhibitory CAR comprises the antigen binding domain, a transmembrane domain and an intracellular domain of an inhibitory molecule. For example, the intracellular domain of the inhibitory CAR can be an intracellular domain of PD1, PD-L1, PD-L2, CTLA4, TIM3, CEACAM (CEACAM-1, CEACAM-3, and/or CEACAM-5), LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, and TGF (e.g., TGF beta).

In one embodiment, when the CAR-expressing cell comprises two or more different CARs, the antigen binding domains of the different CARs can be such that the antigen binding domains do not interact with one another. For example, a cell expressing a first and second CAR can have an antigen binding domain of the first CAR, e.g., as a fragment, e.g., an scFv, that does not form an association with the antigen binding domain of the second CAR, e.g., the antigen binding domain of the second CAR is a VHH.

In some embodiments, the antigen binding domain comprises a single domain antigen binding (SDAB) molecules include molecules whose complementary determining regions are part of a single domain polypeptide. Examples include, but are not limited to, heavy chain variable domains, binding molecules naturally devoid of light chains, single domains derived from conventional 4-chain antibodies, engineered domains and single domain scaffolds other than those derived from antibodies. SDAB molecules may be any of the art, or any future single domain molecules. SDAB molecules may be derived from any species including, but not limited to mouse, human, camel, llama, lamprey, fish, shark, goat, rabbit, and bovine. This term also includes naturally occurring single domain antibody molecules from species other than Camelidae and sharks.

In one aspect, an SDAB molecule can be derived from a variable region of the immunoglobulin found in fish, such as, for example, that which is derived from the immunoglobulin isotype known as Novel Antigen Receptor (NAR) found in the serum of shark. Methods of producing single domain molecules derived from a variable region of NAR ("IgNARs") are described in WO 03/014161 and Streltsov (2005) Protein Sci. 14:2901-2909.

According to another aspect, an SDAB molecule is a naturally occurring single domain antigen binding molecule known as heavy chain devoid of light chains. Such single domain molecules are disclosed in WO 9404678 and Hamers-Casterman, C. et al. (1993) Nature 363:446-448, for example. For clarity reasons, this variable domain derived from a heavy chain molecule naturally devoid of light chain is known herein as a VHH or nanobody to distinguish it from the conventional VH of four chain immunoglobulins. Such a VHH molecule can be derived from Camelidae species, for example in camel, llama, dromedary, alpaca and guanaco. Other species besides Camelidae may produce heavy chain molecules naturally devoid of light chain; such VHHs are within the scope of the invention.

The SDAB molecules can be recombinant, CDR-grafted, humanized, camelized, de-immunized and/or in vitro generated (e.g., selected by phage display).

It has also been discovered, that cells having a plurality of chimeric membrane embedded receptors comprising an antigen binding domain that interactions between the antigen binding domain of the receptors can be undesirable, e.g., because it inhibits the ability of one or more of the antigen binding domains to bind its cognate antigen. Accordingly, disclosed herein are cells having a first and a second non-naturally occurring chimeric membrane embedded receptor comprising antigen binding domains that minimize such interactions. Also disclosed herein are nucleic acids encoding a first and a second non-naturally occurring chimeric membrane embedded receptor comprising an antigen binding domains that minimize such interactions, as well as methods of making and using such cells and nucleic acids. In an embodiment the antigen binding domain of one of the first and the second non-naturally occurring chimeric membrane embedded receptor, comprises an scFv, and the other comprises a single VH domain, e.g., a camelid, shark, or lamprey single VH domain, or a single VH domain derived from a human or mouse sequence.

In some embodiments, a composition herein comprises a first and second CAR, wherein the antigen binding domain of one of the first and the second CAR does not comprise a variable light domain and a variable heavy domain. In some embodiments, the antigen binding domain of one of the first and the second CAR is an scFv, and the other is not an scFv. In some embodiments, the antigen binding domain of one of the first and the second CAR comprises a single VH domain, e.g., a camelid, shark, or lamprey single VH domain, or a single VH domain derived from a human or mouse sequence. In some embodiments, the antigen binding domain of one of the first and the second CAR comprises a nanobody. In some embodiments, the antigen binding domain of one of the first and the second CAR comprises a camelid VHH domain.

In some embodiments, the antigen binding domain of one of the first and the second CAR comprises an scFv, and the other comprises a single VH domain, e.g., a camelid, shark, or lamprey single VH domain, or a single VH domain derived from a human or mouse sequence. In some embodiments, the antigen binding domain of one of the first and the second CAR comprises an scFv, and the other comprises a nanobody. In some embodiments, the antigen binding domain of one of the first and the second CAR comprises an scFv, and the other comprises a camelid VHH domain.

In some embodiments, when present on the surface of a cell, binding of the antigen binding domain of the first CAR to its cognate antigen is not substantially reduced by the presence of the second CAR. In some embodiments, binding of the antigen binding domain of the first CAR to its cognate antigen in the presence of the second CAR is at least 85%, 90%, 95%, 96%, 97%, 98% or 99%, e.g., 85%, 90%, 95%, 96%, 97%, 98% or 99% of binding of the antigen binding domain of the first CAR to its cognate antigen in the absence of the second CAR.

In some embodiments, when present on the surface of a cell, the antigen binding domains of the first and the second CAR, associate with one another less than if both were scFv antigen binding domains. In some embodiments, the antigen binding domains of the first and the second CAR, associate with one another at least 85%, 90%, 95%, 96%, 97%, 98% or 99% less than, e.g., 85%, 90%, 95%, 96%, 97%, 98% or 99% less than if both were scFv antigen binding domains.

### Co-expression of an Agent that Enhances CAR Activity

In another aspect, the CAR-expressing cell described herein can further express another agent, e.g., an agent that enhances the activity or fitness of a CAR-expressing cell.

For example, in one embodiment, the agent can be an agent which inhibits a molecule that modulates or regulates, e.g., inhibits, T cell function. In some embodiments, the molecule that modulates or regulates T cell function is an inhibitory molecule. Inhibitory molecules, e.g., PD1, can, in some embodiments, decrease the ability of a CAR-expressing cell to mount an immune effector response. Examples of inhibitory molecules include PD1, PD-L1, CTLA4, TIM3, LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, or TGF beta.

In embodiments, an agent, e.g., an inhibitory nucleic acid, e.g., a dsRNA, e.g., an siRNA or shRNA; or e.g., an inhibitory protein or system, e.g., a clustered regularly interspaced short palindromic repeats (CRISPR), a transcription-activator like effector nuclease (TALEN), or a zinc finger endonuclease (ZFN), e.g., as described herein, can be used to inhibit expression of a molecule that modulates or regulates, e.g., inhibits, T-cell function in the CAR-expressing cell. In an embodiment the agent is an shRNA, e.g., an shRNA described herein. In an embodiment, the agent that modulates or regulates, e.g., inhibits, T-cell function is inhibited within a CAR-expressing cell. For example, a dsRNA molecule that inhibits expression of a molecule that modulates or regulates, e.g., inhibits, T-cell function is linked to the nucleic acid that encodes a component, e.g., all of the components, of the CAR.

In one embodiment, the agent which inhibits an inhibitory molecule comprises a first polypeptide, e.g., an inhibitory molecule, associated with a second polypeptide that provides a positive signal to the cell, e.g., an intracellular signaling domain described herein. In one embodiment, the agent comprises a first polypeptide, e.g., of an inhibitory molecule such as PD1, PD-L1, CTLA4, TIM3, LAG3, VISTA, BTLA, TIGIT, LAIR1, CD160, 2B4, CD80, CD86, B7-H3 (CD276), B7-H4 (VTCN1), HVEM (TNFRSF14 or CD270), KIR, A2aR, MHC class I, MHC class II, GAL9, adenosine, or TGF beta, or a fragment of any of these (e.g., at least a portion of an extracellular domain of any of these), and a second polypeptide which is an intracellular signaling domain described herein (e.g., comprising a costimulatory domain (e.g., 41BB, CD27 or CD28, e.g., as described herein) and/or a primary signaling domain (e.g., a CD3 zeta signaling domain described herein). In one embodiment, the agent comprises a first polypeptide of PD1 or a fragment thereof (e.g., at least a portion of an extracellular domain of PD1), and a second polypeptide of an intracellular signaling domain described herein (e.g., a CD28 signaling domain described herein and/or a CD3 zeta signaling domain described herein). PD1 is an inhibitory member of the CD28 family of receptors that also includes CD28, CTLA-4, ICOS, and BTLA. PD-1 is expressed on activated B cells, T cells and myeloid cells (Agata et al. 1996 Int. Immunol 8:765-75). Two ligands for PD1, PD-L1 and PD-L2 have been shown to downregulate T cell activation upon binding to PD1 (Freeman et a. 2000 J Exp Med 192:1027-34; Latchman et al. 2001 Nat Immunol 2:261-8; Carter et al. 2002 Eur J Immunol 32:634-43). PD-L1 is abundant in human cancers (Dong et al. 2003 J Mol Med 81:281-7; Blank et al. 2005 Cancer Immunol. Immunother 54:307-314; Konishi et al. 2004 Clin Cancer Res 10:5094). Immune suppression can be reversed by inhibiting the local interaction of PD1 with PD-L1.

In one embodiment, the agent comprises the extracellular domain (ECD) of an inhibitory molecule, e.g., Programmed Death 1 (PD1), can be fused to a transmembrane domain and intracellular signaling domains such as 41BB and CD3 zeta (also referred to herein as a PD1 CAR). In one embodiment, the PD1 CAR, when used in combinations with an XCAR described herein, improves the persistence of the T cell. In one embodiment, the CAR is a PD1 CAR comprising the extracellular domain of PD1 indicated as underlined in SEQ ID NO: 105. In one embodiment, the PD1 CAR comprises the amino acid sequence of SEQ ID NO: 105.

Malpvtalllplalllhaarppgwfldspdrpwnpptfspallvvtegdnatftcsfsntsesfvlnwyrmspsnqtdklaaf
pedrsqpgqdcrfrvtqlpngrdfhmsvvrarrndsgtylcgaislapkaqikeslraelrvterraevptahpspsprpagqfqtlvttt
paprpptpaptiasqplslrpeacrpaaggavhtrgldfacdiyiwaplagtcgvlllslvitlyckrgrkkllyifkqpfmrpvqttqee
dgcscrfpeeeeggcelrvkfsrsadapaykqgqnqlynelnlgrreeydvldkrrgrdpemggkprrknpqeglynelqkdkma
eayseigmkgerrrgkghdglyqglstatkdtydalhmqalppr (SEQ ID NO:105).

In one embodiment, the PD1 CAR comprises the amino acid sequence provided below (SEQ ID NO: 106). 
pgwfldspdrpwnpptfspallvvtegdnatftcsfsntsesfvlnwyrmspsnqtdklaafpedrsqpgqdcrfrvtqlp
ngrdfhmsvvrarrndsgtylcgaislapkaqikeslraelrvterraevptahpspsprpagqfqtlvtttpaprpptpaptiasqplslrp
eacrpaaggavhtrgldfacdiyiwaplagtcgvlllslvitlyckrgrkkllyifkqpfmrpvqttqeedgcscrfpeeeeggcelrvkf
srsadapaykqgqnqlynelnlgrreeydvldkrrgrdpemggkprrknpqeglynelqkdkmaeayseigmkgerrrgkghdgl
yqglstatkdtydalhmqalppr (SEQ ID NO:106).
 In one embodiment, the agent comprises a nucleic acid sequence encoding the PD1 CAR, e.g., the PD1 CAR described herein. In one embodiment, the nucleic acid sequence for the PD1 CAR is shown below, with the PD1 ECD underlined below in SEQ ID NO: 107

atggccctccctgtcactgccctgcttctccccctcgcactcctgctccacgccgctagaccacccggatggtttctggactctc
cggatcgcccgtggaatcccccaaccttctcaccggcactcttggttgtgactgagggcgataatgcgaccttcacgtgctcgttctccaa
cacctccgaatcattcgtgctgaactggtaccgcatgagcccgtcaaaccagaccgacaagctcgccgcgtttccggaagatcggtcgc
aaccgggacaggattgtcggttccgcgtgactcaactgccgaatggcagagacttccacatgagcgtggtccgcgctaggcgaaacga
ctccgggacctacctgtgcggagccatctcgctggcgcctaaggcccaaatcaaagagagcttgagggccgaactgagagtgaccga
gcgcagagctgaggtgccaactgcacatccatccccatcgcctcggcctgcggggcagtttcagaccctggtcacgaccactccggcg
ccgcgcccaccgactccggccccaactatcgcgagccagcccctgtcgctgaggccggaagcatgccgccctgccgccggaggtgc
tgtgcatacccggggattggacttcgcatgcgacatctacatttgggctcctctcgccggaacttgtggcgtgctccttctgtccctggtcat
caccctgtactgcaagcggggtcggaaaaagcttctgtacattttcaagcagcccttcatgaggcccgtgcaaaccacccaggaggagg
acggttgctcctgccggttccccgaagaggaagaaggaggttgcgagctgcgcgtgaagttctcccggagcgccgacgcccccgcct
ataagcagggccagaaccagctgtacaacgaactgaacctgggacggcgggaagagtacgatgtgctggacaagcggcgcggccg
ggaccccgaaatgggcgggaagcctagaagaaagaaccctcaggaaggcctgtataacgagctgcagaaggacaagatggccgag
gcctactccgaaattgggatgaagggagagcggcggaggggaaaggggcacgacggcctgtaccaaggactgtccaccgccacca
aggacacatacgatgccctgcacatgcaggcccttccccctcgc (SEQ ID NO: 107).

In another example, in one embodiment, the agent which enhances the activity of a CAR-expressing cell can be a costimulatory molecule or costimulatory molecule ligand. Examples of costimulatory molecules include MHC class I molecule, BTLA and a Toll ligand receptor, as well as OX40, CD27, CD28, CDS, ICAM-1, LFA-1 (CD11a/CD18), ICOS (CD278), and 4-1BB (CD137). Further examples of such costimulatory molecules include CDS, ICAM-1, GITR, BAFFR, HVEM (LIGHTR), SLAMF7, NKp80 (KLRF1), NKp44, NKp30, NKp46, CD160, CD19, CD4, CD8alpha, CD8beta, IL2R beta, IL2R gamma, IL7R alpha, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, TNFR2, TRANCE/RANKL, DNAM1 (CD226), SLAMF4 (CD244, 2B4), CD84, CD96 (Tactile), CEACAM1, CRTAM, Ly9 (CD229), CD160 (BY55), PSGL1, CD100 (SEMA4D), CD69, SLAMF6 (NTB-A, Ly108), SLAM (SLAMF1, CD150, IPO-3), BLAME (SLAMF8), SELPLG (CD162), LTBR, LAT, GADS, SLP-76, PAG/Cbp, CD19a, and a ligand that specifically binds with CD83., e.g., as described herein. Examples of costimulatory molecule ligands include CD80, CD86, CD40L, ICOSL, CD70, OX40L, 4-1BBL, GITRL, and LIGHT. In embodiments, the costimulatory molecule ligand is a ligand for a costimulatory molecule different from the costimulatory molecule domain of the CAR. In embodiments, the costimulatory molecule ligand is a ligand for a costimulatory molecule that is the same as the costimulatory molecule domain of the CAR. In an embodiment, the costimulatory molecule ligand is 4-1BBL. In an embodiment, the costimulatory ligand is CD80 or CD86. In an embodiment, the costimulatory molecule ligand is CD70. In embodiments, a CAR-expressing immune effector cell described herein can be further engineered to express one or more additional costimulatory molecules or costimulatory molecule ligands.

### Co-expression of CAR with a Chemokine Receptor

In embodiments, the CAR-expressing cell described herein, e.g., CD19 CAR-expressing cell, further comprises a chemokine receptor molecule. Transgenic expression of chemokine receptors CCR2b or CXCR2 in T cells enhances trafficking to CCL2- or CXCL1-secreting solid tumors including melanoma and neuroblastoma (Craddock et al., J Immunother. 2010 Oct; 33(8):780-8 and Kershaw et al., Hum Gene Ther. 2002 Nov 1; 13(16):1971-80). Thus, without wishing to be bound by theory, it is believed that chemokine receptors expressed in CAR-expressing cells that recognize chemokines secreted by tumors, e.g., solid tumors, can improve homing of the CAR-expressing cell to the tumor, facilitate the infiltration of the CAR-expressing cell to the tumor, and enhances antitumor efficacy of the CAR-expressing cell. The chemokine receptor molecule can comprise a naturally occurring or recombinant chemokine receptor or a chemokine-binding fragment thereof. A chemokine receptor molecule suitable for expression in a CAR-expressing cell (e.g., CAR-Tx) described herein include a CXC chemokine receptor (e.g., CXCR1, CXCR2, CXCR3, CXCR4, CXCR5, CXCR6, or CXCR7), a CC chemokine receptor (e.g., CCR1, CCR2, CCR3, CCR4, CCR5, CCR6, CCR7, CCR8, CCR9, CCR10, or CCR11), a CX3C chemokine receptor (e.g., CX3CR1), a XC chemokine receptor (e.g., XCR1), or a chemokine-binding fragment thereof. In one embodiment, the chemokine receptor molecule to be expressed with a CAR described herein is selected based on the chemokine(s) secreted by the tumor. In one embodiment, the CAR-expressing cell described herein further comprises, e.g., expresses, a CCR2b receptor or a CXCR2 receptor. In an embodiment, the CAR described herein and the chemokine receptor molecule are on the same vector or are on two different vectors. In embodiments where the CAR described herein and the chemokine receptor molecule are on the same vector, the CAR and the chemokine receptor molecule are each under control of two different promoters or are under the control of the same promoter.

### Nucleic Acid Constructs Encoding a CAR

The present invention also provides an immune effector cell, e.g., made by a method described herein, that includes a nucleic acid molecules encoding one or more CAR constructs described herein. In one aspect, the nucleic acid molecule is provided as a messenger RNA transcript. In one aspect, the nucleic acid molecule is provided as a DNA construct.

The nucleic acid molecules described herein can be a DNA molecule, an RNA molecule, or a combination thereof. In one embodiment, the nucleic acid molecule is an mRNA encoding a CAR polypeptide as described herein. In other embodiments, the nucleic acid molecule is a vector that includes any of the aforesaid nucleic acid molecules.

In one aspect, the antigen binding domain of a CAR of the invention (e.g., a scFv) is encoded by a nucleic acid molecule whose sequence has been codon optimized for expression in a mammalian cell. In one aspect, entire CAR construct of the invention is encoded by a nucleic acid molecule whose entire sequence has been codon optimized for expression in a mammalian cell. Codon optimization refers to the discovery that the frequency of occurrence of synonymous codons (i.e., codons that code for the same amino acid) in coding DNA is biased in different species. Such codon degeneracy allows an identical polypeptide to be encoded by a variety of nucleotide sequences. A variety of codon optimization methods is known in the art, and include, e.g., methods disclosed in at least US Patent Numbers 5,786,464 and 6,114,148.

Accordingly, in one aspect, an immune effector cell, e.g., made by a method described herein, includes a nucleic acid molecule encoding a chimeric antigen receptor (CAR), wherein the CAR comprises an antigen binding domain that binds to a tumor antigen described herein, a transmembrane domain (e.g., a transmembrane domain described herein), and an intracellular signaling domain (e.g., an intracellular signaling domain described herein) comprising a stimulatory domain, e.g., a costimulatory signaling domain (e.g., a costimulatory signaling domain described herein) and/or a primary signaling domain (e.g., a primary signaling domain described herein, e.g., a zeta chain described herein).

The present invention also provides vectors in which a nucleic acid molecule encoding a CAR, e.g., a nucleic acid molecule described herein, is inserted. Vectors derived from retroviruses such as the lentivirus are suitable tools to achieve long-term gene transfer since they allow long-term, stable integration of a transgene and its propagation in daughter cells. Lentiviral vectors have the added advantage over vectors derived from onco-retroviruses such as murine leukemia viruses in that they can transduce non-proliferating cells, such as hepatocytes. They also have the added advantage of low immunogenicity. A retroviral vector may also be, e.g., a gammaretroviral vector. A gammaretroviral vector may include, e.g., a promoter, a packaging signal (ψ), a primer binding site (PBS), one or more (e.g., two) long terminal repeats (LTR), and a transgene of interest, e.g., a gene encoding a CAR. A gammaretroviral vector may lack viral structural gens such as gag, pol, and env. Exemplary gammaretroviral vectors include Murine Leukemia Virus (MLV), Spleen-Focus Forming Virus (SFFV), and Myeloproliferative Sarcoma Virus (MPSV), and vectors derived therefrom. Other gammaretroviral vectors are described, e.g., in Tobias Maetzig et al., "Gammaretroviral Vectors: Biology, Technology and Application" Viruses. 2011 Jun; 3(6): 677-713.

In another embodiment, the vector comprising the nucleic acid encoding the desired CAR is an adenoviral vector (A5/35). In another embodiment, the expression of nucleic acids encoding CARs can be accomplished using of transposons such as sleeping beauty, crisper, CAS9, and zinc finger nucleases. See below June et al. 2009Nature Reviews Immunology 9.10: 704-716, is incorporated herein by reference.

In brief summary, the expression of natural or synthetic nucleic acids encoding CARs is typically achieved by operably linking a nucleic acid encoding the CAR polypeptide or portions thereof to a promoter, and incorporating the construct into an expression vector. The vectors can be suitable for replication and integration eukaryotes. Typical cloning vectors contain transcription and translation terminators, initiation sequences, and promoters useful for regulation of the expression of the desired nucleic acid sequence.

The nucleic acid can be cloned into a number of types of vectors. For example, the nucleic acid can be cloned into a vector including, but not limited to a plasmid, a phagemid, a phage derivative, an animal virus, and a cosmid. Vectors of particular interest include expression vectors, replication vectors, probe generation vectors, and sequencing vectors.

Further, the expression vector may be provided to a cell in the form of a viral vector. Viral vector technology is well known in the art and is described, for example, in Sambrook et al., 2012, MOLECULAR CLONING: A LABORATORY MANUAL, volumes 1 -4, Cold Spring Harbor Press, NY), and in other virology and molecular biology manuals. Viruses, which are useful as vectors include, but are not limited to, retroviruses, adenoviruses, adeno-associated viruses, herpes viruses, and lentiviruses. In general, a suitable vector contains an origin of replication functional in at least one organism, a promoter sequence, convenient restriction endonuclease sites, and one or more selectable markers, (e.g., WO 01/96584; WO 01/29058; and U.S. Pat. No. 6,326,193).

A number of viral based systems have been developed for gene transfer into mammalian cells. For example, retroviruses provide a convenient platform for gene delivery systems. A selected gene can be inserted into a vector and packaged in retroviral particles using techniques known in the art. The recombinant virus can then be isolated and delivered to cells of the subject either in vivo or ex vivo. A number of retroviral systems are known in the art. In some embodiments, adenovirus vectors are used. A number of adenovirus vectors are known in the art. In one embodiment, lentivirus vectors are used.

Additional promoter elements, e.g., enhancers, regulate the frequency of transcriptional initiation. Typically, these are located in the region 30-110 bp upstream of the start site, although a number of promoters have been shown to contain functional elements downstream of the start site as well. The spacing between promoter elements frequently is flexible, so that promoter function is preserved when elements are inverted or moved relative to one another. In the thymidine kinase (tk) promoter, the spacing between promoter elements can be increased to 50 bp apart before activity begins to decline. Depending on the promoter, it appears that individual elements can function either cooperatively or independently to activate transcription. Exemplary promoters include the CMV IE gene, EF-1α, ubiquitin C, or phosphoglycerokinase (PGK) promoters.

An example of a promoter that is capable of expressing a CAR encoding nucleic acid molecule in a mammalian T cell is the EF1a promoter. The native EF1a promoter drives expression of the alpha subunit of the elongation factor-1 complex, which is responsible for the enzymatic delivery of aminoacyl tRNAs to the ribosome. The EF1a promoter has been extensively used in mammalian expression plasmids and has been shown to be effective in driving CAR expression from nucleic acid molecules cloned into a lentiviral vector. See, e.g., Milone et al., Mol. Ther. 17(8): 1453-1464 (2009). In one aspect, the EF1a promoter comprises the sequence provided in the Examples.

Another example of a promoter is the immediate early cytomegalovirus (CMV) promoter sequence. This promoter sequence is a strong constitutive promoter sequence capable of driving high levels of expression of any polynucleotide sequence operatively linked thereto. However, other constitutive promoter sequences may also be used, including, but not limited to the simian virus 40 (SV40) early promoter, mouse mammary tumor virus (MMTV), human immunodeficiency virus (HIV) long terminal repeat (LTR) promoter, MoMuLV promoter, an avian leukemia virus promoter, an Epstein-Barr virus immediate early promoter, a Rous sarcoma virus promoter, as well as human gene promoters such as, but not limited to, the actin promoter, the myosin promoter, the elongation factor-1α promoter, the hemoglobin promoter, and the creatine kinase promoter. Further, the invention should not be limited to the use of constitutive promoters. Inducible promoters are also contemplated as part of the invention. The use of an inducible promoter provides a molecular switch capable of turning on expression of the polynucleotide sequence which it is operatively linked when such expression is desired, or turning off the expression when expression is not desired. Examples of inducible promoters include, but are not limited to a metallothionine promoter, a glucocorticoid promoter, a progesterone promoter, and a tetracycline promoter.

Another example of a promoter is the phosphoglycerate kinase (PGK) promoter. In embodiments, a truncated PGK promoter (e.g., a PGK promoter with one or more, e.g., 1, 2, 5, 10, 100, 200, 300, or 400, nucleotide deletions when compared to the wild-type PGK promoter sequence) may be desired.

The nucleotide sequences of exemplary PGK promoters are provided below.

### WT PGK Promoter:

ACCCCTCTCTCCAGCCACTAAGCCAGTTGCTCCCTCGGCTGACGGCTGCACG
CGAGGCCTCCGAACGTCTTACGCCTTGTGGCGCGCCCGTCCTTGTCCCGGGTGTGA
TGGCGGGGTGTGGGGCGGAGGGCGTGGCGGGGAAGGGCCGGCGACGAGAGCCGC
GCGGGACGACTCGTCGGCGATAACCGGTGTCGGGTAGCGCCAGCCGCGCGACGGT
AACGAGGGACCGCGACAGGCAGACGCTCCCATGATCACTCTGCACGCCGAAGGCA
AATAGTGCAGGCCGTGCGGCGCTTGGCGTTCCTTGGAAGGGCTGAATCCCCGCCTC
GTCCTTCGCAGCGGCCCCCCGGGTGTTCCCATCGCCGCTTCTAGGCCCACTGCGAC
GCTTGCCTGCACTTCTTACACGCTCTGGGTCCCAGCCGCGGCGACGCAAAGGGCCT
TGGTGCGGGTCTCGTCGGCGCAGGGACGCGTTTGGGTCCCGACGGAACCTTTTCCG
CGTTGGGGTTGGGGCACCATAAGCT (SEQ ID NO: 109)

Exemplary truncated PGK Promoters:
PGK100: 
ACCCCTCTCTCCAGCCACTAAGCCAGTTGCTCCCTCGGCTGACGGCTGCACG
CGAGGCCTCCGAACGTCTTACGCCTTGTGGCGCGCCCGTCCTTGTCCCGGGTGTGA
TGGCGGGGTG (SEQ ID NO: 110)
PGK200: 
ACCCCTCTCTCCAGCCACTAAGCCAGTTGCTCCCTCGGCTGACGGCTGCACG
CGAGGCCTCCGAACGTCTTACGCCTTGTGGCGCGCCCGTCCTTGTCCCGGGTGTGA
TGGCGGGGTGTGGGGCGGAGGGCGTGGCGGGGAAGGGCCGGCGACGAGAGCCGC
GCGGGACGACTCGTCGGCGATAACCGGTGTCGGGTAGCGCCAGCCGCGCGACGGT
AACG (SEQ ID NO:111)
PGK300: 
ACCCCTCTCTCCAGCCACTAAGCCAGTTGCTCCCTCGGCTGACGGCTGCACG
CGAGGCCTCCGAACGTCTTACGCCTTGTGGCGCGCCCGTCCTTGTCCCGGGTGTGA
TGGCGGGGTGTGGGGCGGAGGGCGTGGCGGGGAAGGGCCGGCGACGAGAGCCGC
GCGGGACGACTCGTCGGCGATAACCGGTGTCGGGTAGCGCCAGCCGCGCGACGGT
AACGAGGGACCGCGACAGGCAGACGCTCCCATGATCACTCTGCACGCCGAAGGCA
AATAGTGCAGGCCGTGCGGCGCTTGGCGTTCCTTGGAAGGGCTGAATCCCCG (SEQ
ID NO:112)
PGK400:

ACCCCTCTCTCCAGCCACTAAGCCAGTTGCTCCCTCGGCTGACGGCTGCACG
CGAGGCCTCCGAACGTCTTACGCCTTGTGGCGCGCCCGTCCTTGTCCCGGGTGTGA
TGGCGGGGTGTGGGGCGGAGGGCGTGGCGGGGAAGGGCCGGCGACGAGAGCCGC
GCGGGACGACTCGTCGGCGATAACCGGTGTCGGGTAGCGCCAGCCGCGCGACGGT
AACGAGGGACCGCGACAGGCAGACGCTCCCATGATCACTCTGCACGCCGAAGGCA
AATAGTGCAGGCCGTGCGGCGCTTGGCGTTCCTTGGAAGGGCTGAATCCCCGCCTC
GTCCTTCGCAGCGGCCCCCCGGGTGTTCCCATCGCCGCTTCTAGGCCCACTGCGAC
GCTTGCCTGCACTTCTTACACGCTCTGGGTCCCAGCCG (SEQ ID NO:113)

A vector may also include, e.g., a signal sequence to facilitate secretion, a polyadenylation signal and transcription terminator (e.g., from Bovine Growth Hormone (BGH) gene), an element allowing episomal replication and replication in prokaryotes (e.g. SV40 origin and ColE1 or others known in the art) and/or elements to allow selection (e.g., ampicillin resistance gene and/or zeocin marker).

In order to assess the expression of a CAR polypeptide or portions thereof, the expression vector to be introduced into a cell can also contain either a selectable marker gene or a reporter gene or both to facilitate identification and selection of expressing cells from the population of cells sought to be transfected or infected through viral vectors. In other aspects, the selectable marker may be carried on a separate piece of DNA and used in a co- transfection procedure. Both selectable markers and reporter genes may be flanked with appropriate regulatory sequences to enable expression in the host cells. Useful selectable markers include, for example, antibiotic-resistance genes, such as neo and the like.

Reporter genes are used for identifying potentially transfected cells and for evaluating the functionality of regulatory sequences. In general, a reporter gene is a gene that is not present in or expressed by the recipient organism or tissue and that encodes a polypeptide whose expression is manifested by some easily detectable property, e.g., enzymatic activity. Expression of the reporter gene is assayed at a suitable time after the DNA has been introduced into the recipient cells. Suitable reporter genes may include genes encoding luciferase, beta-galactosidase, chloramphenicol acetyl transferase, secreted alkaline phosphatase, or the green fluorescent protein gene (e.g., Ui-Tei et al., 2000 FEBS Letters 479: 79-82). Suitable expression systems are well known and may be prepared using known techniques or obtained commercially. In general, the construct with the minimal 5' flanking region showing the highest level of expression of reporter gene is identified as the promoter. Such promoter regions may be linked to a reporter gene and used to evaluate agents for the ability to modulate promoter-driven transcription.

In embodiments, the vector may comprise two or more nucleic acid sequences encoding a CAR, e.g., a CAR described herein, e.g., a CD19 CAR, and a second CAR, e.g., an inhibitory CAR or a CAR that specifically binds to an antigen other than CD19. In such embodiments, the two or more nucleic acid sequences encoding the CAR are encoded by a single nucleic molecule in the same frame and as a single polypeptide chain. In this aspect, the two or more CARs, can, e.g., be separated by one or more peptide cleavage sites. (e.g., an auto-cleavage site or a substrate for an intracellular protease). Examples of peptide cleavage sites include T2A, P2A, E2A, or F2A sites.

Methods of introducing and expressing genes into a cell are known in the art. In the context of an expression vector, the vector can be readily introduced into a host cell, e.g., mammalian, bacterial, yeast, or insect cell by any method, e.g., one known in the art. For example, the expression vector can be transferred into a host cell by physical, chemical, or biological means.

Physical methods for introducing a polynucleotide into a host cell include calcium phosphate precipitation, lipofection, particle bombardment, microinjection, electroporation, and the like. Methods for producing cells comprising vectors and/or exogenous nucleic acids are well-known in the art. See, for example, Sambrook et al., 2012, MOLECULAR CLONING: A LABORATORY MANUAL, volumes 1 -4, Cold Spring Harbor Press, NY). A suitable method for the introduction of a polynucleotide into a host cell is calcium phosphate transfection.

Biological methods for introducing a polynucleotide of interest into a host cell include the use of DNA and RNA vectors. Viral vectors, and especially retroviral vectors, have become the most widely used method for inserting genes into mammalian, e.g., human cells. Other viral vectors can be derived from lentivirus, poxviruses, herpes simplex virus I, adenoviruses and adeno-associated viruses, and the like. See, for example, U.S. Pat. Nos. 5,350,674 and 5,585,362.

Chemical means for introducing a polynucleotide into a host cell include colloidal dispersion systems, such as macromolecule complexes, nanocapsules, microspheres, beads, and lipid-based systems including oil-in-water emulsions, micelles, mixed micelles, and liposomes. An exemplary colloidal system for use as a delivery vehicle in vitro and in vivo is a liposome (e.g., an artificial membrane vesicle). Other methods of state-of-the-art targeted delivery of nucleic acids are available, such as delivery of polynucleotides with targeted nanoparticles or other suitable sub-micron sized delivery system.

In the case where a non-viral delivery system is utilized, an exemplary delivery vehicle is a liposome. The use of lipid formulations is contemplated for the introduction of the nucleic acids into a host cell (in vitro, ex vivo or in vivo). In another aspect, the nucleic acid may be associated with a lipid. The nucleic acid associated with a lipid may be encapsulated in the aqueous interior of a liposome, interspersed within the lipid bilayer of a liposome, attached to a liposome via a linking molecule that is associated with both the liposome and the oligonucleotide, entrapped in a liposome, complexed with a liposome, dispersed in a solution containing a lipid, mixed with a lipid, combined with a lipid, contained as a suspension in a lipid, contained or complexed with a micelle, or otherwise associated with a lipid. Lipid, lipid/DNA or lipid/expression vector associated compositions are not limited to any particular structure in solution. For example, they may be present in a bilayer structure, as micelles, or with a "collapsed" structure. They may also simply be interspersed in a solution, possibly forming aggregates that are not uniform in size or shape. Lipids are fatty substances which may be naturally occurring or synthetic lipids. For example, lipids include the fatty droplets that naturally occur in the cytoplasm as well as the class of compounds which contain long-chain aliphatic hydrocarbons and their derivatives, such as fatty acids, alcohols, amines, amino alcohols, and aldehydes.

Lipids suitable for use can be obtained from commercial sources. For example, dimyristyl phosphatidylcholine ("DMPC") can be obtained from Sigma, St. Louis, MO; dicetyl phosphate ("DCP") can be obtained from K & K Laboratories (Plainview, NY); cholesterol ("Choi") can be obtained from Calbiochem-Behring; dimyristyl phosphatidylglycerol ("DMPG") and other lipids may be obtained from Avanti Polar Lipids, Inc. (Birmingham, AL.). Stock solutions of lipids in chloroform or chloroform/methanol can be stored at about - 20°C. Chloroform is used as the only solvent since it is more readily evaporated than methanol. "Liposome" is a generic term encompassing a variety of single and multilamellar lipid vehicles formed by the generation of enclosed lipid bilayers or aggregates. Liposomes can be characterized as having vesicular structures with a phospholipid bilayer membrane and an inner aqueous medium. Multilamellar liposomes have multiple lipid layers separated by aqueous medium. They form spontaneously when phospholipids are suspended in an excess of aqueous solution. The lipid components undergo self-rearrangement before the formation of closed structures and entrap water and dissolved solutes between the lipid bilayers (Ghosh et al., 1991 Glycobiology 5: 505-10). However, compositions that have different structures in solution than the normal vesicular structure are also encompassed. For example, the lipids may assume a micellar structure or merely exist as nonuniform aggregates of lipid molecules. Also contemplated are lipofectamine-nucleic acid complexes.

Regardless of the method used to introduce exogenous nucleic acids into a host cell or otherwise expose a cell to the inhibitor of the present invention, in order to confirm the presence of the recombinant nucleic acid sequence in the host cell, a variety of assays may be performed. Such assays include, for example, "molecular biological" assays well known to those of skill in the art, such as Southern and Northern blotting, RT-PCR and PCR; "biochemical" assays, such as detecting the presence or absence of a particular peptide, e.g., by immunological means (ELISAs and Western blots) or by assays described herein to identify agents falling within the scope of the invention.

### Natural Killer Cell Receptor (NKR) CARs

In an embodiment, the CAR molecule described herein comprises one or more components of a natural killer cell receptor (NKR), thereby forming an NKR-CAR. The NKR component can be a transmembrane domain, a hinge domain, or a cytoplasmic domain from any of the following natural killer cell receptors: killer cell immunoglobulin-like receptor (KIR), e.g., KIR2DL1, KIR2DL2/L3, KIR2DL4, KIR2DL5A, KIR2DL5B, KIR2DS1, KIR2DS2, KIR2DS3, KIR2DS4, DIR2DS5, KIR3DL1/S1, KIR3DL2, KIR3DL3, KIR2DP1, and KIR3DP1; natural cytotoxicity receptor (NCR), e.g., NKp30, NKp44, NKp46; signaling lymphocyte activation molecule (SLAM) family of immune cell receptors, e.g., CD48, CD229, 2B4, CD84, NTB-A, CRACC, BLAME, and CD2F-10; Fc receptor (FcR), e.g., CD16, and CD64; and Ly49 receptors, e.g., LY49A, LY49C. The NKR-CAR molecules described herein may interact with an adaptor molecule or intracellular signaling domain, e.g., DAP12. Exemplary configurations and sequences of CAR molecules comprising NKR components are described in International Publication No. WO2014/145252, the contents of which are hereby incorporated by reference.

### Split CAR

In some embodiments, the CAR-expressing cell uses a split CAR. The split CAR approach is described in more detail in publications WO2014/055442 and WO2014/055657. Briefly, a split CAR system comprises a cell expressing a first CAR having a first antigen binding domain and a costimulatory domain (e.g., 41BB), and the cell also expresses a second CAR having a second antigen binding domain and an intracellular signaling domain (e.g., CD3 zeta). When the cell encounters the first antigen, the costimulatory domain is activated, and the cell proliferates. When the cell encounters the second antigen, the intracellular signaling domain is activated and cell-killing activity begins. Thus, the CAR-expressing cell is only fully activated in the presence of both antigens.

### Strategies for Regulating Chimeric Antigen Receptors

In some embodiments, a regulatable CAR (RCAR) where the CAR activity can be controlled is desirable to optimize the safety and efficacy of a CAR therapy. There are many ways CAR activities can be regulated. For example, inducible apoptosis using, e.g., a caspase fused to a dimerization domain (see, e.g., Di Stasa et al., N Engl. J. Med. 2011 Nov. 3; 365(18):1673-1683), can be used as a safety switch in the CAR therapy of the instant invention. In one embodiment, the cells (e.g., T cells or NK cells) expressing a CAR of the present invention further comprise an inducible apoptosis switch, wherein a human caspase (e.g., caspase 9) or a modified version is fused to a modification of the human FKB protein that allows conditional dimerization. In the presence of a small molecule, such as a rapalog (e.g., AP 1903, AP20187), the inducible caspase (e.g., caspase 9) is activated and leads to the rapid apoptosis and death of the cells (e.g., T cells or NK cells) expressing a CAR of the present invention. Examples of a caspase-based inducible apoptosis switch (or one or more aspects of such a switch) have been described in, e.g., US2004040047; US20110286980; US20140255360; WO1997031899; WO2014151960; WO2014164348; WO2014197638; WO2014197638; all of which are incorporated by reference herein.

In another example, CAR-expressing cells can also express an inducible Caspase-9 (iCaspase-9) molecule that, upon administration of a dimerizer drug (e.g., rimiducid (also called AP1903 (Bellicum Pharmaceuticals) or AP20187 (Ariad)) leads to activation of the Caspase-9 and apoptosis of the cells. The iCaspase-9 molecule contains a chemical inducer of dimerization (CID) binding domain that mediates dimerization in the presence of a CID. This results in inducible and selective depletion of CAR-expressing cells. In some cases, the iCaspase-9 molecule is encoded by a nucleic acid molecule separate from the CAR-encoding vector(s). In some cases, the iCaspase-9 molecule is encoded by the same nucleic acid molecule as the CAR-encoding vector. The iCaspase-9 can provide a safety switch to avoid any toxicity of CAR-expressing cells. See, e.g., Song et al. Cancer Gene Ther. 2008; 15(10):667-75; Clinical Trial Id. No. NCT02107963; and Di Stasi et al. N. Engl. J. Med. 2011; 365:1673-83.

Alternative strategies for regulating the CAR therapy of the instant invention include utilizing small molecules or antibodies that deactivate or turn off CAR activity, e.g., by deleting CAR-expressing cells, e.g., by inducing antibody dependent cell-mediated cytotoxicity (ADCC). For example, CAR-expressing cells described herein may also express an antigen that is recognized by molecules capable of inducing cell death, e.g., ADCC or complement-induced cell death. For example, CAR expressing cells described herein may also express a receptor capable of being targeted by an antibody or antibody fragment. Examples of such receptors include EpCAM, VEGFR, integrins (e.g., integrins αvβ3, α4, αI¾β3, α4β7, α5β1, ανβ3,αν), members of the TNF receptor superfamily (e.g., TRAIL-R1 , TRAIL-R2), PDGF Receptor, interferon receptor, folate receptor, GPNMB, ICAM-1 , HLA-DR, CEA, CA-125, MUC1 , TAG-72, IL-6 receptor, 5T4, GD2, GD3, CD2, CD3, CD4, CD5, CD1 1 , CD1 1 a/LFA-1 , CD15, CD18/ITGB2, CD19, CD20, CD22, CD23/lgE Receptor, CD25, CD28, CD30, CD33, CD38, CD40, CD41 , CD44, CD51 , CD52, CD62L, CD74, CD80, CD125, CD147/basigin, CD152/CTLA-4, CD154/CD40L, CD195/CCR5, CD319/SLAMF7, and EGFR, and truncated versions thereof (e.g., versions preserving one or more extracellular epitopes but lacking one or more regions within the cytoplasmic domain).

For example, a CAR-expressing cell described herein may also express a truncated epidermal growth factor receptor (EGFR) which lacks signaling capacity but retains the epitope that is recognized by molecules capable of inducing ADCC, e.g., cetuximab (ERBITUX^{®}), such that administration of cetuximab induces ADCC and subsequent depletion of the CAR-expressing cells (see, e.g., WO2011/056894, and Jonnalagadda et al., Gene Ther. 2013; 20(8)853-860). Another strategy includes expressing a highly compact marker/suicide gene that combines target epitopes from both CD32 and CD20 antigens in the CAR-expressing cells described herein, which binds rituximab, resulting in selective depletion of the CAR-expressing cells, e.g., by ADCC (see, e.g., Philip et al., Blood. 2014; 124(8)1277-1287). Other methods for depleting CAR-expressing cells described herein include administration of CAMPATH, a monoclonal anti-CD52 antibody that selectively binds and targets mature lymphocytes, e.g., CAR-expressing cells, for destruction, e.g., by inducing ADCC. In other embodiments, the CAR-expressing cell can be selectively targeted using a CAR ligand, e.g., an anti-idiotypic antibody. In some embodiments, the anti-idiotypic antibody can cause effector cell activity, e.g., ADCC or ADC activities, thereby reducing the number of CAR-expressing cells. In other embodiments, the CAR ligand, e.g., the anti-idiotypic antibody, can be coupled to an agent that induces cell killing, e.g., a toxin, thereby reducing the number of CAR-expressing cells. Alternatively, the CAR molecules themselves can be configured such that the activity can be regulated, e.g., turned on and off, as described below.

In other embodiments, a CAR-expressing cell described herein may also express a target protein recognized by the T cell depleting agent. In one embodiment, the target protein is CD20 and the T cell depleting agent is an anti-CD20 antibody, e.g., rituximab. In such embodiment, the T cell depleting agent is administered once it is desirable to reduce or eliminate the CAR-expressing cell, e.g., to mitigate the CAR induced toxicity. In other embodiments, the T cell depleting agent is an anti-CD52 antibody, e.g., alemtuzumab, as described in the Examples herein.

In other embodiments, an RCAR comprises a set of polypeptides, typically two in the simplest embodiments, in which the components of a standard CAR described herein, e.g., an antigen binding domain and an intracellular signalling domain, are partitioned on separate polypeptides or members. In some embodiments, the set of polypeptides include a dimerization switch that, upon the presence of a dimerization molecule, can couple the polypeptides to one another, e.g., can couple an antigen binding domain to an intracellular signalling domain. In one embodiment, a CAR of the present invention utilizes a dimerization switch as those described in, e.g., WO2014127261, which is incorporated by reference herein. Additional description and exemplary configurations of such regulatable CARs are provided herein and in, e.g., paragraphs 527-551 of International Publication No. WO 2015/090229 filed March 13, 2015, which is incorporated by reference in its entirety. In some embodiments, an RCAR involves a switch domain, e.g., a FKBP switch domain, as set out SEQ ID NO: 114, or comprise a fragment of FKBP having the ability to bind with FRB, e.g., as set out in SEQ ID NO: 115. In some embodiments, the RCAR involves a switch domain comprising a FRB sequence, e.g., as set out in SEQ ID NO: 116, or a mutant FRB sequence, e.g., as set out in any of SEQ ID Nos. 117-122.

DVPDYASLGGPSSPKKKRKVSRGVQVETISPGDGRTFPKRGQTCVVHYTGMLE
DGKKFDSSRDRNKPFKFMLGKQEVIRGWEEGVAQMSVGQRAKLTISPDYAYGATGHP
GIIPPHATLVFDVELLKLETSY (SEQ ID NO: 114)

VQVETISPGDGRTFPKRGQTCVVHYTGMLEDGKKFDSSRDRNKPFKFMLGKQE
VIRGWEEGVAQMSVGQRAKLTISPDYAYGATGHPGIIPPHATLVFDVELLKLETS (SEQ
ID NO: 115)


 (SEQ ID NO: 116)

**Table 13. Exemplary mutant FRB having increased affinity for a dimerization molecule.**

| **FRB mutant** | **Amino Acid Sequence** | **SEQ ID NO:** |
|---|---|---|
| E2032I mutant | ILWHEMWHEGLIEASRLYFGERNVKGMFEVLEPLHAMMERGPQTLKETSFNQAYG RDLMEAQEWCRKYMKSGNVKDLTQAWDLYYHVFRRISKTS | 117 |
| E2032L mutant | ILWHEMWHEGLLEASRLYFGERNVKGMFEVLEPLHAMMERGPQTLKETSFNQAYG RDLMEAQEWCRKYMKSGNVKDLTQAWDLYYHVFRRISKTS | 118 |
| T2098L mutant | ILWHEMWHEGLEEASRLYFGERNVKGMFEVLEPLHAMMERGPQTLKETSFNQAYG RDLMEAQEWCRKYMKSGNVKDLLQAWDLYYHVFRRISKTS | 119 |
| E2032, T2098 mutant | ILWHEMWHEGLXEASRLYFGERNVKGMFEVLEPLHAMMERGPQTLKETSFNQAYG RDLMEAQEWCRKYMKSGNVKDLXQAWDLYYHVFRRISKTS | 120 |
| E2032I, T2098L mutant | ILWHEMWHEGLIEASRLYFGERNVKGMFEVLEPLHAMMERGPQTLKETSFNQAYG RDLMEAQEWCRKYMKSGNVKDLLQAWDLYYHVFRRISKTS | 121 |
| E2032L, T2098L mutant | ILWHEMWHEGLLEASRLYFGERNVKGMFEVLEPLHAMMERGPQTLKETSFNQAYG RDLMEAQEWCRKYMKSGNVKDLLQAWDLYYHVFRRISKTS | 122 |

### RNA Transfection

Disclosed herein are methods for producing an in vitro transcribed RNA CAR. RNA CAR and methods of using the same are described, e.g., in paragraphs 553-570 of in International Application WO2015/142675, filed March 13, 2015, which is herein incorporated by reference in its entirety.

An immune effector cell can include a CAR encoded by a messenger RNA (mRNA). In one aspect, the mRNA encoding a CAR described herein is introduced into an immune effector cell, e.g., made by a method described herein, for production of a CAR-expressing cell.

In one embodiment, the *in vitro* transcribed RNA CAR can be introduced to a cell as a form of transient transfection. The RNA is produced by *in vitro* transcription using a polymerase chain reaction (PCR)-generated template. DNA of interest from any source can be directly converted by PCR into a template for in vitro mRNA synthesis using appropriate primers and RNA polymerase. The source of the DNA can be, for example, genomic DNA, plasmid DNA, phage DNA, cDNA, synthetic DNA sequence or any other appropriate source of DNA. The desired temple for *in vitro* transcription is a CAR described herein. For example, the template for the RNA CAR comprises an extracellular region comprising a single chain variable domain of an antibody to a tumor associated antigen described herein; a hinge region (e.g., a hinge region described herein), a transmembrane domain (e.g., a transmembrane domain described herein such as a transmembrane domain of CD8a); and a cytoplasmic region that includes an intracellular signaling domain, e.g., an intracellular signaling domain described herein, e.g., comprising the signaling domain of CD3-zeta and the signaling domain of 4-1BB.

In one embodiment, the DNA to be used for PCR contains an open reading frame. The DNA can be from a naturally occurring DNA sequence from the genome of an organism. In one embodiment, the nucleic acid can include some or all of the 5' and/or 3' untranslated regions (UTRs). The nucleic acid can include exons and introns. In one embodiment, the DNA to be used for PCR is a human nucleic acid sequence. In another embodiment, the DNA to be used for PCR is a human nucleic acid sequence including the 5' and 3' UTRs. The DNA can alternatively be an artificial DNA sequence that is not normally expressed in a naturally occurring organism. An exemplary artificial DNA sequence is one that contains portions of genes that are ligated together to form an open reading frame that encodes a fusion protein. The portions of DNA that are ligated together can be from a single organism or from more than one organism.

PCR is used to generate a template for in vitro transcription of mRNA which is used for transfection. Methods for performing PCR are well known in the art. Primers for use in PCR are designed to have regions that are substantially complementary to regions of the DNA to be used as a template for the PCR. "Substantially complementary," as used herein, refers to sequences of nucleotides where a majority or all of the bases in the primer sequence are complementary, or one or more bases are non-complementary, or mismatched. Substantially complementary sequences are able to anneal or hybridize with the intended DNA target under annealing conditions used for PCR. The primers can be designed to be substantially complementary to any portion of the DNA template. For example, the primers can be designed to amplify the portion of a nucleic acid that is normally transcribed in cells (the open reading frame), including 5' and 3' UTRs. The primers can also be designed to amplify a portion of a nucleic acid that encodes a particular domain of interest. In one embodiment, the primers are designed to amplify the coding region of a human cDNA, including all or portions of the 5' and 3' UTRs. Primers useful for PCR can be generated by synthetic methods that are well known in the art. "Forward primers" are primers that contain a region of nucleotides that are substantially complementary to nucleotides on the DNA template that are upstream of the DNA sequence that is to be amplified. "Upstream" is used herein to refer to a location 5, to the DNA sequence to be amplified relative to the coding strand. "Reverse primers" are primers that contain a region of nucleotides that are substantially complementary to a double-stranded DNA template that are downstream of the DNA sequence that is to be amplified. "Downstream" is used herein to refer to a location 3' to the DNA sequence to be amplified relative to the coding strand.

Any DNA polymerase useful for PCR can be used in the methods disclosed herein. The reagents and polymerase are commercially available from a number of sources.

Chemical structures with the ability to promote stability and/or translation efficiency may also be used. The RNA in embodiments has 5' and 3' UTRs. In one embodiment, the 5' UTR is between one and 3000 nucleotides in length. The length of 5' and 3' UTR sequences to be added to the coding region can be altered by different methods, including, but not limited to, designing primers for PCR that anneal to different regions of the UTRs. Using this approach, one of ordinary skill in the art can modify the 5' and 3' UTR lengths required to achieve optimal translation efficiency following transfection of the transcribed RNA.

The 5' and 3' UTRs can be the naturally occurring, endogenous 5' and 3' UTRs for the nucleic acid of interest. Alternatively, UTR sequences that are not endogenous to the nucleic acid of interest can be added by incorporating the UTR sequences into the forward and reverse primers or by any other modifications of the template. The use of UTR sequences that are not endogenous to the nucleic acid of interest can be useful for modifying the stability and/or translation efficiency of the RNA. For example, it is known that AU-rich elements in 3' UTR sequences can decrease the stability of mRNA. Therefore, 3' UTRs can be selected or designed to increase the stability of the transcribed RNA based on properties of UTRs that are well known in the art.

In one embodiment, the 5' UTR can contain the Kozak sequence of the endogenous nucleic acid. Alternatively, when a 5' UTR that is not endogenous to the nucleic acid of interest is being added by PCR as described above, a consensus Kozak sequence can be redesigned by adding the 5' UTR sequence. Kozak sequences can increase the efficiency of translation of some RNA transcripts, but does not appear to be required for all RNAs to enable efficient translation. The requirement for Kozak sequences for many mRNAs is known in the art. In other embodiments the 5' UTR can be 5'UTR of an RNA virus whose RNA genome is stable in cells. In other embodiments various nucleotide analogues can be used in the 3' or 5' UTR to impede exonuclease degradation of the mRNA.

To enable synthesis of RNA from a DNA template without the need for gene cloning, a promoter of transcription should be attached to the DNA template upstream of the sequence to be transcribed. When a sequence that functions as a promoter for an RNA polymerase is added to the 5' end of the forward primer, the RNA polymerase promoter becomes incorporated into the PCR product upstream of the open reading frame that is to be transcribed. In one embodiment, the promoter is a T7 polymerase promoter, as described elsewhere herein. Other useful promoters include, but are not limited to, T3 and SP6 RNA polymerase promoters. Consensus nucleotide sequences for T7, T3 and SP6 promoters are known in the art.

In an embodiment, the mRNA has both a cap on the 5' end and a 3' poly(A) tail which determine ribosome binding, initiation of translation and stability mRNA in the cell. On a circular DNA template, for instance, plasmid DNA, RNA polymerase produces a long concatameric product which is not suitable for expression in eukaryotic cells. The transcription of plasmid DNA linearized at the end of the 3' UTR results in normal sized mRNA which is not effective in eukaryotic transfection even if it is polyadenylated after transcription.

On a linear DNA template, phage T7 RNA polymerase can extend the 3' end of the transcript beyond the last base of the template (Schenbom and Mierendorf, Nuc Acids Res., 13:6223-36 (1985); Nacheva and Berzal-Herranz, Eur. J. Biochem., 270:1485-65 (2003).

The conventional method of integration of polyA/T stretches into a DNA template is molecular cloning. However polyA/T sequence integrated into plasmid DNA can cause plasmid instability, which is why plasmid DNA templates obtained from bacterial cells are often highly contaminated with deletions and other aberrations. This makes cloning procedures not only laborious and time consuming but often not reliable. That is why a method which allows construction of DNA templates with polyA/T 3' stretch without cloning highly desirable.

The polyA/T segment of the transcriptional DNA template can be produced during PCR by using a reverse primer containing a polyT tail, such as 100T tail (SEQ ID NO: 123) (size can be 50-5000 T (SEQ ID NO: 32)), or after PCR by any other method, including, but not limited to, DNA ligation or in vitro recombination. Poly(A) tails also provide stability to RNAs and reduce their degradation. Generally, the length of a poly(A) tail positively correlates with the stability of the transcribed RNA. In one embodiment, the poly(A) tail is between 100 and 5000 adenosines (e.g., SEQ ID NO: 33).

Poly(A) tails of RNAs can be further extended following in vitro transcription with the use of a poly(A) polymerase, such as E. coli polyA polymerase (E-PAP). In one embodiment, increasing the length of a poly(A) tail from 100 nucleotides to between 300 and 400 nucleotides (SEQ ID NO: 34) results in about a two-fold increase in the translation efficiency of the RNA. Additionally, the attachment of different chemical groups to the 3' end can increase mRNA stability. Such attachment can contain modified/artificial nucleotides, aptamers and other compounds. For example, ATP analogs can be incorporated into the poly(A) tail using poly(A) polymerase. ATP analogs can further increase the stability of the RNA.

5' caps on also provide stability to RNA molecules. In an embodiment, RNAs produced by the methods disclosed herein include a 5' cap. The 5' cap is provided using techniques known in the art and described herein (Cougot, et al., Trends in Biochem. Sci., 29:436-444 (2001); Stepinski, et al., RNA, 7:1468-95 (2001); Elango, et al., Biochim. Biophys. Res. Commun., 330:958-966 (2005)).

The RNAs produced by the methods disclosed herein can also contain an internal ribosome entry site (IRES) sequence. The IRES sequence may be any viral, chromosomal or artificially designed sequence which initiates cap-independent ribosome binding to mRNA and facilitates the initiation of translation. Any solutes suitable for cell electroporation, which can contain factors facilitating cellular permeability and viability such as sugars, peptides, lipids, proteins, antioxidants, and surfactants can be included.

RNA can be introduced into target cells using any of a number of different methods, for instance, commercially available methods which include, but are not limited to, electroporation (Amaxa Nucleofector-II (Amaxa Biosystems, Cologne, Germany)), (ECM 830 (BTX) (Harvard Instruments, Boston, Mass.) or the Gene Pulser II (BioRad, Denver, Colo.), Multiporator (Eppendort, Hamburg Germany), cationic liposome mediated transfection using lipofection, polymer encapsulation, peptide mediated transfection, or biolistic particle delivery systems such as "gene guns" (see, for example, Nishikawa, et al. Hum Gene Ther., 12(8):861-70 (2001).

### Non-viral delivery methods

In some aspects, non-viral methods can be used to deliver a nucleic acid encoding a CAR described herein into a cell or tissue or a subject.

In some embodiments, the non-viral method includes the use of a transposon (also called a transposable element). In some embodiments, a transposon is a piece of DNA that can insert itself at a location in a genome, for example, a piece of DNA that is capable of self-replicating and inserting its copy into a genome, or a piece of DNA that can be spliced out of a longer nucleic acid and inserted into another place in a genome. For example, a transposon comprises a DNA sequence made up of inverted repeats flanking genes for transposition.

Exemplary methods of nucleic acid delivery using a transposon include a Sleeping Beauty transposon system (SBTS) and a piggyBac (PB) transposon system. See, e.g., Aronovich et al. Hum. Mol. Genet. 20.R1(2011):R14-20; Singh et al. Cancer Res. 15(2008):2961-2971; Huang et al. Mol. Ther. 16(2008):580-589; Grabundzija et al. Mol. Ther. 18(2010):1200-1209; Kebriaei et al. Blood. 122.21(2013):166; Williams. Molecular Therapy 16.9(2008):1515-16; Bell et al. Nat. Protoc. 2.12(2007):3153-65; and Ding et al. Cell. 122.3(2005):473-83, all of which are incorporated herein by reference.

The SBTS includes two components: 1) a transposon containing a transgene and 2) a source of transposase enzyme. The transposase can transpose the transposon from a carrier plasmid (or other donor DNA) to a target DNA, such as a host cell chromosome/genome. For example, the transposase binds to the carrier plasmid/donor DNA, cuts the transposon (including transgene(s)) out of the plasmid, and inserts it into the genome of the host cell. See, e.g., Aronovich et al. *supra.*

Exemplary transposons include a pT2-based transposon. See, e.g., Grabundzija et al. Nucleic Acids Res. 41.3(2013):1829-47; and Singh et al. Cancer Res. 68.8(2008): 2961-2971, all of which are incorporated herein by reference. Exemplary transposases include a Tc1/mariner-type transposase, e.g., the SB10 transposase or the SB11 transposase (a hyperactive transposase which can be expressed, e.g., from a cytomegalovirus promoter). See, e.g., Aronovich et al.; Kebriaei et al.; and Grabundzija et al., all of which are incorporated herein by reference.

Use of the SBTS permits efficient integration and expression of a transgene, e.g., a nucleic acid encoding a CAR described herein. Provided herein are methods of generating a cell, e.g., T cell or NK cell, that stably expresses a CAR described herein, e.g., using a transposon system such as SBTS.

In accordance with methods described herein, in some embodiments, one or more nucleic acids, e.g., plasmids, containing the SBTS components are delivered to a cell (e.g., T or NK cell). For example, the nucleic acid(s) are delivered by standard methods of nucleic acid (e.g., plasmid DNA) delivery, e.g., methods described herein, e.g., electroporation, transfection, or lipofection. In some embodiments, the nucleic acid contains a transposon comprising a transgene, e.g., a nucleic acid encoding a CAR described herein. In some embodiments, the nucleic acid contains a transposon comprising a transgene (e.g., a nucleic acid encoding a CAR described herein) as well as a nucleic acid sequence encoding a transposase enzyme. In other embodiments, a system with two nucleic acids is provided, e.g., a dual-plasmid system, e.g., where a first plasmid contains a transposon comprising a transgene, and a second plasmid contains a nucleic acid sequence encoding a transposase enzyme. For example, the first and the second nucleic acids are co-delivered into a host cell.

In some embodiments, cells, e.g., T or NK cells, are generated that express a CAR described herein by using a combination of gene insertion using the SBTS and genetic editing using a nuclease (e.g., Zinc finger nucleases (ZFNs), Transcription Activator-Like Effector Nucleases (TALENs), the CRISPR/Cas system, or engineered meganuclease re-engineered homing endonucleases).

In some embodiments, use of a non-viral method of delivery permits reprogramming of cells, e.g., T or NK cells, and direct infusion of the cells into a subject. Advantages of non-viral vectors include but are not limited to the ease and relatively low cost of producing sufficient amounts required to meet a patient population, stability during storage, and lack of immunogenicity.

### Methods of Manufacture/Production

In some embodiments, the methods disclosed herein further include administering a T cell depleting agent after treatment with the cell (e.g., an immune effector cell as described herein), thereby reducing (e.g., depleting) the CAR-expressing cells (e.g., the CD19CAR-expressing cells). Such T cell depleting agents can be used to effectively deplete CAR-expressing cells (e.g., CD19CAR-expressing cells) to mitigate toxicity. In some embodiments, the CAR-expressing cells were manufactured according to a method herein, e.g., assayed (e.g., before or after transfection or transduction) according to a method herein.

In some embodiments, the T cell depleting agent is administered one, two, three, four, or five weeks after administration of the cell, e.g., the population of immune effector cells, described herein.

In one embodiment, the T cell depleting agent is an agent that depletes CAR-expressing cells, e.g., by inducing antibody dependent cell-mediated cytotoxicity (ADCC) and/or complement-induced cell death. For example, CAR-expressing cells described herein may also express an antigen (e.g., a target antigen) that is recognized by molecules capable of inducing cell death, e.g., ADCC or complement-induced cell death. For example, CAR expressing cells described herein may also express a target protein (e.g., a receptor) capable of being targeted by an antibody or antibody fragment. Examples of such target proteins include, but are not limited to, EpCAM, VEGFR, integrins (e.g., integrins ανβ3,α4, αI3/4β3, α4β7, α5β1, ανβ3,αν), members of the TNF receptor superfamily (e.g., TRAIL-R1 , TRAIL-R2), PDGF Receptor, interferon receptor, folate receptor, GPNMB, ICAM-1, HLA-DR, CEA, CA-125, MUC1, TAG-72, IL-6 receptor, 5T4, GD2, GD3, CD2, CD3, CD4, CD5, CD11 , CD11a/LFA-1, CD15, CD18/ITGB2, CD19, CD20, CD22, CD23/lgE Receptor, CD25, CD28, CD30, CD33, CD38, CD40, CD41 , CD44, CD51 , CD52, CD62L, CD74, CD80, CD125, CD147/basigin, CD152/CTLA-4, CD154/CD40L, CD195/CCR5, CD319/SLAMF7, and EGFR, and truncated versions thereof (e.g., versions preserving one or more extracellular epitopes but lacking one or more regions within the cytoplasmic domain).

In some embodiments, the CAR expressing cell co-expresses the CAR and the target protein, e.g., naturally expresses the target protein or is engineered to express the target protein. For example, the cell, e.g., the population of immune effector cells, can include a nucleic acid (e.g., vector) comprising the CAR nucleic acid (e.g., a CAR nucleic acid as described herein) and a nucleic acid encoding the target protein.

In one embodiment, the T cell depleting agent is a CD52 inhibitor, e.g., an anti-CD52 antibody molecule, e.g., alemtuzumab.

In other embodiments, the cell, e.g., the population of immune effector cells, expresses a CAR molecule as described herein (e.g., CD19CAR) and the target protein recognized by the T cell depleting agent. In one embodiment, the target protein is CD20. In embodiments where the target protein is CD20, the T cell depleting agent is an anti-CD20 antibody, e.g., rituximab.

In further embodiments of any of the aforesaid methods, the methods further include transplanting a cell, e.g., a hematopoietic stem cell, or a bone marrow, into the mammal.

In another aspect, the invention features a method of conditioning a mammal prior to cell transplantation. The method includes administering to the mammal an effective amount of the cell comprising a CAR nucleic acid or polypeptide, e.g., a CD19 CAR nucleic acid or polypeptide. In some embodiments, the cell transplantation is a stem cell transplantation, e.g., a hematopoietic stem cell transplantation, or a bone marrow transplantation. In other embodiments, conditioning a subject prior to cell transplantation includes reducing the number of target-expressing cells in a subject, e.g., CD19-expressing normal cells or CD19-expressing cancer cells.

### Activation and Expansion of Immune Effector Cells (e.g., T cells)

Immune effector cells such as T cells generated or enriched by the methods described herein may be activated and expanded generally using methods as described, for example, in U.S. Patents 6,352,694; 6,534,055; 6,905,680; 6,692,964; 5,858,358; 6,887,466; 6,905,681; 7,144,575; 7,067,318; 7,172,869; 7,232,566; 7,175,843; 5,883,223; 6,905,874; 6,797,514; 6,867,041; and U.S. Patent Application Publication No. 20060121005.

Generally, a population of immune effector cells, e.g., T regulatory cell depleted cells, may be expanded by contact with a surface having attached thereto an agent that stimulates a CD3/TCR complex associated signal and a ligand that stimulates a costimulatory molecule on the surface of the T cells. In particular, T cell populations may be stimulated as described herein, such as by contact with an anti-CD3 antibody, or antigen-binding fragment thereof, or an anti-CD2 antibody immobilized on a surface, or by contact with a protein kinase C activator (e.g., bryostatin) in conjunction with a calcium ionophore. For co-stimulation of an accessory molecule on the surface of the T cells, a ligand that binds the accessory molecule is used. For example, a population of T cells can be contacted with an anti-CD3 antibody and an anti-CD28 antibody, under conditions appropriate for stimulating proliferation of the T cells. To stimulate proliferation of either CD4+ T cells or CD8+ T cells, an anti-CD3 antibody and an anti-CD28 antibody can be used. Examples of an anti-CD28 antibody include 9.3, B-T3, XR-CD28 (Diaclone, Besançon, France) can be used as can other methods commonly known in the art (Berg et al., Transplant Proc. 30(8):3975-3977, 1998; Haanen et al., J. Exp. Med. 190(9):13191328, 1999; Garland et al., J. Immunol Meth. 227(1-2):53-63, 1999).

In certain aspects, the primary stimulatory signal and the costimulatory signal for the T cell may be provided by different protocols. For example, the agents providing each signal may be in solution or coupled to a surface. When coupled to a surface, the agents may be coupled to the same surface (i.e., in "cis" formation) or to separate surfaces (i.e., in "trans" formation). Alternatively, one agent may be coupled to a surface and the other agent in solution. In one aspect, the agent providing the costimulatory signal is bound to a cell surface and the agent providing the primary activation signal is in solution or coupled to a surface. In certain aspects, both agents can be in solution. In one aspect, the agents may be in soluble form, and then cross-linked to a surface, such as a cell expressing Fc receptors or an antibody or other binding agent which will bind to the agents. In this regard, see for example, U.S. Patent Application Publication Nos. 20040101519 and 20060034810 for artificial antigen presenting cells (aAPCs) that are contemplated for use in activating and expanding T cells in the present invention.

In one aspect, the two agents are immobilized on beads, either on the same bead, i.e., "cis," or to separate beads, i.e., "trans." By way of example, the agent providing the primary activation signal is an anti-CD3 antibody or an antigen-binding fragment thereof and the agent providing the costimulatory signal is an anti-CD28 antibody or antigen-binding fragment thereof; and both agents are co-immobilized to the same bead in equivalent molecular amounts. In one aspect, a 1:1 ratio of each antibody bound to the beads for CD4+ T cell expansion and T cell growth is used. In certain aspects of the present invention, a ratio of anti CD3:CD28 antibodies bound to the beads is used such that an increase in T cell expansion is observed as compared to the expansion observed using a ratio of 1:1. In one particular aspect an increase of from about 1 to about 3 fold is observed as compared to the expansion observed using a ratio of 1:1. In one aspect, the ratio of CD3:CD28 antibody bound to the beads ranges from 100:1 to 1:100 and all integer values there between. In one aspect, more anti-CD28 antibody is bound to the particles than anti-CD3 antibody, i.e., the ratio of CD3:CD28 is less than one. In certain aspects, the ratio of anti CD28 antibody to anti CD3 antibody bound to the beads is greater than 2:1. In one particular aspect, a 1:100 CD3:CD28 ratio of antibody bound to beads is used. In one aspect, a 1:75 CD3:CD28 ratio of antibody bound to beads is used. In a further aspect, a 1:50 CD3:CD28 ratio of antibody bound to beads is used. In one aspect, a 1:30 CD3:CD28 ratio of antibody bound to beads is used. In one aspect, a 1:10 CD3:CD28 ratio of antibody bound to beads is used. In one aspect, a 1:3 CD3:CD28 ratio of antibody bound to the beads is used. In yet one aspect, a 3:1 CD3:CD28 ratio of antibody bound to the beads is used.

Ratios of particles to cells from 1:500 to 500:1 and any integer values in between may be used to stimulate T cells or other target cells. As those of ordinary skill in the art can readily appreciate, the ratio of particles to cells may depend on particle size relative to the target cell. For example, small sized beads could only bind a few cells, while larger beads could bind many. In certain aspects the ratio of cells to particles ranges from 1:100 to 100:1 and any integer values in-between and in further aspects the ratio comprises 1:9 to 9:1 and any integer values in between, can also be used to stimulate T cells. The ratio of anti-CD3- and anti-CD28-coupled particles to T cells that result in T cell stimulation can vary as noted above, however certain suitable values include 1:100, 1:50, 1:40, 1:30, 1:20, 1:10, 1:9, 1:8, 1:7, 1:6, 1:5, 1:4, 1:3, 1:2, 1:1, 2:1, 3:1, 4:1, 5:1, 6:1, 7:1, 8:1, 9:1, 10:1, and 15:1 with one suitable ratio being at least 1:1 particles per T cell. In one aspect, a ratio of particles to cells of 1: 1 or less is used. In one particular aspect, a suitable particle: cell ratio is 1:5. In further aspects, the ratio of particles to cells can be varied depending on the day of stimulation. For example, in one aspect, the ratio of particles to cells is from 1:1 to 10:1 on the first day and additional particles are added to the cells every day or every other day thereafter for up to 10 days, at final ratios of from 1:1 to 1:10 (based on cell counts on the day of addition). In one particular aspect, the ratio of particles to cells is 1:1 on the first day of stimulation and adjusted to 1:5 on the third and fifth days of stimulation. In one aspect, particles are added on a daily or every other day basis to a final ratio of 1:1 on the first day, and 1:5 on the third and fifth days of stimulation. In one aspect, the ratio of particles to cells is 2:1 on the first day of stimulation and adjusted to 1:10 on the third and fifth days of stimulation. In one aspect, particles are added on a daily or every other day basis to a final ratio of 1:1 on the first day, and 1:10 on the third and fifth days of stimulation. One of skill in the art will appreciate that a variety of other ratios may be suitable for use in the present invention. In particular, ratios will vary depending on particle size and on cell size and type. In one aspect, the most typical ratios for use are in the neighborhood of 1:1, 2:1 and 3:1 on the first day.

In further aspects, the cells, such as T cells, are combined with agent-coated beads, the beads and the cells are subsequently separated, and then the cells are cultured. In an alternative aspect, prior to culture, the agent-coated beads and cells are not separated but are cultured together. In a further aspect, the beads and cells are first concentrated by application of a force, such as a magnetic force, resulting in increased ligation of cell surface markers, thereby inducing cell stimulation.

By way of example, cell surface proteins may be ligated by allowing paramagnetic beads to which anti-CD3 and anti-CD28 are attached (3x28 beads) to contact the T cells. In one aspect the cells (for example, 10⁴ to 10⁹ T cells) and beads (for example, DYNABEADS^{®} M-450 CD3/CD28 T paramagnetic beads at a ratio of 1:1) are combined in a buffer, for example PBS (without divalent cations such as, calcium and magnesium). Again, those of ordinary skill in the art can readily appreciate any cell concentration may be used. For example, the target cell may be very rare in the sample and comprise only 0.01% of the sample or the entire sample (i.e., 100%) may comprise the target cell of interest. Accordingly, any cell number is within the context of the present invention. In certain aspects, it may be desirable to significantly decrease the volume in which particles and cells are mixed together (i.e., increase the concentration of cells), to ensure maximum contact of cells and particles. For example, in one aspect, a concentration of about 10 billion cells/ml, 9 billion/ml, 8 billion/ml, 7 billion/ml, 6 billion/ml, 5 billion/ml, or 2 billion cells/ml is used. In one aspect, greater than 100 million cells/ml is used. In a further aspect, a concentration of cells of 10, 15, 20, 25, 30, 35, 40, 45, or 50 million cells/ml is used. In yet one aspect, a concentration of cells from 75, 80, 85, 90, 95, or 100 million cells/ml is used. In further aspects, concentrations of 125 or 150 million cells/ml can be used. Using high concentrations can result in increased cell yield, cell activation, and cell expansion. Further, use of high cell concentrations allows more efficient capture of cells that may weakly express target antigens of interest, such as CD28-negative T cells. Such populations of cells may have therapeutic value and would be desirable to obtain in certain aspects. For example, using high concentration of cells allows more efficient selection of CD8+ T cells that normally have weaker CD28 expression.

In one embodiment, cells transduced with a nucleic acid encoding a CAR, e.g., a CAR described herein, e.g., a CD19 CAR described herein, are expanded, e.g., by a method described herein. In one embodiment, the cells are expanded in culture for a period of several hours (e.g., about 2, 3, 4, 5, 6, 7, 8, 9, 10, 15, 18, 21 hours) to about 14 days (e.g., 1, 2, 3, 4, 5, 6, 7, 8, 9, 10, 11, 12, 13 or 14 days). In one embodiment, the cells are expanded for a period of 4 to 9 days. In one embodiment, the cells are expanded for a period of 8 days or less, e.g., 7, 6 or 5 days. In one embodiment, the cells are expanded in culture for 5 days, and the resulting cells are more potent than the same cells expanded in culture for 9 days under the same culture conditions. Potency can be defined, e.g., by various T cell functions, e.g. proliferation, target cell killing, cytokine production, activation, migration, or combinations thereof. In one embodiment, the cells, e.g., a CD19 CAR cell described herein, expanded for 5 days show at least a one, two, three or four fold increase in cells doublings upon antigen stimulation as compared to the same cells expanded in culture for 9 days under the same culture conditions. In one embodiment, the cells, e.g., the cells expressing a CD19 CAR described herein, are expanded in culture for 5 days, and the resulting cells exhibit higher proinflammatory cytokine production, e.g., IFN-γ and/or GM-CSF levels, as compared to the same cells expanded in culture for 9 days under the same culture conditions. In one embodiment, the cells, e.g., a CD19 CAR cell described herein, expanded for 5 days show at least a one, two, three, four, five, ten fold or more increase in pg/ml of proinflammatory cytokine production, e.g., IFN-γ and/or GM-CSF levels, as compared to the same cells expanded in culture for 9 days under the same culture conditions.

Several cycles of stimulation may also be desired such that culture time of T cells can be 60 days or more. Conditions appropriate for T cell culture include an appropriate media (e.g., Minimal Essential Media or RPMI Media 1640 or, X-vivo 15, (Lonza)) that may contain factors necessary for proliferation and viability, including serum (e.g., fetal bovine or human serum), interleukin-2 (IL-2), insulin, IFN-γ, IL-4, IL-7, GM-CSF, IL-10, IL-12, IL-15, TGFβ, and TNF-α or any other additives for the growth of cells known to the skilled artisan. Other additives for the growth of cells include, but are not limited to, surfactant, plasmanate, and reducing agents such as N-acetyl-cysteine and 2-mercaptoethanol. Media can include RPMI 1640, AIM-V, DMEM, MEM, α-MEM, F-12, X-Vivo 15, and X-Vivo 20, Optimizer, with added amino acids, sodium pyruvate, and vitamins, either serum-free or supplemented with an appropriate amount of serum (or plasma) or a defined set of hormones, and/or an amount of cytokine(s) sufficient for the growth and expansion of T cells. Antibiotics, e.g., penicillin and streptomycin, are included only in experimental cultures, not in cultures of cells that are to be infused into a subject. The target cells are maintained under conditions necessary to support growth, for example, an appropriate temperature (e.g., 37° C) and atmosphere (e.g., air plus 5% CO₂).

In one embodiment, the cells are expanded in an appropriate media (e.g., media described herein) that includes one or more interleukin that result in at least a 200-fold (e.g., 200-fold, 250-fold, 300-fold, 350-fold) increase in cells over a 14 day expansion period, e.g., as measured by a method described herein such as flow cytometry. In one embodiment, the cells are expanded in the presence IL-15 and/or IL-7 (e.g., IL-15 and IL-7).

In embodiments, methods described herein, e.g., CAR-expressing cell manufacturing methods, comprise removing T regulatory cells, e.g., CD25+ T cells, from a cell population, e.g., using an anti-CD25 antibody, or fragment thereof, or a CD25-binding ligand, IL-2. Methods of removing T regulatory cells, e.g., CD25+ T cells, from a cell population are described herein. In embodiments, the methods, e.g., manufacturing methods, further comprise contacting a cell population (e.g., a cell population in which T regulatory cells, such as CD25+ T cells, have been depleted; or a cell population that has previously contacted an anti-CD25 antibody, fragment thereof, or CD25-binding ligand) with IL-15 and/or IL-7. For example, the cell population (e.g., that has previously contacted an anti-CD25 antibody, fragment thereof, or CD25-binding ligand) is expanded in the presence of IL-15 and/or IL-7.

In some embodiments a CAR-expressing cell described herein is contacted with a composition comprising a interleukin-15 (IL-15) polypeptide, a interleukin-15 receptor alpha (IL-15Ra) polypeptide, or a combination of both a IL-15 polypeptide and a IL-15Ra polypeptide e.g., hetIL-15, during the manufacturing of the CAR-expressing cell, e.g., ex vivo. In embodiments, a CAR-expressing cell described herein is contacted with a composition comprising a IL-15 polypeptide during the manufacturing of the CAR-expressing cell, e.g., ex vivo. In embodiments, a CAR-expressing cell described herein is contacted with a composition comprising a combination of both a IL-15 polypeptide and a IL-15 Ra polypeptide during the manufacturing of the CAR-expressing cell, e.g., ex vivo. In embodiments, a CAR-expressing cell described herein is contacted with a composition comprising hetIL-15 during the manufacturing of the CAR-expressing cell, e.g., ex vivo.

In one embodiment the CAR-expressing cell described herein is contacted with a composition comprising hetIL-15 during ex vivo expansion. In an embodiment, the CAR-expressing cell described herein is contacted with a composition comprising an IL-15 polypeptide during ex vivo expansion. In an embodiment, the CAR-expressing cell described herein is contacted with a composition comprising both an IL-15 polypeptide and an IL-15Ra polypeptide during ex vivo expansion. In one embodiment the contacting results in the survival and proliferation of a lymphocyte subpopulation, e.g., CD8+ T cells.

T cells that have been exposed to varied stimulation times may exhibit different characteristics. For example, typical blood or apheresed peripheral blood mononuclear cell products have a helper T cell population (TH, CD4+) that is greater than the cytotoxic or suppressor T cell population (TC, CD8+). Ex vivo expansion of T cells by stimulating CD3 and CD28 receptors produces a population of T cells that prior to about days 8-9 consists predominately of TH cells, while after about days 8-9, the population of T cells comprises an increasingly greater population of TC cells. Accordingly, depending on the purpose of treatment, infusing a subject with a T cell population comprising predominately of TH cells may be advantageous. Similarly, if an antigen-specific subset of TC cells has been isolated it may be beneficial to expand this subset to a greater degree.

Further, in addition to CD4 and CD8 markers, other phenotypic markers vary significantly, but in large part, reproducibly during the course of the cell expansion process. Thus, such reproducibility enables the ability to tailor an activated T cell product for specific purposes.

Once a CAR described herein is constructed, various assays can be used to evaluate the activity of the molecule, such as but not limited to, the ability to expand T cells following antigen stimulation, sustain T cell expansion in the absence of re-stimulation, and anti-cancer activities in appropriate in vitro and animal models. Assays to evaluate the effects of a CAR of the present invention are described in further detail below

Western blot analysis of CAR expression in primary T cells can be used to detect the presence of monomers and dimers, e.g., as described in paragraph 695 of International Application WO2015/142675, filed March 13, 2015, which is herein incorporated by reference in its entirety.

*In vitro* expansion of CAR⁺ T cells following antigen stimulation can be measured by flow cytometry. For example, a mixture of CD4⁺ and CD8⁺ T cells are stimulated with αCD3/αCD28 aAPCs followed by transduction with lentiviral vectors expressing GFP under the control of the promoters to be analyzed. Exemplary promoters include the CMV IE gene, EF-1α, ubiquitin C, or phosphoglycerokinase (PGK) promoters. GFP fluorescence is evaluated on day 6 of culture in the CD4⁺ and/or CD8⁺ T cell subsets by flow cytometry. See, *e.g.,* Milone et al., Molecular Therapy 17(8): 1453-1464 (2009). Alternatively, a mixture of CD4⁺ and CD8⁺ T cells are stimulated with αCD3/αCD28 coated magnetic beads on day 0, and transduced with CAR on day 1 using a bicistronic lentiviral vector expressing CAR along with eGFP using a 2A ribosomal skipping sequence. Cultures are re-stimulated with either a cancer associated antigen as described herein⁺ K562 cells (K562-expressing a cancer associated antigen as described herein), wild-type K562 cells (K562 wild type) or K562 cells expressing hCD32 and 4-1BBL in the presence of antiCD3 and anti-CD28 antibody (K562-BBL-3/28) following washing. Exogenous IL-2 is added to the cultures every other day at 100 IU/ml. GFP⁺ T cells are enumerated by flow cytometry using bead-based counting. See, *e.g.,* Milone et al., Molecular Therapy 17(8): 1453-1464 (2009).

Sustained CAR⁺ T cell expansion in the absence of re-stimulation can also be measured. See, *e.g.,* Milone et al., Molecular Therapy 17(8): 1453-1464 (2009). Briefly, mean T cell volume (fl) is measured on day 8 of culture using a Coulter Multisizer III particle counter, a Nexcelom Cellometer Vision or Millipore Scepter, following stimulation with αCD3/αCD28 coated magnetic beads on day 0, and transduction with the indicated CAR on day 1.

Animal models can also be used to measure a CAR-expressing cell activity, e.g., as described in paragraph 698 of International Application WO2015/142675, filed March 13, 2015, which is herein incorporated by reference in its entirety.

Dose dependent CAR treatment response can be evaluated, e.g., as described in paragraph 699 of International Application WO2015/142675, filed March 13, 2015, which is herein incorporated by reference in its entirety.

Assessment of cell proliferation and cytokine production has been previously described, as described in paragraph 700 of International Application WO2015/142675, filed March 13, 2015, which is herein incorporated by reference in its entirety.

Cytotoxicity can be assessed by a standard 51Cr-release assay, e.g., as described in paragraph 701 of International Application WO2015/142675, filed March 13, 2015, which is herein incorporated by reference in its entirety.

Cytotoxicity can also be assessed by measuring changes in adherent cell's electrical impedance, e.g., using an xCELLigence real time cell analyzer (RTCA). In some embodiments, cytotoxicity is measured at multiple time points.

Imaging technologies can be used to evaluate specific trafficking and proliferation of CARs in tumor-bearing animal models, e.g., as described in paragraph 702 of International Application WO2015/142675, filed March 13, 2015, which is herein incorporated by reference in its entirety.

Other assays, including those described in the Example section herein as well as those that are known in the art can also be used to evaluate the CARs described herein.

Alternatively, or in combination to the methods disclosed herein, methods and compositions for one or more of: detection and/or quantification of CAR-expressing cells (e.g., in vitro or in vivo (e.g., clinical monitoring)); immune cell expansion and/or activation; and/or CAR-specific selection, that involve the use of a CAR ligand, are disclosed. In one exemplary embodiment, the CAR ligand is an antibody that binds to the CAR molecule, e.g., binds to the extracellular antigen binding domain of CAR (e.g., an antibody that binds to the antigen binding domain, e.g., an anti-idiotypic antibody; or an antibody that binds to a constant region of the extracellular binding domain). In other embodiments, the CAR ligand is a CAR antigen molecule (e.g., a CAR antigen molecule as described herein).

In one aspect, a method for detecting and/or quantifying CAR-expressing cells is disclosed. For example, the CAR ligand can be used to detect and/or quantify CAR-expressing cells in vitro or in vivo (e.g., clinical monitoring of CAR-expressing cells in a patient, or dosing a patient). The method includes:
providing the CAR ligand (optionally, a labelled CAR ligand, e.g., a CAR ligand that includes a tag, a bead, a radioactive or fluorescent label);
acquiring the CAR-expressing cell (e.g., acquiring a sample containing CAR-expressing cells, such as a manufacturing sample or a clinical sample);
contacting the CAR-expressing cell with the CAR ligand under conditions where binding occurs, thereby detecting the level (e.g., amount) of the CAR-expressing cells present. Binding of the CAR-expressing cell with the CAR ligand can be detected using standard techniques such as FACS, ELISA and the like.

In another aspect, a method of expanding and/or activating cells (e.g., immune effector cells) is disclosed. The method includes:
providing a CAR-expressing cell (e.g., a first CAR-expressing cell or a transiently expressing CAR cell);
contacting said CAR-expressing cell with a CAR ligand, e.g., a CAR ligand as described herein), under conditions where immune cell expansion and/or proliferation occurs, thereby producing the activated and/or expanded cell population.

In certain embodiments, the CAR ligand is present on a substrate (e.g., is immobilized or attached to a substrate, e.g., a non-naturally occurring substrate). In some embodiments, the substrate is a non-cellular substrate. The non-cellular substrate can be a solid support chosen from, e.g., a plate (e.g., a microtiter plate), a membrane (e.g., a nitrocellulose membrane), a matrix, a chip or a bead. In embodiments, the CAR ligand is present in the substrate (e.g., on the substrate surface). The CAR ligand can be immobilized, attached, or associated covalently or non-covalently (e.g., cross-linked) to the substrate. In one embodiment, the CAR ligand is attached (e.g., covalently attached) to a bead. In the aforesaid embodiments, the immune cell population can be expanded in vitro or ex vivo. The method can further include culturing the population of immune cells in the presence of the ligand of the CAR molecule, e.g., using any of the methods described herein.

In other embodiments, the method of expanding and/or activating the cells further comprises addition of a second stimulatory molecule, e.g., CD28. For example, the CAR ligand and the second stimulatory molecule can be immobilized to a substrate, e.g., one or more beads, thereby providing increased cell expansion and/or activation.

In yet another aspect, a method for selecting or enriching for a CAR expressing cell is provided. The method includes contacting the CAR expressing cell with a CAR ligand as described herein; and selecting the cell on the basis of binding of the CAR ligand.

In yet other embodiments, a method for depleting, reducing and/or killing a CAR expressing cell is provided. The method includes contacting the CAR expressing cell with a CAR ligand as described herein; and targeting the cell on the basis of binding of the CAR ligand, thereby reducing the number, and/or killing, the CAR-expressing cell. In one embodiment, the CAR ligand is coupled to a toxic agent (e.g., a toxin or a cell ablative drug). In another embodiment, the anti-idiotypic antibody can cause effector cell activity, e.g., ADCC or ADC activities.

Exemplary anti-CAR antibodies that can be used in the methods disclosed herein are described, e.g., in WO 2014/190273 and by Jena et al., "Chimeric Antigen Receptor (CAR)-Specific Monoclonal Antibody to Detect CD19-Specific T cells in Clinical Trials", PLOS March 2013 8:3 e57838, the contents of which are incorporated by reference.

In some aspects and embodiments, the compositions and methods herein are optimized for a specific subset of T cells, e.g., as described in US Serial No. PCT/US2015/043219 filed July 31, 2015, the contents of which are incorporated herein by reference in their entirety. In some embodiments, the optimized subsets of T cells display an enhanced persistence compared to a control T cell, e.g., a T cell of a different type (e.g., CD8+ or CD4+) expressing the same construct.

In some embodiments, a CD4+ T cell comprises a CAR described herein, which CAR comprises an intracellular signaling domain suitable for (e.g., optimized for, e.g., leading to enhanced persistence in) a CD4+ T cell, e.g., an ICOS domain. In some embodiments, a CD8+ T cell comprises a CAR described herein, which CAR comprises an intracellular signaling domain suitable for (e.g., optimized for, e.g., leading to enhanced persistence of) a CD8+ T cell, e.g., a 4-1BB domain, a CD28 domain, or another costimulatory domain other than an ICOS domain. In some embodiments, the CAR described herein comprises an antigen binding domain described herein, e.g., a CAR comprising an antigen binding domain.

In an aspect, described herein is a method of treating a subject, e.g., a subject having cancer. The method includes administering to said subject, an effective amount of:
1) a CD4+ T cell comprising a CAR (the CARCD4+) comprising:
   an antigen binding domain, e.g., an antigen binding domain described herein;
   a transmembrane domain; and
   an intracellular signaling domain, e.g., a first costimulatory domain, e.g., an ICOS domain; and
2) a CD8+ T cell comprising a CAR (the CARCD8+) comprising:
   an antigen binding domain, e.g., an antigen binding domain described herein;
   a transmembrane domain; and
   an intracellular signaling domain, e.g., a second costimulatory domain, e.g., a 4-1BB domain, a CD28 domain, or another costimulatory domain other than an ICOS domain;
   wherein the CARCD4+ and the CARCD8+ differ from one another.
   Optionally, the method further includes administering:
3) a second CD8+ T cell comprising a CAR (the second CARCD8+) comprising:
   an antigen binding domain, e.g., an antigen binding domain described herein;
   a transmembrane domain; and
   an intracellular signaling domain, wherein the second CARCD8+ comprises an intracellular signaling domain, e.g., a costimulatory signaling domain, not present on the CARCD8+, and, optionally, does not comprise an ICOS signaling domain.

### Biopolymer delivery methods

In some embodiments, one or more CAR-expressing cells as disclosed herein can be administered or delivered to the subject via a biopolymer scaffold, e.g., a biopolymer implant. Biopolymer scaffolds can support or enhance the delivery, expansion, and/or dispersion of the CAR-expressing cells described herein. A biopolymer scaffold comprises a biocompatible (e.g., does not substantially induce an inflammatory or immune response) and/or a biodegradable polymer that can be naturally occurring or synthetic. Exemplary biopolymers are described, e.g., in paragraphs 1004-1006 of International Application WO2015/142675, filed March 13, 2015, which is herein incorporated by reference in its entirety.

### Pharmaceutical compositions and treatments

In some aspects, the disclosure provides a method of treating a patient, comprising administering CAR-expressing cells produced as described herein, optionally in combination with one or more other therapies. In some aspects, the disclosure provides a method of treating a patient, comprising administering a reaction mixture comprising CAR-expressing cells as described herein, optionally in combination with one or more other therapies. In some aspects, the disclosure provides a method of shipping or receiving a reaction mixture comprising CAR-expressing cells as described herein. In some aspects, the disclosure provides a method of treating a patient, comprising receiving a CAR-expressing cell that was produced as described herein, and further comprising administering the CAR-expressing cell to the patient, optionally in combination with one or more other therapies. In some aspects, the disclosure provides a method of treating a patient, comprising producing a CAR-expressing cell as described herein, and further comprising administering the CAR-expressing cell to the patient, optionally in combination with one or more other therapies. The other therapy may be, e.g., a cancer therapy such as chemotherapy.

In an embodiment, cells expressing a CAR described herein are administered to a subject in combination with a molecule that decreases the Treg cell population. Methods that decrease the number of (e.g., deplete) Treg cells are known in the art and include, e.g., CD25 depletion, cyclophosphamide administration, modulating GITR function. Without wishing to be bound by theory, it is believed that reducing the number of Treg cells in a subject prior to apheresis or prior to administration of a CAR-expressing cell described herein reduces the number of unwanted immune cells (e.g., Tregs) in the tumor microenvironment and reduces the subject's risk of relapse.

In one embodiment, a therapy described herein, e.g., a CAR-expressing cell, is administered to a subject in combination with a molecule targeting GITR and/or modulating GITR functions, such as a GITR agonist and/or a GITR antibody that depletes regulatory T cells (Tregs). In embodiments, cells expressing a CAR described herein are administered to a subject in combination with cyclophosphamide. In one embodiment, the GITR binding molecules and/or molecules modulating GITR functions (e.g., GITR agonist and/or Treg depleting GITR antibodies) are administered prior to the CAR-expressing cell. For example, in one embodiment, a GITR agonist can be administered prior to apheresis of the cells. In embodiments, cyclophosphamide is administered to the subject prior to administration (e.g., infusion or re-infusion) of the CAR-expressing cell or prior to apheresis of the cells. In embodiments, cyclophosphamide and an anti-GITR antibody are administered to the subject prior to administration (e.g., infusion or re-infusion) of the CAR-expressing cell or prior to apheresis of the cells. In one embodiment, the subject has cancer (e.g., a solid cancer or a hematological cancer such as ALL or CLL). In one embodiment, the subject has CLL. In embodiments, the subject has ALL. In embodiments, the subject has a solid cancer, e.g., a solid cancer described herein. Exemplary GITR agonists include, e.g., GITR fusion proteins and anti-GITR antibodies (e.g., bivalent anti-GITR antibodies) such as, e.g., a GITR fusion protein described in U.S. Patent No.: 6,111,090, European Patent No.: 090505B1, U.S Patent No.: 8,586,023, PCT Publication Nos.: WO 2010/003118 and 2011/090754, or an anti-GITR antibody described, e.g., in U.S. Patent No.: 7,025,962, European Patent No.: 1947183B1, U.S. Patent No.: 7,812,135, U.S. Patent No.: 8,388,967, U.S. Patent No.: 8,591,886, European Patent No.: EP 1866339, PCT Publication No.: WO 2011/028683, PCT Publication No.: WO 2013/039954, PCT Publication No.: WO2005/007190, PCT Publication No.: WO 2007/133822, PCT Publication No.: WO2005/055808, PCT Publication No.: WO 99/40196, PCT Publication No.: WO 2001/03720, PCT Publication No.: WO99/20758, PCT Publication No.: WO2006/083289, PCT Publication No.: WO 2005/115451, U.S. Patent No.: 7,618,632, and PCT Publication No.: WO 2011/051726.

In one embodiment, a CAR expressing cell described herein is administered to a subject in combination with a GITR agonist, e.g., a GITR agonist described herein. In one embodiment, the GITR agonist is administered prior to the CAR-expressing cell. For example, in one embodiment, the GITR agonist can be administered prior to apheresis of the cells. In one embodiment, the subject has CLL.

The methods described herein can further include formulating a CAR-expressing cell in a pharmaceutical composition. Pharmaceutical compositions may comprise a CAR-expressing cell, e.g., a plurality of CAR-expressing cells, as described herein, in combination with one or more pharmaceutically or physiologically acceptable carriers, diluents or excipients. Such compositions may comprise buffers such as neutral buffered saline, phosphate buffered saline and the like; carbohydrates such as glucose, mannose, sucrose or dextrans, mannitol; proteins; polypeptides or amino acids such as glycine; antioxidants; chelating agents such as EDTA or glutathione; adjuvants (e.g., aluminum hydroxide); and preservatives. Compositions can be formulated, e.g., for intravenous administration.

In one embodiment, the pharmaceutical composition is substantially free of, e.g., there are no detectable levels of a contaminant, e.g., selected from the group consisting of endotoxin, mycoplasma, replication competent lentivirus (RCL), p24, VSV-G nucleic acid, HIV gag, residual anti-CD3/anti-CD28 coated beads, mouse antibodies, pooled human serum, bovine serum albumin, bovine serum, culture media components, vector packaging cell or plasmid components, a bacterium and a fungus. In one embodiment, the bacterium is at least one selected from the group consisting of Alcaligenes faecalis, Candida albicans, Escherichia coli, Haemophilus influenza, Neisseria meningitides, Pseudomonas aeruginosa, Staphylococcus aureus, Streptococcus pneumonia, and Streptococcus pyogenes group A.

When "an immunologically effective amount," "an anti-cancer effective amount," "a cancer-inhibiting effective amount," or "therapeutic amount" is indicated, the precise amount of the compositions to be administered can be determined by a physician with consideration of individual differences in age, weight, tumor size, extent of infection or metastasis, and condition of the patient (subject). It can generally be stated that a pharmaceutical composition comprising the immune effector cells (e.g., T cells, NK cells) described herein may be administered at a dosage of 10⁴ to 10⁹ cells/kg body weight, in some instances 10⁵ to 10⁶ cells/kg body weight, including all integer values within those ranges. T cell compositions may also be administered multiple times at these dosages. The cells can be administered by using infusion techniques that are commonly known in immunotherapy (see, e.g., Rosenberg et al., New Eng. J. of Med. 319:1676, 1988).

In some embodiments, a dose of CAR cells (e.g., CD19 CAR cells) comprises about 1 × 10⁶, 1.1 × 10⁶, 2 × 10⁶, 3.6 × 10⁶, 5 × 10⁶, 1 × 10⁷, 1.8 × 10⁷, 2 × 10⁷, 5 × 10⁷, 1 × 10⁸, 2 × 10⁸, or 5 × 10⁸ cells/kg. In some embodiments, a dose of CAR cells (e.g., CD19 CAR cells) comprises at least about 1 × 10⁶, 1.1 × 10⁶, 2 × 10⁶, 3.6 × 10⁶, 5 × 10⁶, 1 × 10⁷, 1.8 × 10⁷, 2 × 10⁷, 5 × 10⁷, 1 × 10⁸, 2 × 10⁸, or 5 × 10⁸ cells/kg. In some embodiments, a dose of CAR cells (e.g., CD19 CAR cells) comprises up to about 1 × 10⁶, 1.1 × 10⁶, 2 × 10⁶, 3.6 × 10⁶, 5 × 10⁶, 1 × 10⁷, 1.8 × 10⁷, 2 × 10⁷, 5 × 10⁷, 1 × 10⁸, 2 × 10⁸, or 5 × 10⁸ cells/kg. In some embodiments, a dose of CAR cells (e.g., CD19 CAR cells) comprises about 1.1 × 10⁶ - 1.8 × 10⁷ cells/kg. In some embodiments, a dose of CAR cells (e.g., CD19 CAR cells) comprises about 1 × 10⁷, 2 × 10⁷, 5 × 10⁷, 1 × 10⁸, 2 × 10⁸, 5 × 10⁸, 1 × 10⁹, 2 × 10⁹, or 5 × 10⁹ cells. In some embodiments, a dose of CAR cells (e.g., CD19 CAR cells) comprises at least about 1 × 10⁷, 2 × 10⁷, 5 × 10⁷, 1 × 10⁸, 2 × 10⁸, 5 × 10⁸, 1 × 10⁹, 2 × 10⁹, or 5 × 10⁹ cells. In some embodiments, a dose of CAR cells (e.g., CD19 CAR cells) comprises up to about 1 × 10⁷, 2 × 10⁷, 5 × 10⁷, 1 × 10⁸, 2 × 10⁸, 5 × 10⁸, 1 × 10⁹, 2 × 10⁹, or 5 × 10⁹ cells.

In certain aspects, it may be desired to administer activated immune effector cells (e.g., T cells, NK cells) to a subject and then subsequently redraw blood (or have an apheresis performed), activate immune effector cells (e.g., T cells, NK cells) therefrom, and reinfuse the patient with these activated and expanded immune effector cells (e.g., T cells, NK cells). This process can be carried out multiple times every few weeks. In certain aspects, immune effector cells (e.g., T cells, NK cells) can be activated from blood draws of from 10cc to 400cc. In certain aspects, immune effector cells (e.g., T cells, NK cells) are activated from blood draws of 20cc, 30cc, 40cc, 50cc, 60cc, 70cc, 80cc, 90cc, or 100cc.

The administration of the subject compositions may be carried out in any convenient manner. The compositions described herein may be administered to a patient trans arterially, subcutaneously, intradermally, intratumorally, intranodally, intramedullary, intramuscularly, by intravenous (i.v.) injection, or intraperitoneally, e.g., by intradermal or subcutaneous injection. The compositions of immune effector cells (e.g., T cells, NK cells) may be injected directly into a tumor, lymph node, or site of infection.

### EXAMPLES

The invention is further described in detail by reference to the following experimental examples. These examples are provided for purposes of illustration only, and are not intended to be limiting unless otherwise specified. Thus, the invention should in no way be construed as being limited to the following examples, but rather, should be construed to encompass any and all variations which become evident as a result of the teaching provided herein.

### Example 1: Identifying attributes of the starting apheresis material that correlate to manufacturing success and product quality

Monocytes have been identified as a cellular subtype that negatively affects CART manufacturing and final product quality. Monocytes can negatively impact T cell activation and transduction by being a source of inhibitory factors, non-specifically binding/engulfing the anti-CD3/CD28 antibody coated beads, and reducing gene transduction efficiency by serving as a sink for the viral gene vector (Powell et al. 2009). The presence of monocytes in the patient leukapheresis material, after thawing, either alone or in the presence of blast cells, reduced the cumulative population doublings (cPDL) from a mean of 5.5 with batches with no significant monocyte presence (<20% of WBC) to a mean cPDL of 2.7 (monocytes >20% of WBC). Any batch with cPDL <4.5 was classified as "suboptimal" due to the inability to achieve predetermined dose (number of transduced T cells), compromised transduction efficiency (<2%), and/or no measurable potency (<5x background). A significant proportion of manufacturing batches (33%) have presented manufacturing challenges.

Several examples are shown below of poor outcomes when thawed leukopak samples containing high amount of monocytes or B cell blasts are subjected to the current standard CART cell manufacturing protocol (Process B). Because the total white blood cell seeding number is fixed, a direct consequence of patient lots with relatively high percentage of monocytes or CD19+ B cell blasts means that a lower number of T cells can get seeded on Day 0.

Additionally, such a high percentage of monocytes or B cell blasts in the sample that is subsequently bead stimulated, lentivirally transduced and expanded over several days in culture, acts to compromise T cell activation and expansion potential. It is likely that as the non-T cells undergo apoptosis and cell death, the cell culture medium is compromised and toxified by cell debris and is less able to support the selective expansion of healthy T cells.

The results shown in Table 5 below demonstrates the problems and issues that arise when thawed apheresis material containing high levels of monocytes and B cells is put into the current standard CAR T cell manufacturing protocol (Process B). For example, leukopak sample Patient F1 contained 47.1% of CD14+ monocytes after thawing. This sample experienced suboptimal cell growth and was out of specification (OOS) at harvest on Day 11.

**Table 5. Examples of patient apheresis materials containing high amount of monocytes or CD19+ B blast cells and the impact on cell expansion and function**

| Selected examples of Patient lots | (Potential lymphoblast) CD45 (Dim-) | Mono CD14+ of CD45+ | B cells CD3-/CD19&20+ of CD45+ | T cells CD3+/CD19&20-of CD45+ | Grans CD15+ of CD45+ | Issues |
|---|---|---|---|---|---|---|
| Patient F1 | 1.9 | 47.1 | 0.8 | 32.3 | 0.1 | Suboptimal cell growth; Day 11 harvest (OOS); low post-thaw recovery |
| Patient G1 | 81.5 | 1.3 | 81.1 | 16.2 | 0.1 | Suboptimal cell growth |
| Patient H1/H2 | 67.3 | 1.2 | 48.6 | 25.6 | 1.4 | Suboptimal cell growth; H1 terminated at PU, H2 harvested on Day 11; failed potency and dose (OOS) |
| Patient J1 | 61.0 | 0.2 | 54.7 | 42.5 | 0.8 | Suboptimal cell growth |
| Patient L1 | N/A | 50.2 | N/A | 32.9 | 3.1 | Suboptimal cell growth |
| Patient N1 | 23.9 | 29.2 | 27.0 | 42.1 | 0.0 | Low D3 cell recovery and suboptimal cell growth |
| Patient P1 | 55.5 | 0.5 | 63.5 | 30.3 | 0.0 | Low D3, cell recovery and suboptimal cell growth; would fail NVS dose requirement Suboptimal cell growth; failed the dose (OOS) |
| Patient S1 | N/A | 56.5 | N/A | 32.3 | 10.6 | |

The leukopak sample Patient P1 started with 55.5% of blast cells (CD19+ leukemic cells) and showed suboptimal cell growth and T cell recovery and would fail current dosing requirements for clinical use.

### Example 2: Comparison of Elutriation Methods

As an example of the modified elutriation program, the elutriation settings listed in **Table 6** below have been demonstrated to be able to separate lymphocytes from monocytes. In brief, frozen apheresis materials were thawed, diluted with PBS, and then connected to the elutriation device where all the required elutriation media has been connected. The system is primed, followed by introducing the cells into the chamber. In the meantime, elutriation media is also introduced into the chamber to provide the counter flow and to achieve the cell type separation. Typically Fraction 3 (F3) or a combination of Fraction 3 and 4 (F3+F4) contains the target lymphocytes population with the minimal amount of monocytes, granulocytes and other non-lymphocyte cells.

**Table 6: Sample Elutriation Settings**

| Fraction | Flow rate (mL/min) | Centrifugation (rpm) | Volume (mL) |
|---|---|---|---|
| F1 | 30.0 | 2400 | 900 |
| F2 | 30.0 | 2400 | 500 |
| F3 | 70.0 | 2400 | 975 |
| F4 | 72.0 | 2400 | 400 |
| F5 | 82.0 | 0 | 250 |

A head-to-head comparison between the current standard Ficoll process (Process B) and the modified elutriation program (Elutriation) for the respective ability to process apheresis materials containing high amounts of monocytes was performed. After thawing, leukopak samples from 5 different normal donors were split in half and one arm was processed using standard methods (Process B) and the other was processed with modified elutriation. The results obtained are shown in **Table 7.**

**Table 7: Selected examples of healthy donor apheresis materials processed by either the Ficoll step in the current Process B, or by modified elutriation program.**

| **Batch number** | **Post-Thaw Process** | **% Monocytes in apheresis** | **% Monocytes post elutriation** | **% T Cells post elutriation** | **CAR %** |
|---|---|---|---|---|---|
| **4841** | Elutriation | 24 | 2 | 91 | ND |
| | No elutriation (Process B) | 24 | 44 | 38 | ND |
| **4883** | Elutriation | 25 | 10 | 70 | 13 |
| | No elutriation (Process B) | 25 | 33 | 30 | 12 |
| **5049** | Elutriation | 26 | 1 | 65 | 21 |
| | No elutriation (Process B) | 26 | 26 | 25 | 26 |
| **5131** | Elutriation | 31 | 1 | 62 | 16 |
| | No elutriation (Process B) | 31 | 50 | 41 | 20 |
| **5252** | Elutriation | 29 | 1 | 85 | 32 |
| | No elutriation (Process B) | 29 | 52 | 13 | 23 |

The results shown in **Table 7** above clearly show the effectiveness of the new Elutra process in removing monocytes from the cell samples at Day 0 before initiating subsequent manufacturing steps. For example, Donor 4841 was found to contain 24% monocytes in the thawed apheresis sample. After half of the sample was processed with the standard (Process B) protocol, Day 0 cells contained 44% monocytes. Such a high amount of non-T cells negatively affected the T cell yields in cell harvested at the end of the manufacturing process (only 38% of harvested cells were T cells). In contrast, the half of the sample that was manufactured using the modified Elutra process, as described in this invention disclosure, showed only 2% monocyte 'contamination' at Day 0, with a greatly improved T cell yield and recovery at the end of manufacturing (91% of harvested cells were T cells). This separation efficiency was also demonstrated in processing diffuse large B cell lymphoma (DLBCL) patient leukapheresis materials, found to contain high percent of monocytes, as seen in **Table 8** below.

**Table 8: Elutriation of thawed, previously cryopreserved, DLBCL patient leukapheresis material**

| **Patient batch number** | **% Monocytes** | | **% T cells** | |
|---|---|---|---|---|
| | **Leukapheresis** | **Post-Elutra collection** | **Leukapheresis** | **Post-Elutra collection** |
| **Patient 51** | 67.9 | 2.0 | 3.0 | 82.0 |
| **Patient A1** | 48.7 | 3.0 | 52.6 | 66.3 |
| **Patient B1** | 38.9 | 1.5 | 46.2 | 67.8 |
| **Patient C1** | 49.0 | 2.0 | 55.8 | 91.9 |

### Example 3: Modified Wash Step Reduces Stabilizes Cells and Reduces Clumping

In the current manufacturing Process B, patient cellular leukapheresis material is thawed on the Plasmatherm (Genesis), washed using the CellSaver 5+ instrument (Haemonetics), and is then resuspended in either a cell expansion medium based on X-VIVO15 medium (Lonza), called 'Modified Medium' (MM), or into a buffered isotonic saline solution such as phosphate-buffered saline (PBS) for the subsequent Ficoll selection of lymphocytes. Modified Medium is prepared according to the components provided in Table 9 below.

**Table 9 Components for Modified Medium**

| **Component** | **Final Concentration** | **Reagent Description** |
|---|---|---|
| X-VIVO 15 | N/A (base medium) | X-VIVO 15 chemically-defined serum-free medium, without gentamicin or phenol red, Lonza Cat# 04-744Q or equivalent |
| HEPES | 20 mM | 1 M HEPES, Lonza, Cat# 17-737E or equivalent |
| GlutaMAX | 2 mM | GlutaMAX media supplement, Life Technologies, Cat#35050-061 or equivalent |
| N-Acetylcysteine (NAC) | 10 mM | N-Acetyl-cysteine, APP Pharmaceuticals, LLC, Cat#963030 or equivalent |
| MEM Vitamin Mix | 1% | MEM Essential Vitamin Mixture, Lonza, Cat # 13-607C or equivalent |
| Sodium Pyruvate | 1 mM | Lonza, Cat # 13-115E or equivalent |
| IL-2 | 100 IU/mL | IL2 (Proleukin), Novartis, NDC 65483-116-07 or equivalent |
| Human Serum | 5% | Human AB+ serum heat-inactivated |

Transfer of thawed cells into either Modified Medium or into PBS solution can cause the cells to clump. To mitigate this, alternative cell resuspension buffers were investigated. An aqueous solution with 5% dextrose and 0.45% sodium chloride (referred to interchangeably herein as Dextrose/NaCl, 'D5' or D5 1/2 NS) was found to stabilize the cell suspension and prevents clumping. Cells suspended in D5 are stable and do not clump when held at room temperature for at least 2 hours. The D5 solution is also compatible with subsequent manufacturing steps, including the positive selection of T cells by stimulation with the anti-CD3/CD28 CTS Dynabeads (Thermo Fisher).

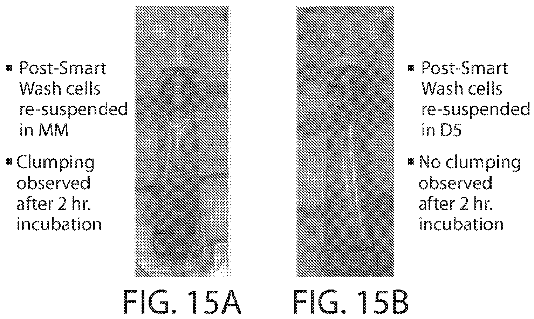

The Sepax 2 RM device (Biosafe) was used to wash thawed leukopak cells to remove subcellular debris, free hemoglobin and cryoprotectants, to achieve volume reduction, and to enable subsequent Ficoll density gradient separation. To reduce monocyte contamination, while also preventing cell clumping, the Sepax 2 SmartWash protocol was performed using D5 medium, instead of Modified Medium. **FIG. 15A and 15B** show photographs documenting extensive clumping of cells after 2 hours in Modified Medium (FIG. 15A), while cells held in D5 solution for the same length of time show little or no clumping (FIG. 15B).

Additional technical details of how to perform the modified process that involves Sepax SmartWash in D5 aqueous solution are provided. The operator opens the Sepax single-use cell processing kit inside of the biosafety cabinet (BSC) and attaches the thawed leukapheresis cell bag and the media bag to the disposable kit. The kit is installed on the Sepax 2 device and the SmartWash procedure, with an output volume of 115mL, is selected. The SmartWash centrifugation procedure pellets the cells which are then resuspended with D5 solution to the final volume of 115mL.

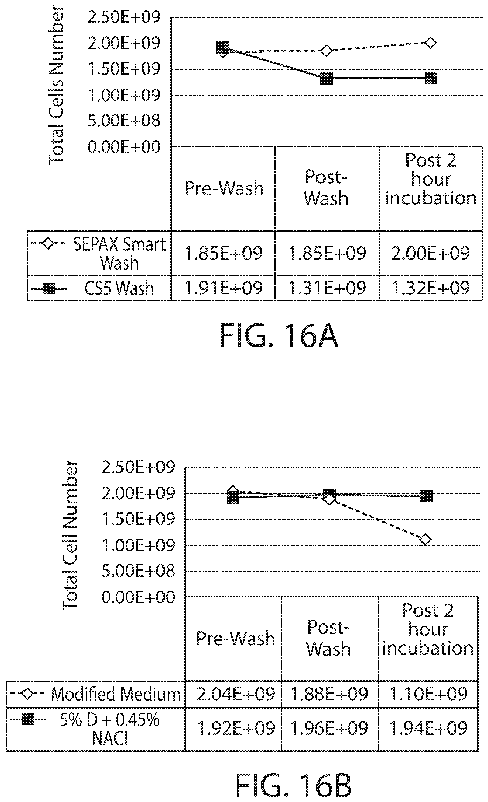

Step by step detail of the modified manufacturing process using D5 solution are given below:
**Step1.** Leukopak is thawed and mixed with aqueous solution of 5% dextrose and 0.45% sodium chloride (7-10 volumes of the solution to one volume of leukopak).
**Step 2.** Cells are allowed to equilibrate for 30 minutes at ambient temperature.
**Step 3.** Cryomedia and debris are removed with Sepax 2 cell wash using the SmartWash program in D5 solution.
**Step 4.** Cells are re-suspended in the aqueous D5 solution.
**Step 5.** Positive selection of T cells is performed in the aqueous D5 solution instead of using the Modified Medium.
**Step 6.** When positive selection in D5 solution is completed, the negative fraction (non-T cells) is removed, and the positive fraction (T cells) is re-suspended in the Modified Medium for the subsequent cell expansion steps.
This revised protocol using D5 aqueous solution to prepare thawed apheresis cells for CAR T cell manufacturing can be performed directly on the Sepax device, and obviates the need to use the CellSaver 5 Plus (CSS+) instrument (Haemonetics) to wash the cells. The results presented in **FIG. 16A** below demonstrate that Sepax SmartWash procedure provides a better cell yield in comparison with CS5+ wash.

As discussed above, the use of the D5 (5% dextrose and 0.45% sodium chloride) aqueous solution acts to prevent undesirable clumping of thawed apheresis cells materials, especially when high percentages of granulocytes (e.g. neutrophils) are present. The results presented in **FIG. 16A** above shows the superiority of the Sepax SmartWash protocol over the CS5+ wash even when both protocols are performed using D5 aqueous solution.

**FIG. 16B** below demonstrates that using D5 aqueous solution prevents cell losses following a CS5+ cell wash and a 2 hour ambient temperature incubation compared to nearly 50% cell losses seen when Modified Medium is used instead of D5.

### Example 4: Comparison of Density Gradient Centrifugation

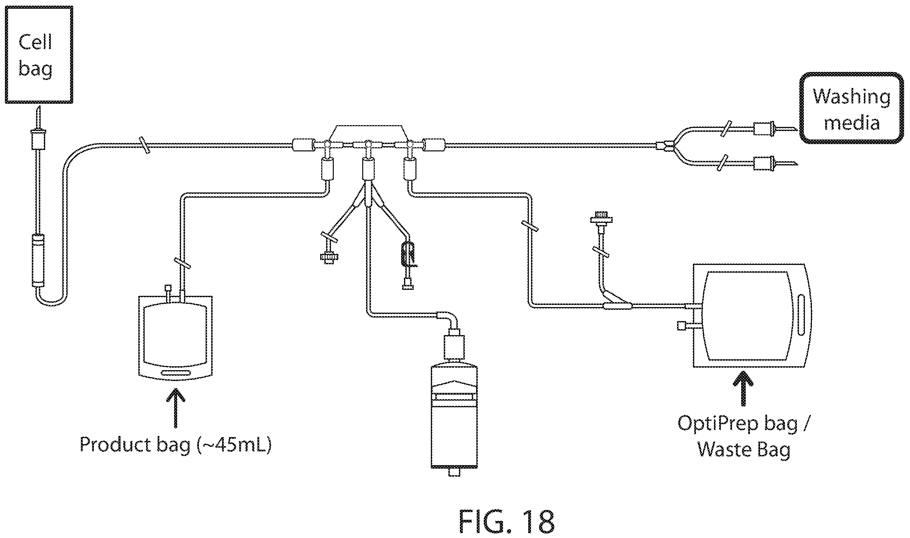

In this example, five head to head comparison runs using cryopreserved then thawed normal donor apheresis materials were compared to determine whether the simplified process involving OptiPrep density gradient medium purification represents a technical advance. Operators performed the Sepax 2 NeatCell protocol and where the protocol called for Ficoll, it was replaced with OptiPrep. **FIG. 18** shows a schematic on how the Sepax 2 NeatCell single use disposable tubing kit (Biosafe CS900.2) is configured with the cell and medium bags.

After Plasmatherm thaw, one half of each normal donor-derived leukopak sample was processed according to the Process B protocol (CS5+ wash, Sepax 2 volume reduction, and Ficoll gradient purification). The other half of each thawed leukopak sample was processed using the Sepax with OptiPrep method we have developed. The cellular materials purified by the 2 different methods were then separately subjected to the same subsequent steps in the CAR T cell manufacturing process (anti-CD3/CD28 bead stimulation, LV transduction, vector washout, and cell expansion). The cell products were harvested at Day9 and subjected to flow cytometry using cell surface marker specific antibodies (anti-CD3, anti-CD19, anti-CD14) to quantify relative cell phenotypes and subset compositions. The attached/inserted Excel file "Sepax with OptiPrep Data Tables" contains the complete data file of these analyses, with overall results summarize in **Table 10** below.

**Table 10. Summary of the results comparing products profiles of thawed normal donor apheresis material prepared using Process B (Ficoll) or by 'Sepax with OptiPrep' method**

| **Results: Mean/Standard Deviation for the five head-to-head runs** | | | | | | |
|---|---|---|---|---|---|---|
| | **Leukopak** | | **Ficoll Product** | | **OptiPrep Product** | |
| | **MEAN** | **SD** | **MEAN** | **SD** | **MEAN** | **SD** |
| Total WBC | 4.43E+09 | 7.69E+08 | 1.01E+09 | 4.49E+08 | 1.26E+09 | 6.11E+08 |
| Abs CD3 | 1.95E+09 | 7.32E+08 | 2.29E+08 | 1.03E+08 | 8.25E+08 | 4.06E+08 |
| Abs CD19 | 7.89E+08 | 3.15E+08 | 2.15E+08 | 1.31E+08 | 8.45E+07 | 4.08E+07 |
| Abs CD14 | 6.39E+08 | 3.58E+08 | 2.78E+08 | 2.44E+08 | 1.20E+08 | 8.64E+07 |
| Abs Other | 8.59E+08 | 2.14E+08 | 2.37E+08 | 1.12E+08 | 2.11E+08 | 1.30E+08 |
| | | | | | | |
| Total WBC Yield | N/A | N/A | 25% | 11% | 28% | 14% |
| CD3+ Yield | N/A | N/A | 15% | 11% | 46% | 31% |
| CD19+ Yield | N/A | N/A | 41% | 36% | 10% | 4% |
| CD14+ Yield | N/A | N/A | 34% | 23% | 16% | 7% |
| Other Yield | N/A | N/A | 27% | 13% | 26% | 14% |
| | | | | | | |
| %CD3 of WBC | 44.04 | 13.02 | 30.85 | 19.46 | 66.02 | 11.13 |
| %CD19 of WBC | 17.27 | 5.57 | 20.55 | 6.15 | 7.08 | 2.14 |
| %CD14 of WBC | 14.27 | 7.61 | 21.64 | 17.07 | 9.24 | 5.74 |
| %Other of WBC | 20.13 | 7.02 | 22.68 | 6.74 | 16.15 | 5.17 |

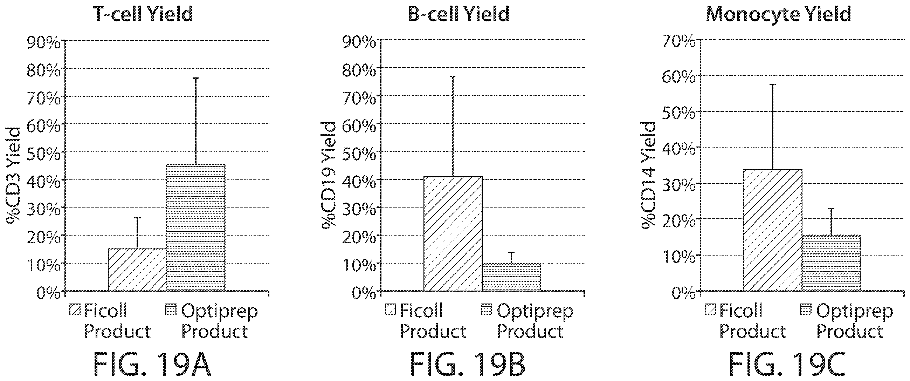

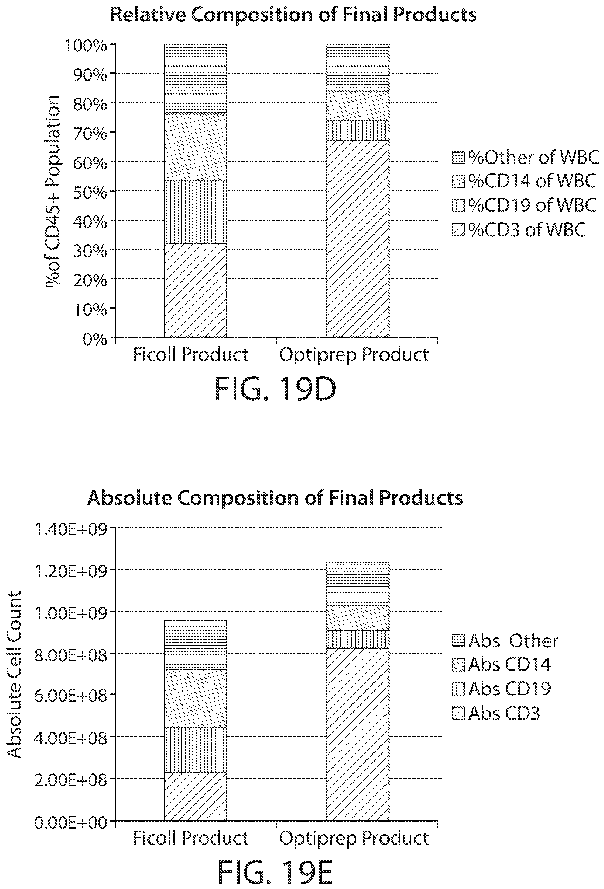

The results of these analyses show that products prepared by the new Sepax with OptiPrep method contain significantly more absolute CD3+ T cells (8.25×10e8 vs. 2.29x10e8), improved yields of CD+ cells (46% *vs.* 15%), fewer CD19+ B cells (10% *vs.* 41%) and fewer CD14+ monocytes (16% *vs.* 34%). In addition, the final product generated by the Sepax with OptiPrep method contains more than double the amount of CD3+ T cells (66.02%) than are present in the product prepared by Process B (30.85%). These data are presented graphically in **FIGs 19A, 19B, 19C****,** **19D**, **and 19E****.**

### Example 5: Comparison of Positive Selection Methods

Three experiments were performed comparing the use of FAST with the current positive selection protocol, Process B. The results of these experiments exhibit reliable T cell enrichment by the optimized FAST protocol, with product T cell purities above 90% independent of input composition. The results also point to very efficient depletion on non-T c ell populations by the FAST approach, including monocytes, B cells and blast (Nalm6) cells.

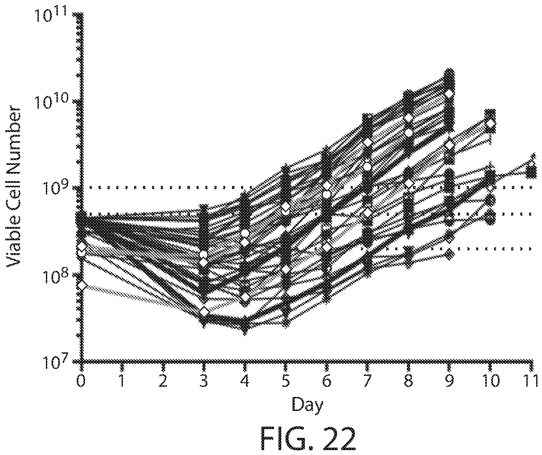

FAST positive selection was implemented for use in patients as a contingency protocol for samples where blast percentage exceeds 20%. To date, three lots were processed using FAST. All three lots successfully expanded to meet dose, with their growth curves being comparable or superior to that of Process B lots. **FIG. 22** provides a graphical overview of the growth curves of clinical FAST lots (white), comparing them to those of Process B (black). Notably, FAST lots provided comparable or superior expansion, with reduced loss of cells between day 0 and day 3, compared to Process B. All clinical FAST lots had > 70% blast in the starting material.

### Experiment 1:

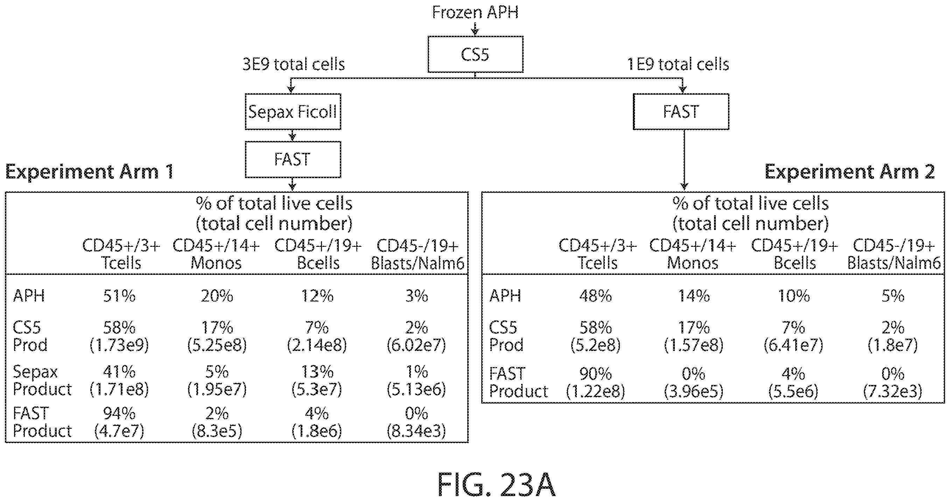

Experiment 1 details: Use of FAST positive selection in lieu of the current positive selection process was first tested in experiment FAST006. This experiment consisted of two experimental arms **(****FIG. 23A****),** which shared a CS5+ wash step. Experiment arm 1 followed Process B using CS5+ wash followed by Sepax 2-Ficoll enrichment, but replaced positive selection with FAST. Experimental arm 2 tested an alternate approach where Sepax 2-Ficoll enrichment was omitted and direct FAST positive selection was performed. Three times as many total cells were allocated for experimental arm 1 (3x10e9 total cells) than to arm 2 (1x10e9 total cells) as shown in the schematic in the top section of **FIG. 23A****.**

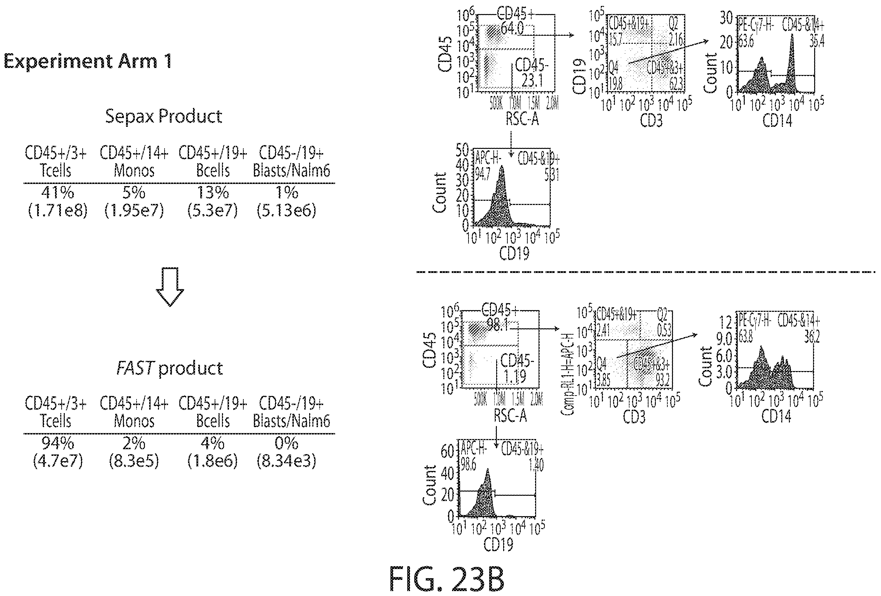

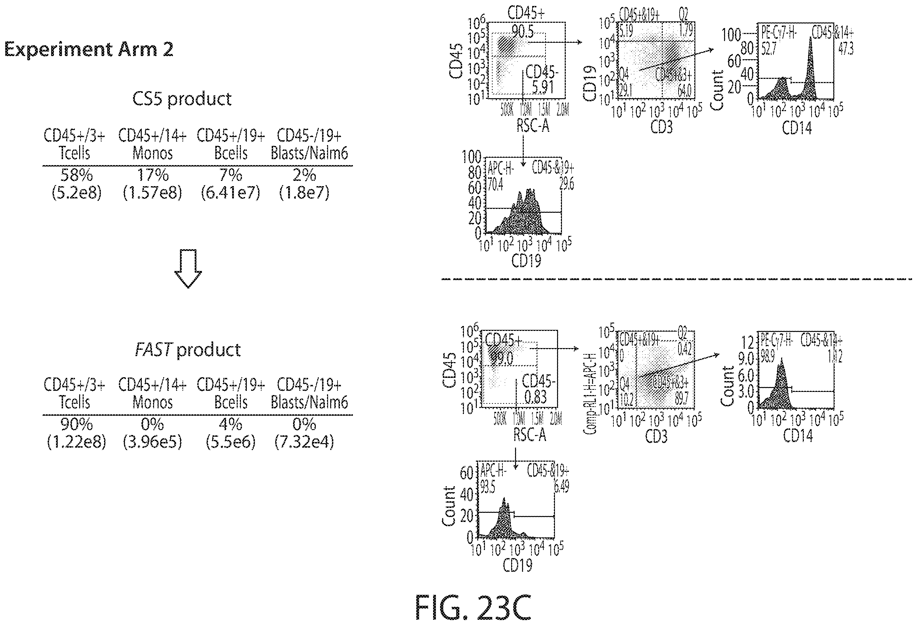

Experiment 1 outcomes: In both experimental arm 1 and 2, optimized FAST positive selection yielded a high final product T cell purity (94% in arm 1, 90% in arm 2), with a high depletion of all non-T cell populations. In experimental arm 1 **(****FIG. 23B****),** the percentage of CD45+/CD3+ T cells recovered after Sepax 2-Ficoll dropped to 41% (from 51% in the original donor material), but T cell purity (94%) was re-established after FAST positive selection. Experimental arm 2 **(****FIG. 23C****),** despite a three-fold lower input cell number, yielded the largest number of T cell in the final product (approximately 3x more T cells), with a final purity of 90%.

Experiment 1 summary: This experiment achieved proof-of-concept results that justify replacing the current mode of positive selection with a modified FAST protocol. Final T cell purity after FAST protocol was 90% or above in both experimental arms. The option of skipping the Sepax 2-Ficoll enrichment step was identified but requires further validation.

### Experiment 2:

Experiment 2 details: In this experiment, use of FAST positive selection in lieu of the current positive selection process was repeated with a donor apheresis that contained a high percentage of monocytes. The experiment consisted of two experimental arms (see schematic at top). Experiment arm 1 followed Process B using CS5+ wash followed by Sepax 2-Ficoll enrichment, but replaced positive selection with FAST. Experimental arm 2 tested an alternate approach where a CS5 wash step was immediate followed by FAST positive selection. One quarter of a frozen leukopak (LKPK) was used for each arm.

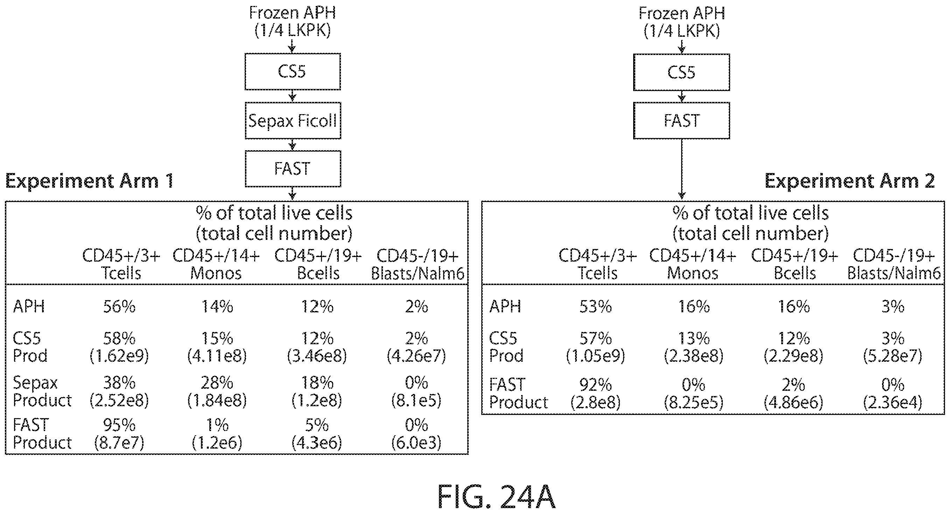

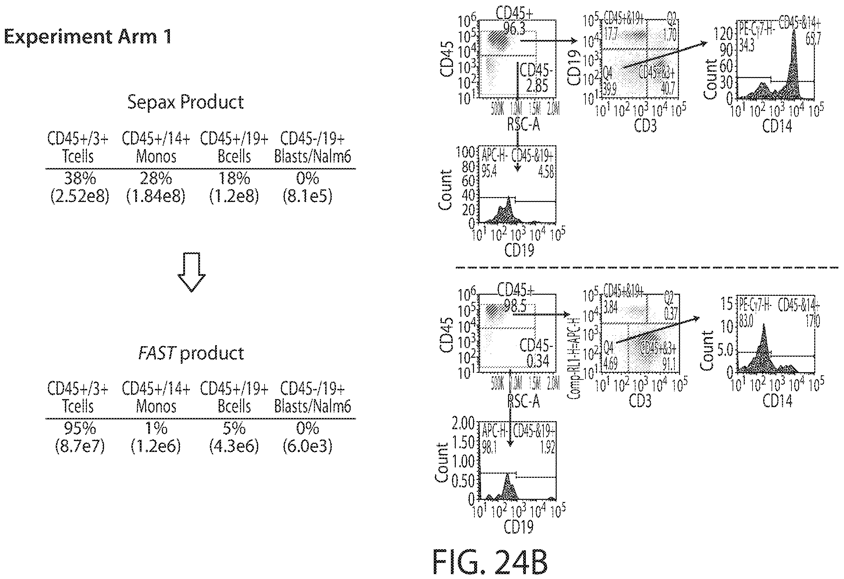

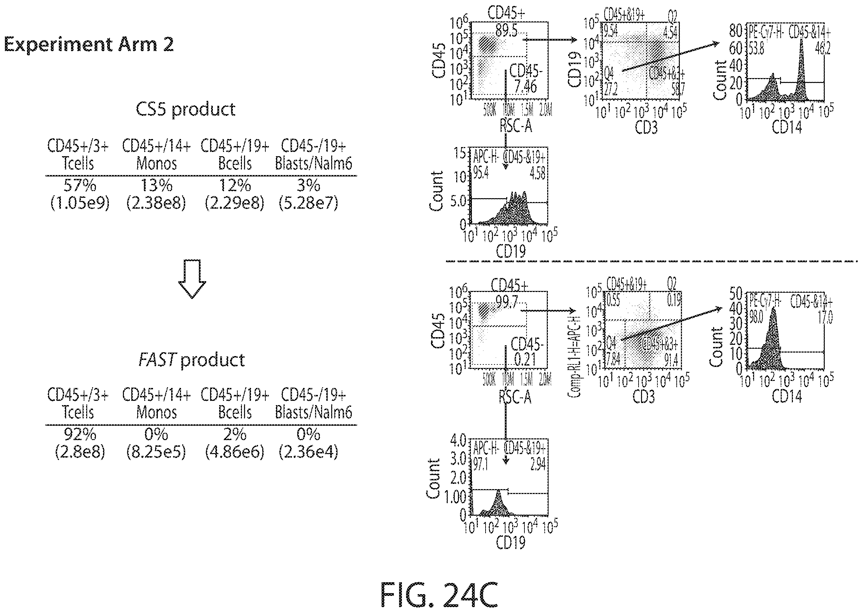

Experiment 2 outcomes: In both experimental arms 1 and 2, optimized FAST positive selection yielded a high final product T cell purity (95% in arm 1, 92% in arm 2), with reliable high depletion of all non-T cell populations. In experimental arm 1 (left side of **FIG. 24A****),** the percentage of CD45+/CD3+ T cells after the Sepax 2-Ficoll step dropped to 38% from 56% in the original donor material, the monocyte percentage was 28%, while B cells constituted 18% of the total sample. After FAST selection, nearly all the monocytes and B cells were removed, which achieved a final T cell purity of 95% **(****FIG. 24B****).** In experimental arm 2 (right side of **FIG. 24A****),** the post-CS5+ wash was directly purified using FAST, which yielded a final product virtually completely depleted of monocytes, with a final T cell purity of 92%. Also in this experiment, implementation of the modified protocol, where Sepax 2-Ficoll enrichment is omitted, yielded the highest T cell number in the final product. Cells from this stage of the experiment were placed back into culture and were transduced. Cells will be harvested at day 10 after wave expansion.

Experiment 2 summary: The use of FAST positive selection as a replacement for the current positive selection protocol was repeated. Whether used after CS5+ wash, or after Sepax 2-Ficoll, the optimized protocol achieved a high removal of the non T cell populations, removing nearly all monocytes present in the sample, and yielded an intermediate product with high T cell purity. Performance of FAST-separated cells in culture, including transduction, will be analyzed and reported.

### Experiment 3:

Experiment 3 details: In this experiment, the use of FAST positive selection to further purify post-elutriation samples was analyzed with a high monocyte donor. In addition, a high B cell blast condition, often observed when tumor-bearing patient apheresis samples are processed, was simulated via spike-in of Nalm6 cells (CD45-/CD19+) into the post-elutriation sample.

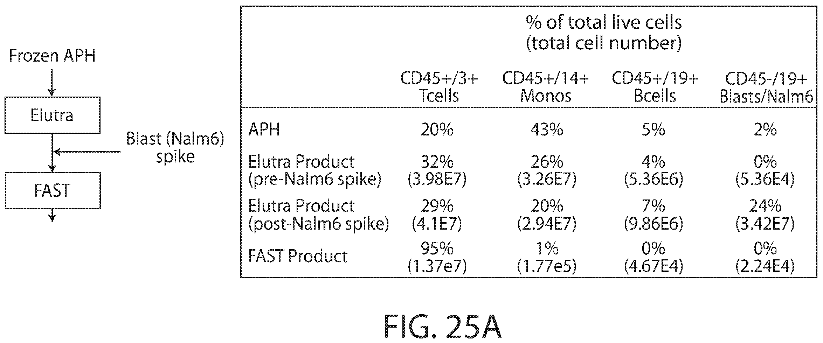

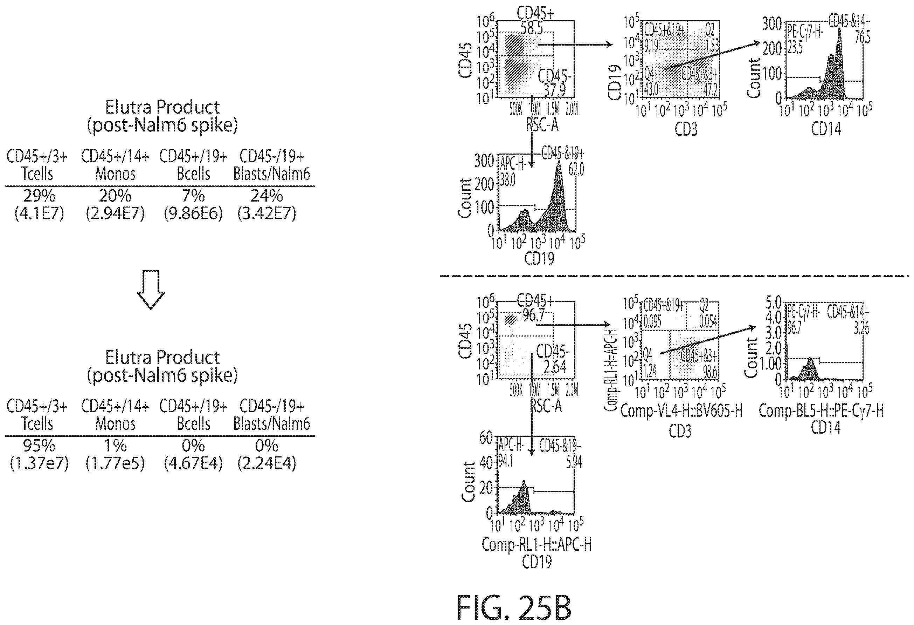

Experiment 3 outcomes: As shown in **FIG. 25A****,** normal donor apheresis material containing 20% T cell and 43% monocytes, was elutriated to reduce the percentage of monocytes in the sample down to 26% and to enrich the T cell percentage to 32%. At this point, a number of Nalm6 cells comparable to T cell and monocytes were spiked-in, leading to a sample that contained 29% T cells, 20% monocytes, and 24% blasts (Nalm6). Processing of this intentionally adulterated sample using FAST positive selection yielded a final product depleted of nearly all monocytes and Nalm6 cells, with a final T cell purity of 95%.

Experiment 3 summary: FAST positive selection can be implemented post-elutriation, and in this experiment was shown to enable high, reliable depletion of both monocytes and blast (Nalm6) cells. Final T cell purity after performing FAST selection on this difficult sample was 95%, starting from a 29% post-elutriation T cell purity.

### Conclusions

FAST positive selection was presented in a series of experiments, and shown to provide reliable T cell enrichment across a range of input sample material. The method was tested with total cell numbers ranging from 1E8 to 2E9 cells, input T cell purities from 20% to 50%, input monocyte percentage from 2% to 30%, and input blast\Nalm6 fractions from 2% to 100%. Under all cases tested to date, FAST yielded a final product with T cell purity of 90% or above. An alternate strategy where the Sepax Ficoll enrichment step is removed to pursue *FAST* positive selection immediately after CS5 was also outlined, showing potential in providing both a highly pure product and the highest number of T cells in the final product.

### Example 6: CD19+ B cell Depletion by Negative Selection

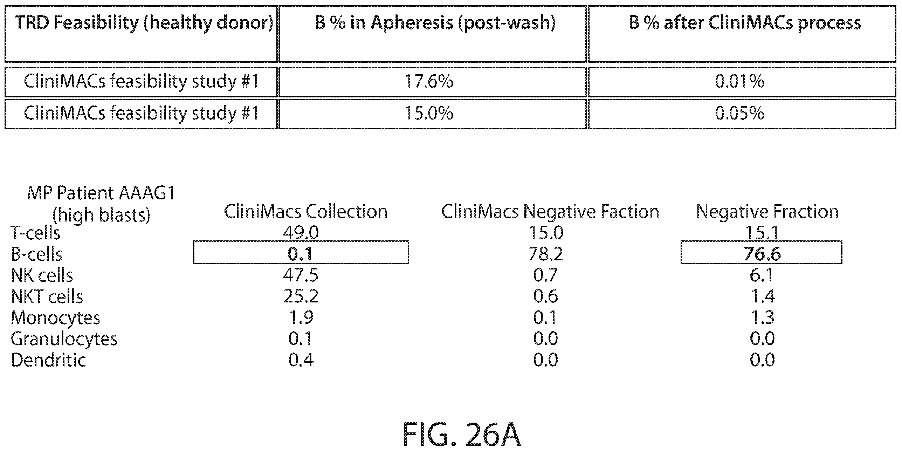

The feasibility of CD19 depletion from apheresis samples was tested. Apheresis samples from a healthy donor and a patient having ALL were used. The apheresis samples were washed and then CD19 negative selection was performed using CliniMACs. The results from the CD19 depletion are shown in Figure 26A. These results show that CD19 depletion using CliniMACs proved to be able to remove B cells from healthy donor apheresis. Moreover, CD19 depletion using CliniMACs was able to remove B cells from an ALL patient apheresis where the apheresis sample had very high % blasts. Specifically, the B cells in the ALL patient apheresis sample was greatly reduced from 77% to <0.1%.

Next, the efficacy of T cell enrichment using a protocol that includes a CD19 depletion step was tested. An enrichment protocol including a CD19 depletion negative selection step was compared with enrichment protocols that do not include a negative selection step, and instead include a positive selection step. The protocol designated TR149 comprises a wash step, density gradient centrifugation using Ficoll and the Sepax2 device, and a positive selection step using CD3/CD28 Dynabeads, also known as Process B. The protocol designated TR150 comprises elutriation, a wash step, and a CD19 depletion step (e.g., negative selection) using CliniMACs, The protocol designated TR151 comprises elutriation, a wash step, and a positive selection step using CD3/CD28 Dynabeads.

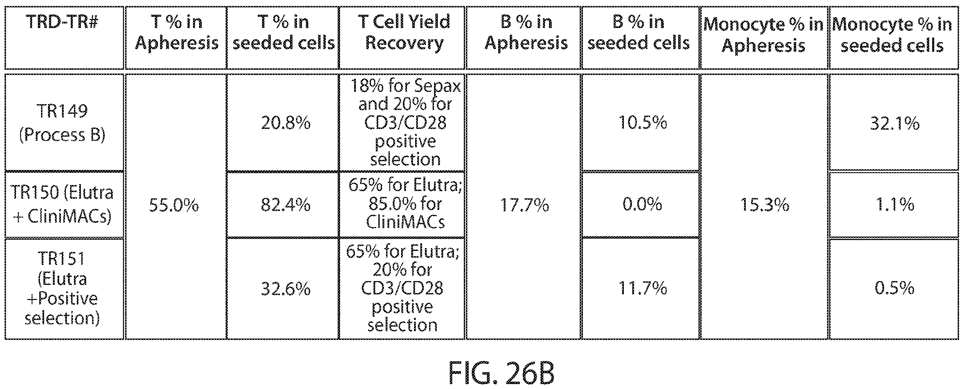

The percentage of T cells, B cells, and monocytes in the apheresis, e.g., before performing the enrichment protocol, and in the sample resulting from the enrichment protocol (referred to herein as "seeded cells") is shown in Figure 26B.

CD19 depletion by negative selection using CliniMACs (TR150) eliminated the B cells (from 17.7% in the apheresis sample) to 0% in the seeded cells. Depletion of B cells from the apheresis sample resulted in an improvement in T cell enrichment compared to the other protocols which did not include a B cell depletion step (e.g., 80% T cells in the seeded cells for TR150 compared to the 20-30% T cells in the seeded cells for TR149 and TR151. In addition, consistent with results from previous studies, the elutriation step (e.g., in TR150 and TR151) reduced the percentage of monocytes from 15% in the apheresis sample to <1.5% in the seeded cells, while still retaining good T cell recovery.

In conclusion, these results demonstrate that CD19 depletion was able to reduce the percentage of B cells to <1% after employing the enrichment protocol, and let to a high T cell yield (>80%).

### Example 7: Potency Assays

Two assays were used to assess the potency of CAR-expressing immune effector cells that were enriched for after different enrichment protocols, in order to determine whether different enrichment conditions resulted in immune effector cells with different CAR-directed potency. Apheresis from two different donors were subjected to different enrichment protocols as shown in Table 11. The cells were then stimulated and transduced to express a CD19 CAR.

**Table 11: Summary of Samples and Enrichment Conditions**

| **Donor** | **Sample** | **Conditions** |
|---|---|---|
| Donor 1 | TR157 | Elutriation |
| | TR158 | Smartwash in D5 + 0.5hr Positive Selection |
| | TR159 | Process B |
| | TR160 | CS5 wash + Sepax-Optiprep w/ positive selection |
| Donor 2 | TR161 | Elutriation |
| | TR162 | 0.5 hr positive selection |
| | TR163 | Process B |
| | TR164 | Sepax-Optiprep alone |

### IFNgamma Release Assay

The cells are co-cultured in the presence of CD19 expressing cells (CD19-expressing K562 cells) as the target cells, mesothelin-expressing cells (mesothelin-expressing K562 cells) as the negative control, or PMA as the positive control. After 24 hours, supernatant from the co-culture was harvested and the levels of IFNγ were quantified by ELISA. IFNγ release was quantified as pg/transduced cells to correct for variation in transduction efficiency.

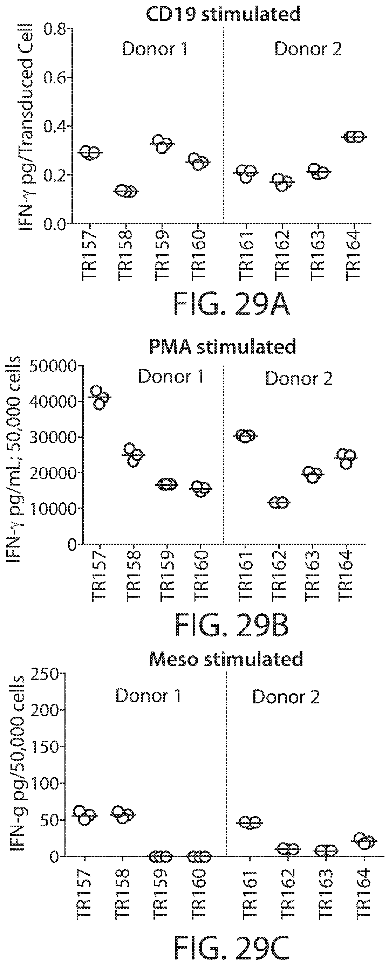

The results show that, CD19 stimulation resulted in IFNγ release as shown in FIG. 29A, in which the level of IFNγ release was within the range of IFNγ release observed when cells were enriched using standard protocols, e.g., Process B. As expected, all samples responded to PMA and released IFNγ after PMA stimulation (FIG. 29B). The highest levels of IFNγ resulted from transduced cells resulting from elutriation. Samples showed minimal response and IFNγ release after mesothelin stimulation (Fig. 29C).

### Cytotoxicity Assay

Cyotoxicity of the CAR-expressing cells generated from the enrichment conditions as described in in Table 11 was tested by co-culturing luciferase expressing CD19 cells (NALM6, target cells) with CAR-expressing cells (effector cells) in varying effector cell:target cell ratios ranging from 1:10,000 to 10:1. After 24 hours, luminescence (relative light units, RLU) was measured on a plate reader. Specific lysis (SpLy) was calculated as: 100-(sample RLU/maxRLU)*100. The ratio effector:target cells plated is recalculated to ratio CTL effector:target cells using the % CAR+ cells (as measured by flow cytometry) in order to correct for the variation in transduction rates.

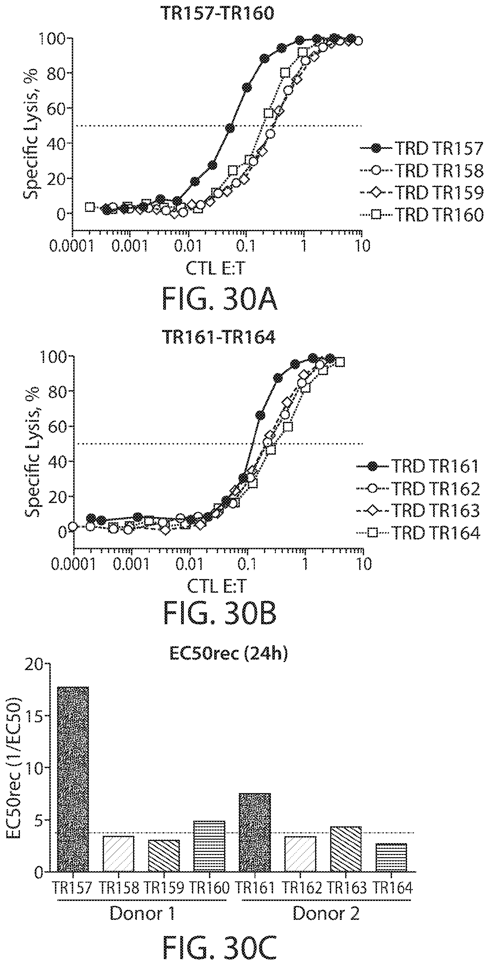

As shown in FIG. 30A and 30B, complete sigmoidal killing curve was reached by all samples, demonstrating specific killing was achieved. Cells with increased killing potency have curves that are farther to the left on the graph. Results from the cytotoxicity assay was also reported as the reciprocal value of EC50. EC50 was calculated from a four-parameter logistic fit of the curve for CTL E:T and % specific lysis. The red dotted line in the graph represents the median levels for 30 healthy donors. As shown in FIG. 30C, all samples had good EC50rec, around or above median levels. The highest EC50rec was from elutriated samples.

### Example 8: Flow-through device

The following example is provided for illustrative purposes only and is not intended to encompass the entire invention. Aspects of this example may be combined with other aspects of the invention described herein.

In this example, T cells were expanded over a 9-day period in culture, then harvested and debeaded using a non flow-through debeading process according to prior procedures or a flow-through debeading process according to the present disclosure. The samples had between approximately 1e8 nucleated viable cells and approximately 3e10 nucleated viable cells. The paramagnetic particle to nucleated viable cell ratio was between approximately 3:1 and 1:3, with samples having lower total nucleated viable cells exhibiting higher paramagnetic particle to nucleated viable cell ratios. The paramagnetic particle to cell ratio was a significant factor in cell recovery. A higher paramagnetic particle to nucleated viable cell ratio increased the chances that a nucleated viable cell is bound to a paramagnetic particle and is lost during the paramagnetic particle removal process.

In the non flow-through debeading process, the sample was collected in a one liter platelet bag (Terumo Medical Corp., Somerset, NJ) and statically placed on top a flat-bed magnetic plate (DYNAMAG^{™} CTS^{™}, Thermo Fisher Scientific, Waltham, MA) of 5 minutes at zero degrees, followed by one minute at a 30 degree inclination. Next, the liquid in the bag, which contained the non-magnetic fraction, was diverted from the bag to form the final product. The magnetic fraction remained inside the bag as waste.

In the flow-through debeading process, the sample was continuously flowed through a CSD400Y9 CRYOSTORE^{™} Conical Bag (OriGen Biomedical, Austin, TX) placed on top a flat-bed magnetic plate (DYNAMAG^{™} CTS^{™}, Thermo Fisher Scientific, Waltham, MA). Due to continuous flow through the bag and over the magnet, the sample was dynamically debeaded, with paramagnetic particles being stripped off the viable nucleated cells as they moved through the bag. The liquid after passaging through the bag for some time formed the final product. The magnetic fraction remained inside the bag as waste. Few viable nucleated cells were trapped in the magnetic faction. This is in contrast to the non flow-through debeading process, which viable nucleated cells bound to paramagnetic particles were attracted to the magnet and lost in the waste.

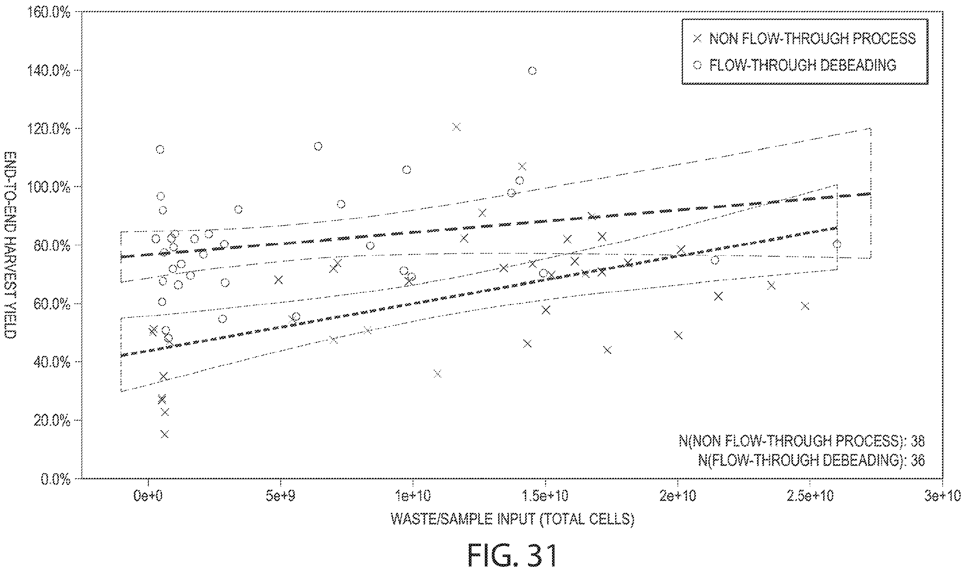

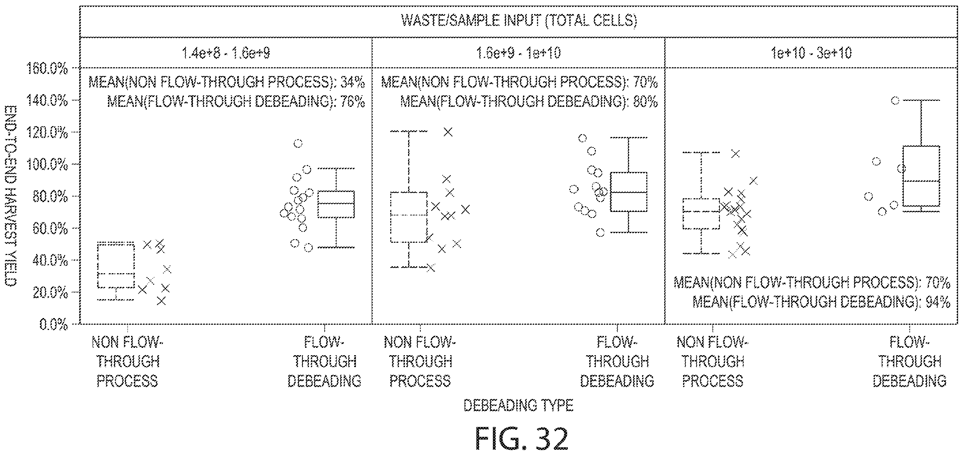

Metadata analysis of thirty-eight non flow-through debeading process runs and thirty-six flow-through debeading process runs showed significant increases in recovery of viable nucleated cells when the flow-through debeading process was used. FIG. 31. The increase in viable nucleated cell recovery was particularly significant at lower numbers of cells in the sample (such as less than 1.6e9 total viable nucleated cells). At such total viable nucleated cell numbers, the flow-through debeading process exhibited a 76% average recovery as compared to only 34% average recovery for the non flow-through debeading process. FIG. 32. A 10-20% increase in recovery was also seen with higher numbers of cells in the sample.

This difference in recovery is due to the ability of the flow-through debeading process to dynamically remove paramagnetic particles from the viable nucleated cells, so that these cells are not lost even if they were initially bound to paramagnetic particles prior to harvest.

### Example 9: Positive selection methods

FAST positive selection can be performed on leukapheresis collected from patients having hematologic malignancies. The patient sample may comprise >20% lymphoblasts. The cells can be present in a flexible container, e.g., a cell bag, e.g., a rectangular Origen CS250 bag, during at least a part of the procedure. First, if the leukapheresis sample is frozen, a thaw step is performed. The cells can then be washed, e.g., using a CellSaver5+ device, e.g., at ambient temperature, e.g., 20-25°C. The wash solution can comprise 5% HABS (human AB serum) MM, e.g., X-VIVO15. The cells can then be contacted with a separation reagent, which comprises a magnetic or paramagnetic bead and a CD3 and CD28-binding antibodies. The cells can then be rotated on a rotator, e.g., at 2-6 rpm, e.g., at 4 rpm, and the rotation may last for, e.g., 10-30 minutes, e.g., 20 minutes. During rotation, the cells may be at ambient temperature, e.g., between 20 and 25 °C. Positive selection can then be performed to enrich for cells that bind the separation reagent, e.g., for 30 sec to 2 minutes, e.g., for 1 minute.

### Example 10: Positive selection methods

FAST positive selection can be performed on leukapheresis collected from patients having hematologic malignancies. The cells can be present in a flexible container, e.g., a cell bag, e.g., a rectangular Origen CS250 bag, during at least a part of the procedure. First, if the leukapheresis sample is frozen, a thaw step is performed. The cells can then be washed, e.g., using a CellSaver5+ device, e.g., at ambient temperature, e.g., 20-25°C. The wash solution can comprise 5% dextrose, 0.45% sodium chloride (e.g., D5 1/2NS). If there is a hold between the wash step and the subsequent step, the cell bag can be placed on a thermal insulating material, e.g., a plurality of layers comprising paper, e.g., paper towels or wipes. The cells can then be contacted with a separation reagent, which comprises a magnetic or paramagnetic bead and a CD3 and CD28-binding antibodies. The cells can then be incubated, e.g., at 37°C, e.g., for 5-15 minutes, e.g., 10 minutes. The cells can then be rotated on a rotator, e.g., at 2-6 rpm, e.g., at 4 rpm, and the rotation may last for, e.g., 10-30 minutes, e.g., 20 minutes. The thermal insulating material may be placed between the flexible container for the cells and one side of the cell plate of the rotator. During rotation, the cells may be at higher than ambient temperature, e.g., between 20 and 37 °C. Positive selection can then be performed to enrich for cells that bind the separation reagent, e.g., for 30 sec to 2 minutes, e.g., for 1 minute.

### EQUIVALENTS

The disclosures of each and every patent, patent application, and publication cited herein are hereby incorporated herein by reference in their entirety. While this invention has been disclosed with reference to specific aspects, it is apparent that other aspects and variations of this invention may be devised by others skilled in the art without departing from the true spirit and scope of the invention. The appended claims are intended to be construed to include all such aspects and equivalent variations.

The following numbered clauses, describing aspects of the invention, are part of the description.
1. A method of making a population of immune effector cells (e.g., T cells) that can be engineered to express a chimeric antigen receptor (CAR), the method comprising:
   a) providing a frozen input sample comprising immune effector cells,
   b) thawing the frozen input sample, to produce a thawed sample, and
   c) performing elutriation on the thawed sample and collecting immune effector cells, thereby producing an output sample comprising immune effector cells that are suitable for expression of a CAR.
2. The method of clause 1, wherein the frozen input sample is a plasma apheresis sample.
3. The method of clauses 1 or 2, further comprising one, two, three or all of:
   d) depleting CD19+ cells under flow conditions;
   e) performing density centrifugation using a medium comprising iodixanol, e.g., 60% iodixanol in water, e.g., Optiprep medium, and/or having a density greater than Ficoll (e.g., greater than 1.077 g/ml, e.g., about 1.32 g/ml);
   f) performing a wash step (e.g., on the thawed sample) with a buffer comprising dextrose and/or sodium chloride, e.g., D5 medium (5% dextrose and 0.45% sodium chloride), e.g., wherein the wash step is performed using a CS5 (CellSaverS+) instrument; and
   g) performing a positive selection of CD3/CD28+ cells under flow conditions.
4. The method of any of clauses 1-3, which comprises a step of adjusting the viscosity of the thawed sample, e.g., by adding an isotonic solution, e.g., PBS, to the thawed sample.
5. The method of any of clauses 1-4, wherein:
   the elutriation is performed using a flow rate of from about 30-82 mL/min or 50-80 mL/min and/or the collection volume is about 250-1250 mL or 300-1000 mL for each fraction;, e.g., the elutriation is performed using a flow rate of about 30, 40, 50, 60, 70, 72, or 82 mL/min, e.g., about 70 or 72 mL/min;
   the elutriation is performed using a collection volume of about 250, 400, 500, 900, or 975 mL, e.g., about 400 or 975 mL; and/or
   the elutriation is performed at about 2400 rpm.
6. The method of any of clauses 1-5, wherein the input sample comprises:
   at least 10%, 15%, 20%, 21%, 22%, 23%, 24%, 25%, 26%, 27%, 28%, 29%, 30%, 31%, 35%, or 40% monocytes;
   less than 60%, 55%, 50%, 45%, 40%, 35%, 30%, 25%, or 20% T cells; and/or
   at least 1%, 2%, 5%, 10%, 15%, 20%, 30%, 35%, 40%, 45%, 50%, 55%, 60%, 65%, 70%, 75%, 80%, 85%, 90%, or 95% B cells.
7. The method of any of clauses 1-6, wherein the output sample comprises:
   less than 20%, 15%, 10%, 9%, 8%, 7%, 6%, 5%, 4%, 2%, 2%, 1%, 0.5%, 0.2%, or 0.1% monocytes;
   at least 50%, 55%, 60%, 65%, 70%, 75%, 80%, 85%, 90%, or 95% T cells;
   less than 20%, 15%, 10%, 9%, 8%, 7%, 6%, 5%, 4%, 2%, 2%, 1%, 0.5%,0 .2%, or 0.1% B cells;
   at least 50%, 55%, 60%, 65%, 70%, 75%, 80%, 85%, 90%, 95%, 96%, 97%, 98%, 99%, 99.5%, 99.7%, or 99.9% CD4+CD25+ cells; and/or
   at least 50%, 55%, 60%, 65%, 70%, 75%, 80%, 85%, 90%, 95%, 96%, 97%, 98%, 99%, 99.5%, 99.7%, or 99.9% CD8+CD25+ cells.
8. The method of any of clauses 1-7, which has a T cell yield recovery of at least 50%, 55%, 60%, 65%, 70%, 75%, 80%, 85%, 90%, or 95% T cells.
9. The method of any of clauses 1-8, wherein the output sample is contacted with a nucleic acid encoding a CAR.
10. The method of clause 9, wherein, after contacting the output sample with a nucleic acid encoding a CAR, the output sample comprises:
   at least 40%, 45%, 50%, 55%, 60%, 65%, 70%, 75%, 80%, 85%, 90%, or 95% CAR+CD4+ central memory cells;
   at least 10%, 15%, 20%, 25%, 30%, 35%, 40%, 45%, or 50% CAR+ cells; and/or
   at least 40%, 45%, 50%, 55%, 60%, 65%, 70%, 75%, 80%, 85%, 90%, or 95% CAR+CD8+ central memory cells.
11. The method of clause 9 or 10, wherein, after contacting the output sample with a nucleic acid encoding a CAR, the output sample:
   produces less than 1, 0.9, 0.8, 0.7, 0.6, 0.5, 0.4, 0.3, 0.2, or 0.1 pg IFN-gamma per transduced cell; and/or
   comprises a cytotoxicity level (e.g., an EC50rec) of at least 5, 6, 7, 8, 9, 10, 11, 12, 13, 14, 15, 16, 17, 18, 19, 20, 25, or 30.
12. A method of making a population of immune effector cells (e.g., T cells) that can be engineered to express a CAR, the method comprising:
   a) providing an input sample comprising immune effector cells, and
   b) performing density centrifugation step using a medium comprising iodixanol, e.g., 60% iodixanol in water, e.g., Optiprep medium and/or having a density greater than Ficoll (e.g., greater than 1.077 g/ml, e.g., about 1.32 g/ml), thereby producing an output sample comprising immune effector cells that are suitable for expression of a CAR.
13. The method of clause 12, further comprising performing one, two, three, or all of:
   c) depleting CD19+ cells under flow conditions;
   d) elutriation on the input sample, wherein the input sample is optionally a thawed input sample;
   e) performing a wash step (e.g., before density centrifugation) with a buffer comprising dextrose and/or sodium chloride, e.g., D5 medium (5% dextrose and 0.45% sodium chloride), e.g., wherein the wash step is performed using a CS5 (CellSaver5+) instrument; and
   f) positive selection of CD3/CD28+ cells under flow conditions.
14. The method of clause 12 or 13, which does not comprise one or more of: using a solution comprising glycol, e.g., a Ficoll solution; or performing a wash step in a buffer comprising dextrose and/or sodium chloride, e.g., D5 medium, e.g., wherein the wash step is performed using a CS5 instrument; or performing a positive selection step.
15. The method of any of clauses 12-14, wherein the density centrifugation is performed using a cell separation device, e.g., a Sepax2 device.
16. The method of any of clauses 12-15, wherein the input sample comprises:
   less than 60%, 55%, 50%, 45%, 40%, 35%, 30%, 25%, 20%, 19%, 18%, 17%, 16%, or 15% T cells;
   at least 10%, 15%, 20%, 21%, 22%, 23%, 24%, 25%, 26%, 27%, 28%, 29%, 30%, 31%, 35%, 40%, 45%, 50%, 55%, 60%, 65%, or 70% monocytes; and/or
   at least 1%, 2%, 5%, 10%, 15%, 20%, 30%, 35%, 40%, 45%, 50%, 55%, 60%, 65%, 70%, 75%, or 80% B cells.
17. The method of any of clauses 12-16, wherein the output sample comprises:
   at least 25%, 30%, 35%, 40%, 45%, 50%, 55%, 60%, 65%, 70%, 75%, 80%, 85%, 90%, or 95% T cells;
   less than 50%, 45%, 40%, 35%, 30%, 25%, 20%, 15%, 10%, 9%, 8%, 7%, 6%, 5%, 4%, 2%, 2%, 1%, 0.5%, 0.2%, or 0.1% monocytes; and/or
   less than 20%, 15%, 10%, 9%, 8%, 7%, 6%, 5%, 4%, 2%, 2%, 1%, 0.5%,0 .2%, 0.1%, 0.05%, or 0.01% B cells.
18. The method of any of clauses 12-17, which has a T cell yield recovery of at least 5%, 10%, 15%, 20%, 25%, 30%, 35%, 40%, 45%, 50%, 55%, 60%, 65%, 70%, 75%, 80%, 85%, 90%, or 95% T cells.
19. A method of making a population of immune effector cells (e.g., T cells) that can be engineered to express a CAR, the method comprising:
   a) providing an input sample comprising immune effector cells, and
   b) removing CD19+ cells from the input sample under flow conditions, e.g., using a flow-through device, thereby producing an output sample comprising immune effector cells that are suitable for expression of a CAR.
20. The method of clause 19, further comprising performing one, two, three or all of:
   c) elutriation on the input sample, wherein the input sample is optionally a thawed input sample;
   d) a density centrifugation step using a medium comprising iodixanol, e.g., 60% iodixanol in water, e.g., Optiprep medium, and/or having a density greater than Ficoll (e.g., greater than 1.077 g/ml, e.g., about 1.32 g/ml),
   e) performing a wash step (e.g., before removing CD19+ cells and/or after the input sample is thawed) with a buffer dextrose and/or sodium chloride, e.g., D5 medium (5% dextrose and 0.45% sodium chloride), e.g., wherein the wash step is performed using a CS5 (CellSaver5+) instrument; and
   f) positive selection of CD3/CD28+ cells under flow conditions.
21. The method of clause 19 or 20, which does not comprise performing elutriation or density centrifugation.
22. The method of any of clauses 19-21, wherein the CD19+ cells comprise B cells, e.g., lymphoblasts.
23. The method of any of clauses 19-37, wherein the input sample comprises:
   at least 1%, 2%, 5%, 10%, 15%, 20%, 30%, 35%, 40%, 45%, or 50% CD19+ cells;
   at least 10%, 15%, 20%, 21%, 22%, 23%, 24%, 25%, 26%, 27%, 28%, 29%, 30%, 31%, 35%, or 40% monocytes; and/or
   less than 60%, 55%, 50%, 45%, 40%, 35%, 30%, 25%, or 20% T cells;
24. The method of any of clauses 19-38, wherein the output sample comprises:
   less than 20%, 15%, 10%, 9%, 8%, 7%, 6%, 5%, 4%, 2%, 2%, 1%, 0.5%,0 .2%, 0.1%, 0.05%, or 0.01% CD19+ cells;
   less than 50%, 45%, 40%, 40%, 35%, 30%, 25%, 20%, 15%, 10%, 5%, 4%, 2%, 2%, or 1% the percentage of CD19+ cells compared to the input sample;
   less than 20%, 15%, 10%, 9%, 8%, 7%, 6%, 5%, 4%, 2%, 2%, 1%, 0.5%,0 .2%, or 0.1% monocytes;
   at least 50%, 55%, 60%, 65%, 70%, 75%, 80%, 85%, 90%, or 95% T cells; and/or less than 20%, 15%, 10%, 9%, 8%, 7%, 6%, 5%, 4%, 2%, 2%, 1%, 0.5%,0 .2%, 0.1%, 0.05%, or 0.01% B cells
25. The method of any of clauses 19-24, wherein CD19+ cells are removed by magnetic separation.
26. The method of clause 25, wherein the magnetic separation comprises:
   contacting the cells with a separation reagent, which separation reagent comprises a magnetic or paramagnetic member and a CD19-binding member;
   flow cytometry; and/or
   use of a magnetic cell separation device, e.g., CliniMACs device.
27. The method of clause 25, wherein CD19+ cells are removed by FACS.
28. The method of any of clauses 19-27, which has a T cell yield recovery of at least 50%, 55%, 60%, 65%, 70%, 75%, 80%, 85%, 90%, or 95% T cells.
29. The method of any of clauses 19-28, wherein the input sample (e.g., the input sample post-wash) comprises at least 1%, 2%, 5%, 10%, 15%, 20%, 30%, 35%, 40%, 45%, 50%, 55%, 60%, 65%, 70%, 75%, or 80% B cells.
30. A method of making a population of immune effector cells (e.g., T cells) that can be engineered to express a CAR, the method comprising:
   a) providing an input sample comprising immune effector cells, and
   b) positively selecting for CD3+/CD28+ cells from the input sample under flow conditions, thereby producing an output sample comprising immune effector cells that are suitable for expression of a CAR.
31. The method of clause 30, which further comprises performing one, two, three, or all of:
   c) depleting CD19+ cells, e.g., under flow conditions;
   d) elutriation on the input sample, wherein the input sample is optionally a thawed input sample;
   e) density centrifugation step using a medium comprising iodixanol, e.g., 60% iodixanol in water, e.g., Optiprep medium, and/or having a density greater than Ficoll (e.g., greater than 1.077 g/ml, e.g., about 1.32 g/ml);
   f) performing a wash step (e.g., before removing CD19+ cells and/or after the input sample is thawed) with a buffer dextrose and/or sodium chloride, e.g., D5 medium (5% dextrose and 0.45% sodium chloride), e.g., wherein the wash step is performed using a CS5 (CellSaver5+) instrument.
32. The method of clause 30, which further comprises:
   performing elutriation, a wash step (optionally), and density centrifugation (e.g., using Ficoll or OptiPrep medium) prior to performing positive selection, and/or
   performing a wash step (optionally) and density centrifugation (e.g., using Ficoll or OptiPrep medium) prior to performing positive selection.
33. The method of any of clauses 30-32, which does not comprise performing elutriation.
34. The method of any of clauses 30-33, which comprises performing a wash with a buffer comprising dextrose and/or sodium chloride, e.g., D5 1/2 NS buffer, e.g., using a CS5+ instrument.
35. The method of any of clauses 30-34, wherein the positive selection for CD3+/CD28+ cells comprises contacting the input sample with a separation reagent, which separation reagent comprises a magnetic or paramagnetic member and a CD3 and/or CD28-binding member.
36. The method of claim any of clauses 30-35, wherein the positive selection for CD3+/CD28+ cells comprises incubating the input sample with a separation reagent for about 10 to 90 minutes, about 10 to 60 minutes, about 10 to 45 minutes, about 12 to 90 minutes, about 12 to 60 minutes, about 12 to 45 minutes, about 15 to 90 minutes, about 15 to 60 minutes, about 15 to 45 minutes, e.g., about 30 minutes or about 20 minutes.
37. The method of clause 36, wherein the separation reagent comprises a bead that is coupled (e.g., covalently or non-covalently coupled) to an anti-CD3 and/or anti-CD28 antibody.
38. The method of any of clauses 30-37, wherein the output sample comprises:
   at least 5%, 6%, 7%, 8%, 9%, 10%, 11%, 12%, 13%, 14%, 15%, or 20% T cells;
   less than 20%, 15%, 10%, 9%, 8%, 7%, 6%, 5%, 4%, 2%, 2%, 1%, 0.5%,0 .2%, or 0.1% monocytes;
   at least 25%, 30%, 35%, 40%, 45%, 50%, 55%, 60%, 65%, 70%, 75%, 80%, 85%, 90%, 95%, 96%, 97%, 98%, or 99% T cells, e.g., CD3+CD45+ T cells.
39. The method of any of clauses 30-38, which is performed with a magnetic device, e.g., Dynamag CTS or other arrangement of magnetic elements and/or which uses an about 3:1 ratio of magnetic separation members (e.g., beads) to T cells.
40. The method of any of clauses 30-39, wherein the positive selection for CD3+/CD28+ cells comprises a separation or dwell time of less than about 6, 5, 6, 3, 2, or 1 minute, or less than about 50, 40, 30, 20, 10, 5, 4, 3, 2, or 1 second.
41. The method of any of clauses 30-40, which comprises flowing a fluid that comprises the immune effector cells and magnetic separation members within an enclosed system, e.g., a chamber or a bag, where magnetic separation occurs, wherein optionally the flowing is performed at a speed such that magnetic separation of the members (optionally bound to immune effector cells) occurs.
42. The method of any of clauses 30-41, which is performed using a device comprising
   at least one cell suspension module;
   at least one flow-through magnetic separation/debeading module;
   at least one non-magnetic output module;
   at least one magnetic output module;
   optionally, at least one magnetic component, external to the magnetic separation/debeading module, that creates magnetic forces and/or gradients; and optionally, at least one buffer module.
43. The method of clause 42, wherein the magnetic separation/debeading module comprises:
   a chamber defined by walls and having an x-direction, a y-direction, and a z-direction;
   an inlet and an outlet arranged on opposite ends of the chamber, e.g., in the x-direction, in the y-direction, or in the z-direction;
   at least two magnets adjacent or proximate to a wall of the chamber and arranged to establish a zero gradient line within the chamber between the inlet and the outlet.
44. The method of any of clauses 30-43, wherein the input sample comprises :
   about 1×10⁷ cells/ml.
   at least about 5%, 10%, 15%, 18%, 20%, 25%, 30%, 35%, 40%, 45%, 50%, 55%, 60%, 65%, 70%, 75%, 80%, 85%, 90%, or 95% B cells, e.g., CD45+CD19+ B cells; and/or
   at least about 5%, 10%, 15%, 18%, 20%, 25%, 30%, 35%, 40%, 45%, 50%, 55%, 60%, 65%, 70%, 75%, 80%, 85%, 90%, or 95% B cells, e.g., CD45-CD19+ B cells.
45. The method of any of clauses 30-44, wherein the output sample comprises:
   less than about 10%, 9%, 8%, 7%, 6%, 5%, 4%, 3%, 2%, or 1% B cells, e.g., CD45+CD19+ B cells;
   less than about 10%, 9%, 8%, 7%, 6%, 5%, 4%, 3%, 2%, or 1% B cells, e.g., CD45-CD19+ B cells.
46. The method of any of the preceding clauses, wherein the input sample comprises:
   at least 5%, 10%, 15%, 20%, 25%, 30%, 35%, 40%, 45%, 50%, 55%, 60%, 65%, 70%, 75%, 80%, 85%, 90%, or 95% monocytes;
   at least 5%, 10%, 15%, 20%, 25%, 30%, 35%, 40%, 45%, 50%, 55%, 60%, 65%, 70%, 75%, 80%, 85%, 90%, or 95% tumor cells, e.g., lymphoblasts; and/or
   less than 60%, 55%, 50%, 45%, 40%, 35%, 30%, 25%, or 20% immune effector cells, e.g., T cells.
47. The method of any of the preceding clauses, wherein the output sample comprises:
   less than 20%, 15%, 10%, 9%, 8%, 7%, 6%, 5%, 4%, 2%, 2%, 1%, 0.5%,0 .2%, or 0.1% monocytes;
   less than 20%, 15%, 10%, 9%, 8%, 7%, 6%, 5%, 4%, 2%, 2%, 1%, 0.5%,0 .2%, or 0.1% tumor cells;
   at least 50%, 55%, 60%, 65%, 70%, 75%, 80%, 85%, 90%, 95%, 96%, 97%, 98%, 99%, 99.5%, 99.8%, or 99.9% immune effector cells, e.g., T cells;
   less than 50%, 45%, 40%, 40%, 35%, 30%, 25%, 20%, 15%, 10%, 5%, 4%, 2%, 2%, or 1% the percentage of monocytes compared to the input sample;
   less than 50%, 45%, 40%, 40%, 35%, 30%, 25%, 20%, 15%, 10%, 5%, 4%, 2%, 2%, or 1% the percentage of tumor cells compared to the input sample; and/or
   at least 50%, 45%, 40%, 40%, 35%, 30%, 25%, 20%, 15%, 10%, 5%, 4%, 2%, 2%, or 1% the percentage of immune effector cells, e.g., T cells, compared to the input sample.
48. The method of any of the preceding clauses, further comprising introducing, e.g., by transduction, a nucleic acid encoding a CAR into one or more of the immune effector cells in the output sample, wherein optionally the CAR comprises an antigen binding domain, a transmembrane domain, and an intracellular signaling domain, e.g., comprising a primary signaling domain and/or a costimulatory signaling domain, and wherein optionally the transduction results in a transduction efficiency of at least about 10%, 15%, 20%, 25%, 30%, 35%, 40%, 45%, or 50%.
49. The method of clause 48, which further comprises a step of assaying the transduction efficiency and/or performing a wash step on the input sample with a buffer comprising dextrose and/or sodium chloride, e.g., D5 medium, e.g., using a CS5+ instrument.
50. The method of any of the preceding clauses, wherein the immune effector cells are human immune effector cells.
51. The method of any of the preceding clauses, wherein the output sample comprises CD8+ T cells and/or CD4+ T cells.
52. The method of any of the preceding clauses, wherein the input sample is from a patient that has a cancer selected from the group consisting of one or more acute leukemias including but not limited to B-cell acute lymphoid leukemia (BALL), T-cell acute lymphoid leukemia (TALL), acute lymphoid leukemia (ALL); one or more chronic leukemias including but not limited to chronic myelogenous leukemia (CML), chronic lymphocytic leukemia (CLL); additional hematologic cancers or hematologic conditions including, but not limited to B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, MALT lymphoma, mantle cell lymphoma, Marginal zone lymphoma, multiple myeloma, myelodysplasia and myelodysplastic syndrome, non-Hodgkin lymphoma, Hodgkin lymphoma, plasmablastic lymphoma, plasmacytoid dendritic cell neoplasm, Waldenstrom macroglobulinemia, preleukemia, atypical and/or non-classical cancers, malignancies, precancerous conditions or proliferative diseases, and any combination thereof.
53. The method of any of the preceding clauses, which further comprises:
   a step of assaying one or more cell surface markers on cells in the output sample, e.g., CD45, CD19, CD3, CD28, CD25, or CD14; and/or
   a step of stimulating the immune effector cells, e.g., by contacting the cells with a reagent that binds CD3 and/or CD28, e.g., a substrate (such as a bead) coupled to CD3 and/or CD28 antibodies, e.g., prior to transduction.
54. A method of making a population of immune effector cells (e.g., T cells) that can be engineered to express a chimeric antigen receptor (CAR), the method comprising:
   i) providing an input sample, e.g., a frozen input sample or a fresh input sample comprising immune effector cells;
   ii) optionally, wherein the input sample is a frozen input sample, thawing the frozen input sample, to produce a thawed sample;
   iii) performing an enrichment step, wherein the enrichment step comprises:
      1. performing elutriation on the input sample, wherein the input sample is optionally a thawed input sample; or
      2. performing density centrifugation step using a medium comprising iodixanol, e.g., 60% iodixanol in water, e.g., Optiprep medium, and/or having a density greater than Ficoll (e.g., greater than 1.077 g/ml, e.g., about 1.32 g/ml); and
   iv) performing a selection step, wherein the selection is a positive selection, e.g., for CD3/CD28+ cells, or a negative selection, e.g., for CD19+, CD25+, or CD14+ cells;
   thereby producing an output sample comprising immune effector cells that are suitable for expression of a CAR.
55. A method of making a population of immune effector cells (e.g., T cells) that can be engineered to express a chimeric antigen receptor (CAR), the method comprising:
   a) providing an input sample, e.g., a frozen input sample or a fresh input sample, comprising immune effector cells;
   b) performing an enrichment step, wherein the enrichment step comprises: performing elutriation or density centrifugation (e.g., using Ficoll or a Optiprep medium);
   c) performing a selection step under flow conditions, e.g., by using a flow-through device, wherein the selection is a positive selection, e.g., for CD3/CD28+ cells, or a negative selection, e.g., for CD19+, CD25+, or CD14+ cells;
   thereby producing an output sample comprising immune effector cells that are suitable for expression of a CAR.
56. The method of clause 54 or 55, further comprising:
   performing a wash step with a buffer comprising dextrose and/or sodium chloride, e.g., D5 medium (5% dextrose and 0.45% sodium chloride), e.g., wherein the washing step is performed using a CS5 (CellSaver5+) instrument;
   stimulating the output sample with an agent that stimulates proliferation of the immune effector cells, e.g., stimulates a CD3/TCR complex associated signal and/or a ligand that stimulates a costimulatory molecule on the surface of the T cells, e.g., an anti-CD3 antibody and an anti-CD28 antibody; and/or
   introducing a nucleic acid encoding a CAR, e.g., by transduction, transfection, or electroporation.
57. A reaction mixture obtained using a method according to any of clauses 1-56.
58. A reaction mixture comprising at least 80%, 85%, 90%, or 95% T cells and less than 10%, 9%, 8%, 7%, 6%, 5%, 4%, 3%, 2%, or 1% monocytes, wherein the total number of cells in the reaction mixture adds up to 100%, wherein optionally one or more of the T cells expresses a CAR.
59. The reaction mixture of clause 58, which comprises:
   at least 1×10⁶, 2×10⁶, 5×10⁶, 1×10⁷, 2×10⁷, 5×10⁷, 1×10⁸, 2×10⁸, or 5×10⁸ cells total;
   less than 10%, 9%, 8%, 7%, 6%, 5%, 4%, 3%, 2%, or 1% B cells; and/or
   less than 10%, 9%, 8%, 7%, 6%, 5%, 4%, 3%, 2%, or 1% cancer cells, e.g.
   lymphoblasts.
60. The reaction mixture of any of clauses 57-59, which further comprises a nucleic acid encoding a CAR, e.g., wherein the nucleic acid is present inside a T cell or outside a T cell.

## Claims

1. A method of making a population of immune effector cells (e.g., T cells) that can be engineered to express a chimeric antigen receptor (CAR), the method comprising:
a) providing an input sample comprising immune effector cells, and
b) positively selecting for CD3+/CD28+ cells from the input sample under flow conditions, thereby producing an output sample comprising immune effector cells that are suitable for expression of a CAR.

2. The method of claim 1, which further comprises performing one, two, three, or all of:
c) depleting CD19+ cells, e.g., under flow conditions;
d) elutriation on the input sample, wherein the input sample is optionally a thawed input sample;
e) density centrifugation step using a medium comprising iodixanol, e.g., 60% iodixanol in water, e.g., Optiprep medium, and/or having a density greater than Ficoll (e.g., greater than 1.077 g/ml, e.g., about 1.32 g/ml);
f) performing a wash step (e.g., before removing CD19+ cells and/or after the input sample is thawed) with a buffer dextrose and/or sodium chloride, e.g., D5 medium (5% dextrose and 0.45% sodium chloride), e.g., wherein the wash step is performed using a CS5 (CellSaver5+) instrument.

3. The method of claim 1 or 2, wherein:
i) the method further comprises performing elutriation, a wash step (optionally), and density centrifugation (e.g., using Ficoll or OptiPrep medium) prior to performing positive selection;
ii) the method further comprises performing a wash step (optionally) and density centrifugation (e.g., using Ficoll or OptiPrep medium) prior to performing positive selection; or
iii) the method does not comprise performing elutriation.

4. A method of making a population of immune effector cells (e.g., T cells) that can be engineered to express a CAR, the method comprising:
i) providing an input sample, e.g., a frozen input sample or a fresh input sample comprising immune effector cells;
ii) optionally, wherein the input sample is a frozen input sample, thawing the frozen input sample, to produce a thawed sample;
iii) performing an enrichment step, wherein the enrichment step comprises:
1. performing elutriation on the input sample, wherein the input sample is optionally a thawed input sample; or
2. performing density centrifugation step using a medium comprising iodixanol, e.g., 60% iodixanol in water, e.g., Optiprep medium, and/or having a density greater than Ficoll (e.g., greater than 1.077 g/ml, e.g., about 1.32 g/ml); and
iv) performing a selection step, wherein the selection is a positive selection, e.g., for CD3/CD28+ cells, or a negative selection, e.g., for CD19+, CD25+, or CD14+ cells;
thereby producing an output sample comprising immune effector cells that are suitable for expression of a CAR.

5. The method of claim 4, further comprising:
performing a wash step with a buffer comprising dextrose and/or sodium chloride, e.g., D5 medium (5% dextrose and 0.45% sodium chloride), e.g., wherein the washing step is performed using a CS5 (CellSaver5+) instrument;
stimulating the output sample with an agent that stimulates proliferation of the immune effector cells, e.g., stimulates a CD3/TCR complex associated signal and/or a ligand that stimulates a costimulatory molecule on the surface of the T cells, e.g., an anti-CD3 antibody and an anti-CD28 antibody; and/or
introducing a nucleic acid encoding a CAR, e.g., by transduction, transfection, or electroporation.

6. The method of any of claims 1-5, wherein:
i) the positive selection for CD3+/CD28+ cells comprises contacting the input sample with a separation reagent, which comprises a magnetic or paramagnetic member and a CD3 and/or CD28-binding member;
ii) the positive selection for CD3+/CD28+ cells comprises incubating the input sample with a separation reagent for about 10 to 90 minutes, about 10 to 60 minutes, about 10 to 45 minutes, about 12 to 90 minutes, about 12 to 60 minutes, about 12 to 45 minutes, about 15 to 90 minutes, about 15 to 60 minutes, or about 15 to 45 minutes, e.g., about 30 minutes or about 20 minutes; optionally wherein the separation reagent comprises a bead that is coupled (e.g., covalently or non-covalently coupled) to an anti-CD3 and/or anti-CD28 antibody;
iii) the positive selection for CD3+/CD28+ cells is performed with a magnetic device, e.g., Dynamag CTS or other arrangement of magnetic elements and/or which uses an about 3:1 ratio of magnetic separation members (e.g., beads) to T cells;
iv) the positive selection for CD3+/CD28+ cells comprises a separation or dwell time of less than about 6, 5, 3, 2, or 1 minute, or less than about 50, 40, 30, 20, 10, 5, 4, 3, 2, or 1 second;
v) the positive selection for CD3+/CD28+ cells comprises flowing a fluid that comprises the immune effector cells and magnetic separation members within an enclosed system, e.g., a chamber or a bag, where magnetic separation occurs, optionally wherein the flowing is performed at a speed such that magnetic separation of the members (optionally bound to immune effector cells) occurs; and/or
vi) the positive selection for CD3+/CD28+ cells is performed using a device comprising:
at least one cell suspension module;
at least one flow-through magnetic separation/debeading module;
at least one non-magnetic output module;
at least one magnetic output module;
optionally, at least one magnetic component, external to the magnetic separation/debeading module, that creates magnetic forces and/or gradients;
and optionally, at least one buffer module;
optionally wherein the magnetic separation/debeading module comprises:
a chamber defined by walls and having an x-direction, a y-direction, and a z-direction;
an inlet and an outlet arranged on opposite ends of the chamber, e.g., in the x-direction, in the y-direction, or in the z-direction; and/or
at least two magnets adjacent or proximate to a wall of the chamber and arranged to establish a zero gradient line within the chamber between the inlet and the outlet.

7. The method of any of claims 1-6, wherein the input sample comprises:
about 1×10⁷ nucleated cells/ml;
at least about 5%, 10%, 15%, 18%, 20%, 25%, 30%, 35%, 40%, 45%, 50%, 55%, 60%, 65%, 70%, 75%, 80%, 85%, 90%, or 95% B cells, e.g., CD45+CD19+ B cells;
at least about 5%, 10%, 15%, 18%, 20%, 25%, 30%, 35%, 40%, 45%, 50%, 55%, 60%, 65%, 70%, 75%, 80%, 85%, 90%, or 95% B cells, e.g., CD45-CD19+ B cells;
at least 5%, 10%, 15%, 20%, 25%, 30%, 35%, 40%, 45%, 50%, 55%, 60%, 65%, 70%, 75%, 80%, 85%, 90%, or 95% monocytes;
at least 5%, 10%, 15%, 20%, 25%, 30%, 35%, 40%, 45%, 50%, 55%, 60%, 65%, 70%, 75%, 80%, 85%, 90%, or 95% tumor cells, e.g., lymphoblasts; and/or
less than 60%, 55%, 50%, 45%, 40%, 35%, 30%, 25%, or 20% immune effector cells, e.g., T cells.

8. The method of any of claims 1-7, wherein the output sample comprises:
less than about 10%, 9%, 8%, 7%, 6%, 5%, 4%, 3%, 2%, or 1% B cells, e.g., CD45+CD19+ B cells;
less than about 10%, 9%, 8%, 7%, 6%, 5%, 4%, 3%, 2%, or 1% B cells, e.g., CD45-CD19+ B cells;
at least 5%, 6%, 7%, 8%, 9%, 10%, 11%, 12%, 13%, 14%, 15%, or 20% T cells;
less than 20%, 15%, 10%, 9%, 8%, 7%, 6%, 5%, 4%, 2%, 1%, 0.5%, 0.2%, or 0.1% monocytes;
at least 25%, 30%, 35%, 40%, 45%, 50%, 55%, 60%, 65%, 70%, 75%, 80%, 85%, 90%, 95%, 96%, 97%, 98%, or 99% T cells, e.g., CD3+CD45+ T cells;
less than 20%, 15%, 10%, 9%, 8%, 7%, 6%, 5%, 4%, 2%, 1%, 0.5%,0 .2%, or 0.1% tumor cells;
at least 50%, 55%, 60%, 65%, 70%, 75%, 80%, 85%, 90%, 95%, 96%, 97%, 98%, 99%, 99.5%, 99.8%, or 99.9% immune effector cells, e.g., T cells;
less than 50%, 45%, 40%, 40%, 35%, 30%, 25%, 20%, 15%, 10%, 5%, 4%, 2%, or 1% the percentage of monocytes compared to the input sample;
less than 50%, 45%, 40%, 40%, 35%, 30%, 25%, 20%, 15%, 10%, 5%, 4%, 2%, or 1% the percentage of tumor cells compared to the input sample; and/or
at least 50%, 45%, 40%, 40%, 35%, 30%, 25%, 20%, 15%, 10%, 5%, 4%, 2%, or 1% the percentage of immune effector cells, e.g., T cells, compared to the input sample.

9. The method of any of the preceding claims, further comprising introducing, e.g., by transduction, a nucleic acid encoding a CAR into one or more of the immune effector cells in the output sample, wherein optionally the CAR comprises an antigen binding domain, a transmembrane domain, and an intracellular signaling domain, e.g., comprising a primary signaling domain and/or a costimulatory signaling domain, and wherein optionally the transduction results in a transduction efficiency of at least about 10%, 15%, 20%, 25%, 30%, 35%, 40%, 45%, or 50%;
optionally wherein the method further comprises a step of assaying the transduction efficiency and/or performing a wash step on the input sample with a buffer comprising dextrose and/or sodium chloride, e.g., D5 medium, e.g., using a CS5+ instrument.

10. The method of any of the preceding claims, wherein the immune effector cells are human immune effector cells.

11. The method of any of the preceding claims, wherein the output sample comprises CD8+ T cells and/or CD4+ T cells.

12. The method of any of the preceding claims, wherein the input sample is from a patient that has a cancer selected from the group consisting of one or more acute leukemias including but not limited to B-cell acute lymphoblastic leukemia (BALL), T-cell acute lymphoblastic leukemia (TALL), acute lymphoblastic leukemia (ALL); one or more chronic leukemias including but not limited to chronic myelogenous leukemia (CML), chronic lymphocytic leukemia (CLL); additional hematologic cancers or hematologic conditions including, but not limited to B cell prolymphocytic leukemia, blastic plasmacytoid dendritic cell neoplasm, Burkitt's lymphoma, diffuse large B cell lymphoma, follicular lymphoma, hairy cell leukemia, small cell- or a large cell-follicular lymphoma, malignant lymphoproliferative conditions, MALT lymphoma, mantle cell lymphoma, Marginal zone lymphoma, multiple myeloma, myelodysplasia and myelodysplastic syndrome, non-Hodgkin lymphoma, Hodgkin lymphoma, plasmablastic lymphoma, plasmacytoid dendritic cell neoplasm, Waldenstrom macroglobulinemia, preleukemia, atypical and/or non-classical cancers, malignancies, precancerous conditions or proliferative diseases, and any combination thereof.

13. The method of any of the preceding claims, which further comprises:
a step of assaying one or more cell surface markers on cells in the output sample, e.g., CD45, CD19, CD3, CD28, CD25, or CD14; and/or
a step of stimulating the immune effector cells, e.g., by contacting the cells with a reagent that binds CD3 and/or CD28, e.g., a substrate (such as a bead) coupled to CD3 and/or CD28 antibodies, e.g., prior to transduction.

14. A reaction mixture obtained using a method according to any of claims 1-13.

15. The reaction mixture of claim 14, wherein:
i) the reaction mixture comprises at least 80%, 85%, 90%, or 95% T cells and less than 10%, 9%, 8%, 7%, 6%, 5%, 4%, 3%, 2%, or 1% monocytes, wherein the total number of cells in the reaction mixture adds up to 100%, wherein optionally one or more of the T cells expresses a CAR; optionally wherein
a) the reaction mixture comprises at least 1×10⁶, 2×10⁶, 5×10⁶, 1×10⁷, 2×10⁷, 5×10⁷, 1×10⁸, 2×10⁸, or 5×10⁸ cells total;
b) the reaction mixture comprises less than 10%, 9%, 8%, 7%, 6%, 5%, 4%, 3%, 2%, or 1% B cells; and/or
c) the reaction mixture comprises less than 10%, 9%, 8%, 7%, 6%, 5%, 4%, 3%, 2%, or 1% cancer cells, e.g. lymphoblasts; and/or
ii) the reaction mixture further comprises a nucleic acid encoding a CAR, e.g., wherein the nucleic acid is present inside a T cell or outside a T cell.
